# EUROPEAN PATENT APPLICATION

(11) **EP 1 690 932 A1**
(43) Date of publication of application: **16.08.2006**
(21) Application number: 04792995.5
(22) Date of filing: 20.10.2004
(51) Int. Cl.: C12N 15/00, C12Q 1/68, C12N 5/10, C07K 14/46, A61K 38/17, A61P 19/00

(54) **BONE AND/OR JOINT DISEASE-ASSOCIATED GENE**

(30) Priority: 20.10.2003 JP 2003359172
(71) Applicant: Teijin Pharma Limited, Tokyo 100-0011 (JP); Komori, Toshihisa, Nagasaki 851-2125 (JP)
(72) Inventor: KOMORI, Toshihisa, Nishisonogi-gun, Nagasaki 851-2125 (JP); KANATANI, Naoko, Nagasaki-shi, Nagasaki 852-8115 (JP); YOSHIDA, Carolina Andrea, Nagasaki-shi, Nagasaki 852-8043 (JP); ZANMA, Akira c/o Teijin Pharma Limited, Hino-shi, Tokyo 191-0065 (JP); KOBAYASHI, Shinji c/o Teijin Pharma Limited, Hino-shi, Tokyo 191-0065 (JP); YAMANA, Kei c/o Teijin Pharma Limited, Hino-shi, Tokyo 191-0065 (JP)
(74) Representative: Hallybone, Huw George
(86) International application number: PCT/JP2004/015879
(87) International publication number: WO 2005/038022

(57) **Abstract**

This invention provides a method for obtaining a gene involved in regulation of cartilage differentiation, in which a transcription factor, preferably Runx2/Cbfa1, is forcedly expressed in a cell that is deficient such transcription factor, preferably in a Runx2/Cbfa1-deficient chondrocyte, and the gene, the expression of which is thereby induced, is selected using DNA chip analysis, subtraction, or other means as well as a Runx2/Cbfa1-deficient chondrocyte useful for carrying out such method. The invention also provides a polynucleotide obtained by such method, a polypeptide encoded by such polynucleotide, an antibody against such polypeptide, a recombinant vector comprising such polynucleotide, a transformant comprising such recombinant DNA vector, a cell expressing such polypeptide, a transgenic animal of such polynucleotide, an animal model of a bone and/or joint disease (preferably osteoarthritis), a method for screening for a therapeutic agent and/or prophylactic agent for a bone and/or joint disease (preferably osteoarthritis) using the aforementioned objects, a candidate compound for a therapeutic agent and/or prophylactic agent selected by such method, a pharmaceutical composition for a bone and/or joint disease (preferably osteoarthritis), and a method for diagnosing such disease.

SEQUENCE LISTING

<110>  Teijin Pharma Limited
       Komori, Toshihisa

<120>  Genes related to Bone and/or Joint Diseases

<130>  PH-2274-PCT

<150>  JP 2003-359172
<151>  2003-10-20

<160>  114

<170>  PatentIn version 3.1

<210>  1
<211>  5220
<212>  DNA
<213>  Mouse

<220>
<221>  CDS
<222>  (274)..(1962)
<223>

<400>  1
cggaactgct tcgactgcaa agcttcaagc gcagcctggg agcggcctgg tggccctatc      60

ccggcagctc cacacagcag aacgccctgg gtccctgaaa ctcgaaaccc gggctcagaa     120

ccagcggaaa ccaaagcgaa atccttgaac ttctctgaac aattgcttcc gggcgtttgc     180

tgagagccgg gggacctgac cggagcccag gccgcgtatg gcgcgcccct gatgtcacac     240

ggacgccagc gaggccagcg ctccggctgc agc atg gac cgc gcg ggg cgc ctg      294
                                     Met Asp Arg Ala Gly Arg Leu
                                     1               5

ggt gcg ggc ctg cgg gga ctc tgc gtg gct gca ctc gtg ctc gtg tgc      342
Gly Ala Gly Leu Arg Gly Leu Cys Val Ala Ala Leu Val Leu Val Cys
        10                  15                  20

gcc gga cac ggg ggc cgc cgc gag gat ggg gga cca gct tgc tac gga      390
Ala Gly His Gly Gly Arg Arg Glu Asp Gly Gly Pro Ala Cys Tyr Gly

    25                  30                  35

gga ttc gac ctc tac ttc atc ctg gac aag tca gga agt gtg ctg cac 438
Gly Phe Asp Leu Tyr Phe Ile Leu Asp Lys Ser Gly Ser Val Leu His
40                  45                  50                  55

cac tgg aat gaa atc tac tac ttc gtg gag cag ttg gct cat aga ttc 486
His Trp Asn Glu Ile Tyr Tyr Phe Val Glu Gln Leu Ala His Arg Phe
                60                  65                  70

atc agc cca cag cta agg atg tcc ttc att gtc ttc tct act cga ggg 534
Ile Ser Pro Gln Leu Arg Met Ser Phe Ile Val Phe Ser Thr Arg Gly
            75                  80                  85

aca act tta atg aaa cta act gag gac agg gaa cag atc cga caa ggc 582
Thr Thr Leu Met Lys Leu Thr Glu Asp Arg Glu Gln Ile Arg Gln Gly
        90                  95                  100

cta gaa gag ctc cag aaa gtt ctg cca gga gga gac act tac atg cac 630
Leu Glu Glu Leu Gln Lys Val Leu Pro Gly Gly Asp Thr Tyr Met His
    105                 110                 115

gaa gga ttc gag agg gcc agt gag cag att tac tat gag aac agt caa 678
Glu Gly Phe Glu Arg Ala Ser Glu Gln Ile Tyr Tyr Glu Asn Ser Gln
120                 125                 130                 135

gga tac agg acg gcg agc gtc atc atc gcg ttg acg gat ggg gag ctg 726
Gly Tyr Arg Thr Ala Ser Val Ile Ile Ala Leu Thr Asp Gly Glu Leu
                140                 145                 150

cac gag gac ctc ttc ttc tac tca gag agg gag gct aac cga tcc cga 774
His Glu Asp Leu Phe Phe Tyr Ser Glu Arg Glu Ala Asn Arg Ser Arg
            155                 160                 165

gac ctt ggt gcg att gtt tac tgc gtt ggc gtg aag gat ttc aat gaa 822
Asp Leu Gly Ala Ile Val Tyr Cys Val Gly Val Lys Asp Phe Asn Glu
        170                 175                 180

act cag ttg gct cgg att gca gac agt aag gac cac gtg ttt cct gtg 870
Thr Gln Leu Ala Arg Ile Ala Asp Ser Lys Asp His Val Phe Pro Val
    185                 190                 195

aac gac ggc ttc cag gct ctc caa ggc att atc cac tca att tta aag 918
Asn Asp Gly Phe Gln Ala Leu Gln Gly Ile Ile His Ser Ile Leu Lys
200                 205                 210                 215

aaa tcc tgc atc gaa att ctg gcg gct gaa cca tcc acc atc tgc gcg 966
Lys Ser Cys Ile Glu Ile Leu Ala Ala Glu Pro Ser Thr Ile Cys Ala
                220                 225                 230

gga gag tcc ttt caa gtg gtc gta aga gga aat ggc ttc cga cat gcc 1014
Gly Glu Ser Phe Gln Val Val Val Arg Gly Asn Gly Phe Arg His Ala
            235                 240                 245

cgc aat gtg gac agg gtc ctc tgc agc ttc aaa atc aat gac tca gtc 1062
Arg Asn Val Asp Arg Val Leu Cys Ser Phe Lys Ile Asn Asp Ser Val
        250                 255                 260

acg ctc aat gag aag ccc ttt gct gtg gaa gac act tat ttg ctg tgc 1110
Thr Leu Asn Glu Lys Pro Phe Ala Val Glu Asp Thr Tyr Leu Leu Cys
    265                 270                 275

cca gca cca atc ttg aaa gaa gtt ggc atg aaa gct gca ctg cag gtc 1158
Pro Ala Pro Ile Leu Lys Glu Val Gly Met Lys Ala Ala Leu Gln Val
280                 285                 290                 295

agc atg aac gac ggc ctg tcc ttc atc tcc agt tct gtc atc atc acc 1206
Ser Met Asn Asp Gly Leu Ser Phe Ile Ser Ser Ser Val Ile Ile Thr
                300                 305                 310

acc aca cac tgt tca gac ggc tcc atc ctg gcg att gct ctg ctg gtc 1254
Thr Thr His Cys Ser Asp Gly Ser Ile Leu Ala Ile Ala Leu Leu Val
            315                 320                 325

ctc ttc ctg ctg ctg gcc ctg gcg ctg ctc tgg tgg ttc tgg ccc ctc 1302
Leu Phe Leu Leu Leu Ala Leu Ala Leu Leu Trp Trp Phe Trp Pro Leu
        330                 335                 340

tgc tgc aca gtg atc atc aag gag gtc cct cca ccc cct gtt gag gag 1350
Cys Cys Thr Val Ile Ile Lys Glu Val Pro Pro Pro Pro Val Glu Glu
    345                 350                 355

agt gag gaa gaa gac gat gat ggt ttg cca aag aag aaa tgg ccc aca 1398
Ser Glu Glu Glu Asp Asp Asp Gly Leu Pro Lys Lys Lys Trp Pro Thr
360                 365                 370                 375

gta gat gcc tct tat tat ggt gga cgc ggt gtg gga ggc att aaa aga 1446
Val Asp Ala Ser Tyr Tyr Gly Gly Arg Gly Val Gly Gly Ile Lys Arg
                380                 385                 390

atg gag gtc cgc tgg gga gaa aag ggc tcc aca gaa gaa ggg gcg aag     1494
Met Glu Val Arg Trp Gly Glu Lys Gly Ser Thr Glu Glu Gly Ala Lys
            395                 400                 405

tta gaa aag gca aag aat gca cga gtc aag atg cca gag caa gaa tat     1542
Leu Glu Lys Ala Lys Asn Ala Arg Val Lys Met Pro Glu Gln Glu Tyr
        410                 415                 420

gag ttc cca gaa ccc cga aac ctc aac aac aac atg cgc cgg cct tcc     1590
Glu Phe Pro Glu Pro Arg Asn Leu Asn Asn Asn Met Arg Arg Pro Ser
    425                 430                 435

tcg cct cgg aag tgg tac tcg ccc atc aag gga aaa ctc gat gcc ttg     1638
Ser Pro Arg Lys Trp Tyr Ser Pro Ile Lys Gly Lys Leu Asp Ala Leu
440                 445                 450                 455

tgg gtt ctg ctg aga aaa gga tat gac cga gtg tct gtg atg agg cca     1686
Trp Val Leu Leu Arg Lys Gly Tyr Asp Arg Val Ser Val Met Arg Pro
                460                 465                 470

cag cca gga gac acg gga cgc tgt atc aac ttc acc aga gtg aag aac     1734
Gln Pro Gly Asp Thr Gly Arg Cys Ile Asn Phe Thr Arg Val Lys Asn
            475                 480                 485

agt cag cca gcc aag tat ccc ctg aac aac acc tac cac ccc agc tcc     1782
Ser Gln Pro Ala Lys Tyr Pro Leu Asn Asn Thr Tyr His Pro Ser Ser
        490                 495                 500

cca cct ccc gct cct atc tac aca ccc cca ccc cct gct ccc cac tgc     1830
Pro Pro Pro Ala Pro Ile Tyr Thr Pro Pro Pro Pro Ala Pro His Cys
    505                 510                 515

cct ccc cca gcc ccc agt gcc ccc act cct ccc att cct tcc cca cca     1878
Pro Pro Pro Ala Pro Ser Ala Pro Thr Pro Pro Ile Pro Ser Pro Pro
520                 525                 530                 535

tcc act ctc ccc cct cct cct cag gcc cca ccc cct aac agg gca cct     1926
Ser Thr Leu Pro Pro Pro Pro Gln Ala Pro Pro Pro Asn Arg Ala Pro
                540                 545                 550

ccc ccc tcc cga cct cct cca agg cct tct gtc tag aacccaaagt     1972
Pro Pro Ser Arg Pro Pro Pro Arg Pro Ser Val
            555                 560

ccgagctctg ggctgcctga gcaactccag caggaggctt ctctgctgaa agaaagatct     2032

gcccagccta tgtggtgagt ggcggctgat gtttgcacga tttaaaagca agtcgtgatg 2092

ggcagaacaa aatgggcatt ttgaactgcc tgaagacaga caatgagaca ataacagtca 2152

cattatagcc tgtgacccct cacctctaga ggaaggttcc cgagatggcc acattgccac 2212

agtgctctca gccagattat gtcccatgaa gaccaggaag aaagtgactt ccaagaatgg 2272

aatgcagcat tggataagaa acacctggct gagattctga cctcactgat ttgactcttg 2332

attcttggac tgggagccag gccatctcca cccctggtac cacccagcaa ctctgaaaat 2392

gtgcagtgtc cctagtatgc atcgaatagg tatccaactg ggatctgcag gttgccttat 2452

aaagagcata tgctctattc tctttcccga acttcctggt ttcccagtga tgagggaagg 2512

ggaaaggtgt tgccatgctt agaagttaga ggacgtcagt gctcagcact gatggagaag 2572

cgttgatggg agtgtccagc tcttacatct agaaatggct ggcttcagca ggcacagttc 2632

ctaaaccaac aagccttgtc attgtcaaag gcaacctact aatgattcac cttaaacatc 2692

aaggttgact gtggcatagg tcagagctga tcacacagaa ccttccccat gaaatcgcaa 2752

ggttcctcat cttcaaatac ccaggacccc agagatttct aaatccagct aagagacagt 2812

agtcctgact tggcaagaaa accattccca gttgttttac tctgaaacag gccgttgtat 2872

gtatggtata tctctccttg gcctttcaac ctgctcacaa gtattaccag ttatgaagca 2932

aggagaaata catccagtgt gtaatagaaa agctctgccc acaatcccca tgtcactcct 2992

ctacattatt ctgaagctgc ttggtcagtg agccctttaa cctcatgtag actctggaca 3052

ctgtcaccca atcatgaaaa cagaggtcat tgtcaaaggc agtgtatagc ctgtacaaaa 3112

atgatgcttc cttcctcagt ttccacaggc cccaaaattc ctgtcttagg ctcctaaacc 3172

tctaaacttt ttcctggaac aaaagatata aaacgggcat aagtttttat gttttgggct 3232

gtgatctcca aagatccttc aagaactcaa gttagcctca ttcttccagc ttgtttagaa 3292

cagaggcatc caggtgtcat gcactccata gacaccaatc cttgttccca aggcagacat 3352

tattaatcaa tctcagcact agttctcaat ttaatccaat tatatttttc cacagtactt 3412

cacatctctt atgacctgtt ggtcatcagt tagaattgag agagataaac actgtttgta 3472

atccctacct tagaaagaaa agcagaggag aatgggggaa ccaccagcat aaaagttatt 3532

atctggggaa aatcgacctg aaagaacgcc caagtccaag acctatggtg ctgacaccaa 3592

agtaacactt tcccaagtgt accccagacc ccactcttct ccctgtggcc accactccct 3652

gcttttcagg agttgtgaaa aagatctcct tcacccttac tgtgccccca tattagaaca 3712

aggcttgttt agtgtagtcc ttgttaaaca ggtgccagaa tgtctcagcc acctgagatg 3772

acattgctgg gccccagaaa accattccaa ggagaatggg ctccccaggc tcagagcatg 3832

caactatgag cccatggcaa ctgttttgac tgctggcagt acaaaacggg ccaccccaca 3892

ttacagctgc aggatttgtg cagccataag aaagtatgaa ccaagatgct ggtgttgctg 3952

ttcaacaagc atgggcttcg gggaaggcag cagactccga gagcaggcct tgtgcagtgt 4012

cccaaggggc tgtggtgaag tgtctgagga aaaatgaatg ctgatacatg gtgattctga 4072

gaagaatttg caaggtttga ccttagaatt tatggaatgt cttccctggt cattcagaat 4132

tatggctaga agtttctaga aaccgtcaag gttaatacct ttcagagtag gtgattacag 4192

gcaggaagag ctttgatgtg gtttacaaag cccatcagtt ctgtgtcatt ccctgtaagc 4252

aacaggagat ggtggttgtg attagcaaac tgcatgtgtt atttgtttga ctccttgtta 4312

ttgtccttac ggaggatttt ttttatataa gccaaatttt gttgtatata ttcatattcc 4372

acgtgacaga tggaagcacg tcctatcagt gtgaataaaa agaacagttg tagtaaatta 4432

ttaaagccag tgatttcatg gcaggttacc ctaccaagct gtgcttgttg atctcccatg 4492

accatactgc ttttacaatg tacaaatagt tcctaggtga cgagaccctc ctttacataa 4552

tgccgatgac agccttgctg ggaactgcgg tccttctgct gtgacagcca gctcgaaaac 4612

aggtcctgcc tggagcttgc cacacacttt agggagacat aagagctgtc tttccccagc 4672

gtcagggaca aagctaccat aaagaagtgg aaaagtcttg gctctccagc ctgggacaga 4732

ggtctctctg gaaccccaag gaagagcaga aatgatcctt gcctgccact gcacacaatg 4792

tgatggtgga aaatccatca aggaataatt gtgagataat gaccgacagt tcaggcgcaa 4852

agggaattca tgctgtgtaa agtgggtgga attcgtttgc aagctatgca aagcctgatc 4912

ttactcacca ggaggatgga aagggttttt ttagttatct gagctcagct gagttatcac 4972

gcttggagaa ccgatttaaa ggaattagaa tatgatttct gaatacacat aacattaaac 5032

tcttctcttt ttctatggta atttagttat ggacgttcag cgtctctgag ttattgttat 5092

aaaagacttg tcatcaccgc actgtgctgt aggagactgg gctgaacctg tacaatggta 5152

taccctggaa gttgcttttt taaaaaaaaa taataataaa cacctaaaat caaaaaaaaa 5212

aaaaaaaa 5220

<210> 2
<211> 562
<212> PRT
<213> Mouse

<400> 2

Met Asp Arg Ala Gly Arg Leu Gly Ala Gly Leu Arg Gly Leu Cys Val
1               5                   10                  15

Ala Ala Leu Val Leu Val Cys Ala Gly His Gly Gly Arg Arg Glu Asp
            20                  25                  30

Gly Gly Pro Ala Cys Tyr Gly Gly Phe Asp Leu Tyr Phe Ile Leu Asp
        35                  40                  45

Lys Ser Gly Ser Val Leu His His Trp Asn Glu Ile Tyr Tyr Phe Val
    50                  55                  60

Glu Gln Leu Ala His Arg Phe Ile Ser Pro Gln Leu Arg Met Ser Phe
65                  70                  75                  80

Ile Val Phe Ser Thr Arg Gly Thr Thr Leu Met Lys Leu Thr Glu Asp
                85                  90                  95

Arg Glu Gln Ile Arg Gln Gly Leu Glu Glu Leu Gln Lys Val Leu Pro
            100                 105                 110

Gly Gly Asp Thr Tyr Met His Glu Gly Phe Glu Arg Ala Ser Glu Gln
        115                 120                 125

Ile Tyr Tyr Glu Asn Ser Gln Gly Tyr Arg Thr Ala Ser Val Ile Ile
    130                 135                 140

Ala Leu Thr Asp Gly Glu Leu His Glu Asp Leu Phe Phe Tyr Ser Glu
145                 150                 155                 160

Arg Glu Ala Asn Arg Ser Arg Asp Leu Gly Ala Ile Val Tyr Cys Val
                165                 170                 175

Gly Val Lys Asp Phe Asn Glu Thr Gln Leu Ala Arg Ile Ala Asp Ser
            180                 185                 190

Lys Asp His Val Phe Pro Val Asn Asp Gly Phe Gln Ala Leu Gln Gly
        195                 200                 205

Ile Ile His Ser Ile Leu Lys Lys Ser Cys Ile Glu Ile Leu Ala Ala
    210                 215                 220

Glu Pro Ser Thr Ile Cys Ala Gly Glu Ser Phe Gln Val Val Val Arg
225                 230                 235                 240

Gly Asn Gly Phe Arg His Ala Arg Asn Val Asp Arg Val Leu Cys Ser
                245                 250                 255

Phe Lys Ile Asn Asp Ser Val Thr Leu Asn Glu Lys Pro Phe Ala Val
            260                 265                 270

Glu Asp Thr Tyr Leu Leu Cys Pro Ala Pro Ile Leu Lys Glu Val Gly
        275                 280                 285

Met Lys Ala Ala Leu Gln Val Ser Met Asn Asp Gly Leu Ser Phe Ile
    290                 295                 300

Ser Ser Ser Val Ile Ile Thr Thr Thr His Cys Ser Asp Gly Ser Ile
305                 310                 315                 320

Leu Ala Ile Ala Leu Leu Val Leu Phe Leu Leu Leu Ala Leu Ala Leu
                325                 330                 335

Leu Trp Trp Phe Trp Pro Leu Cys Cys Thr Val Ile Ile Lys Glu Val
            340                 345                 350

Pro Pro Pro Pro Val Glu Glu Ser Glu Glu Glu Asp Asp Asp Gly Leu
        355                 360                 365

Pro Lys Lys Lys Trp Pro Thr Val Asp Ala Ser Tyr Tyr Gly Gly Arg
    370                 375                 380

Gly Val Gly Gly Ile Lys Arg Met Glu Val Arg Trp Gly Glu Lys Gly
385                 390                 395                 400

Ser Thr Glu Glu Gly Ala Lys Leu Glu Lys Ala Lys Asn Ala Arg Val
                405                 410                 415

Lys Met Pro Glu Gln Glu Tyr Glu Phe Pro Glu Pro Arg Asn Leu Asn
            420                 425                 430

Asn Asn Met Arg Arg Pro Ser Ser Pro Arg Lys Trp Tyr Ser Pro Ile
        435                 440                 445

Lys Gly Lys Leu Asp Ala Leu Trp Val Leu Leu Arg Lys Gly Tyr Asp
    450                 455                 460

Arg Val Ser Val Met Arg Pro Gln Pro Gly Asp Thr Gly Arg Cys Ile
465                 470                 475                 480

Asn Phe Thr Arg Val Lys Asn Ser Gln Pro Ala Lys Tyr Pro Leu Asn
                485                 490                 495

Asn Thr Tyr His Pro Ser Ser Pro Pro Pro Ala Pro Ile Tyr Thr Pro
            500                 505                 510

Pro Pro Pro Ala Pro His Cys Pro Pro Pro Ala Pro Ser Ala Pro Thr
        515                 520                 525

Pro Pro Ile Pro Ser Pro Pro Ser Thr Leu Pro Pro Pro Pro Gln Ala
    530                 535                 540

Pro Pro Pro Asn Arg Ala Pro Pro Pro Ser Arg Pro Pro Pro Arg Pro
545                 550                 555                 560

Ser Val

<210>  3
<211>  1734
<212>  DNA
<213>  Mouse

<220>
<221>  CDS
<222>  (257)..(1012)
<223>

<400> 3
cccacgcgtc cgcgctcctg atctccagag gaccccgggc tgggacaggg gccttggcga 60

ggctgcagct gctgtggcag tagcttggga tggaggtctt tcttgctggg aactgaggag 120

ctgagaggct cctgtcaggc tcctgtccta aactcttggc acttgcggtg gcttgggctt 180

cacacactgt cagacacctt cttggtggcc tcctcggcct caggtttgaa gctggctcca 240

caagggacac ggtgac atg agg ggc aac cca ctg atc cat ctt ctg gcc att 292
Met Arg Gly Asn Pro Leu Ile His Leu Leu Ala Ile
1               5                   10

tcc ttc ctc tgc att ctc tca atg gtg tat tcc cag ctg tgc cca gca 340
Ser Phe Leu Cys Ile Leu Ser Met Val Tyr Ser Gln Leu Cys Pro Ala
        15                  20                  25

ccc tgt gcc tgt cct tgg aca cca ccc cag tgc cca ccg ggg gta ccc 388
Pro Cys Ala Cys Pro Trp Thr Pro Pro Gln Cys Pro Pro Gly Val Pro
    30                  35                  40

ctg gtg ctg gat ggc tgt ggc tgc tgt cga gtg tgt gca cgg agg ctg 436
Leu Val Leu Asp Gly Cys Gly Cys Cys Arg Val Cys Ala Arg Arg Leu
45                  50                  55                  60

ggg gag tcc tgc gac cac ctg cat gtc tgc gac ccc agc cag ggc ctg 484
Gly Glu Ser Cys Asp His Leu His Val Cys Asp Pro Ser Gln Gly Leu
                65                  70                  75

gtt tgt cag cct ggg gca ggc ccc agt ggc cgt ggt gct gtg tgc ctc 532
Val Cys Gln Pro Gly Ala Gly Pro Ser Gly Arg Gly Ala Val Cys Leu
            80                  85                  90

ttc gaa gag gat gac ggg agc tgt gag gtg aat ggc cgc agg tac ctg 580
Phe Glu Glu Asp Asp Gly Ser Cys Glu Val Asn Gly Arg Arg Tyr Leu
        95                  100                 105

gat ggg gag acc ttt aaa ccc aat tgc agg gtt ttg tgc cgc tgt gat 628
Asp Gly Glu Thr Phe Lys Pro Asn Cys Arg Val Leu Cys Arg Cys Asp
    110                 115                 120

gac ggt ggt ttc acc tgc ctg ccg ctg tgc agt gag gat gtg cgg ctg 676
Asp Gly Gly Phe Thr Cys Leu Pro Leu Cys Ser Glu Asp Val Arg Leu
125                 130                 135                 140

ccc agc tgg gac tgc cca cgc ccc agg aga ata cag gtg cca gga agg 724
Pro Ser Trp Asp Cys Pro Arg Pro Arg Arg Ile Gln Val Pro Gly Arg
                145                 150                 155

tgc tgc ccc gag tgg gtg tgt gac cag gca gtg atg cag ccg gca atc 772
Cys Cys Pro Glu Trp Val Cys Asp Gln Ala Val Met Gln Pro Ala Ile
            160                 165                 170

cag ccc tcc tca gcc caa gga cac caa ctt tct gcc ctt gtc act cct 820
Gln Pro Ser Ser Ala Gln Gly His Gln Leu Ser Ala Leu Val Thr Pro
        175                 180                 185

gca tct gcc gat ggc ccc tgt cca aac tgg agc aca gcc tgg ggc ccc 868
Ala Ser Ala Asp Gly Pro Cys Pro Asn Trp Ser Thr Ala Trp Gly Pro
    190                 195                 200

tgc tca acc acc tgt ggg ttg ggc ata gcc acc cga gta tcc aac cag 916
Cys Ser Thr Thr Cys Gly Leu Gly Ile Ala Thr Arg Val Ser Asn Gln
205                 210                 215                 220

aac cga ttc tgc caa ctg gag atc cag cgt cgc ctg tgt ctg tcc aga 964
Asn Arg Phe Cys Gln Leu Glu Ile Gln Arg Arg Leu Cys Leu Ser Arg
                225                 230                 235

ccc tgc ctg gca tcc agg agc cac ggc tca tgg aac agt gcc ttc tag 1012
Pro Cys Leu Ala Ser Arg Ser His Gly Ser Trp Asn Ser Ala Phe
            240                 245                 250

agccattgcg gggatgtgga tacagggcct gccattctca gcaaatgtcc ctaggaccag 1072

gccctggact gatggtagat gcccctctcc atgctcttgg ctgcagttaa ctgtcctggg 1132

tggattcagt gtccagagcc tctgagcgat ccctgctctg tctgaggtgg gggaagcagg 1192

tgaccagctc catttctctg gattctgacc caggcttctg ggttctcctg gctagttcct 1252

caaaacttcc ctgtatgaaa aggacaacca aaaggacctt taaagctaag ctgtactggg 1312

caagcctggc caccatgctg gggatagtga cagtaatagg taccaggcag cagattgcct 1372

gaaacatcca ggtcccttct tggacttcta tgtgcttgtc ccaaagatta tgggtgacct 1432

tgtaagtgtg cctttcctga tctgagaaca ccctgcccgg ctgggaagaa ttttctggga 1492

acatgaagag atggaatcac actattctta agagcgtttg ccaagtccag gaacttgacc 1552

tttgtatttg taaaaataca catctcttaa atgctcacaa agcaagaggc tccacacttc 1612

tggcaggcca gggcctttct cttcagcatg agagagacaa ggaacagtag agtaccctcc 1672

tctggaggac tggcccggtc tggaataaac acccaaatca agtgtggaaa aaaaaaaaaa 1732

aa 1734

<210> 4
<211> 251
<212> PRT
<213> Mouse

<400> 4

Met Arg Gly Asn Pro Leu Ile His Leu Leu Ala Ile Ser Phe Leu Cys
1               5                   10                  15

Ile Leu Ser Met Val Tyr Ser Gln Leu Cys Pro Ala Pro Cys Ala Cys
            20                  25                  30

Pro Trp Thr Pro Pro Gln Cys Pro Pro Gly Val Pro Leu Val Leu Asp
        35                  40                  45

Gly Cys Gly Cys Cys Arg Val Cys Ala Arg Arg Leu Gly Glu Ser Cys
    50                  55                  60

Asp His Leu His Val Cys Asp Pro Ser Gln Gly Leu Val Cys Gln Pro
65                  70                  75                  80

Gly Ala Gly Pro Ser Gly Arg Gly Ala Val Cys Leu Phe Glu Glu Asp
                85                  90                  95

Asp Gly Ser Cys Glu Val Asn Gly Arg Arg Tyr Leu Asp Gly Glu Thr
            100                 105                 110

Phe Lys Pro Asn Cys Arg Val Leu Cys Arg Cys Asp Asp Gly Gly Phe
        115                 120                 125

Thr Cys Leu Pro Leu Cys Ser Glu Asp Val Arg Leu Pro Ser Trp Asp
    130                 135                 140

Cys Pro Arg Pro Arg Arg Ile Gln Val Pro Gly Arg Cys Cys Pro Glu
145                 150                 155                 160

Trp Val Cys Asp Gln Ala Val Met Gln Pro Ala Ile Gln Pro Ser Ser
                165                 170                 175

Ala Gln Gly His Gln Leu Ser Ala Leu Val Thr Pro Ala Ser Ala Asp
            180                 185                 190

Gly Pro Cys Pro Asn Trp Ser Thr Ala Trp Gly Pro Cys Ser Thr Thr
        195                 200                 205

Cys Gly Leu Gly Ile Ala Thr Arg Val Ser Asn Gln Asn Arg Phe Cys
    210                 215                 220

Gln Leu Glu Ile Gln Arg Arg Leu Cys Leu Ser Arg Pro Cys Leu Ala
225                 230                 235                 240

Ser Arg Ser His Gly Ser Trp Asn Ser Ala Phe
                245                 250

<210>  5
<211>  3548
<212>  DNA
<213>  Mouse

<220>
<221>  CDS
<222>  (88)..(2190)

<223>

<400> 5
actggacccg ccctcgaccc gggctgtgct ttggtgttct taagtcgtgc cgcgtgcgca 60

acggtagtga cgcgttttac ccggagc atg gcg gat acc ggc ttg cgc cgc gtg 114
Met Ala Asp Thr Gly Leu Arg Arg Val
1               5

gtt ccc agc gac ctt tat ccc ctt gtg ctc aga ttt ctg cgg gat agc 162
Val Pro Ser Asp Leu Tyr Pro Leu Val Leu Arg Phe Leu Arg Asp Ser
10                  15                  20                  25

caa ctc tcg gag gtg gcc agt aaa ttt gca aaa gcg acc ggc gct aca 210
Gln Leu Ser Glu Val Ala Ser Lys Phe Ala Lys Ala Thr Gly Ala Thr
                30                  35                  40

cag cag gac gcc aat gcc tcg tcc ctc ttg gac atc tat agc ttc tgg 258
Gln Gln Asp Ala Asn Ala Ser Ser Leu Leu Asp Ile Tyr Ser Phe Trp
            45                  50                  55

ctc aag tcc acc aaa gcc cca aag gtg aag tta cag tca aat gga cca 306
Leu Lys Ser Thr Lys Ala Pro Lys Val Lys Leu Gln Ser Asn Gly Pro
        60                  65                  70

gtg acc aag aag gct aag aaa gag act tca tcc agt gac agc agt gag 354
Val Thr Lys Lys Ala Lys Lys Glu Thr Ser Ser Ser Asp Ser Ser Glu
    75                  80                  85

gac agc agt gag gac gag gac aaa aaa gcc cag gga ctt ccc aca cag 402
Asp Ser Ser Glu Asp Glu Asp Lys Lys Ala Gln Gly Leu Pro Thr Gln
90                  95                  100                 105

aag gct gcc gca cag gtc aag cga gcc agt gtg cct cag cat gct gga 450
Lys Ala Ala Ala Gln Val Lys Arg Ala Ser Val Pro Gln His Ala Gly
                110                 115                 120

aag gca gca gcc aaa gct tca gag agc agc agt agt gaa gaa tcc agt 498
Lys Ala Ala Ala Lys Ala Ser Glu Ser Ser Ser Ser Glu Glu Ser Ser
            125                 130                 135

gag gaa gag gaa gag gac aaa aag aaa aag cct gtc cag aag gca gct 546
Glu Glu Glu Glu Glu Asp Lys Lys Lys Lys Pro Val Gln Lys Ala Ala
        140                 145                 150

aag ccc caa gcc aag gca gtc aga cct cct gcg aag aag gca gag agc 594
Lys Pro Gln Ala Lys Ala Val Arg Pro Pro Ala Lys Lys Ala Glu Ser
    155                 160                 165

tct gag tcg gac tca gac tcg gat tcg gac tcc agc tca gag gaa gaa 642
Ser Glu Ser Asp Ser Asp Ser Asp Ser Asp Ser Ser Ser Glu Glu Glu
170                 175                 180                 185

aca cca cag acc cag aag cca aag gca gct gtg gca gca aaa gct cag 690
Thr Pro Gln Thr Gln Lys Pro Lys Ala Ala Val Ala Ala Lys Ala Gln
                190                 195                 200

act aaa gcc gaa gcc aaa cca ggt aca cca gcg aaa gca cag cct aag 738
Thr Lys Ala Glu Ala Lys Pro Gly Thr Pro Ala Lys Ala Gln Pro Lys
            205                 210                 215

gta gcc aat ggc aaa gca gcc gcc agc agc agc agc agc agc agc agc 786
Val Ala Asn Gly Lys Ala Ala Ala Ser Ser Ser Ser Ser Ser Ser Ser
        220                 225                 230

gat gac tca gag gaa gag aag aag gca gct gca cct ccc aag aag act 834
Asp Asp Ser Glu Glu Glu Lys Lys Ala Ala Ala Pro Pro Lys Lys Thr
    235                 240                 245

gta cca aaa aag caa gtc gtg gcc aag gcc cca gtg aaa gta gct gcc 882
Val Pro Lys Lys Gln Val Val Ala Lys Ala Pro Val Lys Val Ala Ala
250                 255                 260                 265

gcc ccc acc cag aag agc tcc agc agt gag gat tct tcc agt gaa gag 930
Ala Pro Thr Gln Lys Ser Ser Ser Ser Glu Asp Ser Ser Ser Glu Glu
                270                 275                 280

gag gag gga cag aga caa ccc atg aag aaa aaa gca ggt ccc tac agt 978
Glu Glu Gly Gln Arg Gln Pro Met Lys Lys Lys Ala Gly Pro Tyr Ser
            285                 290                 295

tca gtt cca cca ccc tct gtt cct tta cca aag aag tcc ccg gga acc 1026
Ser Val Pro Pro Pro Ser Val Pro Leu Pro Lys Lys Ser Pro Gly Thr
        300                 305                 310

cag gct cca aag aaa gct gct gcg cag aca cag cct gca gac agc agt 1074
Gln Ala Pro Lys Lys Ala Ala Ala Gln Thr Gln Pro Ala Asp Ser Ser
    315                 320                 325

gac gac agc agt gac gat tct gat tca agt tct gag gaa gag aaa aaa 1122

Asp Asp Ser Ser Asp Asp Ser Asp Ser Ser Ser Glu Glu Glu Lys Lys
330                 335                 340                 345

cct cca gct aag acg gtc gtc tcc aag aca ccc gcc aaa gca gct cca     1170
Pro Pro Ala Lys Thr Val Val Ser Lys Thr Pro Ala Lys Ala Ala Pro
                350                 355                 360

gtg aag aag aaa gca gaa agc tct tca gac agc tcg gat tct gac agt     1218
Val Lys Lys Lys Ala Glu Ser Ser Ser Asp Ser Ser Asp Ser Asp Ser
            365                 370                 375

tct gag gat gaa gct cct gcc aag cca gtc agt aca acc aag agt ccc     1266
Ser Glu Asp Glu Ala Pro Ala Lys Pro Val Ser Thr Thr Lys Ser Pro
        380                 385                 390

aag cca gct gtc act ccg aag cca tct gca gca aag gca gtg aca act     1314
Lys Pro Ala Val Thr Pro Lys Pro Ser Ala Ala Lys Ala Val Thr Thr
    395                 400                 405

cct aag caa cct gca ggc agt aac cag aaa cct cag agc agg aag gct     1362
Pro Lys Gln Pro Ala Gly Ser Asn Gln Lys Pro Gln Ser Arg Lys Ala
410                 415                 420                 425

gac agc agc tcc agc gag gag gaa agc agc tcc agc gag gag gag gag     1410
Asp Ser Ser Ser Ser Glu Glu Glu Ser Ser Ser Ser Glu Glu Glu Glu
                430                 435                 440

gcc tcc aag aaa agt gcc aca acc ccc aag gcc aag gtg act gct aaa     1458
Ala Ser Lys Lys Ser Ala Thr Thr Pro Lys Ala Lys Val Thr Ala Lys
            445                 450                 455

gca gca ccc gcc aaa cag gcc cct cag gct gct ggg gac agc agc tct     1506
Ala Ala Pro Ala Lys Gln Ala Pro Gln Ala Ala Gly Asp Ser Ser Ser
        460                 465                 470

gac tca gat agt tcc agc agt gaa gag gag gag aag act cct aag ccc     1554
Asp Ser Asp Ser Ser Ser Ser Glu Glu Glu Glu Lys Thr Pro Lys Pro
    475                 480                 485

cca gct aag aag aag gca gca ggt gga gcc gtt tct aca cca gcc cct     1602
Pro Ala Lys Lys Lys Ala Ala Gly Gly Ala Val Ser Thr Pro Ala Pro
490                 495                 500                 505

ggg aag aaa gca gag gcc aag agc agc agc agc agc agc agc agc agc     1650
Gly Lys Lys Ala Glu Ala Lys Ser Ser Ser Ser Ser Ser Ser Ser Ser

                510                 515                 520

tcc gaa gat tcc agt gaa gag gag aaa aaa aag aag ccc aaa gct act 1698
Ser Glu Asp Ser Ser Glu Glu Glu Lys Lys Lys Lys Pro Lys Ala Thr
            525                 530                 535

acc cct aaa ata cag gca agc aag gcc aat ggc act cca gct tct ctg 1746
Thr Pro Lys Ile Gln Ala Ser Lys Ala Asn Gly Thr Pro Ala Ser Leu
        540                 545                 550

aat gga aaa gca gcc aag gaa agt gag gag gaa gag gag gag gaa gaa 1794
Asn Gly Lys Ala Ala Lys Glu Ser Glu Glu Glu Glu Glu Glu Glu Glu
    555                 560                 565

aca gaa gag aag aaa aag gca gct ggg acc aag cca ggt tca ggc aaa 1842
Thr Glu Glu Lys Lys Lys Ala Ala Gly Thr Lys Pro Gly Ser Gly Lys
570                 575                 580                 585

aaa cgg aag cag aat gag acc gca gat gaa gca aca act cct caa gct 1890
Lys Arg Lys Gln Asn Glu Thr Ala Asp Glu Ala Thr Thr Pro Gln Ala
                590                 595                 600

aag aaa gtt aag ctc gag acc ccc aat acg ttt cca aaa agg aag aag 1938
Lys Lys Val Lys Leu Glu Thr Pro Asn Thr Phe Pro Lys Arg Lys Lys
            605                 610                 615

gga gaa aga agg gcg tct tcc cct ttc cga agg gtc agg gag gag gag 1986
Gly Glu Arg Arg Ala Ser Ser Pro Phe Arg Arg Val Arg Glu Glu Glu
        620                 625                 630

att gag gtg gac tct cga gtg gcg gac aat tcc ttt gat gcc aag cga 2034
Ile Glu Val Asp Ser Arg Val Ala Asp Asn Ser Phe Asp Ala Lys Arg
    635                 640                 645

ggt gca gct gga gac tgg ggg gag cga gcc aat cag gtt ctg aag ttc 2082
Gly Ala Ala Gly Asp Trp Gly Glu Arg Ala Asn Gln Val Leu Lys Phe
650                 655                 660                 665

acc aaa ggc aag tcc ttc cgg cat gaa aaa acg aag aag aag cga ggc 2130
Thr Lys Gly Lys Ser Phe Arg His Glu Lys Thr Lys Lys Lys Arg Gly
                670                 675                 680

agc tac cgg gga ggc tcc atc tct gtc cag gtc aat tcc gtc aaa ttc 2178
Ser Tyr Arg Gly Gly Ser Ile Ser Val Gln Val Asn Ser Val Lys Phe
            685                 690                 695

gac agc gag tga cacgtggtca tcctttggca aaggaagggt gattttggga 2230
Asp Ser Glu
        700

gactggcact caactccagt ggacccagaa actccgtgtt aggagacagt tgtgacaagg 2290

acggtgtgga gcaggtcctg aggtgtgtca gcctgcagtc ctctcaggct cctttttctg 2350

gacagctgaa tatcaaggac aaaaaggatt ttttttttta aagaaaccca ttcagttgtc 2410

aattgccttc ctgttctgtg ggtcttcata ctgagagatt tgtatatttt atattaaatc 2470

atgtcataca gatttttgtt gtgattttca gagatgactt ccacagatta aagtcttagc 2530

tgttgcctaa ggcaaagcaa aacaacacat ggtaataatt ttccctactg gaggattctc 2590

tttatgtgaa agccctggtg ggcaatgaca taagtcttgt gatgattgtc tgctaagcat 2650

actctgtgct catcttcatc cattgggccc ggcaccaaag cttctagaag ccagcgtgga 2710

tctaccaact ttgggggata aaattgccat tcttggtgca gtaacctact aattggcagg 2770

caggattctc gagtgtgtga atgccatgca gctgtctttg ttttgttctt ggtagctgtt 2830

actgctacat gtttacagta cttttagttt taatttcgaa gtaagctttt ctgacagaca 2890

ttttgcaaca acttgactgt tgtatattga caagttcatg gatgtatttg attcttatta 2950

acatcaagga caggctggta agtacctgca tggttgagtc actggtcaag gaaatggaga 3010

tgacctatga accctggtgt gaagtagata cactggatct cctgggcacc tggagcagca 3070

gcaggagaca gcccaaggag gcaggagggc tctaactaag caggaccatt ttgtcttgaa 3130

gtcggtacac aggagtaatt atgccctgtt caagttggtc ccctggactc actattagta 3190

ccttacctat aatgttactg atacagacta gccaggaggc agggaggcat agctgggtgg 3250

tagtgtgctt gcttagcatt catagaccaa gtgtgatctc tatcaccgca ggaagtaaag 3310

agcagagaga ttcctgggcc aagggagtgg attataaagc tgtaggatgt gaccattggc 3370

agtgagggca ggggtgggac gtggctggcc tggtgagaaa cggataccgg gcattgcctc 3430

ccctgtttgg tgtccacgct cacctggtaa cctgctaaaa gctgtggcag ctccttgtgg 3490

aaggctgcat ggtcaaagtt ctgtgtctta ccacaaaaca ataaagtgaa tggttcct 3548

<210> 6
<211> 700
<212> PRT
<213> Mouse

<400> 6

Met Ala Asp Thr Gly Leu Arg Arg Val Val Pro Ser Asp Leu Tyr Pro
1               5                   10                  15

Leu Val Leu Arg Phe Leu Arg Asp Ser Gln Leu Ser Glu Val Ala Ser
            20                  25                  30

Lys Phe Ala Lys Ala Thr Gly Ala Thr Gln Gln Asp Ala Asn Ala Ser
        35                  40                  45

Ser Leu Leu Asp Ile Tyr Ser Phe Trp Leu Lys Ser Thr Lys Ala Pro
    50                  55                  60

Lys Val Lys Leu Gln Ser Asn Gly Pro Val Thr Lys Lys Ala Lys Lys
65                  70                  75                  80

Glu Thr Ser Ser Ser Asp Ser Ser Glu Asp Ser Ser Glu Asp Glu Asp
                85                  90                  95

Lys Lys Ala Gln Gly Leu Pro Thr Gln Lys Ala Ala Ala Gln Val Lys
            100                 105                 110

Arg Ala Ser Val Pro Gln His Ala Gly Lys Ala Ala Ala Lys Ala Ser
        115                 120                 125

Glu Ser Ser Ser Ser Glu Glu Ser Ser Glu Glu Glu Glu Glu Asp Lys

130                 135                 140

Lys Lys Lys Pro Val Gln Lys Ala Ala Lys Pro Gln Ala Lys Ala Val
145                 150                 155                 160

Arg Pro Pro Ala Lys Lys Ala Glu Ser Ser Glu Ser Asp Ser Asp Ser
                165                 170                 175

Asp Ser Asp Ser Ser Ser Glu Glu Glu Thr Pro Gln Thr Gln Lys Pro
            180                 185                 190

Lys Ala Ala Val Ala Ala Lys Ala Gln Thr Lys Ala Glu Ala Lys Pro
        195                 200                 205

Gly Thr Pro Ala Lys Ala Gln Pro Lys Val Ala Asn Gly Lys Ala Ala
    210                 215                 220

Ala Ser Ser Ser Ser Ser Ser Ser Ser Asp Asp Ser Glu Glu Glu Lys
225                 230                 235                 240

Lys Ala Ala Ala Pro Pro Lys Lys Thr Val Pro Lys Lys Gln Val Val
                245                 250                 255

Ala Lys Ala Pro Val Lys Val Ala Ala Ala Pro Thr Gln Lys Ser Ser
            260                 265                 270

Ser Ser Glu Asp Ser Ser Ser Glu Glu Glu Glu Gly Gln Arg Gln Pro
        275                 280                 285

Met Lys Lys Lys Ala Gly Pro Tyr Ser Ser Val Pro Pro Pro Ser Val
    290                 295                 300

Pro Leu Pro Lys Lys Ser Pro Gly Thr Gln Ala Pro Lys Lys Ala Ala
305                 310                 315                 320

Ala Gln Thr Gln Pro Ala Asp Ser Ser Asp Asp Ser Ser Asp Asp Ser
                325                 330                 335

Asp Ser Ser Ser Glu Glu Glu Lys Lys Pro Pro Ala Lys Thr Val Val
            340                 345                 350

Ser Lys Thr Pro Ala Lys Ala Ala Pro Val Lys Lys Lys Ala Glu Ser
        355                 360                 365

Ser Ser Asp Ser Ser Asp Ser Asp Ser Ser Glu Asp Glu Ala Pro Ala
    370                 375                 380

Lys Pro Val Ser Thr Thr Lys Ser Pro Lys Pro Ala Val Thr Pro Lys
385                 390                 395                 400

Pro Ser Ala Ala Lys Ala Val Thr Thr Pro Lys Gln Pro Ala Gly Ser
                405                 410                 415

Asn Gln Lys Pro Gln Ser Arg Lys Ala Asp Ser Ser Ser Ser Glu Glu
            420                 425                 430

Glu Ser Ser Ser Ser Glu Glu Glu Glu Ala Ser Lys Lys Ser Ala Thr
        435                 440                 445

Thr Pro Lys Ala Lys Val Thr Ala Lys Ala Ala Pro Ala Lys Gln Ala
    450                 455                 460

Pro Gln Ala Ala Gly Asp Ser Ser Ser Asp Ser Asp Ser Ser Ser Ser
465                 470                 475                 480

Glu Glu Glu Glu Lys Thr Pro Lys Pro Pro Ala Lys Lys Lys Ala Ala
                485                 490                 495

Gly Gly Ala Val Ser Thr Pro Ala Pro Gly Lys Lys Ala Glu Ala Lys
            500                 505                 510

Ser Ser Ser Ser Ser Ser Ser Ser Ser Ser Glu Asp Ser Ser Glu Glu
        515                 520                 525

Glu Lys Lys Lys Lys Pro Lys Ala Thr Thr Pro Lys Ile Gln Ala Ser
    530                 535                 540

Lys Ala Asn Gly Thr Pro Ala Ser Leu Asn Gly Lys Ala Ala Lys Glu
545                 550                 555                 560

Ser Glu Glu Glu Glu Glu Glu Glu Glu Thr Glu Glu Lys Lys Lys Ala
                565                 570                 575

Ala Gly Thr Lys Pro Gly Ser Gly Lys Lys Arg Lys Gln Asn Glu Thr
            580                 585                 590

Ala Asp Glu Ala Thr Thr Pro Gln Ala Lys Lys Val Lys Leu Glu Thr
        595                 600                 605

Pro Asn Thr Phe Pro Lys Arg Lys Lys Gly Glu Arg Arg Ala Ser Ser
    610                 615                 620

Pro Phe Arg Arg Val Arg Glu Glu Glu Ile Glu Val Asp Ser Arg Val
625                 630                 635                 640

Ala Asp Asn Ser Phe Asp Ala Lys Arg Gly Ala Ala Gly Asp Trp Gly
                645                 650                 655

Glu Arg Ala Asn Gln Val Leu Lys Phe Thr Lys Gly Lys Ser Phe Arg
            660                 665                 670

His Glu Lys Thr Lys Lys Lys Arg Gly Ser Tyr Arg Gly Gly Ser Ile
        675                 680                 685

Ser Val Gln Val Asn Ser Val Lys Phe Asp Ser Glu
    690                 695                 700

<210>  7
<211>  4126
<212>  DNA
<213>  Mouse

<220>
<221>  CDS
<222>  (16)..(4050)
<223>

<400>  7
cgtgtttggc tcagc atg gcg gag atg aag agc cct acg aaa gct gag cct      51
                 Met Ala Glu Met Lys Ser Pro Thr Lys Ala Glu Pro
                 1               5                   10

gcg act ccc gca gaa gcg gcg caa agc gac cgc cac agc ctg ctg gag      99
Ala Thr Pro Ala Glu Ala Ala Gln Ser Asp Arg His Ser Leu Leu Glu
        15                  20                  25

cac agc cgc gag ttc ttg gac ttc ttc tgg gac att gcg aaa ccg gat      147
His Ser Arg Glu Phe Leu Asp Phe Phe Trp Asp Ile Ala Lys Pro Asp
    30                  35                  40

cag gaa acg cgg ctc cgg gcc acg gag aag ttg ttg gag tac ttg cgc      195
Gln Glu Thr Arg Leu Arg Ala Thr Glu Lys Leu Leu Glu Tyr Leu Arg
45                  50                  55                  60

aca agg ccc aat gat tcg gag atg aaa tat gcc ctg aag cgc cta atc      243
Thr Arg Pro Asn Asp Ser Glu Met Lys Tyr Ala Leu Lys Arg Leu Ile
                65                  70                  75

act ggg ctt ggg gtg ggc cga gaa gcc gct acg gcc tgc tac agc ctg      291
Thr Gly Leu Gly Val Gly Arg Glu Ala Ala Thr Ala Cys Tyr Ser Leu
            80                  85                  90

gcg ctg gca cag ctg ttg cag tct ttt gaa gac atc cca ttg tgt gac      339
Ala Leu Ala Gln Leu Leu Gln Ser Phe Glu Asp Ile Pro Leu Cys Asp

        95                  100                 105

atc ctg gat cag ata caa gaa aaa tac agc cta caa gcc atg aac aag 387
Ile Leu Asp Gln Ile Gln Glu Lys Tyr Ser Leu Gln Ala Met Asn Lys
    110                 115                 120

gca atg atg aga cct agt ctc ttt gca aac ctt ttt gga gtg cta gcc 435
Ala Met Met Arg Pro Ser Leu Phe Ala Asn Leu Phe Gly Val Leu Ala
125                 130                 135                 140

ctc ttt cag tca ggc cgc cta gtg aag gac aaa gag gcc ctg atg aag 483
Leu Phe Gln Ser Gly Arg Leu Val Lys Asp Lys Glu Ala Leu Met Lys
                145                 150                 155

tcc gtg caa ttg ctg aag atc ctg tcc caa cac ccc aac cac tta cag 531
Ser Val Gln Leu Leu Lys Ile Leu Ser Gln His Pro Asn His Leu Gln
            160                 165                 170

gga cag cca ata aag gct ctg gtg gac atc ctc tct gag gtc cca gag 579
Gly Gln Pro Ile Lys Ala Leu Val Asp Ile Leu Ser Glu Val Pro Glu
        175                 180                 185

tcc atg ttc cag gag atc ctg cca aag gtc ctc aag ggt aac atg aaa 627
Ser Met Phe Gln Glu Ile Leu Pro Lys Val Leu Lys Gly Asn Met Lys
    190                 195                 200

gtg atc ctc cgc tct ccc aag tac ttg gag ctc ttc ctc ctg gct aag 675
Val Ile Leu Arg Ser Pro Lys Tyr Leu Glu Leu Phe Leu Leu Ala Lys
205                 210                 215                 220

cag agg gtg ccg aca aag ctc gag tca ctc atg ggc tcg gtt gac cta 723
Gln Arg Val Pro Thr Lys Leu Glu Ser Leu Met Gly Ser Val Asp Leu
                225                 230                 235

ttc tca gaa gac aat att ccc agt ctg gtg aac atc ctg aag gtg gcc 771
Phe Ser Glu Asp Asn Ile Pro Ser Leu Val Asn Ile Leu Lys Val Ala
            240                 245                 250

gcc aac tct gtc aag aag gag cac aag ctg cct aat gtg gct ctg gac 819
Ala Asn Ser Val Lys Lys Glu His Lys Leu Pro Asn Val Ala Leu Asp
        255                 260                 265

ctg ctc cgc ctg gct ctc aag gag agc aga ttc gaa ctg ttc tgg aag 867
Leu Leu Arg Leu Ala Leu Lys Glu Ser Arg Phe Glu Leu Phe Trp Lys
    270                 275                 280

aag gtt ttg gag gag ggg ctg ctg aag aat ccg tcc tgg aca tcc agc 915
Lys Val Leu Glu Glu Gly Leu Leu Lys Asn Pro Ser Trp Thr Ser Ser
285                 290                 295                 300

tac atg tgc ttc cgc cta ctg ggt gcg tct ctg ccg ctt ctg tca gag 963
Tyr Met Cys Phe Arg Leu Leu Gly Ala Ser Leu Pro Leu Leu Ser Glu
                305                 310                 315

gag cag ttg cag ttg gtg atg cga gga gac ttg atc cgc cat ttt ggg 1011
Glu Gln Leu Gln Leu Val Met Arg Gly Asp Leu Ile Arg His Phe Gly
            320                 325                 330

gag aac atg gtt att tct aag ccc caa aac cta ttt aag atc atc cca 1059
Glu Asn Met Val Ile Ser Lys Pro Gln Asn Leu Phe Lys Ile Ile Pro
        335                 340                 345

gag ata agt aca tac gtg ggt acc ttc cta gag ggg tgc cag gat gac 1107
Glu Ile Ser Thr Tyr Val Gly Thr Phe Leu Glu Gly Cys Gln Asp Asp
    350                 355                 360

cct aag cgg cag ttg act atg atg gtg gcc ttt aca acc atc acc aat 1155
Pro Lys Arg Gln Leu Thr Met Met Val Ala Phe Thr Thr Ile Thr Asn
365                 370                 375                 380

caa ggt ctc cct gtc atg cct acc ttc tgg cgt gtc acg cgg ttt ttg 1203
Gln Gly Leu Pro Val Met Pro Thr Phe Trp Arg Val Thr Arg Phe Leu
                385                 390                 395

aat gct gaa gcc ctg cag agc tat gtg gcc tgg ttg cgg gac atg ttc 1251
Asn Ala Glu Ala Leu Gln Ser Tyr Val Ala Trp Leu Arg Asp Met Phe
            400                 405                 410

ctg cag cct gac ctg aac tcc ttg gtt gac ttc agc act gcc aac cag 1299
Leu Gln Pro Asp Leu Asn Ser Leu Val Asp Phe Ser Thr Ala Asn Gln
        415                 420                 425

aag aga gct cag gac gcc tcg ttg aat gtg cct gag cga gct gta ttc 1347
Lys Arg Ala Gln Asp Ala Ser Leu Asn Val Pro Glu Arg Ala Val Phe
    430                 435                 440

cgg ctc cgg aag tgg atc atc cac cgc ctg gtc agc ctt gtg gat cat 1395
Arg Leu Arg Lys Trp Ile Ile His Arg Leu Val Ser Leu Val Asp His
445                 450                 455                 460

ttg cat ctg gag aag gat gaa gct gtg gtt gag caa ata gcc agg ttt 1443
Leu His Leu Glu Lys Asp Glu Ala Val Val Glu Gln Ile Ala Arg Phe
                465                 470                 475

tgc ttg ttc cat gcc ttc ttt aag acg aag aag gct acg ccc cag atc 1491
Cys Leu Phe His Ala Phe Phe Lys Thr Lys Lys Ala Thr Pro Gln Ile
            480                 485                 490

cca gag acg aag cag cac ttc tcc ttc cct ttg gac gac cgc aac cgt 1539
Pro Glu Thr Lys Gln His Phe Ser Phe Pro Leu Asp Asp Arg Asn Arg
        495                 500                 505

ggg gtc ttt gtc agt gcc ttc ttc agc cta ctg cag acg ctc agt gtg 1587
Gly Val Phe Val Ser Ala Phe Phe Ser Leu Leu Gln Thr Leu Ser Val
    510                 515                 520

aag ttc agg cag aca cca gac ctg gct gaa aat ggg aag cct tgg act 1635
Lys Phe Arg Gln Thr Pro Asp Leu Ala Glu Asn Gly Lys Pro Trp Thr
525                 530                 535                 540

tac cgc ctg gtt cag ttg gca gac atg ctg ttg aac cat aac cgc aat 1683
Tyr Arg Leu Val Gln Leu Ala Asp Met Leu Leu Asn His Asn Arg Asn
                545                 550                 555

gta acc agt gtg aca tcc ttg aca aca cag cag cgt cag gcc tgg gac 1731
Val Thr Ser Val Thr Ser Leu Thr Thr Gln Gln Arg Gln Ala Trp Asp
            560                 565                 570

cag atg atg agt act ctg aag gaa tta gag gcc cgc tcc tct gag acc 1779
Gln Met Met Ser Thr Leu Lys Glu Leu Glu Ala Arg Ser Ser Glu Thr
        575                 580                 585

agg gcc att gcc ttc cag cac ctg ctg ctt ctg gtg ggc ctc cac atc 1827
Arg Ala Ile Ala Phe Gln His Leu Leu Leu Leu Val Gly Leu His Ile
    590                 595                 600

ttc aag tcc cct gca gaa agc tgt gat gtc cta gga gac att cag act 1875
Phe Lys Ser Pro Ala Glu Ser Cys Asp Val Leu Gly Asp Ile Gln Thr
605                 610                 615                 620

tgc atc aag aaa agc atg gag cag aat ccc cgc cga tca cgc tct aga 1923
Cys Ile Lys Lys Ser Met Glu Gln Asn Pro Arg Arg Ser Arg Ser Arg
                625                 630                 635

gcc aaa gcc tcc cag gag cca gta tgg gtg gag gtg atg gtg gag atc 1971

Ala Lys Ala Ser Gln Glu Pro Val Trp Val Glu Val Met Val Glu Ile
            640                 645                 650

ttg ctg tcc ttg ctg gct caa ccc agc aac ttg atg cgc cag gtg gtc 2019
Leu Leu Ser Leu Leu Ala Gln Pro Ser Asn Leu Met Arg Gln Val Val
        655                 660                 665

cgg agt gta ttt ggt cat atc tgt ccc cac ctt act cca cgt tgt ctg 2067
Arg Ser Val Phe Gly His Ile Cys Pro His Leu Thr Pro Arg Cys Leu
    670                 675                 680

cag cta atc ctg gct gtg ctc agc cct gtg aca aac gag gat gag gat 2115
Gln Leu Ile Leu Ala Val Leu Ser Pro Val Thr Asn Glu Asp Glu Asp
685                 690                 695                 700

gac aac gtg gtg gtc act gat gac gct gat gag aag cag ctg cag cat 2163
Asp Asn Val Val Val Thr Asp Asp Ala Asp Glu Lys Gln Leu Gln His
                705                 710                 715

gga gag gac gaa gac tca gat aat gag gac aat aag aac tca gag agt 2211
Gly Glu Asp Glu Asp Ser Asp Asn Glu Asp Asn Lys Asn Ser Glu Ser
            720                 725                 730

gac atg gac agt gag gat ggg gaa gaa agt gaa gag gag gac cgt gac 2259
Asp Met Asp Ser Glu Asp Gly Glu Glu Ser Glu Glu Glu Asp Arg Asp
        735                 740                 745

aaa gat gtg gac cca ggc ttc cgt caa cag ttg atg gaa gtg tta aaa 2307
Lys Asp Val Asp Pro Gly Phe Arg Gln Gln Leu Met Glu Val Leu Lys
    750                 755                 760

gct ggg aat gca ttg ggt gga gtg gac aac gag gag gag gag gag ctt 2355
Ala Gly Asn Ala Leu Gly Gly Val Asp Asn Glu Glu Glu Glu Glu Leu
765                 770                 775                 780

ggg gat gag gcc atg atg gcc ctg gac cag aac ctg gcc agc cta ttt 2403
Gly Asp Glu Ala Met Met Ala Leu Asp Gln Asn Leu Ala Ser Leu Phe
                785                 790                 795

aaa gag cag aag atg cgc atc cag gcc cgg aat gag gag aaa aac aag 2451
Lys Glu Gln Lys Met Arg Ile Gln Ala Arg Asn Glu Glu Lys Asn Lys
            800                 805                 810

cta cag aag gag aag aag ctc cga cgg gac ttc caa atc agg gca cta 2499
Leu Gln Lys Glu Lys Lys Leu Arg Arg Asp Phe Gln Ile Arg Ala Leu

        815                 820                 825

gac ctg atc gag gtg ctg gtg acc aag cag cct gag cac ccc ctg atc 2547
Asp Leu Ile Glu Val Leu Val Thr Lys Gln Pro Glu His Pro Leu Ile
    830                 835                 840

ctg gaa cta ctt gag cca ctg ctg aac gtg atc cag cac agc atg cgc 2595
Leu Glu Leu Leu Glu Pro Leu Leu Asn Val Ile Gln His Ser Met Arg
845                 850                 855                 860

agc aaa ggc tcc acc aag cag gag cag gac ctc ctg cac aag acc gcc 2643
Ser Lys Gly Ser Thr Lys Gln Glu Gln Asp Leu Leu His Lys Thr Ala
                865                 870                 875

cgc atc ttc atg cac cac ctg tgt cgt gcc cgc cgc tac tgc cac gag 2691
Arg Ile Phe Met His His Leu Cys Arg Ala Arg Arg Tyr Cys His Glu
            880                 885                 890

gtg ggg ccg tgt gca gag gct ctg cat gcc cag gtg gag agg ctt gtg 2739
Val Gly Pro Cys Ala Glu Ala Leu His Ala Gln Val Glu Arg Leu Val
        895                 900                 905

cag cag gct ggc agc cag gct gat gcc tct gtc gcc cta tac tat ttc 2787
Gln Gln Ala Gly Ser Gln Ala Asp Ala Ser Val Ala Leu Tyr Tyr Phe
    910                 915                 920

aat gcc tct ctg tac ctg ctg cga gtc ctc aag ggc aac acc aat aag 2835
Asn Ala Ser Leu Tyr Leu Leu Arg Val Leu Lys Gly Asn Thr Asn Lys
925                 930                 935                 940

agg cac caa gat ggt cat aag tta cac gga gct gac aca gag gac tca 2883
Arg His Gln Asp Gly His Lys Leu His Gly Ala Asp Thr Glu Asp Ser
                945                 950                 955

gag gac cag gct gct aac tgc tta gac ttg gac ttt gtg acc cgg gtg 2931
Glu Asp Gln Ala Ala Asn Cys Leu Asp Leu Asp Phe Val Thr Arg Val
            960                 965                 970

tat tca gca tca ctg gaa tct ctt ctg acc aag cgt aac agc tca ctt 2979
Tyr Ser Ala Ser Leu Glu Ser Leu Leu Thr Lys Arg Asn Ser Ser Leu
        975                 980                 985

acg gtc ccc atg ttc ctc agc ctc ttc tcc aga tac cca gtg atc tgt 3027
Thr Val Pro Met Phe Leu Ser Leu Phe Ser Arg Tyr Pro Val Ile Cys
    990                 995                 1000

aag aac ctg ctt ccc gtc ctg gct cag cat gtg gct ggc cca tct 3072
Lys Asn Leu Leu Pro Val Leu Ala Gln His Val Ala Gly Pro Ser
1005                1010                1015

cgg ccc cgc cat cag gcc cag gca tgc ctg atg ctc cag aag act 3117
Arg Pro Arg His Gln Ala Gln Ala Cys Leu Met Leu Gln Lys Thr
1020                1025                1030

ctg tct gca cga gag ctg aga gtc tgt ttt gag gat cct gag tgg 3162
Leu Ser Ala Arg Glu Leu Arg Val Cys Phe Glu Asp Pro Glu Trp
1035                1040                1045

gag cag ctg att acc caa ctc ttg gga aag gcc acc cag acc ctg 3207
Glu Gln Leu Ile Thr Gln Leu Leu Gly Lys Ala Thr Gln Thr Leu
1050                1055                1060

cag act ctt ggg gag gca cag agc aag ggg gag cac cag aag gag 3252
Gln Thr Leu Gly Glu Ala Gln Ser Lys Gly Glu His Gln Lys Glu
1065                1070                1075

cta tcc atc ttg gag ctg ctg aac act ctg ttg agg aca gtc aat 3297
Leu Ser Ile Leu Glu Leu Leu Asn Thr Leu Leu Arg Thr Val Asn
1080                1085                1090

cac gag aag ctg tct gtg gac ctc act gct ccc ttg ggc gtg ctt 3342
His Glu Lys Leu Ser Val Asp Leu Thr Ala Pro Leu Gly Val Leu
1095                1100                1105

cag agc aag caa cag aag ctg cag caa agc ctg cag cag ggg aat 3387
Gln Ser Lys Gln Gln Lys Leu Gln Gln Ser Leu Gln Gln Gly Asn
1110                1115                1120

cac tca tct ggc tcc aat cgc ctc tat gat ctc tac tgg cag gcc 3432
His Ser Ser Gly Ser Asn Arg Leu Tyr Asp Leu Tyr Trp Gln Ala
1125                1130                1135

atg agg atg cta gga gtc caa cgt cca aag tca gaa aag aag aat 3477
Met Arg Met Leu Gly Val Gln Arg Pro Lys Ser Glu Lys Lys Asn
1140                1145                1150

gcc aag gat att cct agt gac acc cag agc ccc gtc agc aca aag 3522
Ala Lys Asp Ile Pro Ser Asp Thr Gln Ser Pro Val Ser Thr Lys
1155                1160                1165

cgg aag aaa aag gga ttc ttg cca gag acc aag aag cga aag aaa 3567
Arg Lys Lys Lys Gly Phe Leu Pro Glu Thr Lys Lys Arg Lys Lys
1170                1175                1180

ctt aaa tct gag ggc acc aca cca gaa aag aat gct gcg tca cag 3612
Leu Lys Ser Glu Gly Thr Thr Pro Glu Lys Asn Ala Ala Ser Gln
1185                1190                1195

cag gat gca gtg aca gag ggt gcc atg cct gct gcc act ggt aaa 3657
Gln Asp Ala Val Thr Glu Gly Ala Met Pro Ala Ala Thr Gly Lys
1200                1205                1210

gac cag ccc ccc agc aca ggc aag aag aaa agg aag agg gta aag 3702
Asp Gln Pro Pro Ser Thr Gly Lys Lys Lys Arg Lys Arg Val Lys
1215                1220                1225

gcc agc acc cca tcc cag gtg aat ggg ata act ggg gcc aag agt 3747
Ala Ser Thr Pro Ser Gln Val Asn Gly Ile Thr Gly Ala Lys Ser
1230                1235                1240

cca gct ccc agt aac ccc acc cta agc ccc agc acc cct gcc aag 3792
Pro Ala Pro Ser Asn Pro Thr Leu Ser Pro Ser Thr Pro Ala Lys
1245                1250                1255

acc cca aaa ctg cag aag aaa aaa gag aag ctg tca cag gtg aat 3837
Thr Pro Lys Leu Gln Lys Lys Lys Glu Lys Leu Ser Gln Val Asn
1260                1265                1270

gga gcc act cct gtg tcc ccc ata gag cct gaa agc aaa aag cat 3882
Gly Ala Thr Pro Val Ser Pro Ile Glu Pro Glu Ser Lys Lys His
1275                1280                1285

cat cag gag gca ctc agc aca aag gag gtc ata aga aag tcc ccc 3927
His Gln Glu Ala Leu Ser Thr Lys Glu Val Ile Arg Lys Ser Pro
1290                1295                1300

cac ccc cag tct gcc ctg cca aag aaa aga gca agg ctg tct ctg 3972
His Pro Gln Ser Ala Leu Pro Lys Lys Arg Ala Arg Leu Ser Leu
1305                1310                1315

gtg agc agg agc ccc agc ctg tta cag agt ggg gtc aag aaa agg 4017
Val Ser Arg Ser Pro Ser Leu Leu Gln Ser Gly Val Lys Lys Arg
1320                1325                1330

aga gtg gcc agc agg aga gtg cag aca cct tga gtgtgtatag 4060

Arg Val Ala Ser Arg Arg Val Gln Thr Pro
1335                1340

tctgtttccc tgccctagag actcctattt tttcaccaat attttaataa acaatccatg 4120

atgcta 4126

<210> 8
<211> 1344
<212> PRT
<213> Mouse

<400> 8

Met Ala Glu Met Lys Ser Pro Thr Lys Ala Glu Pro Ala Thr Pro Ala
1               5                   10                  15

Glu Ala Ala Gln Ser Asp Arg His Ser Leu Leu Glu His Ser Arg Glu
            20                  25                  30

Phe Leu Asp Phe Phe Trp Asp Ile Ala Lys Pro Asp Gln Glu Thr Arg
        35                  40                  45

Leu Arg Ala Thr Glu Lys Leu Leu Glu Tyr Leu Arg Thr Arg Pro Asn
    50                  55                  60

Asp Ser Glu Met Lys Tyr Ala Leu Lys Arg Leu Ile Thr Gly Leu Gly
65                  70                  75                  80

Val Gly Arg Glu Ala Ala Thr Ala Cys Tyr Ser Leu Ala Leu Ala Gln
                85                  90                  95

Leu Leu Gln Ser Phe Glu Asp Ile Pro Leu Cys Asp Ile Leu Asp Gln
            100                 105                 110

Ile Gln Glu Lys Tyr Ser Leu Gln Ala Met Asn Lys Ala Met Met Arg
        115                 120                 125

Pro Ser Leu Phe Ala Asn Leu Phe Gly Val Leu Ala Leu Phe Gln Ser
    130                 135                 140

Gly Arg Leu Val Lys Asp Lys Glu Ala Leu Met Lys Ser Val Gln Leu
145                 150                 155                 160

Leu Lys Ile Leu Ser Gln His Pro Asn His Leu Gln Gly Gln Pro Ile
                165                 170                 175

Lys Ala Leu Val Asp Ile Leu Ser Glu Val Pro Glu Ser Met Phe Gln
            180                 185                 190

Glu Ile Leu Pro Lys Val Leu Lys Gly Asn Met Lys Val Ile Leu Arg
        195                 200                 205

Ser Pro Lys Tyr Leu Glu Leu Phe Leu Leu Ala Lys Gln Arg Val Pro
    210                 215                 220

Thr Lys Leu Glu Ser Leu Met Gly Ser Val Asp Leu Phe Ser Glu Asp
225                 230                 235                 240

Asn Ile Pro Ser Leu Val Asn Ile Leu Lys Val Ala Ala Asn Ser Val
                245                 250                 255

Lys Lys Glu His Lys Leu Pro Asn Val Ala Leu Asp Leu Leu Arg Leu
            260                 265                 270

Ala Leu Lys Glu Ser Arg Phe Glu Leu Phe Trp Lys Lys Val Leu Glu
        275                 280                 285

Glu Gly Leu Leu Lys Asn Pro Ser Trp Thr Ser Ser Tyr Met Cys Phe
    290                 295                 300

Arg Leu Leu Gly Ala Ser Leu Pro Leu Leu Ser Glu Glu Gln Leu Gln
305                 310                 315                 320

Leu Val Met Arg Gly Asp Leu Ile Arg His Phe Gly Glu Asn Met Val
                325                 330                 335

Ile Ser Lys Pro Gln Asn Leu Phe Lys Ile Ile Pro Glu Ile Ser Thr
            340                 345                 350

Tyr Val Gly Thr Phe Leu Glu Gly Cys Gln Asp Asp Pro Lys Arg Gln
        355                 360                 365

Leu Thr Met Met Val Ala Phe Thr Thr Ile Thr Asn Gln Gly Leu Pro
    370                 375                 380

Val Met Pro Thr Phe Trp Arg Val Thr Arg Phe Leu Asn Ala Glu Ala
385                 390                 395                 400

Leu Gln Ser Tyr Val Ala Trp Leu Arg Asp Met Phe Leu Gln Pro Asp
                405                 410                 415

Leu Asn Ser Leu Val Asp Phe Ser Thr Ala Asn Gln Lys Arg Ala Gln
            420                 425                 430

Asp Ala Ser Leu Asn Val Pro Glu Arg Ala Val Phe Arg Leu Arg Lys
        435                 440                 445

Trp Ile Ile His Arg Leu Val Ser Leu Val Asp His Leu His Leu Glu
    450                 455                 460

Lys Asp Glu Ala Val Val Glu Gln Ile Ala Arg Phe Cys Leu Phe His
465                 470                 475                 480

Ala Phe Phe Lys Thr Lys Lys Ala Thr Pro Gln Ile Pro Glu Thr Lys
                485                 490                 495

Gln His Phe Ser Phe Pro Leu Asp Asp Arg Asn Arg Gly Val Phe Val
            500                 505                 510

Ser Ala Phe Phe Ser Leu Leu Gln Thr Leu Ser Val Lys Phe Arg Gln
        515                 520                 525

Thr Pro Asp Leu Ala Glu Asn Gly Lys Pro Trp Thr Tyr Arg Leu Val
    530                 535                 540

Gln Leu Ala Asp Met Leu Leu Asn His Asn Arg Asn Val Thr Ser Val
545                 550                 555                 560

Thr Ser Leu Thr Thr Gln Gln Arg Gln Ala Trp Asp Gln Met Met Ser
                565                 570                 575

Thr Leu Lys Glu Leu Glu Ala Arg Ser Ser Glu Thr Arg Ala Ile Ala
            580                 585                 590

Phe Gln His Leu Leu Leu Leu Val Gly Leu His Ile Phe Lys Ser Pro
        595                 600                 605

Ala Glu Ser Cys Asp Val Leu Gly Asp Ile Gln Thr Cys Ile Lys Lys
    610                 615                 620

Ser Met Glu Gln Asn Pro Arg Arg Ser Arg Ser Arg Ala Lys Ala Ser
625                 630                 635                 640

Gln Glu Pro Val Trp Val Glu Val Met Val Glu Ile Leu Leu Ser Leu
                645                 650                 655

Leu Ala Gln Pro Ser Asn Leu Met Arg Gln Val Val Arg Ser Val Phe

            660                 665                 670

Gly His Ile Cys Pro His Leu Thr Pro Arg Cys Leu Gln Leu Ile Leu
        675                 680                 685

Ala Val Leu Ser Pro Val Thr Asn Glu Asp Glu Asp Asp Asn Val Val
    690                 695                 700

Val Thr Asp Asp Ala Asp Glu Lys Gln Leu Gln His Gly Glu Asp Glu
705                 710                 715                 720

Asp Ser Asp Asn Glu Asp Asn Lys Asn Ser Glu Ser Asp Met Asp Ser
                725                 730                 735

Glu Asp Gly Glu Glu Ser Glu Glu Glu Asp Arg Asp Lys Asp Val Asp
            740                 745                 750

Pro Gly Phe Arg Gln Gln Leu Met Glu Val Leu Lys Ala Gly Asn Ala
        755                 760                 765

Leu Gly Gly Val Asp Asn Glu Glu Glu Glu Glu Leu Gly Asp Glu Ala
    770                 775                 780

Met Met Ala Leu Asp Gln Asn Leu Ala Ser Leu Phe Lys Glu Gln Lys
785                 790                 795                 800

Met Arg Ile Gln Ala Arg Asn Glu Glu Lys Asn Lys Leu Gln Lys Glu
                805                 810                 815

Lys Lys Leu Arg Arg Asp Phe Gln Ile Arg Ala Leu Asp Leu Ile Glu
            820                 825                 830

Val Leu Val Thr Lys Gln Pro Glu His Pro Leu Ile Leu Glu Leu Leu
        835                 840                 845

Glu Pro Leu Leu Asn Val Ile Gln His Ser Met Arg Ser Lys Gly Ser
    850                 855                 860

Thr Lys Gln Glu Gln Asp Leu Leu His Lys Thr Ala Arg Ile Phe Met
865                 870                 875                 880

His His Leu Cys Arg Ala Arg Arg Tyr Cys His Glu Val Gly Pro Cys
                885                 890                 895

Ala Glu Ala Leu His Ala Gln Val Glu Arg Leu Val Gln Gln Ala Gly
            900                 905                 910

Ser Gln Ala Asp Ala Ser Val Ala Leu Tyr Tyr Phe Asn Ala Ser Leu
        915                 920                 925

Tyr Leu Leu Arg Val Leu Lys Gly Asn Thr Asn Lys Arg His Gln Asp
    930                 935                 940

Gly His Lys Leu His Gly Ala Asp Thr Glu Asp Ser Glu Asp Gln Ala
945                 950                 955                 960

Ala Asn Cys Leu Asp Leu Asp Phe Val Thr Arg Val Tyr Ser Ala Ser
                965                 970                 975

Leu Glu Ser Leu Leu Thr Lys Arg Asn Ser Ser Leu Thr Val Pro Met
            980                 985                 990

Phe Leu Ser Leu Phe Ser Arg Tyr  Pro Val Ile Cys Lys  Asn Leu Leu
        995                 1000                 1005

Pro Val  Leu Ala Gln His Val  Ala Gly Pro Ser Arg  Pro Arg His
    1010                 1015                 1020

Gln Ala Gln Ala Cys Leu Met Leu Gln Lys Thr Leu Ser Ala Arg
    1025                1030                1035

Glu Leu Arg Val Cys Phe Glu Asp Pro Glu Trp Glu Gln Leu Ile
    1040                1045                1050

Thr Gln Leu Leu Gly Lys Ala Thr Gln Thr Leu Gln Thr Leu Gly
    1055                1060                1065

Glu Ala Gln Ser Lys Gly Glu His Gln Lys Glu Leu Ser Ile Leu
    1070                1075                1080

Glu Leu Leu Asn Thr Leu Leu Arg Thr Val Asn His Glu Lys Leu
    1085                1090                1095

Ser Val Asp Leu Thr Ala Pro Leu Gly Val Leu Gln Ser Lys Gln
    1100                1105                1110

Gln Lys Leu Gln Gln Ser Leu Gln Gln Gly Asn His Ser Ser Gly
    1115                1120                1125

Ser Asn Arg Leu Tyr Asp Leu Tyr Trp Gln Ala Met Arg Met Leu
    1130                1135                1140

Gly Val Gln Arg Pro Lys Ser Glu Lys Lys Asn Ala Lys Asp Ile
    1145                1150                1155

Pro Ser Asp Thr Gln Ser Pro Val Ser Thr Lys Arg Lys Lys Lys
    1160                1165                1170

Gly Phe Leu Pro Glu Thr Lys Lys Arg Lys Lys Leu Lys Ser Glu
    1175                1180                1185

Gly Thr Thr Pro Glu Lys Asn Ala Ala Ser Gln Gln Asp Ala Val
    1190                1195                1200

Thr Glu Gly Ala Met Pro Ala Ala Thr Gly Lys Asp Gln Pro Pro
    1205                1210                1215

Ser Thr Gly Lys Lys Lys Arg Lys Arg Val Lys Ala Ser Thr Pro
    1220                1225                1230

Ser Gln Val Asn Gly Ile Thr Gly Ala Lys Ser Pro Ala Pro Ser
    1235                1240                1245

Asn Pro Thr Leu Ser Pro Ser Thr Pro Ala Lys Thr Pro Lys Leu
    1250                1255                1260

Gln Lys Lys Lys Glu Lys Leu Ser Gln Val Asn Gly Ala Thr Pro
    1265                1270                1275

Val Ser Pro Ile Glu Pro Glu Ser Lys Lys His His Gln Glu Ala
    1280                1285                1290

Leu Ser Thr Lys Glu Val Ile Arg Lys Ser Pro His Pro Gln Ser
    1295                1300                1305

Ala Leu Pro Lys Lys Arg Ala Arg Leu Ser Leu Val Ser Arg Ser
    1310                1315                1320

Pro Ser Leu Leu Gln Ser Gly Val Lys Lys Arg Arg Val Ala Ser
    1325                1330                1335

Arg Arg Val Gln Thr Pro
    1340

<210> 9

<211>  2851
<212>  DNA
<213>  Mouse

<220>
<221>  CDS
<222>  (76)..(2349)
<223>

<400>  9
acgcgggggg aagtgcagcg tgcgtgcggt ttggtgggtc gctgtgtgcg ctccgcgtgt      60

gcagccgcgt gggcc atg ggg cgg cgg gcg cgg ggc cgg cgg ttc cag cag      111
                 Met Gly Arg Arg Ala Arg Gly Arg Arg Phe Gln Gln
                 1               5                   10

ccg ccg cag cct gag ggc gag gaa gac gcc agc gac ggc ggc aga aag      159
Pro Pro Gln Pro Glu Gly Glu Glu Asp Ala Ser Asp Gly Gly Arg Lys
        15                  20                  25

cga ggc cag gcg ggc tgg gaa ggt ggc tat ccc gag atc gta aag gag      207
Arg Gly Gln Ala Gly Trp Glu Gly Gly Tyr Pro Glu Ile Val Lys Glu
    30                  35                  40

aac aag ctc ttc gag cac tac tat cag gaa ctc aag atc gtg cca gag      255
Asn Lys Leu Phe Glu His Tyr Tyr Gln Glu Leu Lys Ile Val Pro Glu
45                  50                  55                  60

gga gaa tgg gac caa ttc atg gag tca ctc cga gaa cct ctc cca gcc      303
Gly Glu Trp Asp Gln Phe Met Glu Ser Leu Arg Glu Pro Leu Pro Ala
                65                  70                  75

aca ctg aga atc act ggg tac aaa agc cat gcc aaa gag att ctc cat      351
Thr Leu Arg Ile Thr Gly Tyr Lys Ser His Ala Lys Glu Ile Leu His
            80                  85                  90

tgc ttg aag aac aag tac ttt aag gag ttg gag gac ctg gaa gta gat      399
Cys Leu Lys Asn Lys Tyr Phe Lys Glu Leu Glu Asp Leu Glu Val Asp
        95                  100                 105

gga cag aaa gtt gaa gtt cca caa cca cta agc tgg tac cct gaa gaa      447
Gly Gln Lys Val Glu Val Pro Gln Pro Leu Ser Trp Tyr Pro Glu Glu
    110                 115                 120

ctt gcc tgg cat aca aac tta agt cgg aaa atc ttg agg aag tcc ccg      495

Leu Ala Trp His Thr Asn Leu Ser Arg Lys Ile Leu Arg Lys Ser Pro
125                 130                 135                 140

ttg ttg gca aag ttc cat cag ttc ctg gtc agc gag act gag tct gga     543
Leu Leu Ala Lys Phe His Gln Phe Leu Val Ser Glu Thr Glu Ser Gly
                145                 150                 155

aac atc agc cgc cag gag gct gtc agc atg atc ccc cca ctg ctg ctc     591
Asn Ile Ser Arg Gln Glu Ala Val Ser Met Ile Pro Pro Leu Leu Leu
            160                 165                 170

aac gtg gag cca cac cat aag atc tta gac atg tgt gca gcc cct gga     639
Asn Val Glu Pro His His Lys Ile Leu Asp Met Cys Ala Ala Pro Gly
        175                 180                 185

tcc aag acc aca cag tta att gaa atg ttg cat gca gac atg agt gtg     687
Ser Lys Thr Thr Gln Leu Ile Glu Met Leu His Ala Asp Met Ser Val
    190                 195                 200

ccc ttt cca gag gga ttt gta atc gca aat gac gtg gac aac aag cgc     735
Pro Phe Pro Glu Gly Phe Val Ile Ala Asn Asp Val Asp Asn Lys Arg
205                 210                 215                 220

tgc tat ctg ctc gtc cat cag gcc aaa agg ttg agc agt ccc tgc atc     783
Cys Tyr Leu Leu Val His Gln Ala Lys Arg Leu Ser Ser Pro Cys Ile
                225                 230                 235

atg gtg gta aac cat gac gca tcc agc ata cct aga ctt aca gta gat     831
Met Val Val Asn His Asp Ala Ser Ser Ile Pro Arg Leu Thr Val Asp
            240                 245                 250

gtg gac gga agg aaa gag att ctc ttc tat gat cga att tta tgt gat     879
Val Asp Gly Arg Lys Glu Ile Leu Phe Tyr Asp Arg Ile Leu Cys Asp
        255                 260                 265

gtc cct tgc agt ggc gat ggc aca atg aga aaa aac att gat gtc tgg     927
Val Pro Cys Ser Gly Asp Gly Thr Met Arg Lys Asn Ile Asp Val Trp
    270                 275                 280

aag aaa tgg aca acc tta aac agc ttg cag ctc cat ggc ctg cag ctt     975
Lys Lys Trp Thr Thr Leu Asn Ser Leu Gln Leu His Gly Leu Gln Leu
285                 290                 295                 300

cgg att gca act cga ggt gct gag cag ctg gcg gaa ggt ggc agg atg     1023
Arg Ile Ala Thr Arg Gly Ala Glu Gln Leu Ala Glu Gly Gly Arg Met

                305                 310                 315

gtg tat tcc acg tgt tcc ttg aac ccc gtg gag gat gaa gca gtg atc 1071
Val Tyr Ser Thr Cys Ser Leu Asn Pro Val Glu Asp Glu Ala Val Ile
            320                 325                 330

gca gct ctg cta gag aag agt gaa gga gct ctt gag ctt gct gat gtg 1119
Ala Ala Leu Leu Glu Lys Ser Glu Gly Ala Leu Glu Leu Ala Asp Val
        335                 340                 345

tct gct gag ttg cca gga ctg aag tgg atg cct gga gtc tca cag tgg 1167
Ser Ala Glu Leu Pro Gly Leu Lys Trp Met Pro Gly Val Ser Gln Trp
    350                 355                 360

aag gtc atg act aga gac ggg cag tgg ttt gca gac tgg cat gag gtt 1215
Lys Val Met Thr Arg Asp Gly Gln Trp Phe Ala Asp Trp His Glu Val
365                 370                 375                 380

ccc cag ggc agg cat aca caa atc cga cct acc atg ttc cca cca acg 1263
Pro Gln Gly Arg His Thr Gln Ile Arg Pro Thr Met Phe Pro Pro Thr
                385                 390                 395

gac ctg gag aag cta cag gca atg cat cta gag cga tgc ctt cga atc 1311
Asp Leu Glu Lys Leu Gln Ala Met His Leu Glu Arg Cys Leu Arg Ile
            400                 405                 410

ctg ccc cat cat cag aat act gga ggg ttc ttt gtg gca gta ttg gtc 1359
Leu Pro His His Gln Asn Thr Gly Gly Phe Phe Val Ala Val Leu Val
        415                 420                 425

aag aaa gca cca atg ccg tgg aac aaa cgt cag ccc aag gtc cag aat 1407
Lys Lys Ala Pro Met Pro Trp Asn Lys Arg Gln Pro Lys Val Gln Asn
    430                 435                 440

aaa tct gca gaa gcc aga gaa ccc agg gta tcc agc cat gtg gct gcc 1455
Lys Ser Ala Glu Ala Arg Glu Pro Arg Val Ser Ser His Val Ala Ala
445                 450                 455                 460

aca gag gga aat ccc agt gac cag tct gag ctg gaa agt cag atg ata 1503
Thr Glu Gly Asn Pro Ser Asp Gln Ser Glu Leu Glu Ser Gln Met Ile
                465                 470                 475

act gga gct ggt gac tta gaa aca gct cac aac act gag aat aca gag 1551
Thr Gly Ala Gly Asp Leu Glu Thr Ala His Asn Thr Glu Asn Thr Glu
            480                 485                 490

agc aat gag aag aaa gat ggc gtg tgt ggc cct cct cca tca aag aaa 1599
Ser Asn Glu Lys Lys Asp Gly Val Cys Gly Pro Pro Pro Ser Lys Lys
        495                 500                 505

atg aag ttg ttt gga ttt aaa gaa gat cca ttt gta ttc att cct gaa 1647
Met Lys Leu Phe Gly Phe Lys Glu Asp Pro Phe Val Phe Ile Pro Glu
    510                 515                 520

gat gat cct tta ttt cca cct att gag aag ttt tat gcc ttg gat cct 1695
Asp Asp Pro Leu Phe Pro Pro Ile Glu Lys Phe Tyr Ala Leu Asp Pro
525                 530                 535                 540

tca ttc ccg agg atg aat ctg tta acc cga acc aca gaa gga aag aag 1743
Ser Phe Pro Arg Met Asn Leu Leu Thr Arg Thr Thr Glu Gly Lys Lys
                545                 550                 555

cgg cag ctt tat atg gtc tcc aag gag ctg agg aat gta ctg ctg aac 1791
Arg Gln Leu Tyr Met Val Ser Lys Glu Leu Arg Asn Val Leu Leu Asn
            560                 565                 570

aac agc gag aag atg aag gtc att aac act ggg ata aaa gtc tgg tgt 1839
Asn Ser Glu Lys Met Lys Val Ile Asn Thr Gly Ile Lys Val Trp Cys
        575                 580                 585

cga aat aac agt ggt gaa gaa ttc gat tgt gca ttc cgt ttg gca cag 1887
Arg Asn Asn Ser Gly Glu Glu Phe Asp Cys Ala Phe Arg Leu Ala Gln
    590                 595                 600

gag gga ata tat aca ttg tat cca ttt atc aat tca aga atc atc act 1935
Glu Gly Ile Tyr Thr Leu Tyr Pro Phe Ile Asn Ser Arg Ile Ile Thr
605                 610                 615                 620

gta tca atg gaa gac gtg aag aca ctg ttg acc cag gag aac cca ttc 1983
Val Ser Met Glu Asp Val Lys Thr Leu Leu Thr Gln Glu Asn Pro Phe
                625                 630                 635

ttt aga aaa ctg agc agt gag gcc tac agt caa gtc aag gac ctc gca 2031
Phe Arg Lys Leu Ser Ser Glu Ala Tyr Ser Gln Val Lys Asp Leu Ala
            640                 645                 650

aag gga agt gtt gtg ctg aag tat gag cca gat tct gcg aat cca gac 2079
Lys Gly Ser Val Val Leu Lys Tyr Glu Pro Asp Ser Ala Asn Pro Asp
        655                 660                 665

acc ctc cag tgc ccc atc gtg ctg tgt ggg tgg cgg gga aag gcc tct     2127
Thr Leu Gln Cys Pro Ile Val Leu Cys Gly Trp Arg Gly Lys Ala Ser
    670                 675                 680

att cga act ttt gtg ccc aaa aat gag cgg ctt cat tat ctc agg atg     2175
Ile Arg Thr Phe Val Pro Lys Asn Glu Arg Leu His Tyr Leu Arg Met
685                 690                 695                 700

atg ggt ctg gag gta ctg gga gaa aag aag aaa gag gga gtc att ctt     2223
Met Gly Leu Glu Val Leu Gly Glu Lys Lys Lys Glu Gly Val Ile Leu
                705                 710                 715

acc aat gag aat gct gcc agc cca gag cag cct gga gat gag gat gcc     2271
Thr Asn Glu Asn Ala Ala Ser Pro Glu Gln Pro Gly Asp Glu Asp Ala
            720                 725                 730

aag cag aca gca caa gac ccc tgc gtc cca gac tcc gtc cct ggc tgt     2319
Lys Gln Thr Ala Gln Asp Pro Cys Val Pro Asp Ser Val Pro Gly Cys
        735                 740                 745

gat gca gct gca gct gag cca tcc cgg tga aggagtcttc acacaatgaa     2369
Asp Ala Ala Ala Ala Glu Pro Ser Arg
    750                 755

ttggtaccat ccgttgttgg agatcaaacc tagaatgtcc ttagccaggg acctggaaat     2429

gagtggtggc cacagtctgt taggagtcgc ttttgcagag tggatacatg tttctgctgt     2489

taaaggctaa cagcctttgc agaagttcag atcccttgtt ggccagtatt gacctagtct     2549

tctaaaacga cagtaaaagt ctacaagtct ttgggaagca gctctatcta tctatgcttt     2609

aatactttct ggactgcaca ccagtgtgct gtgcagtcat gctgacactg aggtctcagg     2669

tgacttctgt gcctttgtga taaagggtga gataagccat ctcagaggga aggctggtga     2729

atcatgacag cagttttggg gaccctttgt gcttatcctg gagttgtttt aagtggggtg     2789

gagattgctt gggtcatgaa ataaagagct attatgatca tgaaaaaaaa aaaaaaaaaa     2849

aa                                                                    2851

<210>  10
<211>  757

<212>  PRT
<213>  Mouse

<400>  10

Met Gly Arg Arg Ala Arg Gly Arg Arg Phe Gln Gln Pro Pro Gln Pro
1               5                   10                  15

Glu Gly Glu Glu Asp Ala Ser Asp Gly Gly Arg Lys Arg Gly Gln Ala
            20                  25                  30

Gly Trp Glu Gly Gly Tyr Pro Glu Ile Val Lys Glu Asn Lys Leu Phe
        35                  40                  45

Glu His Tyr Tyr Gln Glu Leu Lys Ile Val Pro Glu Gly Glu Trp Asp
    50                  55                  60

Gln Phe Met Glu Ser Leu Arg Glu Pro Leu Pro Ala Thr Leu Arg Ile
65                  70                  75                  80

Thr Gly Tyr Lys Ser His Ala Lys Glu Ile Leu His Cys Leu Lys Asn
                85                  90                  95

Lys Tyr Phe Lys Glu Leu Glu Asp Leu Glu Val Asp Gly Gln Lys Val
            100                 105                 110

Glu Val Pro Gln Pro Leu Ser Trp Tyr Pro Glu Glu Leu Ala Trp His
        115                 120                 125

Thr Asn Leu Ser Arg Lys Ile Leu Arg Lys Ser Pro Leu Leu Ala Lys
    130                 135                 140

Phe His Gln Phe Leu Val Ser Glu Thr Glu Ser Gly Asn Ile Ser Arg
145                 150                 155                 160

Gln Glu Ala Val Ser Met Ile Pro Pro Leu Leu Leu Asn Val Glu Pro
                165                 170                 175

His His Lys Ile Leu Asp Met Cys Ala Ala Pro Gly Ser Lys Thr Thr
            180                 185                 190

Gln Leu Ile Glu Met Leu His Ala Asp Met Ser Val Pro Phe Pro Glu
        195                 200                 205

Gly Phe Val Ile Ala Asn Asp Val Asp Asn Lys Arg Cys Tyr Leu Leu
    210                 215                 220

Val His Gln Ala Lys Arg Leu Ser Ser Pro Cys Ile Met Val Val Asn
225                 230                 235                 240

His Asp Ala Ser Ser Ile Pro Arg Leu Thr Val Asp Val Asp Gly Arg
                245                 250                 255

Lys Glu Ile Leu Phe Tyr Asp Arg Ile Leu Cys Asp Val Pro Cys Ser
            260                 265                 270

Gly Asp Gly Thr Met Arg Lys Asn Ile Asp Val Trp Lys Lys Trp Thr
        275                 280                 285

Thr Leu Asn Ser Leu Gln Leu His Gly Leu Gln Leu Arg Ile Ala Thr
    290                 295                 300

Arg Gly Ala Glu Gln Leu Ala Glu Gly Gly Arg Met Val Tyr Ser Thr
305                 310                 315                 320

Cys Ser Leu Asn Pro Val Glu Asp Glu Ala Val Ile Ala Ala Leu Leu
                325                 330                 335

Glu Lys Ser Glu Gly Ala Leu Glu Leu Ala Asp Val Ser Ala Glu Leu

            340                 345                 350

Pro Gly Leu Lys Trp Met Pro Gly Val Ser Gln Trp Lys Val Met Thr
        355                 360                 365

Arg Asp Gly Gln Trp Phe Ala Asp Trp His Glu Val Pro Gln Gly Arg
    370                 375                 380

His Thr Gln Ile Arg Pro Thr Met Phe Pro Pro Thr Asp Leu Glu Lys
385                 390                 395                 400

Leu Gln Ala Met His Leu Glu Arg Cys Leu Arg Ile Leu Pro His His
                405                 410                 415

Gln Asn Thr Gly Gly Phe Phe Val Ala Val Leu Val Lys Lys Ala Pro
            420                 425                 430

Met Pro Trp Asn Lys Arg Gln Pro Lys Val Gln Asn Lys Ser Ala Glu
        435                 440                 445

Ala Arg Glu Pro Arg Val Ser Ser His Val Ala Ala Thr Glu Gly Asn
    450                 455                 460

Pro Ser Asp Gln Ser Glu Leu Glu Ser Gln Met Ile Thr Gly Ala Gly
465                 470                 475                 480

Asp Leu Glu Thr Ala His Asn Thr Glu Asn Thr Glu Ser Asn Glu Lys
                485                 490                 495

Lys Asp Gly Val Cys Gly Pro Pro Pro Ser Lys Lys Met Lys Leu Phe
            500                 505                 510

Gly Phe Lys Glu Asp Pro Phe Val Phe Ile Pro Glu Asp Asp Pro Leu
        515                 520                 525

Phe Pro Pro Ile Glu Lys Phe Tyr Ala Leu Asp Pro Ser Phe Pro Arg
    530                 535                 540

Met Asn Leu Leu Thr Arg Thr Thr Glu Gly Lys Lys Arg Gln Leu Tyr
545                 550                 555                 560

Met Val Ser Lys Glu Leu Arg Asn Val Leu Leu Asn Asn Ser Glu Lys
                565                 570                 575

Met Lys Val Ile Asn Thr Gly Ile Lys Val Trp Cys Arg Asn Asn Ser
            580                 585                 590

Gly Glu Glu Phe Asp Cys Ala Phe Arg Leu Ala Gln Glu Gly Ile Tyr
        595                 600                 605

Thr Leu Tyr Pro Phe Ile Asn Ser Arg Ile Ile Thr Val Ser Met Glu
    610                 615                 620

Asp Val Lys Thr Leu Leu Thr Gln Glu Asn Pro Phe Phe Arg Lys Leu
625                 630                 635                 640

Ser Ser Glu Ala Tyr Ser Gln Val Lys Asp Leu Ala Lys Gly Ser Val
                645                 650                 655

Val Leu Lys Tyr Glu Pro Asp Ser Ala Asn Pro Asp Thr Leu Gln Cys
            660                 665                 670

Pro Ile Val Leu Cys Gly Trp Arg Gly Lys Ala Ser Ile Arg Thr Phe
        675                 680                 685

Val Pro Lys Asn Glu Arg Leu His Tyr Leu Arg Met Met Gly Leu Glu
    690                 695                 700

Val Leu Gly Glu Lys Lys Lys Glu Gly Val Ile Leu Thr Asn Glu Asn
705                 710                 715                 720

Ala Ala Ser Pro Glu Gln Pro Gly Asp Glu Asp Ala Lys Gln Thr Ala
                725                 730                 735

Gln Asp Pro Cys Val Pro Asp Ser Val Pro Gly Cys Asp Ala Ala Ala
            740                 745                 750

Ala Glu Pro Ser Arg
        755

<210>  11
<211>  1695
<212>  DNA
<213>  Mouse

<220>
<221>  CDS
<222>  (14)..(1234)
<223>

<400>  11
acttaaggct gcc atg ggg ccc agt gct cct ctg ctg ctc ctc ttc ttt        49
               Met Gly Pro Ser Ala Pro Leu Leu Leu Leu Phe Phe
               1               5                   10

ttg tca tgg acg gga ccc ctt cag gga cag cag cac cac ctt gtg gag        97
Leu Ser Trp Thr Gly Pro Leu Gln Gly Gln Gln His His Leu Val Glu
        15                  20                  25

tac atg gaa cgc cga cta gct gcc tta gag gaa cgg ctg gcc caa tgc       145
Tyr Met Glu Arg Arg Leu Ala Ala Leu Glu Glu Arg Leu Ala Gln Cys
    30                  35                  40

cag gat cag agt agt cgg cat gct gcc gag ctt cgg gac ttc aaa aac       193
Gln Asp Gln Ser Ser Arg His Ala Ala Glu Leu Arg Asp Phe Lys Asn
45                  50                  55                  60

aag atg ttg cct ctc ctg gag gtg gca gag aag gag cgg gag acc ctc       241

Lys Met Leu Pro Leu Leu Glu Val Ala Glu Lys Glu Arg Glu Thr Leu
                65                  70                  75

aga act gaa gca gac tcc atc tca gga aga gtg gac cgt ctt gaa agg 289
Arg Thr Glu Ala Asp Ser Ile Ser Gly Arg Val Asp Arg Leu Glu Arg
            80                  85                  90

gag gta gac tat ctg gag aca cag aac cca gct ttg ccc tgt gta gag 337
Glu Val Asp Tyr Leu Glu Thr Gln Asn Pro Ala Leu Pro Cys Val Glu
        95                  100                 105

ctg gat gag aag gtg act gga ggt cct gga gcc aaa ggc aag ggc cga 385
Leu Asp Glu Lys Val Thr Gly Gly Pro Gly Ala Lys Gly Lys Gly Arg
    110                 115                 120

aga aat gag aaa tac gat atg gtg acg gac tgt agc tac aca gtc gct 433
Arg Asn Glu Lys Tyr Asp Met Val Thr Asp Cys Ser Tyr Thr Val Ala
125                 130                 135                 140

cag gtg agg tca atg aag atc ctg aag cgg ttt ggt ggt tca gct ggc 481
Gln Val Arg Ser Met Lys Ile Leu Lys Arg Phe Gly Gly Ser Ala Gly
                145                 150                 155

cta tgg acc aag gat ccg ctg ggg cca gca gag aag atc tac gtg tta 529
Leu Trp Thr Lys Asp Pro Leu Gly Pro Ala Glu Lys Ile Tyr Val Leu
            160                 165                 170

gac ggc acc cag aac gac acg gct ttt gtc ttc cca agg ctg cgt gac 577
Asp Gly Thr Gln Asn Asp Thr Ala Phe Val Phe Pro Arg Leu Arg Asp
        175                 180                 185

ttc acc ctt gcc atg gct gcc cgg aaa gct tcc cga att cgg gtg ccc 625
Phe Thr Leu Ala Met Ala Ala Arg Lys Ala Ser Arg Ile Arg Val Pro
    190                 195                 200

ttc ccc tgg gta ggc acg ggg cag ctg gtg tac ggt ggc ttc ctt tat 673
Phe Pro Trp Val Gly Thr Gly Gln Leu Val Tyr Gly Gly Phe Leu Tyr
205                 210                 215                 220

tat gct cga agg cct cct gga gga cct gga ggg ggt ggt gaa ttg gag 721
Tyr Ala Arg Arg Pro Pro Gly Gly Pro Gly Gly Gly Gly Glu Leu Glu
                225                 230                 235

aac act ctg cag ctg atc aaa ttt cac ttg gca aac cga aca gtg gtg 769
Asn Thr Leu Gln Leu Ile Lys Phe His Leu Ala Asn Arg Thr Val Val

240                 245                 250

gat agc tca gtg ttc cct gca gag agc ctg ata ccc ccc tac ggc ctg      817
Asp Ser Ser Val Phe Pro Ala Glu Ser Leu Ile Pro Pro Tyr Gly Leu
        255                 260                 265

aca gca gat aca tat atc gac ctg gca gct gat gag gag ggc ctg tgg      865
Thr Ala Asp Thr Tyr Ile Asp Leu Ala Ala Asp Glu Glu Gly Leu Trp
    270                 275                 280

gct gtc tat gcc act cga gat gat gac agg cat ttg tgt cta gcc aag      913
Ala Val Tyr Ala Thr Arg Asp Asp Asp Arg His Leu Cys Leu Ala Lys
285                 290                 295                 300

tta gac cca cag aca ctt gac aca gag cag cag tgg gac aca cca tgt      961
Leu Asp Pro Gln Thr Leu Asp Thr Glu Gln Gln Trp Asp Thr Pro Cys
                305                 310                 315

ccc aga gag aac gca gag gct gcg ttt gtc atc tgt ggg acc ctg tac     1009
Pro Arg Glu Asn Ala Glu Ala Ala Phe Val Ile Cys Gly Thr Leu Tyr
            320                 325                 330

gtt gtc tat aac acc cgc cct gcc agt agg gct cgt att cag tgt tcc     1057
Val Val Tyr Asn Thr Arg Pro Ala Ser Arg Ala Arg Ile Gln Cys Ser
        335                 340                 345

ttc gat gcc agt ggt act ctc gcc cct gaa agg gca gca ctc tcc tat     1105
Phe Asp Ala Ser Gly Thr Leu Ala Pro Glu Arg Ala Ala Leu Ser Tyr
    350                 355                 360

ttt cca cgc cga tat ggt gcc cat gcc agc ctt cgc tat aac ccc cgt     1153
Phe Pro Arg Arg Tyr Gly Ala His Ala Ser Leu Arg Tyr Asn Pro Arg
365                 370                 375                 380

gag cgc cag ctg tat gcc tgg gat gat ggc tat cag att gtc tac aaa     1201
Glu Arg Gln Leu Tyr Ala Trp Asp Asp Gly Tyr Gln Ile Val Tyr Lys
                385                 390                 395

ttg gag atg aag aag aag gag gag gaa gtt taa gcagctagcc ttgtgctctt     1254
Leu Glu Met Lys Lys Lys Glu Glu Glu Val
            400                 405

gattcttatg cccagacatt tatattcctg tgagctctcc tgcagttcat ccttcaaaac     1314

gaaggccagt ggtggtagct catataccct aatttctaaa ggacaaccaa attctcaagc     1374

ccctctgttt tatgcagaac tccagatcct gggtagcatt ttagaactga acagcaaaca 1434

aacaccctaa atcttcactc ctgccctatg tccacaaagt ttagttccaa actcagagcc 1494

ctgtcctttg gagagggtca accccagaca gcaggcgaca gcattcttgc cctcagtatg 1554

accgaaggga gagaactcag agacaaagct gccctccctc ccttccccct ccagtgtagg 1614

ggagaatggg gctttcccca catcactttg tatggtaaca gtttgcatta aaaggaaaac 1674

ccacaaaaaa aaaaaaaaaa a 1695

<210> 12
<211> 406
<212> PRT
<213> Mouse

<400> 12

Met Gly Pro Ser Ala Pro Leu Leu Leu Leu Phe Phe Leu Ser Trp Thr
1               5                   10                  15

Gly Pro Leu Gln Gly Gln Gln His His Leu Val Glu Tyr Met Glu Arg
            20                  25                  30

Arg Leu Ala Ala Leu Glu Glu Arg Leu Ala Gln Cys Gln Asp Gln Ser
        35                  40                  45

Ser Arg His Ala Ala Glu Leu Arg Asp Phe Lys Asn Lys Met Leu Pro
    50                  55                  60

Leu Leu Glu Val Ala Glu Lys Glu Arg Glu Thr Leu Arg Thr Glu Ala
65                  70                  75                  80

Asp Ser Ile Ser Gly Arg Val Asp Arg Leu Glu Arg Glu Val Asp Tyr
                85                  90                  95

Leu Glu Thr Gln Asn Pro Ala Leu Pro Cys Val Glu Leu Asp Glu Lys
            100                 105                 110

Val Thr Gly Gly Pro Gly Ala Lys Gly Lys Gly Arg Arg Asn Glu Lys
        115                 120                 125

Tyr Asp Met Val Thr Asp Cys Ser Tyr Thr Val Ala Gln Val Arg Ser
    130                 135                 140

Met Lys Ile Leu Lys Arg Phe Gly Gly Ser Ala Gly Leu Trp Thr Lys
145                 150                 155                 160

Asp Pro Leu Gly Pro Ala Glu Lys Ile Tyr Val Leu Asp Gly Thr Gln
                165                 170                 175

Asn Asp Thr Ala Phe Val Phe Pro Arg Leu Arg Asp Phe Thr Leu Ala
            180                 185                 190

Met Ala Ala Arg Lys Ala Ser Arg Ile Arg Val Pro Phe Pro Trp Val
        195                 200                 205

Gly Thr Gly Gln Leu Val Tyr Gly Gly Phe Leu Tyr Tyr Ala Arg Arg
    210                 215                 220

Pro Pro Gly Gly Pro Gly Gly Gly Gly Glu Leu Glu Asn Thr Leu Gln
225                 230                 235                 240

Leu Ile Lys Phe His Leu Ala Asn Arg Thr Val Val Asp Ser Ser Val
                245                 250                 255

Phe Pro Ala Glu Ser Leu Ile Pro Pro Tyr Gly Leu Thr Ala Asp Thr
            260                 265                 270

Tyr Ile Asp Leu Ala Ala Asp Glu Glu Gly Leu Trp Ala Val Tyr Ala

275                 280                 285

Thr Arg Asp Asp Asp Arg His Leu Cys Leu Ala Lys Leu Asp Pro Gln
    290                 295                 300

Thr Leu Asp Thr Glu Gln Gln Trp Asp Thr Pro Cys Pro Arg Glu Asn
305                 310                 315                 320

Ala Glu Ala Ala Phe Val Ile Cys Gly Thr Leu Tyr Val Val Tyr Asn
                325                 330                 335

Thr Arg Pro Ala Ser Arg Ala Arg Ile Gln Cys Ser Phe Asp Ala Ser
            340                 345                 350

Gly Thr Leu Ala Pro Glu Arg Ala Ala Leu Ser Tyr Phe Pro Arg Arg
        355                 360                 365

Tyr Gly Ala His Ala Ser Leu Arg Tyr Asn Pro Arg Glu Arg Gln Leu
    370                 375                 380

Tyr Ala Trp Asp Asp Gly Tyr Gln Ile Val Tyr Lys Leu Glu Met Lys
385                 390                 395                 400

Lys Lys Glu Glu Glu Val
                405

<210>  13
<211>  1610
<212>  DNA
<213>  Mouse

<220>
<221>  CDS
<222>  (48)..(1193)
<223>

<400> 13
   gctgggtact aggagaagcc atcatgcaca cctctactga agccagg atg ggc atg 56
   Met Gly Met
   1
   agg gcg gca ctg aca ggc ttt gcg gtc ctg atg ctg ctc cag agc tgc 104
   Arg Ala Ala Leu Thr Gly Phe Ala Val Leu Met Leu Leu Gln Ser Cys
   5 10 15
   tct gcg tac aag ctg gtc tgc tac ttc acc agc tgg tcc cag tac cgg 152
   Ser Ala Tyr Lys Leu Val Cys Tyr Phe Thr Ser Trp Ser Gln Tyr Arg
   20 25 30 35
   gaa ggc gtt gga agc ttc tta cca gac gcc atc caa cct ttc ctg tgc 200
   Glu Gly Val Gly Ser Phe Leu Pro Asp Ala Ile Gln Pro Phe Leu Cys
   40 45 50
   acc cac atc atc tac agc ttt gcc aac atc agc agc gac aac atg ctt 248
   Thr His Ile Ile Tyr Ser Phe Ala Asn Ile Ser Ser Asp Asn Met Leu
   55 60 65
   agc aca tgg gag tgg aat gac gag tcg aac tat gac aag ctg aat aaa 296
   Ser Thr Trp Glu Trp Asn Asp Glu Ser Asn Tyr Asp Lys Leu Asn Lys
   70 75 80
   ctg aag acc aga aac acc aac ctg aag acc ctc ctg tct gtt gga ggg 344
   Leu Lys Thr Arg Asn Thr Asn Leu Lys Thr Leu Leu Ser Val Gly Gly
   85 90 95
   tgg aaa ttt ggc gaa aaa aga ttt tcc gag att gcc tcc aac act gag 392
   Trp Lys Phe Gly Glu Lys Arg Phe Ser Glu Ile Ala Ser Asn Thr Glu
   100 105 110 115
   aga cgc act gct ttc gtc cgg tcg gta gcc ccg ttc ctg cgt tct tat 440
   Arg Arg Thr Ala Phe Val Arg Ser Val Ala Pro Phe Leu Arg Ser Tyr
   120 125 130
   ggc ttt gat ggg ctg gat ctc gcc tgg ctc tac cct cgc tta aga gac 488
   Gly Phe Asp Gly Leu Asp Leu Ala Trp Leu Tyr Pro Arg Leu Arg Asp
   135 140 145
   aag cag tat ttc tcc acc ctg atc aag gaa ctg aat gcg gaa ttc aca 536
   Lys Gln Tyr Phe Ser Thr Leu Ile Lys Glu Leu Asn Ala Glu Phe Thr
   150 155 160
   aag gag gtc cag cca ggc aga gag aaa ctc ctg ctc agc gca gct ttg 584
   Lys Glu Val Gln Pro Gly Arg Glu Lys Leu Leu Leu Ser Ala Ala Leu
   165 170 175
   tca gca gga aag gtg gcc att gac act ggc tat gac atc gcc cag ata 632
   Ser Ala Gly Lys Val Ala Ile Asp Thr Gly Tyr Asp Ile Ala Gln Ile
   180 185 190 195
   gcc caa cac ctg gat ttt atc aat ctc atg acc tac gat ttc cat gga 680
   Ala Gln His Leu Asp Phe Ile Asn Leu Met Thr Tyr Asp Phe His Gly
   200 205 210
   gtc tgg cgc caa atc aca ggc cat cac agc ccc ctc ttc caa ggc cag 728
   Val Trp Arg Gln Ile Thr Gly His His Ser Pro Leu Phe Gln Gly Gln
   215 220 225
   aag gac act agg ttt gac aga tac agc aat gtg aac tat gcc gtg cag 776
   Lys Asp Thr Arg Phe Asp Arg Tyr Ser Asn Val Asn Tyr Ala Val Gln
   230 235 240
   tac atg ata cgt ctg gga gcc cag gcc agc aag cta ctg atg ggc atc 824
   Tyr Met Ile Arg Leu Gly Ala Gln Ala Ser Lys Leu Leu Met Gly Ile
   245 250 255
   ccc acc ttt ggg aag agc ttc act ctg gca tct tct gaa aat cag ttg 872
   Pro Thr Phe Gly Lys Ser Phe Thr Leu Ala Ser Ser Glu Asn Gln Leu
   260 265 270 275
   gga gct cca atc tca ggg gaa gga tta cca ggc cgg ttc acc aag gag 920
   Gly Ala Pro Ile Ser Gly Glu Gly Leu Pro Gly Arg Phe Thr Lys Glu
   280 285 290
   gca ggg acc ctg gcc tac tac gag ata tgc gac ttc ctc aaa gga gct 968
   Ala Gly Thr Leu Ala Tyr Tyr Glu Ile Cys Asp Phe Leu Lys Gly Ala
   295 300 305
   gaa gta cat cga ctc tcc aac gag aag gtt ccc ttc gct acc aag ggc 1016
   Glu Val His Arg Leu Ser Asn Glu Lys Val Pro Phe Ala Thr Lys Gly
   310 315 320
   aac cag tgg gtg ggg tat gag gac aag gag agt gtc aaa aac aag gtt 1064
   Asn Gln Trp Val Gly Tyr Glu Asp Lys Glu Ser Val Lys Asn Lys Val
   325 330 335
   ggg ttc ctg aag gag aag aag ctg gca gga gcc atg gtg tgg gca ctg 1112
   Gly Phe Leu Lys Glu Lys Lys Leu Ala Gly Ala Met Val Trp Ala Leu
   340                 345                 350                 355
   gat ttg gat gat ttc cag ggc acc tgt cag ccg aag gaa ttc ttc ccg      1160
   Asp Leu Asp Asp Phe Gln Gly Thr Cys Gln Pro Lys Glu Phe Phe Pro
                   360                 365                 370
   ctc acc aac gcc atc aag gat gcc ctg gct tag ctcccctttt cccatatggt    1213
   Leu Thr Asn Ala Ile Lys Asp Ala Leu Ala
               375                 380
   acccccactc tctggccagg agtttaatct cttgcaatgt taagtccccc aactgagcct      1273
   cagtttctcc ttcccttggc acctgtgtaa ggggccacag caggctcagc tatggagaac      1333
   agggaactag ggtaggacga tggtggggtt gtgagagtca cagtgtgagc agatacacaa      1393
   ccctgttaag gaatgcaaat tctcagactc taacctccct ttacccagcc tgaccaaagg      1453
   acaccacttg gatcaagtag gcaaatatct tacaggattg agggaccata ctaattatac      1513
   cctctgcaaa gcccaacttg aatccttccc ttaggaactt aatcgtccca cttccctttc      1573
   cctaattcca cagctgttca ataaagcgcc agaacct                              1610

<210>  14
<211>  381
<212>  PRT
<213>  Mouse

<400>  14

Met Gly Met Arg Ala Ala Leu Thr Gly Phe Ala Val Leu Met Leu Leu
1               5                   10                  15

Gln Ser Cys Ser Ala Tyr Lys Leu Val Cys Tyr Phe Thr Ser Trp Ser
            20                  25                  30

Gln Tyr Arg Glu Gly Val Gly Ser Phe Leu Pro Asp Ala Ile Gln Pro
        35                  40                  45

Phe Leu Cys Thr His Ile Ile Tyr Ser Phe Ala Asn Ile Ser Ser Asp
    50                  55                  60

Asn Met Leu Ser Thr Trp Glu Trp Asn Asp Glu Ser Asn Tyr Asp Lys
65                  70                  75                  80

Leu Asn Lys Leu Lys Thr Arg Asn Thr Asn Leu Lys Thr Leu Leu Ser
                85                  90                  95

Val Gly Gly Trp Lys Phe Gly Glu Lys Arg Phe Ser Glu Ile Ala Ser
            100                 105                 110

Asn Thr Glu Arg Arg Thr Ala Phe Val Arg Ser Val Ala Pro Phe Leu
        115                 120                 125

Arg Ser Tyr Gly Phe Asp Gly Leu Asp Leu Ala Trp Leu Tyr Pro Arg
    130                 135                 140

Leu Arg Asp Lys Gln Tyr Phe Ser Thr Leu Ile Lys Glu Leu Asn Ala
145                 150                 155                 160

Glu Phe Thr Lys Glu Val Gln Pro Gly Arg Glu Lys Leu Leu Leu Ser
                165                 170                 175

Ala Ala Leu Ser Ala Gly Lys Val Ala Ile Asp Thr Gly Tyr Asp Ile
            180                 185                 190

Ala Gln Ile Ala Gln His Leu Asp Phe Ile Asn Leu Met Thr Tyr Asp
        195                 200                 205

Phe His Gly Val Trp Arg Gln Ile Thr Gly His His Ser Pro Leu Phe
    210                 215                 220

Gln Gly Gln Lys Asp Thr Arg Phe Asp Arg Tyr Ser Asn Val Asn Tyr

225                 230                 235                 240

Ala Val Gln Tyr Met Ile Arg Leu Gly Ala Gln Ala Ser Lys Leu Leu
                245                 250                 255

Met Gly Ile Pro Thr Phe Gly Lys Ser Phe Thr Leu Ala Ser Ser Glu
            260                 265                 270

Asn Gln Leu Gly Ala Pro Ile Ser Gly Glu Gly Leu Pro Gly Arg Phe
        275                 280                 285

Thr Lys Glu Ala Gly Thr Leu Ala Tyr Tyr Glu Ile Cys Asp Phe Leu
    290                 295                 300

Lys Gly Ala Glu Val His Arg Leu Ser Asn Glu Lys Val Pro Phe Ala
305                 310                 315                 320

Thr Lys Gly Asn Gln Trp Val Gly Tyr Glu Asp Lys Glu Ser Val Lys
                325                 330                 335

Asn Lys Val Gly Phe Leu Lys Glu Lys Lys Leu Ala Gly Ala Met Val
            340                 345                 350

Trp Ala Leu Asp Leu Asp Asp Phe Gln Gly Thr Cys Gln Pro Lys Glu
        355                 360                 365

Phe Phe Pro Leu Thr Asn Ala Ile Lys Asp Ala Leu Ala
    370                 375                 380

<210>  15
<211>  2002
<212>  DNA
<213>  Mouse

<220>

<221>  CDS
<222>  (178)..(1689)
<223>

<400>  15
gaattccgga agggggctct gacccgttcc gagcgccaac gcagcctctg tagcccgcaa      60

gtcttcgtcg cttgctccgg gctctgaagt ccggggccac caggggccgc agcgctgggg     120

ggtcggtcta gctgcgagga tccgggctgc ccacgaagcg aagggcgggc gcccagg        177

atg gga tgc gtg aag tcc agg ttc ctc cga gat gga agc aag gcc tca       225
Met Gly Cys Val Lys Ser Arg Phe Leu Arg Asp Gly Ser Lys Ala Ser
1               5                   10                  15

aaa aca gag cca agt gcc aat cag aag ggc cct gtg tat gtg ccg gat       273
Lys Thr Glu Pro Ser Ala Asn Gln Lys Gly Pro Val Tyr Val Pro Asp
            20                  25                  30

ccc acg tcc tcc agc aag ctg gga cca aac aac agc aac agc atg ccc       321
Pro Thr Ser Ser Ser Lys Leu Gly Pro Asn Asn Ser Asn Ser Met Pro
        35                  40                  45

cca ggg ttt gtg gag ggc tct gag gat acc att gtg gtc gca ctg tac       369
Pro Gly Phe Val Glu Gly Ser Glu Asp Thr Ile Val Val Ala Leu Tyr
    50                  55                  60

gac tat gag gct att cac cgt gaa gac ctc agc ttc cag aag gga gac       417
Asp Tyr Glu Ala Ile His Arg Glu Asp Leu Ser Phe Gln Lys Gly Asp
65                  70                  75                  80

cag atg gtg gtt ctg gag gag gct ggg gag tgg tgg aag gca cgg tcc       465
Gln Met Val Val Leu Glu Glu Ala Gly Glu Trp Trp Lys Ala Arg Ser
                85                  90                  95

ctg gct acc aag aag gaa ggc tac atc cca agc aac tat gtg gct cga       513
Leu Ala Thr Lys Lys Glu Gly Tyr Ile Pro Ser Asn Tyr Val Ala Arg
            100                 105                 110

gtt aac tct ttg gag aca gaa gag tgg ttc ttc aag ggg atc agc cgg       561
Val Asn Ser Leu Glu Thr Glu Glu Trp Phe Phe Lys Gly Ile Ser Arg
        115                 120                 125

aag gat gca gag cgc cac ctc ctg gct cca ggc aac atg ctg ggc tcc       609
Lys Asp Ala Glu Arg His Leu Leu Ala Pro Gly Asn Met Leu Gly Ser

130                 135                 140

ttc atg atc cgg gac agt gag acc acc aaa ggg agc tac tcg ttg tct     657
Phe Met Ile Arg Asp Ser Glu Thr Thr Lys Gly Ser Tyr Ser Leu Ser
145                 150                 155                 160

gtt cga gac ttt gac ccc cag cac gga gac acc gtg aag cac tat aag     705
Val Arg Asp Phe Asp Pro Gln His Gly Asp Thr Val Lys His Tyr Lys
                165                 170                 175

atc cgg acg ctg gac agt gga ggc ttc tac atc tct cca agg agc acc     753
Ile Arg Thr Leu Asp Ser Gly Gly Phe Tyr Ile Ser Pro Arg Ser Thr
            180                 185                 190

ttc agc agc ctg cag gaa ctc gtg ctc cac tac aag aag ggg aag gat     801
Phe Ser Ser Leu Gln Glu Leu Val Leu His Tyr Lys Lys Gly Lys Asp
        195                 200                 205

ggg ctc tgc cag aag ctg tca gtg ccc tgt gtg tct ccc aaa ccc cag     849
Gly Leu Cys Gln Lys Leu Ser Val Pro Cys Val Ser Pro Lys Pro Gln
    210                 215                 220

aag cca tgg gag aaa gat gct tgg gag att cct cga gaa tcc ctc cag     897
Lys Pro Trp Glu Lys Asp Ala Trp Glu Ile Pro Arg Glu Ser Leu Gln
225                 230                 235                 240

atg gag aag aaa ctt gga gct ggg cag ttt gga gaa gtg tgg atg gcc     945
Met Glu Lys Lys Leu Gly Ala Gly Gln Phe Gly Glu Val Trp Met Ala
                245                 250                 255

acc tac aac aag cac acc aaa gtg gcg gtg aag aca atg aag cca ggg     993
Thr Tyr Asn Lys His Thr Lys Val Ala Val Lys Thr Met Lys Pro Gly
            260                 265                 270

agc atg tcc gtg gag gcc ttc ctg gct gag gcc aac ctg atg aag tcg     1041
Ser Met Ser Val Glu Ala Phe Leu Ala Glu Ala Asn Leu Met Lys Ser
        275                 280                 285

ctg cag cat gac aaa ctg gtg aag cta cac gct gtg gtc tct cag gag     1089
Leu Gln His Asp Lys Leu Val Lys Leu His Ala Val Val Ser Gln Glu
    290                 295                 300

ccc atc ttt att gtc acg gag ttc atg gcc aaa gga agc ctg ctg gac     1137
Pro Ile Phe Ile Val Thr Glu Phe Met Ala Lys Gly Ser Leu Leu Asp
305                 310                 315                 320

ttt ctc aag agt gaa gaa ggc agc aag cag cca ctg cca aaa ctc att 1185
Phe Leu Lys Ser Glu Glu Gly Ser Lys Gln Pro Leu Pro Lys Leu Ile
                325                 330                 335

gac ttc tca gcc cag atc tca gaa ggc atg gcc ttc att gag cag agg 1233
Asp Phe Ser Ala Gln Ile Ser Glu Gly Met Ala Phe Ile Glu Gln Arg
            340                 345                 350

aac tac atc cac cga gac ctg agg gct gcc aac atc tta gtc tct gca 1281
Asn Tyr Ile His Arg Asp Leu Arg Ala Ala Asn Ile Leu Val Ser Ala
        355                 360                 365

tca ctg gtg tgt aag att gct gac ttt gga ctg gca cga atc atc gag 1329
Ser Leu Val Cys Lys Ile Ala Asp Phe Gly Leu Ala Arg Ile Ile Glu
    370                 375                 380

gac aat gag tac aca gct cgg gaa gga gcc aag ttc ccc atc aag tgg 1377
Asp Asn Glu Tyr Thr Ala Arg Glu Gly Ala Lys Phe Pro Ile Lys Trp
385                 390                 395                 400

aca gct cct gaa gcc atc aac ttt ggt tcc ttc acc atc aag tca gat 1425
Thr Ala Pro Glu Ala Ile Asn Phe Gly Ser Phe Thr Ile Lys Ser Asp
                405                 410                 415

gtc tgg tcc ttt ggt atc ctg ctg atg gaa att gtc acc tat ggc cgg 1473
Val Trp Ser Phe Gly Ile Leu Leu Met Glu Ile Val Thr Tyr Gly Arg
            420                 425                 430

atc cct tac cca ggt atg tca aac cca gag gtg att cgg gca cta gag 1521
Ile Pro Tyr Pro Gly Met Ser Asn Pro Glu Val Ile Arg Ala Leu Glu
        435                 440                 445

cat ggg tac cgt atg cct cga cca gat aac tgt cca gaa gag ctc tac 1569
His Gly Tyr Arg Met Pro Arg Pro Asp Asn Cys Pro Glu Glu Leu Tyr
    450                 455                 460

aat atc atg atc cgc tgc tgg aag aac cgc ccc gag gaa cgg ccc acc 1617
Asn Ile Met Ile Arg Cys Trp Lys Asn Arg Pro Glu Glu Arg Pro Thr
465                 470                 475                 480

ttt gaa tac atc cag agt gtg ctg gat gac ttc tac acg gcc act gag 1665
Phe Glu Tyr Ile Gln Ser Val Leu Asp Asp Phe Tyr Thr Ala Thr Glu
                485                 490                 495

agc cag tat cag cag cag cct tga cagcagtaag gacatgagca gagccagaag     1719
Ser Gln Tyr Gln Gln Gln Pro
            500

ccccatcagt gccttgacac gcccaacttg ctgggcccac tctcagacac cacaccacac     1779

acactgcagc tgttgagtgg gtgggaggac ttcacaatct ctttctgact ctagtcatct     1839

gcaatccgcc actctcaggg cctccaagtt ggtatgtctc atttgcctgg aatgactgaa     1899

ttcaatctat agctgtgatt taagtggaaa ctgttagaat agtatttaaa taaaagatat     1959

gaatgtcaaa aaaaaaaaaa aaaaaaaaaa aaaaaaaaaa aaa                       2002

<210>  16
<211>  503
<212>  PRT
<213>  Mouse

<400>  16

Met Gly Cys Val Lys Ser Arg Phe Leu Arg Asp Gly Ser Lys Ala Ser
1               5                   10                  15

Lys Thr Glu Pro Ser Ala Asn Gln Lys Gly Pro Val Tyr Val Pro Asp
            20                  25                  30

Pro Thr Ser Ser Ser Lys Leu Gly Pro Asn Asn Ser Asn Ser Met Pro
        35                  40                  45

Pro Gly Phe Val Glu Gly Ser Glu Asp Thr Ile Val Val Ala Leu Tyr
    50                  55                  60

Asp Tyr Glu Ala Ile His Arg Glu Asp Leu Ser Phe Gln Lys Gly Asp
65                  70                  75                  80

Gln Met Val Val Leu Glu Glu Ala Gly Glu Trp Trp Lys Ala Arg Ser
                85                  90                  95

Leu Ala Thr Lys Lys Glu Gly Tyr Ile Pro Ser Asn Tyr Val Ala Arg
            100                 105                 110

Val Asn Ser Leu Glu Thr Glu Glu Trp Phe Phe Lys Gly Ile Ser Arg
        115                 120                 125

Lys Asp Ala Glu Arg His Leu Leu Ala Pro Gly Asn Met Leu Gly Ser
    130                 135                 140

Phe Met Ile Arg Asp Ser Glu Thr Thr Lys Gly Ser Tyr Ser Leu Ser
145                 150                 155                 160

Val Arg Asp Phe Asp Pro Gln His Gly Asp Thr Val Lys His Tyr Lys
                165                 170                 175

Ile Arg Thr Leu Asp Ser Gly Gly Phe Tyr Ile Ser Pro Arg Ser Thr
            180                 185                 190

Phe Ser Ser Leu Gln Glu Leu Val Leu His Tyr Lys Lys Gly Lys Asp
        195                 200                 205

Gly Leu Cys Gln Lys Leu Ser Val Pro Cys Val Ser Pro Lys Pro Gln
    210                 215                 220

Lys Pro Trp Glu Lys Asp Ala Trp Glu Ile Pro Arg Glu Ser Leu Gln
225                 230                 235                 240

Met Glu Lys Lys Leu Gly Ala Gly Gln Phe Gly Glu Val Trp Met Ala
                245                 250                 255

Thr Tyr Asn Lys His Thr Lys Val Ala Val Lys Thr Met Lys Pro Gly
            260                 265                 270

Ser Met Ser Val Glu Ala Phe Leu Ala Glu Ala Asn Leu Met Lys Ser
        275                 280                 285

Leu Gln His Asp Lys Leu Val Lys Leu His Ala Val Val Ser Gln Glu
    290                 295                 300

Pro Ile Phe Ile Val Thr Glu Phe Met Ala Lys Gly Ser Leu Leu Asp
305                 310                 315                 320

Phe Leu Lys Ser Glu Glu Gly Ser Lys Gln Pro Leu Pro Lys Leu Ile
                325                 330                 335

Asp Phe Ser Ala Gln Ile Ser Glu Gly Met Ala Phe Ile Glu Gln Arg
            340                 345                 350

Asn Tyr Ile His Arg Asp Leu Arg Ala Ala Asn Ile Leu Val Ser Ala
        355                 360                 365

Ser Leu Val Cys Lys Ile Ala Asp Phe Gly Leu Ala Arg Ile Ile Glu
    370                 375                 380

Asp Asn Glu Tyr Thr Ala Arg Glu Gly Ala Lys Phe Pro Ile Lys Trp
385                 390                 395                 400

Thr Ala Pro Glu Ala Ile Asn Phe Gly Ser Phe Thr Ile Lys Ser Asp
                405                 410                 415

Val Trp Ser Phe Gly Ile Leu Leu Met Glu Ile Val Thr Tyr Gly Arg
            420                 425                 430

Ile Pro Tyr Pro Gly Met Ser Asn Pro Glu Val Ile Arg Ala Leu Glu
        435                 440                 445

His Gly Tyr Arg Met Pro Arg Pro Asp Asn Cys Pro Glu Glu Leu Tyr

450                 455                 460

Asn Ile Met Ile Arg Cys Trp Lys Asn Arg Pro Glu Glu Arg Pro Thr
465                 470                 475                 480

Phe Glu Tyr Ile Gln Ser Val Leu Asp Asp Phe Tyr Thr Ala Thr Glu
                485                 490                 495

Ser Gln Tyr Gln Gln Gln Pro
            500

<210>  17
<211>  3064
<212>  DNA
<213>  Mouse

<220>
<221>  CDS
<222>  (133)..(2463)
<223>

<400>  17
ctcactttgc ccgggtcccg ggaggccgtc gacttatcct aggagctcgg agcttttctt      60

ctgggcaacc agggcgcttc cagacagagt tcctgctgcc acccaccacc cccctctcag     120

cacaggacaa gg atg gag ctc cat ttt ggc tcc tgc ctc tcc ggc tgt ttg     171
              Met Glu Leu His Phe Gly Ser Cys Leu Ser Gly Cys Leu
              1               5                   10

gct ctg ctt gtc ttg ctg cct tcc ctg agc cta gca cag tac gag ggc       219
Ala Leu Leu Val Leu Leu Pro Ser Leu Ser Leu Ala Gln Tyr Glu Gly
    15                  20                  25

tgg ccc tac cag ctc cag tac cct gag tac ttc cag cag ccc gct cct       267
Trp Pro Tyr Gln Leu Gln Tyr Pro Glu Tyr Phe Gln Gln Pro Ala Pro
30                  35                  40                  45

gag cac cat cag cgg cag gtg ccc tcc gat gtg gtc aag atc cag gtc       315
Glu His His Gln Arg Gln Val Pro Ser Asp Val Val Lys Ile Gln Val
                50                  55                  60

cgc ctg gcg ggc cag aag agg aag cac aat gag ggc cgc gtg gag gtc 363
Arg Leu Ala Gly Gln Lys Arg Lys His Asn Glu Gly Arg Val Glu Val
            65                  70                  75

tac tac gaa ggc cag tgg ggc acg gtg tgc gac gat gac ttc tcg atc 411
Tyr Tyr Glu Gly Gln Trp Gly Thr Val Cys Asp Asp Asp Phe Ser Ile
        80                  85                  90

cat gcc gcc cat gtg gtc tgc cgg caa gtg ggc tat gta gag gcc aag 459
His Ala Ala His Val Val Cys Arg Gln Val Gly Tyr Val Glu Ala Lys
    95                  100                 105

tcc tgg gct gcc agc tcc tcc tac ggt cca ggc gaa ggc ccc atc tgg 507
Ser Trp Ala Ala Ser Ser Ser Tyr Gly Pro Gly Glu Gly Pro Ile Trp
110                 115                 120                 125

ttg gac aat atc tac tgt act ggc aaa gag tcg acc ctg gca tct tgc 555
Leu Asp Asn Ile Tyr Cys Thr Gly Lys Glu Ser Thr Leu Ala Ser Cys
                130                 135                 140

tcc tcc aat ggc tgg ggt gtc act gac tgc aag cac act gaa gac gtt 603
Ser Ser Asn Gly Trp Gly Val Thr Asp Cys Lys His Thr Glu Asp Val
            145                 150                 155

gga gtg gtg tgt agt gag aaa aga att cct ggc ttc aaa ttt gac aat 651
Gly Val Val Cys Ser Glu Lys Arg Ile Pro Gly Phe Lys Phe Asp Asn
        160                 165                 170

tcg ttg atc aac caa ata gag agc cta aat ata cag gtg gaa gac atc 699
Ser Leu Ile Asn Gln Ile Glu Ser Leu Asn Ile Gln Val Glu Asp Ile
    175                 180                 185

cgg att cgg ccc atc ctt tct gcc ttt cgc cat cgc aag cct gtg aca 747
Arg Ile Arg Pro Ile Leu Ser Ala Phe Arg His Arg Lys Pro Val Thr
190                 195                 200                 205

gag ggc tac gtg gag gtg aag gag ggc aag gct tgg aag cag atc tgc 795
Glu Gly Tyr Val Glu Val Lys Glu Gly Lys Ala Trp Lys Gln Ile Cys
                210                 215                 220

aac aaa cac tgg aca gcc aag aat tcc cac gtg gtc tgt ggc atg ttc 843
Asn Lys His Trp Thr Ala Lys Asn Ser His Val Val Cys Gly Met Phe
            225                 230                 235

ggc ttc cct gca gag aag acc tac aac ccc aaa gcc tat aaa acc ttt 891
Gly Phe Pro Ala Glu Lys Thr Tyr Asn Pro Lys Ala Tyr Lys Thr Phe
        240                 245                 250

gcc tcg cgg agg aag ctg cgt tac tgg aag ttt tct atg aac tgc acg 939
Ala Ser Arg Arg Lys Leu Arg Tyr Trp Lys Phe Ser Met Asn Cys Thr
    255                 260                 265

ggc act gaa gcg cat atc tcc agc tgc aag ctg ggc cct tcc gtg acc 987
Gly Thr Glu Ala His Ile Ser Ser Cys Lys Leu Gly Pro Ser Val Thr
270                 275                 280                 285

cgg gac cct gtg aag aac gcc acc tgt gag aac ggg cag cca gct gtg 1035
Arg Asp Pro Val Lys Asn Ala Thr Cys Glu Asn Gly Gln Pro Ala Val
                290                 295                 300

gtc agt tgt gtg cct agc cag atc ttc agc ccc gat gga ccc tca agg 1083
Val Ser Cys Val Pro Ser Gln Ile Phe Ser Pro Asp Gly Pro Ser Arg
            305                 310                 315

ttc cgg aaa gcc tac aag cca gag caa ccc ttg gtg cgc ctg aga ggt 1131
Phe Arg Lys Ala Tyr Lys Pro Glu Gln Pro Leu Val Arg Leu Arg Gly
        320                 325                 330

gga gcc cag gtc ggg gag ggc cga gtg gag gtg ctg aag aat gga gaa 1179
Gly Ala Gln Val Gly Glu Gly Arg Val Glu Val Leu Lys Asn Gly Glu
    335                 340                 345

tgg gga acc atc tgc gat gac aag tgg gac ctg gta tct gcc agt gtg 1227
Trp Gly Thr Ile Cys Asp Asp Lys Trp Asp Leu Val Ser Ala Ser Val
350                 355                 360                 365

gtc tgc cga gag ctg ggc ttt ggg acc gct aaa gag gcc atc aca ggc 1275
Val Cys Arg Glu Leu Gly Phe Gly Thr Ala Lys Glu Ala Ile Thr Gly
                370                 375                 380

tcc aga cta ggg caa ggg att ggg ccc atc cat ctc aat gaa gtc cag 1323
Ser Arg Leu Gly Gln Gly Ile Gly Pro Ile His Leu Asn Glu Val Gln
            385                 390                 395

tgc aca ggg act gag aag tcc atc ata gac tgc aaa ttc aac aca gag 1371
Cys Thr Gly Thr Glu Lys Ser Ile Ile Asp Cys Lys Phe Asn Thr Glu
        400                 405                 410

tct caa ggc tgc aac cat gaa gaa gat gcc ggg gtg cga tgc aac atc 1419

Ser Gln Gly Cys Asn His Glu Glu Asp Ala Gly Val Arg Cys Asn Ile
    415                 420                 425

ccc atc atg ggt ttc cag aaa aag gtg cgc ctg aat gga ggc cgc aat      1467
Pro Ile Met Gly Phe Gln Lys Lys Val Arg Leu Asn Gly Gly Arg Asn
430                 435                 440                 445

cct tat gag ggc cga gtg gag gtg cta aca gag aga aat ggg tcc ctt      1515
Pro Tyr Glu Gly Arg Val Glu Val Leu Thr Glu Arg Asn Gly Ser Leu
                450                 455                 460

gtt tgg ggg act gta tgc ggc cag aac tgg ggc att gtg gaa gcc atg      1563
Val Trp Gly Thr Val Cys Gly Gln Asn Trp Gly Ile Val Glu Ala Met
            465                 470                 475

gtg gtc tgc cgg cag cta ggc ctg ggc ttt gcc agc aat gcc ttt cag      1611
Val Val Cys Arg Gln Leu Gly Leu Gly Phe Ala Ser Asn Ala Phe Gln
        480                 485                 490

gag acc tgg tac tgg cat gga aat atc ttc gcc aac aac gtg gtc atg      1659
Glu Thr Trp Tyr Trp His Gly Asn Ile Phe Ala Asn Asn Val Val Met
    495                 500                 505

agt gga gtg aag tgc tca gga acg gag ctg tcc cta gca cac tgc cgc      1707
Ser Gly Val Lys Cys Ser Gly Thr Glu Leu Ser Leu Ala His Cys Arg
510                 515                 520                 525

cat gac gag gag gtg gcc tgc ccc gag ggc ggg gtg cgg ttt ggt gct      1755
His Asp Glu Glu Val Ala Cys Pro Glu Gly Gly Val Arg Phe Gly Ala
                530                 535                 540

gga gtc gcc tgc tcg gaa act gca cct gac ctg gtg ctt aat gct gag      1803
Gly Val Ala Cys Ser Glu Thr Ala Pro Asp Leu Val Leu Asn Ala Glu
            545                 550                 555

att gtc cag cag act gcc tac ctg gag gac agg ccc atg tcc ttg ctg      1851
Ile Val Gln Gln Thr Ala Tyr Leu Glu Asp Arg Pro Met Ser Leu Leu
        560                 565                 570

cag tgt gcc atg gag gag aac tgc ctc tcc gcc tcc gct gtg cac acc      1899
Gln Cys Ala Met Glu Glu Asn Cys Leu Ser Ala Ser Ala Val His Thr
    575                 580                 585

gac ccc acc aga ggc cac cgg cgc ctt tta cgc ttc tcc tcc cag atc      1947
Asp Pro Thr Arg Gly His Arg Arg Leu Leu Arg Phe Ser Ser Gln Ile

590                 595                 600                 605

cac aac aat ggc cag tct gac ttc cgc ccc aag aat ggc cgc cat gcg     1995
His Asn Asn Gly Gln Ser Asp Phe Arg Pro Lys Asn Gly Arg His Ala
                610                 615                 620

tgg att tgg cac gac tgc cac agg cac tac cac agc atg gaa gtc ttc     2043
Trp Ile Trp His Asp Cys His Arg His Tyr His Ser Met Glu Val Phe
            625                 630                 635

act tac tat gac ctg ctg agc ctc aac ggc acc aag gtg gct gag ggc     2091
Thr Tyr Tyr Asp Leu Leu Ser Leu Asn Gly Thr Lys Val Ala Glu Gly
        640                 645                 650

cac aag gcc agc ttc tgc ctg gag gac act gag tgt gag gga gac att     2139
His Lys Ala Ser Phe Cys Leu Glu Asp Thr Glu Cys Glu Gly Asp Ile
    655                 660                 665

cag aag agt tac gag tgt gcc aac ttt gga gaa caa ggc atc acc atg     2187
Gln Lys Ser Tyr Glu Cys Ala Asn Phe Gly Glu Gln Gly Ile Thr Met
670                 675                 680                 685

ggc tgc tgg gac atg tac cgt cat gac att gac tgc cag tgg ata gac     2235
Gly Cys Trp Asp Met Tyr Arg His Asp Ile Asp Cys Gln Trp Ile Asp
                690                 695                 700

atc acc gat gtg ccc cct gga gac tac ctg ttc cag gtt gtc att aac     2283
Ile Thr Asp Val Pro Pro Gly Asp Tyr Leu Phe Gln Val Val Ile Asn
            705                 710                 715

ccc aac tat gaa gtg cca gaa tca gat ttc tct aac aac atc atg aag     2331
Pro Asn Tyr Glu Val Pro Glu Ser Asp Phe Ser Asn Asn Ile Met Lys
        720                 725                 730

tgc agg agc cgc tat gat ggc tac cgc atc tgg atg tac aac tgt cac     2379
Cys Arg Ser Arg Tyr Asp Gly Tyr Arg Ile Trp Met Tyr Asn Cys His
    735                 740                 745

gta ggt gga gcc ttc agt gag gag aca gaa cag aag ttc gaa cac ttc     2427
Val Gly Gly Ala Phe Ser Glu Glu Thr Glu Gln Lys Phe Glu His Phe
750                 755                 760                 765

agt gga ctt cta aat aac cag ctc tct gta cag taa agaagatcct          2473
Ser Gly Leu Leu Asn Asn Gln Leu Ser Val Gln
                770                 775

gggccaggca tgatggctca tgcctgtaat ccctgcactc atgctgaggc aggaggattg 2533

ccacaagatt tccactctgg acattaaacc aagcttcagt ttcaaaagaa atgaatgaaa 2593

gaaaggaagg aaggaaggaa ggaaggaagg aaggaaggaa ggaaggaagg aagaaagggg 2653

aaagggaaag ggaagaaaaa tgacttaatg gtcacttact gactcctggg ggaatactga 2713

ttaccacctc ttttctagcc agatccagct gagaagaaag gtgctcattc actccccaga 2773

cactgccgtg tgtccctgtc ctgaggcctt aggggcaggg ctcgggcaca tggccatgga 2833

aacttgatga caagcttaga gcagcttatc ccatccgagc tttggcatgt cccaagtgtg 2893

acatcatctg tgctctgcac agaggggccg ttttcttctg ggaacacagc aggcatgaac 2953

tcagcaactg cagaggtgat cgggctgaac tccgtttttc cccttcttag gtcatttctg 3013

gaaaacttga atatcaagac ctctgtatta agtttgtttg gttttttgtt t 3064

<210> 18
<211> 776
<212> PRT
<213> Mouse

<400> 18

Met Glu Leu His Phe Gly Ser Cys Leu Ser Gly Cys Leu Ala Leu Leu
1               5                   10                  15

Val Leu Leu Pro Ser Leu Ser Leu Ala Gln Tyr Glu Gly Trp Pro Tyr
            20                  25                  30

Gln Leu Gln Tyr Pro Glu Tyr Phe Gln Gln Pro Ala Pro Glu His His
        35                  40                  45

Gln Arg Gln Val Pro Ser Asp Val Val Lys Ile Gln Val Arg Leu Ala
    50                  55                  60

Gly Gln Lys Arg Lys His Asn Glu Gly Arg Val Glu Val Tyr Tyr Glu
65                  70                  75                  80

Gly Gln Trp Gly Thr Val Cys Asp Asp Asp Phe Ser Ile His Ala Ala
                85                  90                  95

His Val Val Cys Arg Gln Val Gly Tyr Val Glu Ala Lys Ser Trp Ala
            100                 105                 110

Ala Ser Ser Ser Tyr Gly Pro Gly Glu Gly Pro Ile Trp Leu Asp Asn
        115                 120                 125

Ile Tyr Cys Thr Gly Lys Glu Ser Thr Leu Ala Ser Cys Ser Ser Asn
    130                 135                 140

Gly Trp Gly Val Thr Asp Cys Lys His Thr Glu Asp Val Gly Val Val
145                 150                 155                 160

Cys Ser Glu Lys Arg Ile Pro Gly Phe Lys Phe Asp Asn Ser Leu Ile
                165                 170                 175

Asn Gln Ile Glu Ser Leu Asn Ile Gln Val Glu Asp Ile Arg Ile Arg
            180                 185                 190

Pro Ile Leu Ser Ala Phe Arg His Arg Lys Pro Val Thr Glu Gly Tyr
        195                 200                 205

Val Glu Val Lys Glu Gly Lys Ala Trp Lys Gln Ile Cys Asn Lys His
    210                 215                 220

Trp Thr Ala Lys Asn Ser His Val Val Cys Gly Met Phe Gly Phe Pro
225                 230                 235                 240

Ala Glu Lys Thr Tyr Asn Pro Lys Ala Tyr Lys Thr Phe Ala Ser Arg

                245                 250                 255

Arg Lys Leu Arg Tyr Trp Lys Phe Ser Met Asn Cys Thr Gly Thr Glu
            260                 265                 270

Ala His Ile Ser Ser Cys Lys Leu Gly Pro Ser Val Thr Arg Asp Pro
        275                 280                 285

Val Lys Asn Ala Thr Cys Glu Asn Gly Gln Pro Ala Val Val Ser Cys
    290                 295                 300

Val Pro Ser Gln Ile Phe Ser Pro Asp Gly Pro Ser Arg Phe Arg Lys
305                 310                 315                 320

Ala Tyr Lys Pro Glu Gln Pro Leu Val Arg Leu Arg Gly Gly Ala Gln
                325                 330                 335

Val Gly Glu Gly Arg Val Glu Val Leu Lys Asn Gly Glu Trp Gly Thr
            340                 345                 350

Ile Cys Asp Asp Lys Trp Asp Leu Val Ser Ala Ser Val Val Cys Arg
        355                 360                 365

Glu Leu Gly Phe Gly Thr Ala Lys Glu Ala Ile Thr Gly Ser Arg Leu
    370                 375                 380

Gly Gln Gly Ile Gly Pro Ile His Leu Asn Glu Val Gln Cys Thr Gly
385                 390                 395                 400

Thr Glu Lys Ser Ile Ile Asp Cys Lys Phe Asn Thr Glu Ser Gln Gly
                405                 410                 415

Cys Asn His Glu Glu Asp Ala Gly Val Arg Cys Asn Ile Pro Ile Met
            420                 425                 430

Gly Phe Gln Lys Lys Val Arg Leu Asn Gly Gly Arg Asn Pro Tyr Glu
        435                 440                 445

Gly Arg Val Glu Val Leu Thr Glu Arg Asn Gly Ser Leu Val Trp Gly
    450                 455                 460

Thr Val Cys Gly Gln Asn Trp Gly Ile Val Glu Ala Met Val Val Cys
465                 470                 475                 480

Arg Gln Leu Gly Leu Gly Phe Ala Ser Asn Ala Phe Gln Glu Thr Trp
                485                 490                 495

Tyr Trp His Gly Asn Ile Phe Ala Asn Asn Val Val Met Ser Gly Val
            500                 505                 510

Lys Cys Ser Gly Thr Glu Leu Ser Leu Ala His Cys Arg His Asp Glu
        515                 520                 525

Glu Val Ala Cys Pro Glu Gly Gly Val Arg Phe Gly Ala Gly Val Ala
    530                 535                 540

Cys Ser Glu Thr Ala Pro Asp Leu Val Leu Asn Ala Glu Ile Val Gln
545                 550                 555                 560

Gln Thr Ala Tyr Leu Glu Asp Arg Pro Met Ser Leu Leu Gln Cys Ala
                565                 570                 575

Met Glu Glu Asn Cys Leu Ser Ala Ser Ala Val His Thr Asp Pro Thr
            580                 585                 590

Arg Gly His Arg Arg Leu Leu Arg Phe Ser Ser Gln Ile His Asn Asn
        595                 600                 605

Gly Gln Ser Asp Phe Arg Pro Lys Asn Gly Arg His Ala Trp Ile Trp
    610                 615                 620

His Asp Cys His Arg His Tyr His Ser Met Glu Val Phe Thr Tyr Tyr
625                 630                 635                 640

Asp Leu Leu Ser Leu Asn Gly Thr Lys Val Ala Glu Gly His Lys Ala
                645                 650                 655

Ser Phe Cys Leu Glu Asp Thr Glu Cys Glu Gly Asp Ile Gln Lys Ser
            660                 665                 670

Tyr Glu Cys Ala Asn Phe Gly Glu Gln Gly Ile Thr Met Gly Cys Trp
        675                 680                 685

Asp Met Tyr Arg His Asp Ile Asp Cys Gln Trp Ile Asp Ile Thr Asp
    690                 695                 700

Val Pro Pro Gly Asp Tyr Leu Phe Gln Val Val Ile Asn Pro Asn Tyr
705                 710                 715                 720

Glu Val Pro Glu Ser Asp Phe Ser Asn Asn Ile Met Lys Cys Arg Ser
                725                 730                 735

Arg Tyr Asp Gly Tyr Arg Ile Trp Met Tyr Asn Cys His Val Gly Gly
            740                 745                 750

Ala Phe Ser Glu Glu Thr Glu Gln Lys Phe Glu His Phe Ser Gly Leu
        755                 760                 765

Leu Asn Asn Gln Leu Ser Val Gln
    770                 775

<210> 19
<211> 3276
<212> DNA
<213> Mouse

<220>
<221> CDS
<222> (148)..(2763)
<223>

<400> 19
ttgggccagt ggcctgtcac tgggttctcc gctcttatgt tggaaagtgt gagggacaca 60

ggagcgacct gcatacctga gcctccgaga gagaagctgc actctggcat ccagtagcag 120

caggagacta agcagataaa aggagcc atg act tct ggc gct agt cat atg ctg 174
Met Thr Ser Gly Ala Ser His Met Leu
1               5

gaa gct gcc ctg gag cag atg gac ggg atc att gca ggc act aaa aca 222
Glu Ala Ala Leu Glu Gln Met Asp Gly Ile Ile Ala Gly Thr Lys Thr
10                  15                  20                  25

gct gca gat ttt agt gat ggt acc tgt gag cct ggg ctg tct ccc cca 270
Ala Ala Asp Phe Ser Asp Gly Thr Cys Glu Pro Gly Leu Ser Pro Pro
                30                  35                  40

tcc acc tgc ttg aac tcc atg cct gtg ctc cat ctc att gag gac ctg 318
Ser Thr Cys Leu Asn Ser Met Pro Val Leu His Leu Ile Glu Asp Leu
            45                  50                  55

aga cta gcc ttg gag atg ttg gca ctt cct cag gaa aga gaa gcc ctc 366
Arg Leu Ala Leu Glu Met Leu Ala Leu Pro Gln Glu Arg Glu Ala Leu
        60                  65                  70

ctg agc cag gtc cct ggc cca aca gct acc tac ata aag gag tgg ttt 414
Leu Ser Gln Val Pro Gly Pro Thr Ala Thr Tyr Ile Lys Glu Trp Phe
    75                  80                  85

gag gac agc ttg tcc cag gta aat cac cac ggt gct gct agt aat gaa 462
Glu Asp Ser Leu Ser Gln Val Asn His His Gly Ala Ala Ser Asn Glu
90                  95                  100                 105

acc tac cag gaa cgc ctg gca cgg cta gaa gga gat aaa gag tcc ctc 510
Thr Tyr Gln Glu Arg Leu Ala Arg Leu Glu Gly Asp Lys Glu Ser Leu

                110                 115                 120

ata cta cag gtg agc gtc ctc aca gat caa gtg gaa gcc caa gga gag 558
Ile Leu Gln Val Ser Val Leu Thr Asp Gln Val Glu Ala Gln Gly Glu
            125                 130                 135

aag att cgg gac ctt gaa gtg tgt ctg gaa ggc cac cag gtg aag ctc 606
Lys Ile Arg Asp Leu Glu Val Cys Leu Glu Gly His Gln Val Lys Leu
        140                 145                 150

aat gct gca gaa gag atg ctt cag cag gag ctg cta agt cgc aca tct 654
Asn Ala Ala Glu Glu Met Leu Gln Gln Glu Leu Leu Ser Arg Thr Ser
    155                 160                 165

ctg gag acc cag aag ttg gat ctg atg act gag gtg tct gag ctg aag 702
Leu Glu Thr Gln Lys Leu Asp Leu Met Thr Glu Val Ser Glu Leu Lys
170                 175                 180                 185

ctc aag ctg gtt ggt atg gaa aaa gaa cag aaa gaa caa gaa gaa aaa 750
Leu Lys Leu Val Gly Met Glu Lys Glu Gln Lys Glu Gln Glu Glu Lys
                190                 195                 200

cag aga aaa gca gag gag tta ctg caa gag ctt aag cac ctc aaa atc 798
Gln Arg Lys Ala Glu Glu Leu Leu Gln Glu Leu Lys His Leu Lys Ile
            205                 210                 215

aag gtg gag gag ctg gag aac gag cgg aac cag tat gag tgg gag ctg 846
Lys Val Glu Glu Leu Glu Asn Glu Arg Asn Gln Tyr Glu Trp Glu Leu
        220                 225                 230

aag gcc acc aag gct gag gta gcc cag ctg caa gaa cag gtg gcc cta 894
Lys Ala Thr Lys Ala Glu Val Ala Gln Leu Gln Glu Gln Val Ala Leu
    235                 240                 245

aaa gat gca gaa att gag cgt ctc cac agc cag ctc tcc cgg agt gca 942
Lys Asp Ala Glu Ile Glu Arg Leu His Ser Gln Leu Ser Arg Ser Ala
250                 255                 260                 265

gct ctc cac agc gac cat gca gag cga gat caa gaa atc cac cgt ctg 990
Ala Leu His Ser Asp His Ala Glu Arg Asp Gln Glu Ile His Arg Leu
                270                 275                 280

aaa atg ggg atg gaa aca ctg ctg gtt gcc aat gag gat aag gac cgt 1038
Lys Met Gly Met Glu Thr Leu Leu Val Ala Asn Glu Asp Lys Asp Arg
            285                 290                 295

cgg ata gag gag ctg aca ggg ctg ttg aac aag tac cta agg gta aag 1086
Arg Ile Glu Glu Leu Thr Gly Leu Leu Asn Lys Tyr Leu Arg Val Lys
        300                 305                 310

gag att gtg atg gca act cag ggg cct tca gaa aga acc ctc tcc atc 1134
Glu Ile Val Met Ala Thr Gln Gly Pro Ser Glu Arg Thr Leu Ser Ile
    315                 320                 325

aat gaa gat gaa ata gag gga agc ttc cga aaa tgg aat acc aca aat 1182
Asn Glu Asp Glu Ile Glu Gly Ser Phe Arg Lys Trp Asn Thr Thr Asn
330                 335                 340                 345

aaa agc cca gag gaa gtc ccg aag caa gag ata tca cca cga tgc agc 1230
Lys Ser Pro Glu Glu Val Pro Lys Gln Glu Ile Ser Pro Arg Cys Ser
                350                 355                 360

tct ccc acc cca gga cca cct cct ttg cca cag aaa tca ctg gag agc 1278
Ser Pro Thr Pro Gly Pro Pro Pro Leu Pro Gln Lys Ser Leu Glu Ser
            365                 370                 375

agg gct cag aag aaa ctc tcc tgc agt cta gaa gac ttg aga cgt gaa 1326
Arg Ala Gln Lys Lys Leu Ser Cys Ser Leu Glu Asp Leu Arg Arg Glu
        380                 385                 390

tct ggg gat aag tgt gtc gat ggg aac cag ctg tcc cca gtg gga gag 1374
Ser Gly Asp Lys Cys Val Asp Gly Asn Gln Leu Ser Pro Val Gly Glu
    395                 400                 405

ccc aag gac agc tct ttc cta gcg gag cag aaa tac ccc aca tta cct 1422
Pro Lys Asp Ser Ser Phe Leu Ala Glu Gln Lys Tyr Pro Thr Leu Pro
410                 415                 420                 425

ggg aag ctt tca gga gcc aca ccc aat gga gaa gct gcc aaa tct cct 1470
Gly Lys Leu Ser Gly Ala Thr Pro Asn Gly Glu Ala Ala Lys Ser Pro
                430                 435                 440

ccc act gcc tcc ctc cag cct gac tct tca ggg agc agc cag cca aag 1518
Pro Thr Ala Ser Leu Gln Pro Asp Ser Ser Gly Ser Ser Gln Pro Lys
            445                 450                 455

ctg aga gac aca gaa gga ggc tgg gaa gat ata gtc tca tct gct tcg 1566
Leu Arg Asp Thr Glu Gly Gly Trp Glu Asp Ile Val Ser Ser Ala Ser
        460                 465                 470

tct ggg act gag tca agc cct cag tct ccc gtg aca cca gat ggc aaa 1614
Ser Gly Thr Glu Ser Ser Pro Gln Ser Pro Val Thr Pro Asp Gly Lys
    475                 480                 485

cgg agc ccc aaa ggc att aag aag ttc tgg gga aag atc cga aga act 1662
Arg Ser Pro Lys Gly Ile Lys Lys Phe Trp Gly Lys Ile Arg Arg Thr
490                 495                 500                 505

cag tct gga aac ttc aat act gat gca ccg ggg atg gca gag ttt cga 1710
Gln Ser Gly Asn Phe Asn Thr Asp Ala Pro Gly Met Ala Glu Phe Arg
                510                 515                 520

cga ggt ggg ctc cga gca act gcg gga cca agg ctt tct agg acc agg 1758
Arg Gly Gly Leu Arg Ala Thr Ala Gly Pro Arg Leu Ser Arg Thr Arg
            525                 530                 535

gac acc aag gga cag aaa tgt gat gcc aat gcc ccc ttt gcc cag tgg 1806
Asp Thr Lys Gly Gln Lys Cys Asp Ala Asn Ala Pro Phe Ala Gln Trp
        540                 545                 550

agc aca gaa cgc gta tgt aca tgg atg gag gat ttc ggc ctg ggt cag 1854
Ser Thr Glu Arg Val Cys Thr Trp Met Glu Asp Phe Gly Leu Gly Gln
    555                 560                 565

tat gtg atc ttt gcc aga cag tgg gtg aca tct gga cat aca cta ctg 1902
Tyr Val Ile Phe Ala Arg Gln Trp Val Thr Ser Gly His Thr Leu Leu
570                 575                 580                 585

aca gct acc cct cag gac atg gaa aag gag cta ggg att aaa cac ccc 1950
Thr Ala Thr Pro Gln Asp Met Glu Lys Glu Leu Gly Ile Lys His Pro
                590                 595                 600

ctc cac agg aag aag ctg gtt tta gca gtg aaa gct atc aac gcc aag 1998
Leu His Arg Lys Lys Leu Val Leu Ala Val Lys Ala Ile Asn Ala Lys
            605                 610                 615

caa gaa gaa acg tct gcg ctg ctg gac cac att tgg gtg aca cgg tgg 2046
Gln Glu Glu Thr Ser Ala Leu Leu Asp His Ile Trp Val Thr Arg Trp
        620                 625                 630

ctt gat gat att ggc tta ccc caa tac aaa gac cag ttt cat gaa tca 2094
Leu Asp Asp Ile Gly Leu Pro Gln Tyr Lys Asp Gln Phe His Glu Ser
    635                 640                 645

aga gtt gat ggg cga atg ctg caa tac cta act gtg aat gat cta ctc 2142

Arg Val Asp Gly Arg Met Leu Gln Tyr Leu Thr Val Asn Asp Leu Leu
650                 655                 660                 665

ttc tta aaa gtc acc agc caa cta cat cat ctc agc atc aaa tgt gct     2190
Phe Leu Lys Val Thr Ser Gln Leu His His Leu Ser Ile Lys Cys Ala
                670                 675                 680

att cac gtg cta cat gtc aac aag ttc aac ccc aac tgc ctg cac agg     2238
Ile His Val Leu His Val Asn Lys Phe Asn Pro Asn Cys Leu His Arg
            685                 690                 695

agg cct gct gat gag agt aac ctt tcc cct tcc gaa gtt gtg cag tgg     2286
Arg Pro Ala Asp Glu Ser Asn Leu Ser Pro Ser Glu Val Val Gln Trp
        700                 705                 710

tcc aac cac agg gta atg gag tgg ctg cga tcc gtg gac ctg gca gag     2334
Ser Asn His Arg Val Met Glu Trp Leu Arg Ser Val Asp Leu Ala Glu
    715                 720                 725

tat gca ccc aac ctt cga ggg agt ggc gtc cat ggc ggc ctc att atc     2382
Tyr Ala Pro Asn Leu Arg Gly Ser Gly Val His Gly Gly Leu Ile Ile
730                 735                 740                 745

ctg gag cct cgc ttt act ggg gac acc ctg gct atg ctt ctt aac atc     2430
Leu Glu Pro Arg Phe Thr Gly Asp Thr Leu Ala Met Leu Leu Asn Ile
                750                 755                 760

ccc cca cag aag acg ctc ctc agg cgc cat ctg acc acc aag ttc aac     2478
Pro Pro Gln Lys Thr Leu Leu Arg Arg His Leu Thr Thr Lys Phe Asn
            765                 770                 775

gcc ctg att ggt cct gag gct gaa cag gaa aag cga gac aaa atg gcc     2526
Ala Leu Ile Gly Pro Glu Ala Glu Gln Glu Lys Arg Asp Lys Met Ala
        780                 785                 790

tca ccc gcc tac aca cct ctg acc acc acc gcc aaa gtt cgg cct agg     2574
Ser Pro Ala Tyr Thr Pro Leu Thr Thr Thr Ala Lys Val Arg Pro Arg
    795                 800                 805

aaa ctt gga ttt tca cat ttt gga aac atg aga aaa aag aag ttt gat     2622
Lys Leu Gly Phe Ser His Phe Gly Asn Met Arg Lys Lys Lys Phe Asp
810                 815                 820                 825

gaa tct aca gat tac att tgc ccc atg gag cct gga gat gct gtc agt     2670
Glu Ser Thr Asp Tyr Ile Cys Pro Met Glu Pro Gly Asp Ala Val Ser

                830                 835                 840

gac agc cac agg gtc tac gga gtc tac cgg ggc ctc agt ccc ctt gac 2718
Asp Ser His Arg Val Tyr Gly Val Tyr Arg Gly Leu Ser Pro Leu Asp
            845                 850                 855

aac cat gaa cta gat ggt ttg gac cag gtg gga cag ata agc tga 2763
Asn His Glu Leu Asp Gly Leu Asp Gln Val Gly Gln Ile Ser
        860                 865                 870

tgaccctgtt atctgccttc tctgtgcacg gagagctcac agtaacactg tgtgtcacca 2823

cgtaactgca cttcaccctc gtccttttgc atgatctaca gaaaacaatt gtgtctttgg 2883

gctggtctca ctacacactt taaggatggt cagaatatgc agttatacag ccacaaaggg 2943

gacagacttt ggggaactat agccaaatgt ggactctggg aaaacacctg cagacattag 3003

tagatgttta gtaattcata gtatccatgt ttcagcttga aacacatgag cagagggcag 3063

gccctgggta atcgcaaaag gttcagttct cagacgctgc cctattcttt cagtcgaggg 3123

aagaattcaa gtgccttagg tttgtgagcc acaaagtctt ggctgagatc aaagtgccaa 3183

cagcggatgt ttggaccata gtgacaatgt aatttgattg taccttacta tagagtggcc 3243

acttgtttct gataataaaa acaatattta tgt 3276

<210> 20
<211> 871
<212> PRT
<213> Mouse

<400> 20

Met Thr Ser Gly Ala Ser His Met Leu Glu Ala Ala Leu Glu Gln Met
1               5                   10                  15

Asp Gly Ile Ile Ala Gly Thr Lys Thr Ala Ala Asp Phe Ser Asp Gly
            20                  25                  30

Thr Cys Glu Pro Gly Leu Ser Pro Pro Ser Thr Cys Leu Asn Ser Met

        35                  40                  45

Pro Val Leu His Leu Ile Glu Asp Leu Arg Leu Ala Leu Glu Met Leu
    50                  55                  60

Ala Leu Pro Gln Glu Arg Glu Ala Leu Leu Ser Gln Val Pro Gly Pro
65                  70                  75                  80

Thr Ala Thr Tyr Ile Lys Glu Trp Phe Glu Asp Ser Leu Ser Gln Val
                85                  90                  95

Asn His His Gly Ala Ala Ser Asn Glu Thr Tyr Gln Glu Arg Leu Ala
            100                 105                 110

Arg Leu Glu Gly Asp Lys Glu Ser Leu Ile Leu Gln Val Ser Val Leu
        115                 120                 125

Thr Asp Gln Val Glu Ala Gln Gly Glu Lys Ile Arg Asp Leu Glu Val
    130                 135                 140

Cys Leu Glu Gly His Gln Val Lys Leu Asn Ala Ala Glu Glu Met Leu
145                 150                 155                 160

Gln Gln Glu Leu Leu Ser Arg Thr Ser Leu Glu Thr Gln Lys Leu Asp
                165                 170                 175

Leu Met Thr Glu Val Ser Glu Leu Lys Leu Lys Leu Val Gly Met Glu
            180                 185                 190

Lys Glu Gln Lys Glu Gln Glu Glu Lys Gln Arg Lys Ala Glu Glu Leu
        195                 200                 205

Leu Gln Glu Leu Lys His Leu Lys Ile Lys Val Glu Glu Leu Glu Asn
    210                 215                 220

Glu Arg Asn Gln Tyr Glu Trp Glu Leu Lys Ala Thr Lys Ala Glu Val
225                 230                 235                 240

Ala Gln Leu Gln Glu Gln Val Ala Leu Lys Asp Ala Glu Ile Glu Arg
                245                 250                 255

Leu His Ser Gln Leu Ser Arg Ser Ala Ala Leu His Ser Asp His Ala
            260                 265                 270

Glu Arg Asp Gln Glu Ile His Arg Leu Lys Met Gly Met Glu Thr Leu
        275                 280                 285

Leu Val Ala Asn Glu Asp Lys Asp Arg Arg Ile Glu Glu Leu Thr Gly
    290                 295                 300

Leu Leu Asn Lys Tyr Leu Arg Val Lys Glu Ile Val Met Ala Thr Gln
305                 310                 315                 320

Gly Pro Ser Glu Arg Thr Leu Ser Ile Asn Glu Asp Glu Ile Glu Gly
                325                 330                 335

Ser Phe Arg Lys Trp Asn Thr Thr Asn Lys Ser Pro Glu Glu Val Pro
            340                 345                 350

Lys Gln Glu Ile Ser Pro Arg Cys Ser Ser Pro Thr Pro Gly Pro Pro
        355                 360                 365

Pro Leu Pro Gln Lys Ser Leu Glu Ser Arg Ala Gln Lys Lys Leu Ser
    370                 375                 380

Cys Ser Leu Glu Asp Leu Arg Arg Glu Ser Gly Asp Lys Cys Val Asp
385                 390                 395                 400

Gly Asn Gln Leu Ser Pro Val Gly Glu Pro Lys Asp Ser Ser Phe Leu
                405                 410                 415

Ala Glu Gln Lys Tyr Pro Thr Leu Pro Gly Lys Leu Ser Gly Ala Thr
            420                 425                 430

Pro Asn Gly Glu Ala Ala Lys Ser Pro Pro Thr Ala Ser Leu Gln Pro
        435                 440                 445

Asp Ser Ser Gly Ser Ser Gln Pro Lys Leu Arg Asp Thr Glu Gly Gly
    450                 455                 460

Trp Glu Asp Ile Val Ser Ser Ala Ser Ser Gly Thr Glu Ser Ser Pro
465                 470                 475                 480

Gln Ser Pro Val Thr Pro Asp Gly Lys Arg Ser Pro Lys Gly Ile Lys
                485                 490                 495

Lys Phe Trp Gly Lys Ile Arg Arg Thr Gln Ser Gly Asn Phe Asn Thr
            500                 505                 510

Asp Ala Pro Gly Met Ala Glu Phe Arg Arg Gly Gly Leu Arg Ala Thr
        515                 520                 525

Ala Gly Pro Arg Leu Ser Arg Thr Arg Asp Thr Lys Gly Gln Lys Cys
    530                 535                 540

Asp Ala Asn Ala Pro Phe Ala Gln Trp Ser Thr Glu Arg Val Cys Thr
545                 550                 555                 560

Trp Met Glu Asp Phe Gly Leu Gly Gln Tyr Val Ile Phe Ala Arg Gln
                565                 570                 575

Trp Val Thr Ser Gly His Thr Leu Leu Thr Ala Thr Pro Gln Asp Met
            580                 585                 590

Glu Lys Glu Leu Gly Ile Lys His Pro Leu His Arg Lys Lys Leu Val
        595                 600                 605

Leu Ala Val Lys Ala Ile Asn Ala Lys Gln Glu Glu Thr Ser Ala Leu
    610                 615                 620

Leu Asp His Ile Trp Val Thr Arg Trp Leu Asp Asp Ile Gly Leu Pro
625                 630                 635                 640

Gln Tyr Lys Asp Gln Phe His Glu Ser Arg Val Asp Gly Arg Met Leu
                645                 650                 655

Gln Tyr Leu Thr Val Asn Asp Leu Leu Phe Leu Lys Val Thr Ser Gln
            660                 665                 670

Leu His His Leu Ser Ile Lys Cys Ala Ile His Val Leu His Val Asn
        675                 680                 685

Lys Phe Asn Pro Asn Cys Leu His Arg Arg Pro Ala Asp Glu Ser Asn
    690                 695                 700

Leu Ser Pro Ser Glu Val Val Gln Trp Ser Asn His Arg Val Met Glu
705                 710                 715                 720

Trp Leu Arg Ser Val Asp Leu Ala Glu Tyr Ala Pro Asn Leu Arg Gly
                725                 730                 735

Ser Gly Val His Gly Gly Leu Ile Ile Leu Glu Pro Arg Phe Thr Gly
            740                 745                 750

Asp Thr Leu Ala Met Leu Leu Asn Ile Pro Pro Gln Lys Thr Leu Leu

755                 760                 765

Arg Arg His Leu Thr Thr Lys Phe Asn Ala Leu Ile Gly Pro Glu Ala
    770                 775                 780

Glu Gln Glu Lys Arg Asp Lys Met Ala Ser Pro Ala Tyr Thr Pro Leu
785                 790                 795                 800

Thr Thr Thr Ala Lys Val Arg Pro Arg Lys Leu Gly Phe Ser His Phe
                805                 810                 815

Gly Asn Met Arg Lys Lys Lys Phe Asp Glu Ser Thr Asp Tyr Ile Cys
            820                 825                 830

Pro Met Glu Pro Gly Asp Ala Val Ser Asp Ser His Arg Val Tyr Gly
        835                 840                 845

Val Tyr Arg Gly Leu Ser Pro Leu Asp Asn His Glu Leu Asp Gly Leu
    850                 855                 860

Asp Gln Val Gly Gln Ile Ser
865                 870

<210>  21
<211>  5020
<212>  DNA
<213>  Mouse

<220>
<221>  CDS
<222>  (174)..(1277)
<223>

<400>  21
tgcagggcta gtctgttggc ctgacgtcag atgtcgcttt gacaaacgcc cccgggggct      60

gaggaaggct ctccgctgct ctgatgggcc agcccagtcg tggcccagct ccctggagag     120

gcatccgcat cctctgggct gagccgtagc tcctgtgacg ctgacttcca ggc atg 176
Met
1

agg tgg ctc ctg ccc tgg acg ctg gca gcc gtg gca gtc ctg agg gtg 224
Arg Trp Leu Leu Pro Trp Thr Leu Ala Ala Val Ala Val Leu Arg Val
            5                   10                  15

ggc aac atc ctg gcc acg gcc ctc tct cca acc ccc aca aca atg acc 272
Gly Asn Ile Leu Ala Thr Ala Leu Ser Pro Thr Pro Thr Thr Met Thr
        20                  25                  30

ttc acc cca gca cca cta gag gaa acg act aca cgc ccc gaa ttc tgc 320
Phe Thr Pro Ala Pro Leu Glu Glu Thr Thr Thr Arg Pro Glu Phe Cys
    35                  40                  45

aag tgg cca tgt gag tgc cca caa tcc cca cct cgc tgc cca ctg ggc 368
Lys Trp Pro Cys Glu Cys Pro Gln Ser Pro Pro Arg Cys Pro Leu Gly
50                  55                  60                  65

gtc agc cta atc aca gat ggc tgt gaa tgc tgt aag ata tgt gcc cag 416
Val Ser Leu Ile Thr Asp Gly Cys Glu Cys Cys Lys Ile Cys Ala Gln
                70                  75                  80

cag ctt ggg gac aac tgc aca gag gct gcc atc tgt gac cca cac cgg 464
Gln Leu Gly Asp Asn Cys Thr Glu Ala Ala Ile Cys Asp Pro His Arg
            85                  90                  95

ggc ctc tac tgc gat tac agt ggg gat cgc ccg agg tac gca ata gga 512
Gly Leu Tyr Cys Asp Tyr Ser Gly Asp Arg Pro Arg Tyr Ala Ile Gly
        100                 105                 110

gtg tgt gca cag gtg gtc ggt gtg ggc tgt gtc ctg gat ggc gta cgc 560
Val Cys Ala Gln Val Val Gly Val Gly Cys Val Leu Asp Gly Val Arg
    115                 120                 125

tac acc aat ggc gag tcc ttc caa ccc aac tgc agg tac aac tgt acc 608
Tyr Thr Asn Gly Glu Ser Phe Gln Pro Asn Cys Arg Tyr Asn Cys Thr
130                 135                 140                 145

tgc att gat ggc acg gtg ggc tgc aca ccg ctg tgc cta agc ccc agg 656
Cys Ile Asp Gly Thr Val Gly Cys Thr Pro Leu Cys Leu Ser Pro Arg
                150                 155                 160

ccc cca cgc ctc tgg tgc cgc cag ccc cgg cac gtg aga gtc cct ggc 704
Pro Pro Arg Leu Trp Cys Arg Gln Pro Arg His Val Arg Val Pro Gly
            165                 170                 175

cag tgc tgt gag cag tgg gtg tgt gat gat gac gca agg aga cca cgc 752
Gln Cys Cys Glu Gln Trp Val Cys Asp Asp Asp Ala Arg Arg Pro Arg
        180                 185                 190

cag act gca ctg ttg gac acc aga gcc ttt gca gcg tca ggc gcc gtg 800
Gln Thr Ala Leu Leu Asp Thr Arg Ala Phe Ala Ala Ser Gly Ala Val
    195                 200                 205

gag caa cgg tat gag aac tgc ata gcc tac act agt ccc tgg agc ccc 848
Glu Gln Arg Tyr Glu Asn Cys Ile Ala Tyr Thr Ser Pro Trp Ser Pro
210                 215                 220                 225

tgc tct acc acc tgt ggc cta ggt atc tcc act cgg atc tct aac gtc 896
Cys Ser Thr Thr Cys Gly Leu Gly Ile Ser Thr Arg Ile Ser Asn Val
                230                 235                 240

aat gcc cgg tgc tgg cca gag cag gaa agt cgc ctc tgc aac ctg cgg 944
Asn Ala Arg Cys Trp Pro Glu Gln Glu Ser Arg Leu Cys Asn Leu Arg
            245                 250                 255

cca tgt gat gtg gac atc caa cta cac atc aag gca ggg aag aaa tgc 992
Pro Cys Asp Val Asp Ile Gln Leu His Ile Lys Ala Gly Lys Lys Cys
        260                 265                 270

ctg gct gtg tac cag cca gag gag gcc acg aac ttc act ctc gca ggc 1040
Leu Ala Val Tyr Gln Pro Glu Glu Ala Thr Asn Phe Thr Leu Ala Gly
    275                 280                 285

tgt gtc agc aca cgc acc tac cga ccc aag tac tgc gga gtc tgt act 1088
Cys Val Ser Thr Arg Thr Tyr Arg Pro Lys Tyr Cys Gly Val Cys Thr
290                 295                 300                 305

gac aat agg tgt tgc atc ccc tac aag tcc aag acc atc agt gtg gat 1136
Asp Asn Arg Cys Cys Ile Pro Tyr Lys Ser Lys Thr Ile Ser Val Asp
                310                 315                 320

ttc cag tgt cca gag ggg cca ggt ttc tcc cgg cag gtc cta tgg att 1184
Phe Gln Cys Pro Glu Gly Pro Gly Phe Ser Arg Gln Val Leu Trp Ile
            325                 330                 335

aat gct tgc ttc tgc aac ctg agc tgc agg aat cct aac gat atc ttt 1232

Asn Ala Cys Phe Cys Asn Leu Ser Cys Arg Asn Pro Asn Asp Ile Phe
        340                 345                 350

gct gac ttg gaa tct tac cct gac ttc gaa gag att gcc aat tag        1277
Ala Asp Leu Glu Ser Tyr Pro Asp Phe Glu Glu Ile Ala Asn
    355                 360                 365

gtgggtgtgt ggctcagggt aaagttccat gctgcaaagc agccagccct ttgtggtcca    1337

ggacttcaca attgagcctt atttcatcta cttcctactc gattctgaat tcccagtttc    1397

tgttcctgtt ttgacaatcg taatggccca ggagagtgct gctcaggctc agacaatggg    1457

ttcctccttg gggacattct acatcattcc aaggaaaaca catctctgac tgttcacaat    1517

ggaagcaaag cctggcccag ctagtctggc tccagcctgg gcaagttgtc agaagttgtg    1577

atgggattgt ccaaggaaaa gcatcagctg aagaaccagt atcatgaagt ccttcctcag    1637

atgccaagcc tagggatgct gggatccttt cagacagatg gatgggattg gggacacagg    1697

aataagctat tattttaccc ttgccaaatg atactatcct gggtatttct gcctaaaaca    1757

taccaaaagt gttcttgttc cactgatctg tatatcacaa gtcaccaaac attttccagg    1817

tgaggaccat agttgtgtca ttctgttttg ctattgaaaa tcattttaaa aagaaggaaa    1877

aaaaaaaaaa gaaaagaaaa gaaagtcatt tcattaactt gggcactttc tcctctcacc    1937

ccatattcta taaagggcta aacttgggtt cttgttgatc aggaatgtaa tttgagaagt    1997

cttacttttg cagggagatg gtagccctca attcatcccg tagatgggac aaggccagcc    2057

aatctttcaa gccatagctg ggcaggtcac tgaatctgct gctggccaag ttcttaggac    2117

aattagccaa aatctgggcc tctctctccc tagggttcat gggagttggt agggaccgta    2177

gagtgacttg tctgttgtct caaaaagtaa gatggaaaga tgttctcatg gcccttagaa    2237

gactcttttg aagtctacgc cagacctaac agaatatgtg catcaaacaa acaagtggat    2297

caccctccca tggcctgggt gacctctagc agtcacccag tgactgtggt aaggccacag    2357

tagtccctgg acccaggaca aatctttttt gtttcagtga cctactttac agcctcagtg    2417

tctatgaaga aagttaactc agttttctca tctgaagaca gaagtcgacc agccattcag 2477

aaatggggat ctatttagta gtggtaaacg ttaaagagtt tgtaagctaa agaaatattt 2537

tttttcctaa gtgataatga ggttgttacc tattttagag gtagaatttt ctgtcatcat 2597

ccttttattg tgttctacat actacccatt gttctcaata tttgccacac atttactcat 2657

tgaaacttgc tgataacaca gaggacttcc cttaatgatt tctgttcttg acactgtgaa 2717

atatcaattc aaagaggcta ataagttcaa tcaaagtcac ctatctgaag actcatagcc 2777

agagaagatt gccttcatat ttcccctttg cttctggacc ttgtaaacat gtcagggcag 2837

gaaagcatac agaagtcagc agcttgccct cttagtattg gctctctagc ccctttcacg 2897

taagaggaga gtgtgtctag gtactaagga tccaacctaa actggacaga aaagggcatg 2957

tgcattatcc cctgtggctc ctcctgccct ggaatcagaa ctagcttaca gcatgctttt 3017

atatgatgat tcagtttgcc ccacaccata catatgggca tatgggagaa ctatacacac 3077

actcataaat ggatttgtaa gtaggaaagg ggacttgaga tttcccctcc ttcctgcaac 3137

ttcccaaagg cctggcctta tgcaaggcaa acagtggtgc ttaagatgta ttttgggaag 3197

aaaatactat atatatatat tcctattttt ccttagaagc cttttcttcc aggatctcat 3257

cttaacagta agaaatcctc tcttaagaga ggcctagtat tttgagtaaa catatgttca 3317

tatgtgtaca agtacttgta tgtaaatata agttaccact gtgggttatt aattaggtaa 3377

attttatgtg gtatcatatt tgattttctt ctattggaaa aatctgcctt aacagctaac 3437

tctgtaagaa cttccatagg agccataagc tagggtcttc ccaggtatcc atcctctttt 3497

gggaaactga gctgagcatc ttcaaccaaa ggagttaggg tgatcattgg gaataggaga 3557

aagggatggc cagggtagct ccatcgttat ttagaaacag acctggcata cagaacgacc 3617

agaggaacca accttctttg aaccaaggga aaaagacttg gatgtaatat atagaagctt 3677

tttctaatag tcagaaacag actttaattg tatggcctgg ttcaaggaaa gttaagaatg 3737

tccattatcg ttaaaaacaa aagtccatta ttgatagctt atttggtgct aagccctacg 3797

gtgcattttg tccggctatc acttagcaga gccatgctca gtacacagtc tttctctatt 3857

caataaatag aaacgctgaa gctcaaaggc actgaggagc tgaggctcag aagcatgttt 3917

agtccactct atcagggggg gaagagatct tgacggaacc taaatgacta ctattggaaa 3977

ctcatatttg aaagctttca gaagtcccac ccccacccca tccccagatg cttagaaact 4037

aaagaagcaa tgaggatgag ctagccttca gtgaagaggt tcactactgc accaagagtg 4097

aatgtcttag gtgtacttag tcattggaca gggagacctg agtgagtttg tgaacctgca 4157

gcttactaaa ccttacaatg agcatttgga gagccaagac tgcttctcgg cgctttactg 4217

acatggcttg cttaatcttc tcagtgagcc caagagtcag ggcgttacca ctgcccattt 4277

tagggctgag aaagcaaaat ctccaggagt taagtgattt gctcaagttt ttaaccaacc 4337

gaggcactgc agataaactc cgaagcccag tgtcatgtaa catgcccatg ccatctctcc 4397

ggacacgcag cccattttcc tgttcctaaa ccaaaggctc agagtcacca gaaccaactc 4457

acaggacagt gcagaaattc taatgtcgag ggtgattaga gactgatcaa agaaagtaat 4517

ttcaaatgat atgattgttt gtaagcaccc tagttaattc tggactacat atgcatagag 4577

attgtgaaga acattacagc ctgtgactat aacgttgact tctgtcattt ctttttaaag 4637

acttgttttt tttttttact caaaggaccc acagtgacag ccctgaatgg ttgagaagca 4697

ttgattagct gtgagtcctg catatgtatg tatgtgtgtg tgtgtgtgtg tatttgtatg 4757

tacttatcta ttttcaaact gtgattgtgt atttaaatat tcctcctgcc attttgtaag 4817

tgattacgca taaagaaaca cctttgaatg tcctaataaa ggagagctag cccttgggcg 4877

gcctgtcaca ttttgccaca ttcctcattt ttctcatgat ctgtgtagca gggaatgtgt 4937

ttgttcaacc atgatgagtt ttcattgttc aaattctttg tttacagctt ttctccttaa 4997

agcaataaat catcagcaac agt 5020

<210> 22

<211>  367
<212>  PRT
<213>  Mouse

<400>  22

Met Arg Trp Leu Leu Pro Trp Thr Leu Ala Ala Val Ala Val Leu Arg
1               5                   10                  15

Val Gly Asn Ile Leu Ala Thr Ala Leu Ser Pro Thr Pro Thr Thr Met
            20                  25                  30

Thr Phe Thr Pro Ala Pro Leu Glu Glu Thr Thr Thr Arg Pro Glu Phe
        35                  40                  45

Cys Lys Trp Pro Cys Glu Cys Pro Gln Ser Pro Pro Arg Cys Pro Leu
    50                  55                  60

Gly Val Ser Leu Ile Thr Asp Gly Cys Glu Cys Cys Lys Ile Cys Ala
65                  70                  75                  80

Gln Gln Leu Gly Asp Asn Cys Thr Glu Ala Ala Ile Cys Asp Pro His
                85                  90                  95

Arg Gly Leu Tyr Cys Asp Tyr Ser Gly Asp Arg Pro Arg Tyr Ala Ile
            100                 105                 110

Gly Val Cys Ala Gln Val Val Gly Val Gly Cys Val Leu Asp Gly Val
        115                 120                 125

Arg Tyr Thr Asn Gly Glu Ser Phe Gln Pro Asn Cys Arg Tyr Asn Cys
    130                 135                 140

Thr Cys Ile Asp Gly Thr Val Gly Cys Thr Pro Leu Cys Leu Ser Pro
145                 150                 155                 160

Arg Pro Pro Arg Leu Trp Cys Arg Gln Pro Arg His Val Arg Val Pro
                165                 170                 175

Gly Gln Cys Cys Glu Gln Trp Val Cys Asp Asp Asp Ala Arg Arg Pro
            180                 185                 190

Arg Gln Thr Ala Leu Leu Asp Thr Arg Ala Phe Ala Ala Ser Gly Ala
        195                 200                 205

Val Glu Gln Arg Tyr Glu Asn Cys Ile Ala Tyr Thr Ser Pro Trp Ser
    210                 215                 220

Pro Cys Ser Thr Thr Cys Gly Leu Gly Ile Ser Thr Arg Ile Ser Asn
225                 230                 235                 240

Val Asn Ala Arg Cys Trp Pro Glu Gln Glu Ser Arg Leu Cys Asn Leu
                245                 250                 255

Arg Pro Cys Asp Val Asp Ile Gln Leu His Ile Lys Ala Gly Lys Lys
            260                 265                 270

Cys Leu Ala Val Tyr Gln Pro Glu Glu Ala Thr Asn Phe Thr Leu Ala
        275                 280                 285

Gly Cys Val Ser Thr Arg Thr Tyr Arg Pro Lys Tyr Cys Gly Val Cys
    290                 295                 300

Thr Asp Asn Arg Cys Cys Ile Pro Tyr Lys Ser Lys Thr Ile Ser Val
305                 310                 315                 320

Asp Phe Gln Cys Pro Glu Gly Pro Gly Phe Ser Arg Gln Val Leu Trp
                325                 330                 335

Ile Asn Ala Cys Phe Cys Asn Leu Ser Cys Arg Asn Pro Asn Asp Ile
        340                 345                 350

Phe Ala Asp Leu Glu Ser Tyr Pro Asp Phe Glu Glu Ile Ala Asn
    355                 360                 365

<210> 23
<211> 1580
<212> DNA
<213> Mouse

<220>
<221> CDS
<222> (318)..(794)
<223>

<400> 23
ctgagaagga aaccgcatct tcagacttct gctcactcac gagaggagct agggtcatcg      60

gacccctaag atcctttcac atcgctccga ctcgccggaa cactcctcac cagtttcctc     120

agccacagcc ctacctgtga gctccggact tccccggctt tctgtggatg ctcaggcaga     180

ctgcagagac ctagcctagg acagcagtga gggggacact cctctcctct cctctcctct     240

ggtatcagcg tctcccccaa gggggacgag catggtgatt gtgccttgaa ggaccttggc     300

tctggatgtc tgagaag atg ctg gtc atg aag ctg ttc act tgc ttc tta     350
                   Met Leu Val Met Lys Leu Phe Thr Cys Phe Leu
                   1               5                   10

cag gtc cta gct ggg ttg gct gtg cat tcc cag ggg gcc ctg tct gct     398
Gln Val Leu Ala Gly Leu Ala Val His Ser Gln Gly Ala Leu Ser Ala
        15                  20                  25

ggg aac aac tca aca gaa gtg gaa gtg gtg cct ttc aac gaa gtg tgg     446
Gly Asn Asn Ser Thr Glu Val Glu Val Val Pro Phe Asn Glu Val Trp
    30                  35                  40

ggt cgc agc tac tgt cgg ccc atg gag aag ctg gtg tac atc ttg gat     494
Gly Arg Ser Tyr Cys Arg Pro Met Glu Lys Leu Val Tyr Ile Leu Asp
45                  50                  55

gaa tac cct gat gag gtg tct cac ata ttc agt ccg tcc tgt gtc ctt     542
Glu Tyr Pro Asp Glu Val Ser His Ile Phe Ser Pro Ser Cys Val Leu
60                  65                  70                  75

ctg agt cgc tgt agt ggc tgc tgt ggt gat gaa ggt ctg cac tgt gtg     590
Leu Ser Arg Cys Ser Gly Cys Cys Gly Asp Glu Gly Leu His Cys Val
                80                  85                  90

ccg ata aag aca gcc aac atc act atg cag atc ttg aag att ccc ccc     638
Pro Ile Lys Thr Ala Asn Ile Thr Met Gln Ile Leu Lys Ile Pro Pro
            95                  100                 105

aat cgg gat cca cat ttc tat gtg gag atg aca ttt tct cag gat gtg     686
Asn Arg Asp Pro His Phe Tyr Val Glu Met Thr Phe Ser Gln Asp Val
        110                 115                 120

ctc tgt gaa tgc aga cct att ctg gag acg aca aag gca gaa agg agg     734
Leu Cys Glu Cys Arg Pro Ile Leu Glu Thr Thr Lys Ala Glu Arg Arg
    125                 130                 135

aaa acc aag ggg aag agg aag agg agt aga aac tca cag act gag gaa     782
Lys Thr Lys Gly Lys Arg Lys Arg Ser Arg Asn Ser Gln Thr Glu Glu
140                 145                 150                 155

ccc cac ccg tga tgtcatttcc cggaggtaac cagccactca gaggagagcc     834
Pro His Pro

ccacacccag ctcacgtatt tattaccgtc accctctcag aaccctccct gctggtacct     894

accctctatt tattagccga ctcgtccctg ctgaatgacg tgccccctcc aagataaggg     954

gcatggaagg acaagacgct caggaattca gtgccttaaa acagaacgag agagaaagaa     1014

agaagccagc cacggatctg tgggagcttc ggcttgggaa gaagcaagac atggacatgg     1074

ccttacaagg ggcaagccgc accccagagg ccctggctct ccagggaact ggagaagaga     1134

agaggaggcc tagaacctgc cctgattccc agctccaccc agagagcagc tctggccccg     1194

gctgcattga aggcatgtag aggggaccca gtctactgta tcctggagat gggacaggac     1254

attcggctct ggagagcaga gcttgcctgt ggagtttgcc cttcagccta gaagtttatg     1314

cttcaccctt tgcaaaggtc ctttgtcccc tctctggaac acaggcagag tggcctgggg     1374

ctgagcagag ggcaggctgg aggagcagta gcccgtggac tttgaccgat acaagtctgg 1434

gttgtgtgtc ttcgtggtac agctacggga agtgagccga ctcctggagg gcccttgcag 1494

cccaccggtt gtctctgccg ggactaactg ccaagccaga ttctcttgaa taaagcattc 1554

tagtctggaa aaaaaaaaaa aaaaaa 1580

<210> 24
<211> 158
<212> PRT
<213> Mouse

<400> 24

Met Leu Val Met Lys Leu Phe Thr Cys Phe Leu Gln Val Leu Ala Gly
1               5                   10                  15

Leu Ala Val His Ser Gln Gly Ala Leu Ser Ala Gly Asn Asn Ser Thr
            20                  25                  30

Glu Val Glu Val Val Pro Phe Asn Glu Val Trp Gly Arg Ser Tyr Cys
        35                  40                  45

Arg Pro Met Glu Lys Leu Val Tyr Ile Leu Asp Glu Tyr Pro Asp Glu
    50                  55                  60

Val Ser His Ile Phe Ser Pro Ser Cys Val Leu Leu Ser Arg Cys Ser
65                  70                  75                  80

Gly Cys Cys Gly Asp Glu Gly Leu His Cys Val Pro Ile Lys Thr Ala
                85                  90                  95

Asn Ile Thr Met Gln Ile Leu Lys Ile Pro Pro Asn Arg Asp Pro His
            100                 105                 110

Phe Tyr Val Glu Met Thr Phe Ser Gln Asp Val Leu Cys Glu Cys Arg
        115                 120                 125

Pro Ile Leu Glu Thr Thr Lys Ala Glu Arg Arg Lys Thr Lys Gly Lys
    130                 135                 140

Arg Lys Arg Ser Arg Asn Ser Gln Thr Glu Glu Pro His Pro
145                 150                 155

<210>  25
<211>  2879
<212>  DNA
<213>  Mouse

<220>
<221>  CDS
<222>  (223)..(2124)
<223>

<400>  25
ggcacgaggg ccacccgagt cgccggcgtc gccacctgca cttggctctg gacccccgcg      60

gacagagccc cggccggccg ccgcttcccg ccgcctgccc tgcccacctg ccaggtatta     120

ccacttaaag aaaccctttt accggcaaac ctatgctaaa gagtataagt aacaaaggaa     180

accaaacagt tatctgtcaa gtaacaagca tttaatgaca ga atg gct cac ctt        234
                                               Met Ala His Leu
                                               1

aag cga cta gta aaa ttg cac att aaa aga cat tac cac aga aag ttc       282
Lys Arg Leu Val Lys Leu His Ile Lys Arg His Tyr His Arg Lys Phe
5                   10                  15                  20

tgg aag ctg ggt gca gtc atc ttt ttc ttt tta gta gtt ctg att tta       330
Trp Lys Leu Gly Ala Val Ile Phe Phe Phe Leu Val Val Leu Ile Leu
                25                  30                  35

atg caa aga gaa gta agt gtt cag tat tcc aag gag gaa tca aag atg       378
Met Gln Arg Glu Val Ser Val Gln Tyr Ser Lys Glu Glu Ser Lys Met
            40                  45                  50

gag agg aac ttg aaa aac aaa aac aaa atg ttg gat ttt atg ctc gaa 426
Glu Arg Asn Leu Lys Asn Lys Asn Lys Met Leu Asp Phe Met Leu Glu
        55                  60                  65

gct gta aat aat att aaa gat gca atg cca aag atg caa ata gga gcg 474
Ala Val Asn Asn Ile Lys Asp Ala Met Pro Lys Met Gln Ile Gly Ala
    70                  75                  80

ccc att aag gag aat atc gac gtc cgc gag aga ccc tgt ctg caa ggg 522
Pro Ile Lys Glu Asn Ile Asp Val Arg Glu Arg Pro Cys Leu Gln Gly
85                  90                  95                  100

tac tac aca gcc gcg gag ttg aag ccg gtt ttt gat cgc cca cct cag 570
Tyr Tyr Thr Ala Ala Glu Leu Lys Pro Val Phe Asp Arg Pro Pro Gln
                105                 110                 115

gat tct aac gca cct ggt gct tct ggc aag ccg ttt aag atc acc cac 618
Asp Ser Asn Ala Pro Gly Ala Ser Gly Lys Pro Phe Lys Ile Thr His
            120                 125                 130

ctc agc ccg gag gag cag aag gag aaa gag cga ggg gaa acg aag cac 666
Leu Ser Pro Glu Glu Gln Lys Glu Lys Glu Arg Gly Glu Thr Lys His
        135                 140                 145

tgc ttc aac gcc ttt gca agt gac aga att tct ctg cac cgg gac ctt 714
Cys Phe Asn Ala Phe Ala Ser Asp Arg Ile Ser Leu His Arg Asp Leu
    150                 155                 160

ggg cct gac acc cga cca cct gaa tgt att gaa caa aaa ttt aag cgc 762
Gly Pro Asp Thr Arg Pro Pro Glu Cys Ile Glu Gln Lys Phe Lys Arg
165                 170                 175                 180

tgc ccg ccc ctg cct acc acc agt gtc ata ata gtc ttt cac aat gaa 810
Cys Pro Pro Leu Pro Thr Thr Ser Val Ile Ile Val Phe His Asn Glu
                185                 190                 195

gca tgg tcc acg ctg ctt agg acc gtc cac agt gtg ctc tat tct tca 858
Ala Trp Ser Thr Leu Leu Arg Thr Val His Ser Val Leu Tyr Ser Ser
            200                 205                 210

cct gcc ata ctg ctg aag gag atc att ttg gtg gat gat gct agt gta 906
Pro Ala Ile Leu Leu Lys Glu Ile Ile Leu Val Asp Asp Ala Ser Val
        215                 220                 225

gac gac tac ctg cat gaa aag ctg gag gaa tac ata aaa cag ttt tct 954

Asp Asp Tyr Leu His Glu Lys Leu Glu Glu Tyr Ile Lys Gln Phe Ser
    230                 235                 240

att gtg aaa ata gtc agg cag caa gaa agg aaa ggc ctg atc acc gcg     1002
Ile Val Lys Ile Val Arg Gln Gln Glu Arg Lys Gly Leu Ile Thr Ala
245                 250                 255                 260

cgg ttg cta ggg gca gct gta gca act gcc gag acg ctc acg ttc tta     1050
Arg Leu Leu Gly Ala Ala Val Ala Thr Ala Glu Thr Leu Thr Phe Leu
                265                 270                 275

gat gct cac tgt gag tgc ttc tat ggc tgg ctg gaa cct ctg ctg gcc     1098
Asp Ala His Cys Glu Cys Phe Tyr Gly Trp Leu Glu Pro Leu Leu Ala
            280                 285                 290

agg ata gct gag aac tac act gcc gtg gtg agt cca gac atc gca tcc     1146
Arg Ile Ala Glu Asn Tyr Thr Ala Val Val Ser Pro Asp Ile Ala Ser
        295                 300                 305

ata gat cta aac aca ttt gaa ttc aac aag cct tct ccg tac gga agc     1194
Ile Asp Leu Asn Thr Phe Glu Phe Asn Lys Pro Ser Pro Tyr Gly Ser
    310                 315                 320

aac cat aac cgt gga aat ttt gac tgg agc ctt tcc ttt ggc tgg gag     1242
Asn His Asn Arg Gly Asn Phe Asp Trp Ser Leu Ser Phe Gly Trp Glu
325                 330                 335                 340

tca ctt cct gat cat gag aag caa aga agg aaa gat gaa acc tac cca     1290
Ser Leu Pro Asp His Glu Lys Gln Arg Arg Lys Asp Glu Thr Tyr Pro
                345                 350                 355

att aag acc ccc acc ttt gca gga ggc ctt ttt tct ata tct aaa aaa     1338
Ile Lys Thr Pro Thr Phe Ala Gly Gly Leu Phe Ser Ile Ser Lys Lys
            360                 365                 370

tat ttt gag cac att gga agt tac gat gaa gaa atg gaa atc tgg gga     1386
Tyr Phe Glu His Ile Gly Ser Tyr Asp Glu Glu Met Glu Ile Trp Gly
        375                 380                 385

ggt gaa aat ata gaa atg tca ttc cga gtg tgg caa tgt ggt ggg cag     1434
Gly Glu Asn Ile Glu Met Ser Phe Arg Val Trp Gln Cys Gly Gly Gln
    390                 395                 400

ttg gag att atg cct tgc tct gtt gtt gga cat gtt ttt cgc agc aaa     1482
Leu Glu Ile Met Pro Cys Ser Val Val Gly His Val Phe Arg Ser Lys

405                 410                 415                 420

agc cct cat acc ttc cca aaa ggc acg cag gtg att gct cgt aac caa      1530
Ser Pro His Thr Phe Pro Lys Gly Thr Gln Val Ile Ala Arg Asn Gln
                425                 430                 435

gtt cgc ctt gca gag gtc tgg atg gac gaa tac aag gaa ata ttt tat      1578
Val Arg Leu Ala Glu Val Trp Met Asp Glu Tyr Lys Glu Ile Phe Tyr
            440                 445                 450

agg aga aac aca gat gca gca aaa atc gtt aag caa aaa tca ttt ggt      1626
Arg Arg Asn Thr Asp Ala Ala Lys Ile Val Lys Gln Lys Ser Phe Gly
        455                 460                 465

gat ctt tcc aaa aga ttt gaa ata aag aaa cgc ctt cag tgt aaa aat      1674
Asp Leu Ser Lys Arg Phe Glu Ile Lys Lys Arg Leu Gln Cys Lys Asn
    470                 475                 480

ttt acc tgg tac ctg aac act att tac ccg gaa gcg tat gtg cca gac      1722
Phe Thr Trp Tyr Leu Asn Thr Ile Tyr Pro Glu Ala Tyr Val Pro Asp
485                 490                 495                 500

ctt aat cct gtt ata tct gga tat att aag agt gtg ggt caa cct tta      1770
Leu Asn Pro Val Ile Ser Gly Tyr Ile Lys Ser Val Gly Gln Pro Leu
                505                 510                 515

tgt ctg gat gtt ggt gag aat aac cag gga ggc aaa cca ttg att ctg      1818
Cys Leu Asp Val Gly Glu Asn Asn Gln Gly Gly Lys Pro Leu Ile Leu
            520                 525                 530

tac acg tgc cac ggc ctc ggg gga aat cag tac ttc gag tat tct gct      1866
Tyr Thr Cys His Gly Leu Gly Gly Asn Gln Tyr Phe Glu Tyr Ser Ala
        535                 540                 545

cag cgt gaa atc cgg cac aac atc cag aag gag ctg tgt ctt cat gct      1914
Gln Arg Glu Ile Arg His Asn Ile Gln Lys Glu Leu Cys Leu His Ala
    550                 555                 560

act cag ggt gtc gtc cag ctg aag gca tgt gtc tat aaa ggt cac agg      1962
Thr Gln Gly Val Val Gln Leu Lys Ala Cys Val Tyr Lys Gly His Arg
565                 570                 575                 580

acc atc gcc cct gga gaa cag ata tgg gag att cgg aag gac caa ctt      2010
Thr Ile Ala Pro Gly Glu Gln Ile Trp Glu Ile Arg Lys Asp Gln Leu
                585                 590                 595

cta tat aat cca tta ttt aaa atg tgc ctt tca tca aat gga gag cat     2058
Leu Tyr Asn Pro Leu Phe Lys Met Cys Leu Ser Ser Asn Gly Glu His
            600                 605                 610

cca aac tta gtg cca tgt gac gca aca gat cta ctc caa aaa tgg att     2106
Pro Asn Leu Val Pro Cys Asp Ala Thr Asp Leu Leu Gln Lys Trp Ile
        615                 620                 625

ttt agc caa aat gaa taa gtgttcctta aaattaagga gttgaaaagg            2154
Phe Ser Gln Asn Glu
    630

acatactctt cctcataaaa ctgtgactag gcatacactg tagttgttga aaattatgca   2214

aaagcagcta attgtaactt attccaagtg catttcctta tttatatctt aagatgtcta   2274

tgtagaaccg ctgcagaaac cctggggttt ctgtctgaaa gcacagcaac tagaatacca   2334

aagacgattc tgaaatgtcc agatgtagaa gagagatgtt tacagtgtga agaaaataat   2394

ttccttagta aagtgaggtg tggttgtaca cttggggatc tacacacctg catccacaca   2454

ctcacagctg aaaatgtttt ccctaatttt aggggcaata gaaaaagatt tgatactgta   2514

tttttataac tatatagaaa tggatcaatg aaggccagtc atttggcctt tcggtacaaa   2574

ccaggaactt tttatcgatc tagaattcat tgttttaaaa tccaggtaaa cttttgttgt   2634

ctcttgttga cttgtctgtc aaatatttcc ttaaacatga agttgataag gagaggacta   2694

tttttaacat ttaaattttt ggaaaattta caaatatttt ttagaagccc actccacttg   2754

atgcacatga gtcttcccca aatagctttt ctgaagtgac tatactgtgt gttttctcag   2814

agtacttttt aaaaaataaa taaactttat aaattattag ctgttaaaaa aaaaaaaaaa   2874

aaaaa                                                               2879

<210>  26
<211>  633
<212>  PRT
<213>  Mouse

<400> 26

Met Ala His Leu Lys Arg Leu Val Lys Leu His Ile Lys Arg His Tyr
1               5                   10                  15

His Arg Lys Phe Trp Lys Leu Gly Ala Val Ile Phe Phe Phe Leu Val
            20                  25                  30

Val Leu Ile Leu Met Gln Arg Glu Val Ser Val Gln Tyr Ser Lys Glu
        35                  40                  45

Glu Ser Lys Met Glu Arg Asn Leu Lys Asn Lys Asn Lys Met Leu Asp
    50                  55                  60

Phe Met Leu Glu Ala Val Asn Asn Ile Lys Asp Ala Met Pro Lys Met
65                  70                  75                  80

Gln Ile Gly Ala Pro Ile Lys Glu Asn Ile Asp Val Arg Glu Arg Pro
                85                  90                  95

Cys Leu Gln Gly Tyr Tyr Thr Ala Ala Glu Leu Lys Pro Val Phe Asp
            100                 105                 110

Arg Pro Pro Gln Asp Ser Asn Ala Pro Gly Ala Ser Gly Lys Pro Phe
        115                 120                 125

Lys Ile Thr His Leu Ser Pro Glu Glu Gln Lys Glu Lys Glu Arg Gly
    130                 135                 140

Glu Thr Lys His Cys Phe Asn Ala Phe Ala Ser Asp Arg Ile Ser Leu
145                 150                 155                 160

His Arg Asp Leu Gly Pro Asp Thr Arg Pro Pro Glu Cys Ile Glu Gln
                165                 170                 175

Lys Phe Lys Arg Cys Pro Pro Leu Pro Thr Thr Ser Val Ile Ile Val
            180                 185                 190

Phe His Asn Glu Ala Trp Ser Thr Leu Leu Arg Thr Val His Ser Val
        195                 200                 205

Leu Tyr Ser Ser Pro Ala Ile Leu Leu Lys Glu Ile Ile Leu Val Asp
    210                 215                 220

Asp Ala Ser Val Asp Asp Tyr Leu His Glu Lys Leu Glu Glu Tyr Ile
225                 230                 235                 240

Lys Gln Phe Ser Ile Val Lys Ile Val Arg Gln Gln Glu Arg Lys Gly
                245                 250                 255

Leu Ile Thr Ala Arg Leu Leu Gly Ala Ala Val Ala Thr Ala Glu Thr
            260                 265                 270

Leu Thr Phe Leu Asp Ala His Cys Glu Cys Phe Tyr Gly Trp Leu Glu
        275                 280                 285

Pro Leu Leu Ala Arg Ile Ala Glu Asn Tyr Thr Ala Val Val Ser Pro
    290                 295                 300

Asp Ile Ala Ser Ile Asp Leu Asn Thr Phe Glu Phe Asn Lys Pro Ser
305                 310                 315                 320

Pro Tyr Gly Ser Asn His Asn Arg Gly Asn Phe Asp Trp Ser Leu Ser
                325                 330                 335

Phe Gly Trp Glu Ser Leu Pro Asp His Glu Lys Gln Arg Arg Lys Asp
            340                 345                 350

Glu Thr Tyr Pro Ile Lys Thr Pro Thr Phe Ala Gly Gly Leu Phe Ser
        355                 360                 365

Ile Ser Lys Lys Tyr Phe Glu His Ile Gly Ser Tyr Asp Glu Glu Met
    370                 375                 380

Glu Ile Trp Gly Gly Glu Asn Ile Glu Met Ser Phe Arg Val Trp Gln
385                 390                 395                 400

Cys Gly Gly Gln Leu Glu Ile Met Pro Cys Ser Val Val Gly His Val
                405                 410                 415

Phe Arg Ser Lys Ser Pro His Thr Phe Pro Lys Gly Thr Gln Val Ile
            420                 425                 430

Ala Arg Asn Gln Val Arg Leu Ala Glu Val Trp Met Asp Glu Tyr Lys
        435                 440                 445

Glu Ile Phe Tyr Arg Arg Asn Thr Asp Ala Ala Lys Ile Val Lys Gln
    450                 455                 460

Lys Ser Phe Gly Asp Leu Ser Lys Arg Phe Glu Ile Lys Lys Arg Leu
465                 470                 475                 480

Gln Cys Lys Asn Phe Thr Trp Tyr Leu Asn Thr Ile Tyr Pro Glu Ala
                485                 490                 495

Tyr Val Pro Asp Leu Asn Pro Val Ile Ser Gly Tyr Ile Lys Ser Val
            500                 505                 510

Gly Gln Pro Leu Cys Leu Asp Val Gly Glu Asn Asn Gln Gly Gly Lys
        515                 520                 525

Pro Leu Ile Leu Tyr Thr Cys His Gly Leu Gly Gly Asn Gln Tyr Phe

530                 535                 540

Glu Tyr Ser Ala Gln Arg Glu Ile Arg His Asn Ile Gln Lys Glu Leu
545                 550                 555                 560

Cys Leu His Ala Thr Gln Gly Val Val Gln Leu Lys Ala Cys Val Tyr
                565                 570                 575

Lys Gly His Arg Thr Ile Ala Pro Gly Glu Gln Ile Trp Glu Ile Arg
            580                 585                 590

Lys Asp Gln Leu Leu Tyr Asn Pro Leu Phe Lys Met Cys Leu Ser Ser
        595                 600                 605

Asn Gly Glu His Pro Asn Leu Val Pro Cys Asp Ala Thr Asp Leu Leu
    610                 615                 620

Gln Lys Trp Ile Phe Ser Gln Asn Glu
625                 630

<210>  27
<211>  5540
<212>  DNA
<213>  human

<220>
<221>  CDS
<222>  (144)..(1838)
<223>

<400>  27
aattgcttcc ggggagttgc gagggagcga gggggaataa aggacccgcg aggaagggcc      60
cgcggatggc gcgtccctga gggtcgtggc gagttcgcgg agcgtgggaa ggagcggacc     120
ctgctctccc cgggctgcgg gcc atg gcc acg gcg gag cgg aga gcc ctc ggc     173
atc ggc ttc cag tgg ctc tct ttg gcc act ctg gtg ctc atc tgc gcc 221
ggg caa ggg gga cgc agg gag gat ggg ggt cca gcc tgc tac ggc gga 269
ttt gac ctg tac ttc att ttg gac aaa tca gga agt gtg ctg cac cac 317
tgg aat gaa atc tat tac ttt gtg gaa cag ttg gct cac aaa ttc atc 365
agc cca cag ttg aga atg tcc ttt att gtt ttc tcc acc cga gga aca 413
acc tta atg aaa ctg aca gaa gac aga gaa caa atc cgt caa ggc cta 461
gaa gaa ctc cag aaa gtt ctg cca gga gga gac act tac atg cat gaa 509
gga ttt gaa agg gcc agt gag cag att tat tat gaa aac aga caa ggg 557
tac agg aca gcc agc gtc atc att gct ttg act gat gga gaa ctc cat 605
gaa gat ctc ttt ttc tat tca gag agg gag gct aat agg tct cga gat 653
ctt ggt gca att gtt tac tgt gtt ggt gtg aaa gat ttc aat gag aca 701
cag ctg gcc cgg att gcg gac agt aag gat cat gtg ttt ccc gtg aat 749
gac ggc ttt cag gct ctg caa ggc atc atc cac tca att ttg aag aag 797
tcc tgc atc gaa att cta gca gct gaa cca tcc acc ata tgt gca gga 845
gag tca ttt caa gtt gtc gtg aga gga aac ggc ttc cga cat gcc cgc 893
aac gtg gac agg gtc ctc tgc agc ttc aag atc aat gac tcg gtc aca 941
ctc aat gag aag ccc ttt tct gtg gaa gat act tat tta ctg tgt cca 989
gcg cct atc tta aaa gaa gtt ggc atg aaa gct gca ctc cag gtc agc 1037
atg aac gat ggc ctc tct ttt atc tcc agt tct gtc atc atc acc acc 1085
aca cac tgt tct gac ggt tcc atc ctg gcc atc gcc ctg ctg atc ctg 1133
ttc ctg ctc cta gcc ctg gct ctc ctc tgg tgg ttc tgg ccc ctc tgc 1181
tgc act gtg att atc aag gag gtc cct cca ccc cct gcc gag gag agt 1229
gag gaa gaa gat gat gat ggt ctg cct aag aaa aag tgg cca acg gta 1277
gac gcc tct tat tat ggt ggg aga ggc gtt gga ggc att aaa aga atg        1325
gag gtt cgt tgg gga gaa aag ggc tcc aca gaa gaa ggt gct aag ttg        1373
gaa aag gca aag aat gca aga gtc aag atg ccg gag cag gaa tat gaa        1421
ttc cct gag ccg cga aat ctc aac aac aat atg cgt cgg cct tct tcc        1469
ccc cgg aag tgg tac tct cca atc aag gga aaa ctc gat gcc ttg tgg        1517
gtc cta ctg agg aaa gga tat gat cgt gtg tct gtg atg cgt cca cag        1565
cca gga gac acg ggg cgc tgc atc aac ttc acc agg gtc aag aac aac        1613
cag cca gcc aag tac cca ctc aac aac gcc tac cac acc tcc tcg ccg        1661
cct cct gcc ccc atc tac act ccc cca cct cct gcg ccc cac tgc cct        1709
ccc ccg ccc ccc agc gcc cct acc cct ccc atc ccg tcc cca cct tcc        1757
acc ctt ccc cct cct ccc cag gct cca cct ccc aac agg gca cct cct        1805
ccc tcc cgc cct cct cca agg cct tct gtc tag agcccaaagt tcctgctctg    1858
ggctctctca gaaacttcag gagatgttag aacaagtctt tccagttaga gaagaggagt    1918
ggtgataaag cccactgacc ttcacacatt ctaaaaattg gttggcaatg ccagtatacc    1978
aacaatcatg atcagctgaa agaaacagat attttaaatt gccagaaaac aaatgatgag    2038
gcaactacag tcagatttat agccagccat ctatcacctc tagaaggttc cagagacagt    2098
gaaactgcaa gatgctctca acaggattat gtctcatgga gaccagtaag aaaatcattt    2158
atctgaaggt gaaatgcaga gttggataag aaatacattg ctgggtttct aaaatgctgc    2218
cttcctgcct ctactccacc tccatccctg gactttggac ccttggccta ggagcctaag    2278
gaccttcacc cctgtgcacc acccaagaaa gaggaaaact ttgcctacaa ctttggaaat    2338
gctggggtcc ctggtgtggt aagaaactca acatcagacg ggtatgcaga aggatgttct    2398
tctgggattt gcaggtacat aaaaaatgta tggcatcttt tccttgcaaa ttcttccagt    2458
ttccaagtga gaaggggagc aggtgtttac tgatggaaaa ggtatgttgc tatgttgatg    2518
tgtaagtgaa atcagttgtg tgcaatagac aggggcgtat tcatgggagc atcagccagt    2578
ttctaaaacc cacaggccat cagcagctag aggtggctgg ctttggccag acatggaccc    2638
taaatcaaca gacaatggca ttgtcgaaga gcaacctgtt aatgaatcat gttaaaaatc    2698
aaggtttggc ttcagtttaa atcacttgag gtatgaagtt tatcctgttt tccagagata    2758
aacataagtt gatcttccca aaataccatc attaggacct atcacacaat atcactagtt    2818
tttttgtttt gtttgttttt tgtttttttt cttggtaaag ccatgcacca cagacttctg    2878
ggcagagctg agagacaatg gtcctgacat aataaggatc tttgattaac ccccataagg    2938
catgtgtgtg tatacaaata tacttctctt tggcttttcg acatagaacc tcagctgtta    2998
accaagggga aatacatcag atctgcaaca cagaaatgct ctgcctgaaa tttccaccat    3058
gcctaggact caccccattt atccaggtct ttctggatct gtttaatcaa taagccctat 3118
aatcacttgc taaacactgg gcttcatcac ccagggataa aaacagagat cattgtcttg 3178
gacctcctgc atcagcctat tcaaaattat ctctctctct agctttccac aaatcctaaa 3238
attcctgtcc caagccaccc aaattctcag atcttttctg gaacaaggca gaatataaaa 3298
taaatataca tttagtggct tgggctatgg tctccaaaga tccttcaaaa atacatcaag 3358
ccagcttcat tcactcactt tacttagaac agagatataa gggcctggga tgcatttatt 3418
ttatcaatac caatttttgt ggccatggca gacattgcta atcaatcaca gcactatttc 3478
ctattaagcc cactgatttc ttcacaatcc ttctcaaatt acaattccaa agagccgcca 3538
ctcaacagtc agatgaaccc aacagtcaga tgagagaaat gaaccctact tgctatctct 3598
atcttagaaa gcaaaaacaa acaggagttt ccagggagaa tgggaaagcc agggggcata 3658
aaaggtacag tcaggggaaa atagatctag gcagagtgcc ttagtcaggg accacgggcg 3718
ctgaatctgc agtgccaaca ccaaactgac acatctccag gtgtacctcc aaccctagcc 3778
ttctcccaca gctgcctaca acagagtctc ccagccttct cagagagcta aaaccagaaa 3838
tttccagact catgaaagca accccccagc ctctccccaa ccctgccgca ttgtctaatt 3898
tttagaacac taggcttctt ctttcatgta gttcctcata agcaggggcc agaatatctc 3958
agccacctgc agtgacattg ctggacccct gaaaaccatt ccataggaga atgggttccc 4018
caggctcaca gtgtagagac attgagccca tcacaactgt tttgactgct ggcagtctaa 4078
aacagtccac ccaccccatg gcactgccgc gtgattcccg cggccattca gaagttcaag 4138
ccgagatgct gacgttgctg agcaacgaga tggtgagcat cagtgcaaat gcaccattca 4198
gcacatcagt catatgccca gtgcagttac aagatgttgt ttcggcaaag cattttgatg 4258
gaatagggaa ctgcaaatgt atgatgattt tgaaaaggct cagcaggatt tgttcttaaa 4318
ccgactcagt gtgtcatccc cggttattta gaattacagt taagaaggag aaacttctat 4378
aagactgtat gaacaaggtg atatcttcat agtgggctat tacaggcagg aaaatgtttt 4438
aactggttta caaaatccat caatacttgt gtcattccct gtaaaaggca ggagacatgt 4498
gattatgatc aggaaactgc acaaaattat tgttttcagc ccccgtgtta ttgtcctttt 4558
gaactgtttt ttttttatta aagccaaatt tgtgttgtat atattcgtat tccatgtgtt 4618
agatggaagc atttcctatc cagtgtgaat aaaaagaaca gttgtagtaa attattataa 4678
agccgatgat atttcatggc aggttattct accaagctgt gcttgttggt ttttcccatg 4738
actgtattgc ttttataaat gtacaaatag ttactgaaat gacgagaccc ttgtttgcac 4798
agcattaata agaaccttga taagaaccat attctgttga cagccagctc acagtttctt 4858
gcctgaagct tggtgcaccc tccagtgaga cacaagatct ctcttttacc aaagttgaga 4918
acagagctgg tggattaatt aatagtcttc gatatctggc catgggtaac ctcattgtaa 4978
ctatcatcag aatgggcaga gatgatcttg aagtgtcaca tacactaaag tccaaacact 5038
atgtcagatg ggggtaaaat ccattaaaga acaggaaaaa ataattataa gatgataagc 5098
aaatgtttca gcccaatgtc aacccagtta aaaaaaaaat taatgctgtg taaaatggtt 5158
gaattagttt gcaaactata taaagacata tgcagtaaaa agtctgttaa tgcacatcct 5218
gtgggaatgg agtgttctaa ccaattgcct tttcttgtta tctgagctct cctatattat 5278
catactcaga taaccaaatt aaaagaatta gaatatgatt tttaatacac ttaacattaa 5338
actcttctaa ctttcttctt tctgtgataa ttcagaagat agttatggat cttcaatgcc 5398
tctgagtcat tgttataaaa aatcagttat cactatacca tgctatagga gactgggcaa 5458
aacctgtaca atgacaaccc tggaagttgc tttttttaaa aaaataataa atttcttaaa 5518
tcaaaaaaaa aaaaaaaaaa aa 5540

<210> 28
<211> 564
<212> PRT
<213> human

<400> 28

<210>  29
<211>  1433
<212>  DNA
<213>  human

<220>
<221>  CDS
<222>  (148)..(900)
<223>

<400>  29
tgtgtgtgtg tgtgtgtgag cgcgcgcgcg cgcgcgcgtg tgtactcgtg cgtgtgcctg      60

tgtgtgcctg ggagtgacct cacagctgcc ggaacataaa gactcacagg tccgcctccc     120

aggctcaaag ctggctctgc aggggac atg aga ggc aca ccg aag acc cac ctc     174
                              Met Arg Gly Thr Pro Lys Thr His Leu
                              1               5

ctg gcc ttc tcc ctc ctc tgc ctc ctc tca aag gtg cgt acc cag ctg       222
Leu Ala Phe Ser Leu Leu Cys Leu Leu Ser Lys Val Arg Thr Gln Leu
10                  15                  20                  25

tgc ccg aca cca tgt acc tgc ccc tgg cca cct ccc cga tgc ccg ctg       270
Cys Pro Thr Pro Cys Thr Cys Pro Trp Pro Pro Pro Arg Cys Pro Leu
                30                  35                  40

gga gta ccc ctg gtg ctg gat ggc tgt ggc tgc tgc cgg gta tgt gca       318
Gly Val Pro Leu Val Leu Asp Gly Cys Gly Cys Cys Arg Val Cys Ala
            45                  50                  55

cgg cgg ctg ggg gag ccc tgc gac caa ctc cac gtc tgc gac gcc agc       366
Arg Arg Leu Gly Glu Pro Cys Asp Gln Leu His Val Cys Asp Ala Ser

        60                  65                  70

cag ggc ctg gtc tgc cag ccc ggg gca gga ccc ggt ggc cgg ggg gcc 414
Gln Gly Leu Val Cys Gln Pro Gly Ala Gly Pro Gly Gly Arg Gly Ala
    75                  80                  85

ctg tgc ctc ttg gca gag gac gac agc agc tgt gag gtg aac ggc cgc 462
Leu Cys Leu Leu Ala Glu Asp Asp Ser Ser Cys Glu Val Asn Gly Arg
90                  95                  100                 105

ctg tat cgg gaa ggg gag acc ttc cag ccc cac tgc agc atc cgc tgc 510
Leu Tyr Arg Glu Gly Glu Thr Phe Gln Pro His Cys Ser Ile Arg Cys
                110                 115                 120

cgc tgc gag gac ggc ggc ttc acc tgc gtg ccg ctg tgc agc gag gat 558
Arg Cys Glu Asp Gly Gly Phe Thr Cys Val Pro Leu Cys Ser Glu Asp
            125                 130                 135

gtg cgg ctg ccc agc tgg gac tgc ccc cac ccc agg agg gtc gag gtc 606
Val Arg Leu Pro Ser Trp Asp Cys Pro His Pro Arg Arg Val Glu Val
        140                 145                 150

ctg ggc aag tgc tgc cct gag tgg gtg tgc ggc caa gga ggg gga ctg 654
Leu Gly Lys Cys Cys Pro Glu Trp Val Cys Gly Gln Gly Gly Gly Leu
    155                 160                 165

ggg acc cag ccc ctt cca gcc caa gga ccc cag ttt tct ggc ctt gtc 702
Gly Thr Gln Pro Leu Pro Ala Gln Gly Pro Gln Phe Ser Gly Leu Val
170                 175                 180                 185

tct tcc ctg ccc cct ggt gtc ccc tgc cca gaa tgg agc acg gcc tgg 750
Ser Ser Leu Pro Pro Gly Val Pro Cys Pro Glu Trp Ser Thr Ala Trp
                190                 195                 200

gga ccc tgc tcg acc acc tgt ggg ctg ggc atg gcc acc cgg gtg tcc 798
Gly Pro Cys Ser Thr Thr Cys Gly Leu Gly Met Ala Thr Arg Val Ser
            205                 210                 215

aac cag aac cgc ttc tgc cga ctg gag acc cag cgc cgc ctg tgc ctg 846
Asn Gln Asn Arg Phe Cys Arg Leu Glu Thr Gln Arg Arg Leu Cys Leu
        220                 225                 230

tcc agg ccc tgc cca ccc tcc agg ggt cgc agt cca caa aac agt gcc 894
Ser Arg Pro Cys Pro Pro Ser Arg Gly Arg Ser Pro Gln Asn Ser Ala
    235                 240                 245

ttc tag agccgggctg ggaatgggga cacggtgtcc accatcccca gctggtggcc 950
Phe
250

ctgtgcctgg gccctgggct gatggaagat ggtccgtgcc caggcccttg gctgcaggca 1010

acactttagc ttgggtccac catgcagaac accaatatta acacgctgcc tggtctgtct 1070

ggatcccgag gtatggcaga ggtgcaagac ctagtcccct ttcctctaac tcactgccta 1130

ggaggctggc caaggtgtcc agggtcctct agcccactcc ctgcctacac acacagccta 1190

tatcaaacat gcacacgggc gagctttctc tccgacttcc cctgggcaag agatgggaca 1250

agcagtccct taatattgag gctgcagcag gtgctgggct ggactggcca tttttctggg 1310

ggtaggatga agagaaggca cacagagatt ctggatctcc tgctgccttt tctggagttt 1370

gtaaaattgt tcctgaatac aagcctatgc gtgaaaaaaa aaaaaaaaaa aaaaaaaaaa 1430

aaa 1433

<210> 30
<211> 250
<212> PRT
<213> human

<400> 30

Met Arg Gly Thr Pro Lys Thr His Leu Leu Ala Phe Ser Leu Leu Cys
1               5                   10                  15

Leu Leu Ser Lys Val Arg Thr Gln Leu Cys Pro Thr Pro Cys Thr Cys
            20                  25                  30

Pro Trp Pro Pro Pro Arg Cys Pro Leu Gly Val Pro Leu Val Leu Asp
        35                  40                  45

Gly Cys Gly Cys Cys Arg Val Cys Ala Arg Arg Leu Gly Glu Pro Cys
    50                  55                  60

Asp Gln Leu His Val Cys Asp Ala Ser Gln Gly Leu Val Cys Gln Pro
65                  70                  75                  80

Gly Ala Gly Pro Gly Gly Arg Gly Ala Leu Cys Leu Leu Ala Glu Asp
                85                  90                  95

Asp Ser Ser Cys Glu Val Asn Gly Arg Leu Tyr Arg Glu Gly Glu Thr
            100                 105                 110

Phe Gln Pro His Cys Ser Ile Arg Cys Arg Cys Glu Asp Gly Gly Phe
        115                 120                 125

Thr Cys Val Pro Leu Cys Ser Glu Asp Val Arg Leu Pro Ser Trp Asp
    130                 135                 140

Cys Pro His Pro Arg Arg Val Glu Val Leu Gly Lys Cys Cys Pro Glu
145                 150                 155                 160

Trp Val Cys Gly Gln Gly Gly Gly Leu Gly Thr Gln Pro Leu Pro Ala
                165                 170                 175

Gln Gly Pro Gln Phe Ser Gly Leu Val Ser Ser Leu Pro Pro Gly Val
            180                 185                 190

Pro Cys Pro Glu Trp Ser Thr Ala Trp Gly Pro Cys Ser Thr Thr Cys
        195                 200                 205

Gly Leu Gly Met Ala Thr Arg Val Ser Asn Gln Asn Arg Phe Cys Arg
    210                 215                 220

Leu Glu Thr Gln Arg Arg Leu Cys Leu Ser Arg Pro Cys Pro Pro Ser
225                 230                 235                 240

Arg Gly Arg Ser Pro Gln Asn Ser Ala Phe
                245                 250

<210>  31
<211>  2502
<212>  DNA
<213>  human

<220>
<221>  CDS
<222>  (55)..(2154)
<223>

<400>  31
gaattcgtgg gtcgtgctgc gtcgacaacg gtagtgacgc gtattgcctg gagg atg      57
                                                            Met
                                                            1

gcg gac gcc ggc att cgc cgc gtg gtt ccc agc gac ctg tat ccc ctc      105
Ala Asp Ala Gly Ile Arg Arg Val Val Pro Ser Asp Leu Tyr Pro Leu
            5                   10                  15

gtg ctc ggc ttc ctg cgc gat aac caa ctc tca gag gtg gcc aat aag      153
Val Leu Gly Phe Leu Arg Asp Asn Gln Leu Ser Glu Val Ala Asn Lys
        20                  25                  30

ttc gcc aaa gcg aca gga gct aca cag cag gat gcc aat gcc tct tcc      201
Phe Ala Lys Ala Thr Gly Ala Thr Gln Gln Asp Ala Asn Ala Ser Ser
    35                  40                  45

ctc tta gac atc tat agc ttc tgg ctc aag tct gcc aag gtc cca gag      249
Leu Leu Asp Ile Tyr Ser Phe Trp Leu Lys Ser Ala Lys Val Pro Glu
50                  55                  60                  65

cga aag tta cag gca aat gga cca gtg gct aag aaa gct aag aag aag      297
Arg Lys Leu Gln Ala Asn Gly Pro Val Ala Lys Lys Ala Lys Lys Lys
                70                  75                  80

gcc tca tcc agt gac agt gag gac agc agc gag gag gag gag gaa gtt      345
Ala Ser Ser Ser Asp Ser Glu Asp Ser Ser Glu Glu Glu Glu Glu Val
            85                  90                  95

caa ggg cct cca gca aag aag gct gct gta cct gcc aag cga gtc ggt      393

Gln Gly Pro Pro Ala Lys Lys Ala Ala Val Pro Ala Lys Arg Val Gly
        100                 105                 110

ctg cct cct ggg aag gct gca gcc aaa gca tca gag agt agc agc agt      441
Leu Pro Pro Gly Lys Ala Ala Ala Lys Ala Ser Glu Ser Ser Ser Ser
    115                 120                 125

gaa gag tcc aga gat gat gat gat gag gag gac caa aag aaa cag cct      489
Glu Glu Ser Arg Asp Asp Asp Asp Glu Glu Asp Gln Lys Lys Gln Pro
130                 135                 140                 145

gtc cag aag gga gtt aag ccc caa gcc aag gca gcc aaa gct cct cct      537
Val Gln Lys Gly Val Lys Pro Gln Ala Lys Ala Ala Lys Ala Pro Pro
                150                 155                 160

aag aag gcc aag agc tct gat tct gat tct gac tca agc tcc gag gat      585
Lys Lys Ala Lys Ser Ser Asp Ser Asp Ser Asp Ser Ser Ser Glu Asp
            165                 170                 175

gag cca cca aag aac cag aag cca aag ata aca cct gtg aca gtt aaa      633
Glu Pro Pro Lys Asn Gln Lys Pro Lys Ile Thr Pro Val Thr Val Lys
        180                 185                 190

gct cag act aaa gcc cct ccc aaa cca gct cga gca gca cct aaa ata      681
Ala Gln Thr Lys Ala Pro Pro Lys Pro Ala Arg Ala Ala Pro Lys Ile
    195                 200                 205

gcc aat ggt aaa gca gcc agt agc agc agt agc agc agc agc agc agt      729
Ala Asn Gly Lys Ala Ala Ser Ser Ser Ser Ser Ser Ser Ser Ser Ser
210                 215                 220                 225

agc agt gat gac tca gag gag gag aag gca gca gcc acc ccc aag aag      777
Ser Ser Asp Asp Ser Glu Glu Glu Lys Ala Ala Ala Thr Pro Lys Lys
                230                 235                 240

act gta cct aaa aag caa gtt gtg gcc aaa gcc cca gtg aaa gca gct      825
Thr Val Pro Lys Lys Gln Val Val Ala Lys Ala Pro Val Lys Ala Ala
            245                 250                 255

acc acc cct acc cgg aag agt tct agc agt gag gat tcc tcc agt gac      873
Thr Thr Pro Thr Arg Lys Ser Ser Ser Ser Glu Asp Ser Ser Ser Asp
        260                 265                 270

gag gaa gag gag caa aaa aaa ccc atg aaa aat aaa cca ggt ccc tac      921
Glu Glu Glu Glu Gln Lys Lys Pro Met Lys Asn Lys Pro Gly Pro Tyr

    275                 280                 285

agt tac gcc ccc ccg cct tct gct ccc cca cca aag aag tct ctg gga 969
Ser Tyr Ala Pro Pro Pro Ser Ala Pro Pro Pro Lys Lys Ser Leu Gly
290                 295                 300                 305

acc cag cct ccc aag aag gct gtg gag aag cag cag cct gtg gaa agc 1017
Thr Gln Pro Pro Lys Lys Ala Val Glu Lys Gln Gln Pro Val Glu Ser
                310                 315                 320

agt gaa gac agc agt gat gag tct gat tca agt tct gaa gaa gag aag 1065
Ser Glu Asp Ser Ser Asp Glu Ser Asp Ser Ser Ser Glu Glu Glu Lys
            325                 330                 335

aaa ccc cca act aag gca gta gtc tct aaa gca acc act aaa cca cct 1113
Lys Pro Pro Thr Lys Ala Val Val Ser Lys Ala Thr Thr Lys Pro Pro
        340                 345                 350

cca gca aag aaa gca gca gag agc tct tca gac agc tca gac tct gac 1161
Pro Ala Lys Lys Ala Ala Glu Ser Ser Ser Asp Ser Ser Asp Ser Asp
    355                 360                 365

agc tct gag gat gat gaa gct cct tct aag cca gct ggt acc acc aag 1209
Ser Ser Glu Asp Asp Glu Ala Pro Ser Lys Pro Ala Gly Thr Thr Lys
370                 375                 380                 385

aat tct tca aat aag cca gct gtc acc acc aag tca cct gca gtg aag 1257
Asn Ser Ser Asn Lys Pro Ala Val Thr Thr Lys Ser Pro Ala Val Lys
                390                 395                 400

cca gct gca gcc ccc aag caa cct gtg ggc ggt ggc cag aag ctt ctg 1305
Pro Ala Ala Ala Pro Lys Gln Pro Val Gly Gly Gly Gln Lys Leu Leu
            405                 410                 415

acg aga aag gct gac agc agc tcc agc gag gaa gag agc agc tcc agt 1353
Thr Arg Lys Ala Asp Ser Ser Ser Ser Glu Glu Glu Ser Ser Ser Ser
        420                 425                 430

gag gag gag aag aca aag aag atg gtg gcc acc act aag ccc aag gcg 1401
Glu Glu Glu Lys Thr Lys Lys Met Val Ala Thr Thr Lys Pro Lys Ala
    435                 440                 445

act gcc aaa gca gct cta tct ctg cct gcc aag cag gct cct cag ggt 1449
Thr Ala Lys Ala Ala Leu Ser Leu Pro Ala Lys Gln Ala Pro Gln Gly
450                 455                 460                 465

agt agg gac agc agc tct gat tca gac agc tcc agc agt gag gag gag 1497
Ser Arg Asp Ser Ser Ser Asp Ser Asp Ser Ser Ser Ser Glu Glu Glu
                470                 475                 480

gaa gag aag aca tct aag tct gca gtt aag aag aag cca cag aag gta 1545
Glu Glu Lys Thr Ser Lys Ser Ala Val Lys Lys Lys Pro Gln Lys Val
            485                 490                 495

gca gga ggt gca gcc cct tcc aag cca gcc tct gca aag aaa gga aag 1593
Ala Gly Gly Ala Ala Pro Ser Lys Pro Ala Ser Ala Lys Lys Gly Lys
        500                 505                 510

gct gag agc agc aac agt tct tct tct gat gac tcc agt gag gaa gag 1641
Ala Glu Ser Ser Asn Ser Ser Ser Ser Asp Asp Ser Ser Glu Glu Glu
    515                 520                 525

gaa gag aag ctc aag ggc aag ggc tct cca aga cca caa gcc ccc aag 1689
Glu Glu Lys Leu Lys Gly Lys Gly Ser Pro Arg Pro Gln Ala Pro Lys
530                 535                 540                 545

gcc aat ggc acc tct gca ctg act gcc cag aat gga aaa gca gct aag 1737
Ala Asn Gly Thr Ser Ala Leu Thr Ala Gln Asn Gly Lys Ala Ala Lys
                550                 555                 560

aac agt gag gag gag gaa gaa gaa aag aaa aag gcg gca gtg gta gtt 1785
Asn Ser Glu Glu Glu Glu Glu Glu Lys Lys Lys Ala Ala Val Val Val
            565                 570                 575

tcc aaa tca ggt tca tta aag aag cgg aag cag aat gag gct gcc aag 1833
Ser Lys Ser Gly Ser Leu Lys Lys Arg Lys Gln Asn Glu Ala Ala Lys
        580                 585                 590

gag gca gag act cct cag gcc aag aag ata aag ctt cag acc cct aac 1881
Glu Ala Glu Thr Pro Gln Ala Lys Lys Ile Lys Leu Gln Thr Pro Asn
    595                 600                 605

aca ttt cca aaa agg aag aaa gga gaa aaa agg gca tca tcc cca ttc 1929
Thr Phe Pro Lys Arg Lys Lys Gly Glu Lys Arg Ala Ser Ser Pro Phe
610                 615                 620                 625

cga agg gtc agg gag gag gaa att gag gtg gat tca cga gtt gcg gac 1977
Arg Arg Val Arg Glu Glu Glu Ile Glu Val Asp Ser Arg Val Ala Asp
                630                 635                 640

aac tcc ttt gat gcc aag cga ggt gca gcc gga gac tgg gga gag cga 2025
Asn Ser Phe Asp Ala Lys Arg Gly Ala Ala Gly Asp Trp Gly Glu Arg
            645                 650                 655

gcc aat cag gtt ttg aag ttc acc aaa ggc aag tcc ttt cgg cat gag 2073
Ala Asn Gln Val Leu Lys Phe Thr Lys Gly Lys Ser Phe Arg His Glu
        660                 665                 670

aaa acc aag aag aag cgg ggc agc tac cgg gga ggc tca atc tct gtc 2121
Lys Thr Lys Lys Lys Arg Gly Ser Tyr Arg Gly Gly Ser Ile Ser Val
    675                 680                 685

cag gtc aat tct att aag ttt gac agc gag tga cctgaggcca tcttcggtga 2174
Gln Val Asn Ser Ile Lys Phe Asp Ser Glu
690                 695

agcaagggtg atgatcggag actacttact ttctccagtg gacctgggaa ccctcaggtc 2234

tctaggtgag ggtcttgatg aggacagaag tttagagtag gtcctaagac tttacagtgt 2294

aacatcctct ctggtccttt tctgtgttcc tagttttgta cagacttgtt tttgagtgtt 2354

gagtagcagg gacaaaataa gggaatgtta ttttttaaga aaattcattt tcattgttgt 2414

ctccttcctt ttctgtgaaa gtcctcatac tgagaaattt gtatatttta tattaaatca 2474

cttactattg aaaaaaaaa aggaattc 2502

<210> 32
<211> 699
<212> PRT
<213> human

<400> 32

Met Ala Asp Ala Gly Ile Arg Arg Val Val Pro Ser Asp Leu Tyr Pro
1               5                   10                  15

Leu Val Leu Gly Phe Leu Arg Asp Asn Gln Leu Ser Glu Val Ala Asn
            20                  25                  30

Lys Phe Ala Lys Ala Thr Gly Ala Thr Gln Gln Asp Ala Asn Ala Ser

35                  40                  45

Ser Leu Leu Asp Ile Tyr Ser Phe Trp Leu Lys Ser Ala Lys Val Pro
    50                  55                  60

Glu Arg Lys Leu Gln Ala Asn Gly Pro Val Ala Lys Lys Ala Lys Lys
65                  70                  75                  80

Lys Ala Ser Ser Ser Asp Ser Glu Asp Ser Ser Glu Glu Glu Glu Glu
                85                  90                  95

Val Gln Gly Pro Pro Ala Lys Lys Ala Ala Val Pro Ala Lys Arg Val
            100                 105                 110

Gly Leu Pro Pro Gly Lys Ala Ala Ala Lys Ala Ser Glu Ser Ser Ser
        115                 120                 125

Ser Glu Glu Ser Arg Asp Asp Asp Asp Glu Glu Asp Gln Lys Lys Gln
    130                 135                 140

Pro Val Gln Lys Gly Val Lys Pro Gln Ala Lys Ala Ala Lys Ala Pro
145                 150                 155                 160

Pro Lys Lys Ala Lys Ser Ser Asp Ser Asp Ser Asp Ser Ser Ser Glu
                165                 170                 175

Asp Glu Pro Pro Lys Asn Gln Lys Pro Lys Ile Thr Pro Val Thr Val
            180                 185                 190

Lys Ala Gln Thr Lys Ala Pro Pro Lys Pro Ala Arg Ala Ala Pro Lys
        195                 200                 205

Ile Ala Asn Gly Lys Ala Ala Ser Ser Ser Ser Ser Ser Ser Ser Ser
    210                 215                 220

Ser Ser Ser Asp Asp Ser Glu Glu Glu Lys Ala Ala Ala Thr Pro Lys
225                 230                 235                 240

Lys Thr Val Pro Lys Lys Gln Val Val Ala Lys Ala Pro Val Lys Ala
                245                 250                 255

Ala Thr Thr Pro Thr Arg Lys Ser Ser Ser Ser Glu Asp Ser Ser Ser
            260                 265                 270

Asp Glu Glu Glu Glu Gln Lys Lys Pro Met Lys Asn Lys Pro Gly Pro
        275                 280                 285

Tyr Ser Tyr Ala Pro Pro Pro Ser Ala Pro Pro Pro Lys Lys Ser Leu
    290                 295                 300

Gly Thr Gln Pro Pro Lys Lys Ala Val Glu Lys Gln Gln Pro Val Glu
305                 310                 315                 320

Ser Ser Glu Asp Ser Ser Asp Glu Ser Asp Ser Ser Ser Glu Glu Glu
                325                 330                 335

Lys Lys Pro Pro Thr Lys Ala Val Val Ser Lys Ala Thr Thr Lys Pro
            340                 345                 350

Pro Pro Ala Lys Lys Ala Ala Glu Ser Ser Ser Asp Ser Ser Asp Ser
        355                 360                 365

Asp Ser Ser Glu Asp Asp Glu Ala Pro Ser Lys Pro Ala Gly Thr Thr
    370                 375                 380

Lys Asn Ser Ser Asn Lys Pro Ala Val Thr Thr Lys Ser Pro Ala Val
385                 390                 395                 400

Lys Pro Ala Ala Ala Pro Lys Gln Pro Val Gly Gly Gly Gln Lys Leu
                405                 410                 415

Leu Thr Arg Lys Ala Asp Ser Ser Ser Ser Glu Glu Glu Ser Ser Ser
            420                 425                 430

Ser Glu Glu Glu Lys Thr Lys Lys Met Val Ala Thr Thr Lys Pro Lys
        435                 440                 445

Ala Thr Ala Lys Ala Ala Leu Ser Leu Pro Ala Lys Gln Ala Pro Gln
    450                 455                 460

Gly Ser Arg Asp Ser Ser Ser Asp Ser Asp Ser Ser Ser Ser Glu Glu
465                 470                 475                 480

Glu Glu Glu Lys Thr Ser Lys Ser Ala Val Lys Lys Lys Pro Gln Lys
                485                 490                 495

Val Ala Gly Gly Ala Ala Pro Ser Lys Pro Ala Ser Ala Lys Lys Gly
            500                 505                 510

Lys Ala Glu Ser Ser Asn Ser Ser Ser Ser Asp Asp Ser Ser Glu Glu
        515                 520                 525

Glu Glu Glu Lys Leu Lys Gly Lys Gly Ser Pro Arg Pro Gln Ala Pro
    530                 535                 540

Lys Ala Asn Gly Thr Ser Ala Leu Thr Ala Gln Asn Gly Lys Ala Ala
545                 550                 555                 560

Lys Asn Ser Glu Glu Glu Glu Glu Glu Lys Lys Lys Ala Ala Val Val
                565                 570                 575

Val Ser Lys Ser Gly Ser Leu Lys Lys Arg Lys Gln Asn Glu Ala Ala
            580                 585                 590

Lys Glu Ala Glu Thr Pro Gln Ala Lys Lys Ile Lys Leu Gln Thr Pro
        595                 600                 605

Asn Thr Phe Pro Lys Arg Lys Lys Gly Glu Lys Arg Ala Ser Ser Pro
    610                 615                 620

Phe Arg Arg Val Arg Glu Glu Glu Ile Glu Val Asp Ser Arg Val Ala
625                 630                 635                 640

Asp Asn Ser Phe Asp Ala Lys Arg Gly Ala Ala Gly Asp Trp Gly Glu
                645                 650                 655

Arg Ala Asn Gln Val Leu Lys Phe Thr Lys Gly Lys Ser Phe Arg His
            660                 665                 670

Glu Lys Thr Lys Lys Lys Arg Gly Ser Tyr Arg Gly Gly Ser Ile Ser
        675                 680                 685

Val Gln Val Asn Ser Ile Lys Phe Asp Ser Glu
    690                 695

<210>  33
<211>  4538
<212>  DNA
<213>  human

<220>
<221>  CDS
<222>  (26)..(4012)
<223>

<400>  33
tcgtgtttcg gtgagtgtgg cggag atg gag agc cgg gat ccc gcc cag ccg        52
                            Met Glu Ser Arg Asp Pro Ala Gln Pro

                            1               5

atg tcg cct gga gaa gcg acg cag agt ggc gcc cgg cct gcc gac cgc 100
Met Ser Pro Gly Glu Ala Thr Gln Ser Gly Ala Arg Pro Ala Asp Arg
10                  15                  20                  25

tat ggc cta ttg aag cac agt cgc gag ttc ttg gac ttc ttc tgg gac 148
Tyr Gly Leu Leu Lys His Ser Arg Glu Phe Leu Asp Phe Phe Trp Asp
                30                  35                  40

att gcg aag cct gag cag gag acg cga ctt gcg gcc acg gag aag ctg 196
Ile Ala Lys Pro Glu Gln Glu Thr Arg Leu Ala Ala Thr Glu Lys Leu
            45                  50                  55

ctg gag tat ctg cgt ggc agg ccg aag ggg tcc gag atg aaa tat gcc 244
Leu Glu Tyr Leu Arg Gly Arg Pro Lys Gly Ser Glu Met Lys Tyr Ala
        60                  65                  70

ctg aag cgt cta atc acg gga ctc ggg gtc ggg cga gaa aca gcc cgg 292
Leu Lys Arg Leu Ile Thr Gly Leu Gly Val Gly Arg Glu Thr Ala Arg
    75                  80                  85

ccc tgc tac agt ttg gcc ctg gca cag ctg tta cag tct ttt gaa gac 340
Pro Cys Tyr Ser Leu Ala Leu Ala Gln Leu Leu Gln Ser Phe Glu Asp
90                  95                  100                 105

ctc ccc ttg tgc agc atc ctg cag cag ata caa gaa aaa tat gac ctg 388
Leu Pro Leu Cys Ser Ile Leu Gln Gln Ile Gln Glu Lys Tyr Asp Leu
                110                 115                 120

cat cag gtg aag aag gca atg ctg aga cct gct ctc ttt gca aac ctg 436
His Gln Val Lys Lys Ala Met Leu Arg Pro Ala Leu Phe Ala Asn Leu
            125                 130                 135

ttt gga gtg ctc gcc ctc ttt cag tca ggt cgg ctg gtg aag gac cag 484
Phe Gly Val Leu Ala Leu Phe Gln Ser Gly Arg Leu Val Lys Asp Gln
        140                 145                 150

gag gca ctg atg aag tcg gtg aag ctg ctg cag gcc ctg gcc cag tac 532
Glu Ala Leu Met Lys Ser Val Lys Leu Leu Gln Ala Leu Ala Gln Tyr
    155                 160                 165

caa aac cac ttg cag gag cag ccc cgg aag gcc ctg gtg gac atc ctc 580
Gln Asn His Leu Gln Glu Gln Pro Arg Lys Ala Leu Val Asp Ile Leu
170                 175                 180                 185

tcc gag gtc tcg aag gcc aca ttg cag gag atc ctg ccg gag gtc ctc 628
Ser Glu Val Ser Lys Ala Thr Leu Gln Glu Ile Leu Pro Glu Val Leu
                190                 195                 200

aaa gcc gac ttg aat ata ata ctc agc tcc cct gaa cag cta gag ctc 676
Lys Ala Asp Leu Asn Ile Ile Leu Ser Ser Pro Glu Gln Leu Glu Leu
            205                 210                 215

ttc ctc ctg gcc cag cag aag gtg ccc tcc aag ctc aag aag ctg gtg 724
Phe Leu Leu Ala Gln Gln Lys Val Pro Ser Lys Leu Lys Lys Leu Val
        220                 225                 230

gga tcc gtg aac cta ttc tca gat gag aat gtc ccc agg ctg gtg aat 772
Gly Ser Val Asn Leu Phe Ser Asp Glu Asn Val Pro Arg Leu Val Asn
    235                 240                 245

gtg ctg aag atg gcc gcc tcc tct gtg aag aag gac cgc aag ctg ccc 820
Val Leu Lys Met Ala Ala Ser Ser Val Lys Lys Asp Arg Lys Leu Pro
250                 255                 260                 265

gcc att gct ctg gac ctg ctc cgc ctg gcg ctc aag gaa gac aag ttc 868
Ala Ile Ala Leu Asp Leu Leu Arg Leu Ala Leu Lys Glu Asp Lys Phe
                270                 275                 280

cca cgg ttc tgg aag gag gtg gtg gaa caa ggg ctg ctg aag atg cag 916
Pro Arg Phe Trp Lys Glu Val Val Glu Gln Gly Leu Leu Lys Met Gln
            285                 290                 295

ttc tgg cca gcc agc tac ctg tgt ttc cac ctg ctg ggc gcg gcc ctg 964
Phe Trp Pro Ala Ser Tyr Leu Cys Phe His Leu Leu Gly Ala Ala Leu
        300                 305                 310

ccc ctg ctg acc aag gag cag ctg cac ctg gtg atg cag gga gac gtg 1012
Pro Leu Leu Thr Lys Glu Gln Leu His Leu Val Met Gln Gly Asp Val
    315                 320                 325

atc cgc cat tac ggg gag cac gtg tgc act gct aag ctc cca aag cag 1060
Ile Arg His Tyr Gly Glu His Val Cys Thr Ala Lys Leu Pro Lys Gln
330                 335                 340                 345

ttc aag ttt gcc cca gag atg gac gat tac gtg ggc acc ttc cta gag 1108
Phe Lys Phe Ala Pro Glu Met Asp Asp Tyr Val Gly Thr Phe Leu Glu
                350                 355                 360

ggg tgc cag gat gac cct gag cgg cag ctg gcc gtg cta gtg gcc ttc 1156
Gly Cys Gln Asp Asp Pro Glu Arg Gln Leu Ala Val Leu Val Ala Phe
            365                 370                 375

tca tct gtc acc aac caa ggc ctc cct gtc acg cct act ttc tgg cgg 1204
Ser Ser Val Thr Asn Gln Gly Leu Pro Val Thr Pro Thr Phe Trp Arg
        380                 385                 390

gtc gtg cgg ttc ctg agc cct ccg gcc ctg cag ggc tat gtg gcc tgg 1252
Val Val Arg Phe Leu Ser Pro Pro Ala Leu Gln Gly Tyr Val Ala Trp
    395                 400                 405

ctg cgg gcc atg ttt ctc cag cca gac ctg gac tcc ttg gtt gac ttc 1300
Leu Arg Ala Met Phe Leu Gln Pro Asp Leu Asp Ser Leu Val Asp Phe
410                 415                 420                 425

agc acc aac aac cag aag aaa gcc cag gat tca tcg ctc cac atg cct 1348
Ser Thr Asn Asn Gln Lys Lys Ala Gln Asp Ser Ser Leu His Met Pro
                430                 435                 440

gag cga gct gtg ttc cgg ctg agg aaa tgg atc atc ttt cga ttg gtg 1396
Glu Arg Ala Val Phe Arg Leu Arg Lys Trp Ile Ile Phe Arg Leu Val
            445                 450                 455

agc att gtg gac agc ctg cac ctg gag atg gag gag gcc ttg act gag 1444
Ser Ile Val Asp Ser Leu His Leu Glu Met Glu Glu Ala Leu Thr Glu
        460                 465                 470

cag gtg gcc agg ttt tgt ttg ttc cac tcg ttc ttt gtc aca aag aag 1492
Gln Val Ala Arg Phe Cys Leu Phe His Ser Phe Phe Val Thr Lys Lys
    475                 480                 485

ccc aca tcc cag atc cct gag aca aag cac ccg ttc tcc ttc cct ttg 1540
Pro Thr Ser Gln Ile Pro Glu Thr Lys His Pro Phe Ser Phe Pro Leu
490                 495                 500                 505

gaa aac cag gcc cga gag gct gtc agc agt gcc ttc ttc agt ctg ttg 1588
Glu Asn Gln Ala Arg Glu Ala Val Ser Ser Ala Phe Phe Ser Leu Leu
                510                 515                 520

cag acc ctc agc acg cag ttc aag cag gca ccg ggc cag acc cag ggt 1636
Gln Thr Leu Ser Thr Gln Phe Lys Gln Ala Pro Gly Gln Thr Gln Gly
            525                 530                 535

ggg cag ccc tgg acc tac cac ctg gtg caa ttc gca gac ctc ctg ttg 1684

Gly Gln Pro Trp Thr Tyr His Leu Val Gln Phe Ala Asp Leu Leu Leu
        540                 545                 550

aat cac agc cac aac gtg acc acc gtg aca ccc ttc act gcg cag cag 1732
Asn His Ser His Asn Val Thr Thr Val Thr Pro Phe Thr Ala Gln Gln
    555                 560                 565

cac cag gcc tgg gac cgg atg ctg cag act ctg aag gag ctg gag gcc 1780
His Gln Ala Trp Asp Arg Met Leu Gln Thr Leu Lys Glu Leu Glu Ala
570                 575                 580                 585

cac tcc gca gag gcc agg gct gct gcc ttc cag cac ctt ctg ctc ttc 1828
His Ser Ala Glu Ala Arg Ala Ala Ala Phe Gln His Leu Leu Leu Phe
                590                 595                 600

gtg ggc atc cac ctc ctc aag tcc cct gca gag agc tgt gac ctg ctg 1876
Val Gly Ile His Leu Leu Lys Ser Pro Ala Glu Ser Cys Asp Leu Leu
            605                 610                 615

ggt gac atc cag acc tgc atc agg aaa agt ctg gga gag aag ccc cgc 1924
Gly Asp Ile Gln Thr Cys Ile Arg Lys Ser Leu Gly Glu Lys Pro Arg
        620                 625                 630

cgg agc cgc acc aag acc atc gac ccc cag gaa ccc ccg tgg gta gag 1972
Arg Ser Arg Thr Lys Thr Ile Asp Pro Gln Glu Pro Pro Trp Val Glu
    635                 640                 645

gtg ctg gtg gag atc ttg ctg gcc ctg ttg gcc cag ccc agc cac ctc 2020
Val Leu Val Glu Ile Leu Leu Ala Leu Leu Ala Gln Pro Ser His Leu
650                 655                 660                 665

atg cgc cag gtg gcc cgg agc gtg ttt ggc cac atc tgc tcc cac ctg 2068
Met Arg Gln Val Ala Arg Ser Val Phe Gly His Ile Cys Ser His Leu
                670                 675                 680

acc ccg cgt gcc ctg cag cta att ctg gat gtg ctg aac ccc gag acc 2116
Thr Pro Arg Ala Leu Gln Leu Ile Leu Asp Val Leu Asn Pro Glu Thr
            685                 690                 695

agt gag gat gag aat gac cgt gtg gtg gtg acg gac gat tct gat gag 2164
Ser Glu Asp Glu Asn Asp Arg Val Val Val Thr Asp Asp Ser Asp Glu
        700                 705                 710

cgg cgg ctg aag ggt gca gag gac aag agc gag gaa ggt gag gac aac 2212
Arg Arg Leu Lys Gly Ala Glu Asp Lys Ser Glu Glu Gly Glu Asp Asn

715                 720                 725

aga agc tca gag agt gaa gag gag agc gag ggg gag gag agc gag gag        2260
Arg Ser Ser Glu Ser Glu Glu Glu Ser Glu Gly Glu Glu Ser Glu Glu
730                 735                 740                 745

gag gag cgc gac ggg gac gtg gat cag ggc ttc cgg gaa cag ctg atg        2308
Glu Glu Arg Asp Gly Asp Val Asp Gln Gly Phe Arg Glu Gln Leu Met
                750                 755                 760

acc gtg ctg cag gct ggg aag gcg ctg ggt gga gag gac agt gag aac        2356
Thr Val Leu Gln Ala Gly Lys Ala Leu Gly Gly Glu Asp Ser Glu Asn
            765                 770                 775

gag gag gag ctg ggg gat gag gcc atg atg gcc ctg gac cag agc ctc        2404
Glu Glu Glu Leu Gly Asp Glu Ala Met Met Ala Leu Asp Gln Ser Leu
        780                 785                 790

gcc agc ctc ttt gcc gag cag aag ctg cgt atc cag gcc cgg cga gac        2452
Ala Ser Leu Phe Ala Glu Gln Lys Leu Arg Ile Gln Ala Arg Arg Asp
    795                 800                 805

gag aag aac aag ctg cag aag gag aag gct ctg cgg cgc gac ttc cag        2500
Glu Lys Asn Lys Leu Gln Lys Glu Lys Ala Leu Arg Arg Asp Phe Gln
810                 815                 820                 825

atc cgg gtg ctg gac ctg gtg gag gtg cta gtg acc aag cag ccc gag        2548
Ile Arg Val Leu Asp Leu Val Glu Val Leu Val Thr Lys Gln Pro Glu
                830                 835                 840

aat gcc ctg gtc ctg gag ctg ctg gag ccg ctg ctg agc atc atc cgg        2596
Asn Ala Leu Val Leu Glu Leu Leu Glu Pro Leu Leu Ser Ile Ile Arg
            845                 850                 855

cgc agc ctg cgc agc agc agc tcc aaa cag gag cag gac ctt ctg cac        2644
Arg Ser Leu Arg Ser Ser Ser Ser Lys Gln Glu Gln Asp Leu Leu His
        860                 865                 870

aag acg gcg cgc atc ttc acg cat cac ctg tgc cgt gcc cgg cgc tac        2692
Lys Thr Ala Arg Ile Phe Thr His His Leu Cys Arg Ala Arg Arg Tyr
    875                 880                 885

tgc cac gac ttg ggt gag cgc gca ggg gcc ctg cac gcc cag gtg gag        2740
Cys His Asp Leu Gly Glu Arg Ala Gly Ala Leu His Ala Gln Val Glu
890                 895                 900                 905

cgg ttg gtg cag cag gct ggc cgc cag ccc gac tcc ccc acc gcc ctc 2788
Arg Leu Val Gln Gln Ala Gly Arg Gln Pro Asp Ser Pro Thr Ala Leu
                910                 915                 920

tac cac ttc aac gcc tct ctc tac ctg ctc cgg gtc ttg aag ggc aac 2836
Tyr His Phe Asn Ala Ser Leu Tyr Leu Leu Arg Val Leu Lys Gly Asn
            925                 930                 935

act gct gag ggc tgc gtg cat gag aca cag gag aag cag aaa gct ggc 2884
Thr Ala Glu Gly Cys Val His Glu Thr Gln Glu Lys Gln Lys Ala Gly
        940                 945                 950

act gac ccc agc cac atg ccc acg ggc ccg cag gct gcc agc tgc ttg 2932
Thr Asp Pro Ser His Met Pro Thr Gly Pro Gln Ala Ala Ser Cys Leu
    955                 960                 965

gac ttg aac ctg gtg acc cgg gtg tac tcg aca gca ctg agc tcc ttc 2980
Asp Leu Asn Leu Val Thr Arg Val Tyr Ser Thr Ala Leu Ser Ser Phe
970                 975                 980                 985

ctg acc aag cgc aac agc ccc ctc aca gtt ccc atg ttc ctc agc ctc 3028
Leu Thr Lys Arg Asn Ser Pro Leu Thr Val Pro Met Phe Leu Ser Leu
                990                 995                 1000

ttc tcc cgg cac ccg gtg ctc tgt cag agc ctg ctc ccc atc ctg 3073
Phe Ser Arg His Pro Val Leu Cys Gln Ser Leu Leu Pro Ile Leu
            1005                1010                1015

gtc cag cat atc acg ggc ccg gtg cgg ccc cgt cat cag gcc tgc 3118
Val Gln His Ile Thr Gly Pro Val Arg Pro Arg His Gln Ala Cys
            1020                1025                1030

ctg ctg ctc cag aag acc ctg tcc atg cgg gag gtg agg tcg tgc 3163
Leu Leu Leu Gln Lys Thr Leu Ser Met Arg Glu Val Arg Ser Cys
            1035                1040                1045

ttt gag gac ccc gag tgg aag cag ctg atg ggc cag gtc cta gca 3208
Phe Glu Asp Pro Glu Trp Lys Gln Leu Met Gly Gln Val Leu Ala
            1050                1055                1060

aag gtc acc gag aac ttg cgc gtg ctg ggg gag gcg cag acc aag 3253
Lys Val Thr Glu Asn Leu Arg Val Leu Gly Glu Ala Gln Thr Lys
            1065                1070                1075

gcg cag cat cag cag gca ctg tcc tcc ctg gag ctg ctc aac gtt 3298
Ala Gln His Gln Gln Ala Leu Ser Ser Leu Glu Leu Leu Asn Val
            1080                1085                1090

ctc ttc agg acc tgc aaa cat gag aag ctg acc ttg gac ctg acg 3343
Leu Phe Arg Thr Cys Lys His Glu Lys Leu Thr Leu Asp Leu Thr
            1095                1100                1105

gtg ctc ctg ggt gtg ctg cag ggg caa cag cag agc cta cag cag 3388
Val Leu Leu Gly Val Leu Gln Gly Gln Gln Gln Ser Leu Gln Gln
            1110                1115                1120

ggg gca cac tcc acc ggc tcc agc cgc ctg cac gac ctc tac tgg 3433
Gly Ala His Ser Thr Gly Ser Ser Arg Leu His Asp Leu Tyr Trp
            1125                1130                1135

cag gcc atg aaa acc ctg gga gtc cag cgc ccc aag ttg gag aag 3478
Gln Ala Met Lys Thr Leu Gly Val Gln Arg Pro Lys Leu Glu Lys
            1140                1145                1150

aag gat gcc aag gag atc ccc agt gcc acc cag agc ccc atc agt 3523
Lys Asp Ala Lys Glu Ile Pro Ser Ala Thr Gln Ser Pro Ile Ser
            1155                1160                1165

aag aag cgg aag aaa aag gga ttc ttg cca gag acg aag aag cgc 3568
Lys Lys Arg Lys Lys Lys Gly Phe Leu Pro Glu Thr Lys Lys Arg
            1170                1175                1180

aag aaa cgc aag tca gag gat ggc acg cca gcg gag gat ggc aca 3613
Lys Lys Arg Lys Ser Glu Asp Gly Thr Pro Ala Glu Asp Gly Thr
            1185                1190                1195

cct gca gcc acc ggc ggg agc cag ccc ccc agc atg ggc agg aag 3658
Pro Ala Ala Thr Gly Gly Ser Gln Pro Pro Ser Met Gly Arg Lys
            1200                1205                1210

aag agg aac agg aca aag gct aag gtc cca gcc cag gca aac ggg 3703
Lys Arg Asn Arg Thr Lys Ala Lys Val Pro Ala Gln Ala Asn Gly
            1215                1220                1225

acg cca acc acc aag agt cca gcc cct ggc gcc ccc acc cgg agc 3748
Thr Pro Thr Thr Lys Ser Pro Ala Pro Gly Ala Pro Thr Arg Ser
            1230                1235                1240

ccc agc acc cct gcc aaa tcc cca aaa ctg cag aag aaa aac cag 3793

Pro Ser Thr Pro Ala Lys Ser Pro Lys Leu Gln Lys Lys Asn Gln
            1245                1250                1255

aag ccg tcc cag gtg aat gga gct ccc ggg tcc ccc acg gaa cct 3838
Lys Pro Ser Gln Val Asn Gly Ala Pro Gly Ser Pro Thr Glu Pro
            1260                1265                1270

gca ggc caa aag cag cat cag aag gct ctt ccc aaa aag ggg gtc 3883
Ala Gly Gln Lys Gln His Gln Lys Ala Leu Pro Lys Lys Gly Val
            1275                1280                1285

ttg ggc aaa tca cca ctg tcc gcg ctg gca cgg aaa aag gca agg 3928
Leu Gly Lys Ser Pro Leu Ser Ala Leu Ala Arg Lys Lys Ala Arg
            1290                1295                1300

ctg tct ttg gtc atc agg agt ccc agc ctg ctt cag agt ggg gcc 3973
Leu Ser Leu Val Ile Arg Ser Pro Ser Leu Leu Gln Ser Gly Ala
            1305                1310                1315

aag aag aaa gca cag gtg agg aag gca ggg aag ccc tga gcacaggtac 4022
Lys Lys Lys Ala Gln Val Arg Lys Ala Gly Lys Pro
            1320                1325

gggcccccct cagcccctgc ctccatctgc ctgagacgcc tatttttttt tttttaaaac 4082

catgatttta atacgcaagc tgtttctaag gcgctgccac tggggagggt ggctgttgcc 4142

gcctgcccgg gcatcctgct ctggcaagca cagcctgagc cattcctgcg ggggtcccag 4202

ggtgcagaga cctccccacc cccagttctg ggctgggatc ctggctccag ggccatgtcc 4262

agggctctgg tgtttgcctg ggttggtgca tgttgatgtg ctggctgcag gcaggtgtga 4322

ccatctctcg tgcctgccac ctctttgccc ccaggctttt ttgctgtgag ggagccacca 4382

gggggtgatt taaataggtt tatttcttca tttacaagag gaatatattt ggcttctctc 4442

ttaagactct gagattcaca atcagcagct ctaaaaaata aaggagcagt ttggcttccg 4502

gaaggaagag gaggctaaaa aaaaaaaaaa aaaaaa 4538

<210> 34
<211> 1328
<212> PRT

<213>  human

<400>  34

Met Glu Ser Arg Asp Pro Ala Gln Pro Met Ser Pro Gly Glu Ala Thr
1               5                   10                  15

Gln Ser Gly Ala Arg Pro Ala Asp Arg Tyr Gly Leu Leu Lys His Ser
            20                  25                  30

Arg Glu Phe Leu Asp Phe Phe Trp Asp Ile Ala Lys Pro Glu Gln Glu
        35                  40                  45

Thr Arg Leu Ala Ala Thr Glu Lys Leu Leu Glu Tyr Leu Arg Gly Arg
    50                  55                  60

Pro Lys Gly Ser Glu Met Lys Tyr Ala Leu Lys Arg Leu Ile Thr Gly
65                  70                  75                  80

Leu Gly Val Gly Arg Glu Thr Ala Arg Pro Cys Tyr Ser Leu Ala Leu
                85                  90                  95

Ala Gln Leu Leu Gln Ser Phe Glu Asp Leu Pro Leu Cys Ser Ile Leu
            100                 105                 110

Gln Gln Ile Gln Glu Lys Tyr Asp Leu His Gln Val Lys Lys Ala Met
        115                 120                 125

Leu Arg Pro Ala Leu Phe Ala Asn Leu Phe Gly Val Leu Ala Leu Phe
    130                 135                 140

Gln Ser Gly Arg Leu Val Lys Asp Gln Glu Ala Leu Met Lys Ser Val
145                 150                 155                 160

Lys Leu Leu Gln Ala Leu Ala Gln Tyr Gln Asn His Leu Gln Glu Gln

                165                 170                 175

Pro Arg Lys Ala Leu Val Asp Ile Leu Ser Glu Val Ser Lys Ala Thr
            180                 185                 190

Leu Gln Glu Ile Leu Pro Glu Val Leu Lys Ala Asp Leu Asn Ile Ile
        195                 200                 205

Leu Ser Ser Pro Glu Gln Leu Glu Leu Phe Leu Leu Ala Gln Gln Lys
    210                 215                 220

Val Pro Ser Lys Leu Lys Lys Leu Val Gly Ser Val Asn Leu Phe Ser
225                 230                 235                 240

Asp Glu Asn Val Pro Arg Leu Val Asn Val Leu Lys Met Ala Ala Ser
                245                 250                 255

Ser Val Lys Lys Asp Arg Lys Leu Pro Ala Ile Ala Leu Asp Leu Leu
            260                 265                 270

Arg Leu Ala Leu Lys Glu Asp Lys Phe Pro Arg Phe Trp Lys Glu Val
        275                 280                 285

Val Glu Gln Gly Leu Leu Lys Met Gln Phe Trp Pro Ala Ser Tyr Leu
    290                 295                 300

Cys Phe His Leu Leu Gly Ala Ala Leu Pro Leu Leu Thr Lys Glu Gln
305                 310                 315                 320

Leu His Leu Val Met Gln Gly Asp Val Ile Arg His Tyr Gly Glu His
                325                 330                 335

Val Cys Thr Ala Lys Leu Pro Lys Gln Phe Lys Phe Ala Pro Glu Met
            340                 345                 350

Asp Asp Tyr Val Gly Thr Phe Leu Glu Gly Cys Gln Asp Asp Pro Glu
        355                 360                 365

Arg Gln Leu Ala Val Leu Val Ala Phe Ser Ser Val Thr Asn Gln Gly
    370                 375                 380

Leu Pro Val Thr Pro Thr Phe Trp Arg Val Val Arg Phe Leu Ser Pro
385                 390                 395                 400

Pro Ala Leu Gln Gly Tyr Val Ala Trp Leu Arg Ala Met Phe Leu Gln
                405                 410                 415

Pro Asp Leu Asp Ser Leu Val Asp Phe Ser Thr Asn Asn Gln Lys Lys
            420                 425                 430

Ala Gln Asp Ser Ser Leu His Met Pro Glu Arg Ala Val Phe Arg Leu
        435                 440                 445

Arg Lys Trp Ile Ile Phe Arg Leu Val Ser Ile Val Asp Ser Leu His
    450                 455                 460

Leu Glu Met Glu Glu Ala Leu Thr Glu Gln Val Ala Arg Phe Cys Leu
465                 470                 475                 480

Phe His Ser Phe Phe Val Thr Lys Lys Pro Thr Ser Gln Ile Pro Glu
                485                 490                 495

Thr Lys His Pro Phe Ser Phe Pro Leu Glu Asn Gln Ala Arg Glu Ala
            500                 505                 510

Val Ser Ser Ala Phe Phe Ser Leu Leu Gln Thr Leu Ser Thr Gln Phe
        515                 520                 525

Lys Gln Ala Pro Gly Gln Thr Gln Gly Gly Gln Pro Trp Thr Tyr His
    530                 535                 540

Leu Val Gln Phe Ala Asp Leu Leu Leu Asn His Ser His Asn Val Thr
545                 550                 555                 560

Thr Val Thr Pro Phe Thr Ala Gln Gln His Gln Ala Trp Asp Arg Met
                565                 570                 575

Leu Gln Thr Leu Lys Glu Leu Glu Ala His Ser Ala Glu Ala Arg Ala
            580                 585                 590

Ala Ala Phe Gln His Leu Leu Leu Phe Val Gly Ile His Leu Leu Lys
        595                 600                 605

Ser Pro Ala Glu Ser Cys Asp Leu Leu Gly Asp Ile Gln Thr Cys Ile
    610                 615                 620

Arg Lys Ser Leu Gly Glu Lys Pro Arg Arg Ser Arg Thr Lys Thr Ile
625                 630                 635                 640

Asp Pro Gln Glu Pro Pro Trp Val Glu Val Leu Val Glu Ile Leu Leu
                645                 650                 655

Ala Leu Leu Ala Gln Pro Ser His Leu Met Arg Gln Val Ala Arg Ser
            660                 665                 670

Val Phe Gly His Ile Cys Ser His Leu Thr Pro Arg Ala Leu Gln Leu
        675                 680                 685

Ile Leu Asp Val Leu Asn Pro Glu Thr Ser Glu Asp Glu Asn Asp Arg
    690                 695                 700

Val Val Val Thr Asp Asp Ser Asp Glu Arg Arg Leu Lys Gly Ala Glu
705                 710                 715                 720

Asp Lys Ser Glu Glu Gly Glu Asp Asn Arg Ser Ser Glu Ser Glu Glu
                725                 730                 735

Glu Ser Glu Gly Glu Glu Ser Glu Glu Glu Glu Arg Asp Gly Asp Val
            740                 745                 750

Asp Gln Gly Phe Arg Glu Gln Leu Met Thr Val Leu Gln Ala Gly Lys
        755                 760                 765

Ala Leu Gly Gly Glu Asp Ser Glu Asn Glu Glu Glu Leu Gly Asp Glu
    770                 775                 780

Ala Met Met Ala Leu Asp Gln Ser Leu Ala Ser Leu Phe Ala Glu Gln
785                 790                 795                 800

Lys Leu Arg Ile Gln Ala Arg Arg Asp Glu Lys Asn Lys Leu Gln Lys
                805                 810                 815

Glu Lys Ala Leu Arg Arg Asp Phe Gln Ile Arg Val Leu Asp Leu Val
            820                 825                 830

Glu Val Leu Val Thr Lys Gln Pro Glu Asn Ala Leu Val Leu Glu Leu
        835                 840                 845

Leu Glu Pro Leu Leu Ser Ile Ile Arg Arg Ser Leu Arg Ser Ser Ser
    850                 855                 860

Ser Lys Gln Glu Gln Asp Leu Leu His Lys Thr Ala Arg Ile Phe Thr
865                 870                 875                 880

His His Leu Cys Arg Ala Arg Arg Tyr Cys His Asp Leu Gly Glu Arg

                885                 890                 895

Ala Gly Ala Leu His Ala Gln Val Glu Arg Leu Val Gln Gln Ala Gly
            900                 905                 910

Arg Gln Pro Asp Ser Pro Thr Ala Leu Tyr His Phe Asn Ala Ser Leu
        915                 920                 925

Tyr Leu Leu Arg Val Leu Lys Gly Asn Thr Ala Glu Gly Cys Val His
    930                 935                 940

Glu Thr Gln Glu Lys Gln Lys Ala Gly Thr Asp Pro Ser His Met Pro
945                 950                 955                 960

Thr Gly Pro Gln Ala Ala Ser Cys Leu Asp Leu Asn Leu Val Thr Arg
                965                 970                 975

Val Tyr Ser Thr Ala Leu Ser Ser Phe Leu Thr Lys Arg Asn Ser Pro
            980                 985                 990

Leu Thr Val Pro Met Phe Leu Ser Leu Phe Ser Arg His Pro Val Leu
        995                 1000                1005

Cys Gln Ser Leu Leu Pro Ile Leu Val Gln His Ile Thr Gly Pro
    1010                1015                1020

Val Arg Pro Arg His Gln Ala Cys Leu Leu Leu Gln Lys Thr Leu
    1025                1030                1035

Ser Met Arg Glu Val Arg Ser Cys Phe Glu Asp Pro Glu Trp Lys
    1040                1045                1050

Gln Leu Met Gly Gln Val Leu Ala Lys Val Thr Glu Asn Leu Arg
    1055                1060                1065

Val Leu Gly Glu Ala Gln Thr Lys Ala Gln His Gln Gln Ala Leu
    1070                1075                1080

Ser Ser Leu Glu Leu Leu Asn Val Leu Phe Arg Thr Cys Lys His
    1085                1090                1095

Glu Lys Leu Thr Leu Asp Leu Thr Val Leu Leu Gly Val Leu Gln
    1100                1105                1110

Gly Gln Gln Gln Ser Leu Gln Gln Gly Ala His Ser Thr Gly Ser
    1115                1120                1125

Ser Arg Leu His Asp Leu Tyr Trp Gln Ala Met Lys Thr Leu Gly
    1130                1135                1140

Val Gln Arg Pro Lys Leu Glu Lys Lys Asp Ala Lys Glu Ile Pro
    1145                1150                1155

Ser Ala Thr Gln Ser Pro Ile Ser Lys Lys Arg Lys Lys Lys Gly
    1160                1165                1170

Phe Leu Pro Glu Thr Lys Lys Arg Lys Lys Arg Lys Ser Glu Asp
    1175                1180                1185

Gly Thr Pro Ala Glu Asp Gly Thr Pro Ala Ala Thr Gly Gly Ser
    1190                1195                1200

Gln Pro Pro Ser Met Gly Arg Lys Lys Arg Asn Arg Thr Lys Ala
    1205                1210                1215

Lys Val Pro Ala Gln Ala Asn Gly Thr Pro Thr Thr Lys Ser Pro
    1220                1225                1230

Ala Pro Gly Ala Pro Thr Arg Ser Pro Ser Thr Pro Ala Lys Ser
    1235                1240                1245

Pro Lys Leu Gln Lys Lys Asn Gln Lys Pro Ser Gln Val Asn Gly
    1250                1255                1260

Ala Pro Gly Ser Pro Thr Glu Pro Ala Gly Gln Lys Gln His Gln
    1265                1270                1275

Lys Ala Leu Pro Lys Lys Gly Val Leu Gly Lys Ser Pro Leu Ser
    1280                1285                1290

Ala Leu Ala Arg Lys Lys Ala Arg Leu Ser Leu Val Ile Arg Ser
    1295                1300                1305

Pro Ser Leu Leu Gln Ser Gly Ala Lys Lys Lys Ala Gln Val Arg
    1310                1315                1320

Lys Ala Gly Lys Pro
    1325

<210>  35
<211>  2427
<212>  DNA
<213>  human

<220>
<221>  CDS
<222>  (86)..(1681)
<223>

<400>  35
gggatttgtt attgcgaatg atgtggacaa caagcgctgc tacctgctcg tccatcaagc      60

caagaggctg agcagcccct gcatc atg gtg gtc aac cat gat gcc tcc agc      112
                            Met Val Val Asn His Asp Ala Ser Ser
                            1               5

ata ccc agg ctc cag ata gat gtg gac ggc agg aaa gag atc ctc ttc 160
Ile Pro Arg Leu Gln Ile Asp Val Asp Gly Arg Lys Glu Ile Leu Phe
10                  15                  20                  25

tat gat cga att tta tgt gat gtc cct tgc agt gga gac ggc act atg 208
Tyr Asp Arg Ile Leu Cys Asp Val Pro Cys Ser Gly Asp Gly Thr Met
                30                  35                  40

aga aaa aac att gat gtt tgg aaa aag tgg acc acc tta aat agc ttg 256
Arg Lys Asn Ile Asp Val Trp Lys Lys Trp Thr Thr Leu Asn Ser Leu
            45                  50                  55

cag cta cat ggc tta cag ctg cgg att gca aca cgc ggg gct gaa cag 304
Gln Leu His Gly Leu Gln Leu Arg Ile Ala Thr Arg Gly Ala Glu Gln
        60                  65                  70

ctg gct gaa ggt gga agg gtg gtg tat tcc acg tgt tca cta aac cct 352
Leu Ala Glu Gly Gly Arg Val Val Tyr Ser Thr Cys Ser Leu Asn Pro
    75                  80                  85

att gag gat gaa gca gtc ata gca tct tta ctg gaa aaa agt gaa ggt 400
Ile Glu Asp Glu Ala Val Ile Ala Ser Leu Leu Glu Lys Ser Glu Gly
90                  95                  100                 105

gct ttg gag ctt gct gat gtg tct aat gaa ctg cca ggg ctg aag tgg 448
Ala Leu Glu Leu Ala Asp Val Ser Asn Glu Leu Pro Gly Leu Lys Trp
                110                 115                 120

atg cct gga atc aca cag tgg aag gta atg acg aaa gat ggg cag tgg 496
Met Pro Gly Ile Thr Gln Trp Lys Val Met Thr Lys Asp Gly Gln Trp
            125                 130                 135

ttt aca gac tgg gac gct gtt cct cac agc aga cac acc cag atc cga 544
Phe Thr Asp Trp Asp Ala Val Pro His Ser Arg His Thr Gln Ile Arg
        140                 145                 150

cct acc atg ttc cct ccg aag gac cca gaa aag ctg cag gcc atg cac 592
Pro Thr Met Phe Pro Pro Lys Asp Pro Glu Lys Leu Gln Ala Met His
    155                 160                 165

ctg gag cga tgc ctt agg ata tta ccc cat cat cag aat act gga ggg 640
Leu Glu Arg Cys Leu Arg Ile Leu Pro His His Gln Asn Thr Gly Gly
170                 175                 180                 185

ttt ttt gtg gca gta ttg gtg aaa aaa tct tca atg ccg tgg aat aaa 688
Phe Phe Val Ala Val Leu Val Lys Lys Ser Ser Met Pro Trp Asn Lys
                190                 195                 200

cgt cag cca aag ctt cag ggt aaa tct gca gag acc aga gaa agc aca 736
Arg Gln Pro Lys Leu Gln Gly Lys Ser Ala Glu Thr Arg Glu Ser Thr
            205                 210                 215

cag ctg agc cct gca gat ctc aca gaa ggg aaa ccc aca gat ccc tct 784
Gln Leu Ser Pro Ala Asp Leu Thr Glu Gly Lys Pro Thr Asp Pro Ser
        220                 225                 230

aag ctg gaa agt ccg tca ttc aca gga act ggt gac aca gaa ata gct 832
Lys Leu Glu Ser Pro Ser Phe Thr Gly Thr Gly Asp Thr Glu Ile Ala
    235                 240                 245

cat gca act gag gat tta gag aat aat ggc agt aag aaa gat ggc gtg 880
His Ala Thr Glu Asp Leu Glu Asn Asn Gly Ser Lys Lys Asp Gly Val
250                 255                 260                 265

tgt ggt cct cct cca tca aag aaa atg aag tta ttt gga ttt aaa gaa 928
Cys Gly Pro Pro Pro Ser Lys Lys Met Lys Leu Phe Gly Phe Lys Glu
                270                 275                 280

gat cca ttt gta ttt att cct gaa gat gac cca tta ttt cca cct att 976
Asp Pro Phe Val Phe Ile Pro Glu Asp Asp Pro Leu Phe Pro Pro Ile
            285                 290                 295

gag aaa ttt tat gct ttg gat cct tca ttc cca agg atg aat ttg tta 1024
Glu Lys Phe Tyr Ala Leu Asp Pro Ser Phe Pro Arg Met Asn Leu Leu
        300                 305                 310

act cgg act aca gaa ggg aag aaa agg cag ctc tac atg gtt tct aag 1072
Thr Arg Thr Thr Glu Gly Lys Lys Arg Gln Leu Tyr Met Val Ser Lys
    315                 320                 325

gag ttg cgg aat gtg ctg ctg aat aac agt gag aag atg aag gtt att 1120
Glu Leu Arg Asn Val Leu Leu Asn Asn Ser Glu Lys Met Lys Val Ile
330                 335                 340                 345

aac acg ggg atc aaa gtc tgg tgt aga aat aac agc ggt gaa gag ttt 1168
Asn Thr Gly Ile Lys Val Trp Cys Arg Asn Asn Ser Gly Glu Glu Phe
                350                 355                 360

gac tgt gct ttc cgg ctg gca cag gag gga ata tat aca ttg tat cca 1216

Asp Cys Ala Phe Arg Leu Ala Gln Glu Gly Ile Tyr Thr Leu Tyr Pro
            365                 370                 375

ttt att aac tca aga att att act gta tca atg gaa gat gtt aag ata      1264
Phe Ile Asn Ser Arg Ile Ile Thr Val Ser Met Glu Asp Val Lys Ile
        380                 385                 390

ctg ttg acc cag gaa aat ccc ttt ttt aga aaa ctc agc agt gag acc      1312
Leu Leu Thr Gln Glu Asn Pro Phe Phe Arg Lys Leu Ser Ser Glu Thr
    395                 400                 405

tac agt caa gca aag gac ctg gca aag gga agc atc gtg ctg aag tat      1360
Tyr Ser Gln Ala Lys Asp Leu Ala Lys Gly Ser Ile Val Leu Lys Tyr
410                 415                 420                 425

gaa cca gat tct gcg aat cca gac gct cta cag tgt ccc atc gtc tta      1408
Glu Pro Asp Ser Ala Asn Pro Asp Ala Leu Gln Cys Pro Ile Val Leu
                430                 435                 440

tgc gga tgg cgg gga aag gcc tcc att cga act ttt gtg ccc aag aat      1456
Cys Gly Trp Arg Gly Lys Ala Ser Ile Arg Thr Phe Val Pro Lys Asn
            445                 450                 455

gaa cgg ctt cac tat ctc agg atg atg ggg ctg gag gta ttg gga gaa      1504
Glu Arg Leu His Tyr Leu Arg Met Met Gly Leu Glu Val Leu Gly Glu
        460                 465                 470

aag aag aag gaa ggg gtt atc ctc aca aat gag agt gca gcc agc acc      1552
Lys Lys Lys Glu Gly Val Ile Leu Thr Asn Glu Ser Ala Ala Ser Thr
    475                 480                 485

gga cag cca gac aat gac gtg act gag gga cag aga gca gga gag ccc      1600
Gly Gln Pro Asp Asn Asp Val Thr Glu Gly Gln Arg Ala Gly Glu Pro
490                 495                 500                 505

aac agc cca gat gca gaa gag gcc aac agt cca gac gtg aca gca ggc      1648
Asn Ser Pro Asp Ala Glu Glu Ala Asn Ser Pro Asp Val Thr Ala Gly
                510                 515                 520

tgt gac ccg gcg ggg gtc cat cca ccc cgg tga gcaggcccaa ggcagcgggg      1701
Cys Asp Pro Ala Gly Val His Pro Pro Arg
            525                 530

gcccacaccc ctcacacgca aaactggctt cttctggtca ctggtgtctg aaaccaaatc      1761

cagagcagcc tgtggcctgt aaagcatata tttctagtga ctgcagactg gtgggatcat 1821

aggagccttc tgaatgacca ggactgcttt ctttggagct gatgaaaatg tactctttta 1881

gcgtgttaga aatcacttgt tttattttgt tttctttggc caagctgggt ctagtgtttc 1941

ttttgctggg aatagacttt caaaagttgt acttctatca agaaacaaaa ctgcccttgc 2001

agaaatttca ggtcttttgt taagcctgta ttggtcttaa ggtgcagtat tttttaaatt 2061

attatttata gaaagaatct ataaattctt ggggaagtgt gttataagct ttaataatta 2121

cattgagctg cacctcagtg gtgtgtcatt aacatgcagt ggggttaata tctgaggcct 2181

cagatgactt tgtgcctttt ggaataaagg gtaaaataaa ctctcccaga gtaagagctg 2241

tatcgtgaat tgtcatacta attattgagg gggacttatg tgcttttatt gaatggagtg 2301

ctttacaatt tttattttta aatggggttg ggatccttgg aatatttcaa taaaattgat 2361

aaaatataaa aaaaaaaaaa aaaaaaaaaa aaaaaaaaaa aaaaaaaaaa aaaaaaaaaa 2421

aaaaaa 2427

<210> 36
<211> 531
<212> PRT
<213> human

<400> 36

Met Val Val Asn His Asp Ala Ser Ser Ile Pro Arg Leu Gln Ile Asp
1               5                   10                  15

Val Asp Gly Arg Lys Glu Ile Leu Phe Tyr Asp Arg Ile Leu Cys Asp
            20                  25                  30

Val Pro Cys Ser Gly Asp Gly Thr Met Arg Lys Asn Ile Asp Val Trp
        35                  40                  45

Lys Lys Trp Thr Thr Leu Asn Ser Leu Gln Leu His Gly Leu Gln Leu

50                  55                  60

Arg Ile Ala Thr Arg Gly Ala Glu Gln Leu Ala Glu Gly Gly Arg Val
65                  70                  75                  80

Val Tyr Ser Thr Cys Ser Leu Asn Pro Ile Glu Asp Glu Ala Val Ile
                85                  90                  95

Ala Ser Leu Leu Glu Lys Ser Glu Gly Ala Leu Glu Leu Ala Asp Val
            100                 105                 110

Ser Asn Glu Leu Pro Gly Leu Lys Trp Met Pro Gly Ile Thr Gln Trp
        115                 120                 125

Lys Val Met Thr Lys Asp Gly Gln Trp Phe Thr Asp Trp Asp Ala Val
    130                 135                 140

Pro His Ser Arg His Thr Gln Ile Arg Pro Thr Met Phe Pro Pro Lys
145                 150                 155                 160

Asp Pro Glu Lys Leu Gln Ala Met His Leu Glu Arg Cys Leu Arg Ile
                165                 170                 175

Leu Pro His His Gln Asn Thr Gly Gly Phe Phe Val Ala Val Leu Val
            180                 185                 190

Lys Lys Ser Ser Met Pro Trp Asn Lys Arg Gln Pro Lys Leu Gln Gly
        195                 200                 205

Lys Ser Ala Glu Thr Arg Glu Ser Thr Gln Leu Ser Pro Ala Asp Leu
    210                 215                 220

Thr Glu Gly Lys Pro Thr Asp Pro Ser Lys Leu Glu Ser Pro Ser Phe
225                 230                 235                 240

Thr Gly Thr Gly Asp Thr Glu Ile Ala His Ala Thr Glu Asp Leu Glu
                245                 250                 255

Asn Asn Gly Ser Lys Lys Asp Gly Val Cys Gly Pro Pro Pro Ser Lys
            260                 265                 270

Lys Met Lys Leu Phe Gly Phe Lys Glu Asp Pro Phe Val Phe Ile Pro
        275                 280                 285

Glu Asp Asp Pro Leu Phe Pro Pro Ile Glu Lys Phe Tyr Ala Leu Asp
    290                 295                 300

Pro Ser Phe Pro Arg Met Asn Leu Leu Thr Arg Thr Thr Glu Gly Lys
305                 310                 315                 320

Lys Arg Gln Leu Tyr Met Val Ser Lys Glu Leu Arg Asn Val Leu Leu
                325                 330                 335

Asn Asn Ser Glu Lys Met Lys Val Ile Asn Thr Gly Ile Lys Val Trp
            340                 345                 350

Cys Arg Asn Asn Ser Gly Glu Glu Phe Asp Cys Ala Phe Arg Leu Ala
        355                 360                 365

Gln Glu Gly Ile Tyr Thr Leu Tyr Pro Phe Ile Asn Ser Arg Ile Ile
    370                 375                 380

Thr Val Ser Met Glu Asp Val Lys Ile Leu Leu Thr Gln Glu Asn Pro
385                 390                 395                 400

Phe Phe Arg Lys Leu Ser Ser Glu Thr Tyr Ser Gln Ala Lys Asp Leu
                405                 410                 415

Ala Lys Gly Ser Ile Val Leu Lys Tyr Glu Pro Asp Ser Ala Asn Pro
            420                 425                 430

Asp Ala Leu Gln Cys Pro Ile Val Leu Cys Gly Trp Arg Gly Lys Ala
        435                 440                 445

Ser Ile Arg Thr Phe Val Pro Lys Asn Glu Arg Leu His Tyr Leu Arg
    450                 455                 460

Met Met Gly Leu Glu Val Leu Gly Glu Lys Lys Lys Glu Gly Val Ile
465                 470                 475                 480

Leu Thr Asn Glu Ser Ala Ala Ser Thr Gly Gln Pro Asp Asn Asp Val
                485                 490                 495

Thr Glu Gly Gln Arg Ala Gly Glu Pro Asn Ser Pro Asp Ala Glu Glu
            500                 505                 510

Ala Asn Ser Pro Asp Val Thr Ala Gly Cys Asp Pro Ala Gly Val His
        515                 520                 525

Pro Pro Arg
    530

<210>  37
<211>  1792
<212>  DNA
<213>  human

<220>
<221>  CDS
<222>  (61)..(1281)
<223>

<400>  37
cttgagggga agaggctgac tgtacgttcc ttctactctg gcaccactct ccaggctgcc      60

atg ggg ccc agc acc cct ctc ctc atc ttg ttc ctt ttg tca tgg tcg 108
Met Gly Pro Ser Thr Pro Leu Leu Ile Leu Phe Leu Leu Ser Trp Ser
1               5                   10                  15

gga ccc ctc caa gga cag cag cac cac ctt gtg gag tac atg gaa cgc 156
Gly Pro Leu Gln Gly Gln Gln His His Leu Val Glu Tyr Met Glu Arg
            20                  25                  30

cga cta gct gct tta gag gaa cgg ctg gcc cag tgc cag gac cag agt 204
Arg Leu Ala Ala Leu Glu Glu Arg Leu Ala Gln Cys Gln Asp Gln Ser
        35                  40                  45

agt cgg cat gct gct gag ctg cgg gac ttc aag aac aag atg ctg cca 252
Ser Arg His Ala Ala Glu Leu Arg Asp Phe Lys Asn Lys Met Leu Pro
    50                  55                  60

ctg ctg gag gtg gca gag aag gag cgg gag gca ctc aga act gag gcc 300
Leu Leu Glu Val Ala Glu Lys Glu Arg Glu Ala Leu Arg Thr Glu Ala
65                  70                  75                  80

gac acc atc tcc ggg aga gtg gat cgt ctg gag cgg gag gta gac tat 348
Asp Thr Ile Ser Gly Arg Val Asp Arg Leu Glu Arg Glu Val Asp Tyr
                85                  90                  95

ctg gag acc cag aac cca gct ctg ccc tgt gta gag ttt gat gag aag 396
Leu Glu Thr Gln Asn Pro Ala Leu Pro Cys Val Glu Phe Asp Glu Lys
            100                 105                 110

gtg act gga ggc cct ggg acc aaa ggc aag gga aga agg aat gag aag 444
Val Thr Gly Gly Pro Gly Thr Lys Gly Lys Gly Arg Arg Asn Glu Lys
        115                 120                 125

tac gat atg gtg aca gac tgt ggc tac aca atc tct caa gtg aga tca 492
Tyr Asp Met Val Thr Asp Cys Gly Tyr Thr Ile Ser Gln Val Arg Ser
    130                 135                 140

atg aag att ctg aag cga ttt ggt ggc cca gct ggt cta tgg acc aag 540

145                 150                 155                 160

gat cca ctg ggg caa aca gag aag atc tac gtg tta gat ggg aca cag 588

                165                 170                 175

aat gac aca gcc ttt gtc ttc cca agg ctg cgt gac ttc acc ctt gcc 636

            180                 185                 190

atg gct gcc cgg aaa gct tcc cga gtc cgg gtg ccc ttc ccc tgg gta 684

        195                 200                 205

ggc aca ggg cag ctg gta tat ggt ggc ttt ctt tat ttt gct cgg agg 732

    210                 215                 220

cct cct gga aga cct ggt gga ggt ggt gag atg gag aac act ttg cag 780

225                 230                 235                 240

cta atc aaa ttc cac ctg gca aac cga aca gtg gtg gac agc tca gta 828

                245                 250                 255

ttc cca gca gag ggg ctg atc ccc ccc tac ggc ttg aca gca gac acc 876

            260                 265                 270

tac atc gac ctg gca gct gat gag gaa ggt ctt tgg gct gtc tat gcc 924

        275                 280                 285

acc cgg gag gat gac agg cac ttg tgt ctg gcc aag tta gat cca cag 972

    290                 295                 300

aca ctg gac aca gag cag cag tgg gac aca cca tgt ccc aga gag aat 1020

305                 310                 315                 320

gct gag gct gcc ttt gtc atc tgt ggg acc ctc tat gtc gtc tat aac 1068

                325                 330                 335

acc cgt cct gcc agt cgg gcc cgc atc cag tgc tcc ttt gat gcc agc 1116

            340                 345                 350

ggc acc ctg acc cct gaa cgg gca gca ctc cct tat ttt ccc cgc aga 1164


        355                 360                 365

tat ggt gcc cat gcc agc ctc cgc tat aac ccc cga gaa cgc cag ctc      1212

    370                 375                 380

tat gcc tgg gat gat ggc tac cag att gtc tat aag ctg gag atg agg      1260

385                 390                 395                 400

aag aaa gag gag gag gtt tga ggagctagcc ttgtttttg catctttctc      1311

                405

actcccatac atttatatta tatccccact aaatttcttg ttcctcattc ttcaaatgtg      1371

ggccagttgt ggctcaaatc ctctatattt ttagccaatg gcaatcaaat tctttcagct      1431

cctttgtttc atacggaact ccagatcctg agtaatcctt ttagagcccg aagagtcaaa      1491

accctcaatg ttccctcctg ctctcctgcc ccatgtcaac aaatttcagg ctaaggatgc      1551

cccagaccca gggctctaac cttgtatgcg ggcaggccca gggagcaggc agcagtgttc      1611

ttcccctcag agtgacttgg ggagggagaa ataggaggag acgtccagct ctgtcctctc      1671

ttcctcactc ctcccttcag tgtcctgagg aacaggactt tctccacatt gttttgtatt      1731

gcaacatttt gcattaaaag gaaaatccac tgctaaaaaa aaaaaaaaaa aaaaaaaaaa      1791

a                                                                      1792

<210>  38
<211>  406
<212>  PRT
<213>  human

<400>  38

Met Gly Pro Ser Thr Pro Leu Leu Ile Leu Phe Leu Leu Ser Trp Ser
1               5                   10                  15

Gly Pro Leu Gln Gly Gln Gln His His Leu Val Glu Tyr Met Glu Arg
            20                  25                  30

Arg Leu Ala Ala Leu Glu Glu Arg Leu Ala Gln Cys Gln Asp Gln Ser
        35                  40                  45

Ser Arg His Ala Ala Glu Leu Arg Asp Phe Lys Asn Lys Met Leu Pro
    50                  55                  60

Leu Leu Glu Val Ala Glu Lys Glu Arg Glu Ala Leu Arg Thr Glu Ala
65                  70                  75                  80

Asp Thr Ile Ser Gly Arg Val Asp Arg Leu Glu Arg Glu Val Asp Tyr
                85                  90                  95

Leu Glu Thr Gln Asn Pro Ala Leu Pro Cys Val Glu Phe Asp Glu Lys
            100                 105                 110

Val Thr Gly Gly Pro Gly Thr Lys Gly Lys Gly Arg Arg Asn Glu Lys
        115                 120                 125

Tyr Asp Met Val Thr Asp Cys Gly Tyr Thr Ile Ser Gln Val Arg Ser
    130                 135                 140

Met Lys Ile Leu Lys Arg Phe Gly Gly Pro Ala Gly Leu Trp Thr Lys
145                 150                 155                 160

Asp Pro Leu Gly Gln Thr Glu Lys Ile Tyr Val Leu Asp Gly Thr Gln
                165                 170                 175

Asn Asp Thr Ala Phe Val Phe Pro Arg Leu Arg Asp Phe Thr Leu Ala
            180                 185                 190

Met Ala Ala Arg Lys Ala Ser Arg Val Arg Val Pro Phe Pro Trp Val

195                 200                 205

Gly Thr Gly Gln Leu Val Tyr Gly Gly Phe Leu Tyr Phe Ala Arg Arg
    210                 215                 220

Pro Pro Gly Arg Pro Gly Gly Gly Gly Glu Met Glu Asn Thr Leu Gln
225                 230                 235                 240

Leu Ile Lys Phe His Leu Ala Asn Arg Thr Val Val Asp Ser Ser Val
                245                 250                 255

Phe Pro Ala Glu Gly Leu Ile Pro Pro Tyr Gly Leu Thr Ala Asp Thr
            260                 265                 270

Tyr Ile Asp Leu Ala Ala Asp Glu Glu Gly Leu Trp Ala Val Tyr Ala
        275                 280                 285

Thr Arg Glu Asp Asp Arg His Leu Cys Leu Ala Lys Leu Asp Pro Gln
    290                 295                 300

Thr Leu Asp Thr Glu Gln Gln Trp Asp Thr Pro Cys Pro Arg Glu Asn
305                 310                 315                 320

Ala Glu Ala Ala Phe Val Ile Cys Gly Thr Leu Tyr Val Val Tyr Asn
                325                 330                 335

Thr Arg Pro Ala Ser Arg Ala Arg Ile Gln Cys Ser Phe Asp Ala Ser
            340                 345                 350

Gly Thr Leu Thr Pro Glu Arg Ala Ala Leu Pro Tyr Phe Pro Arg Arg
        355                 360                 365

Tyr Gly Ala His Ala Ser Leu Arg Tyr Asn Pro Arg Glu Arg Gln Leu
    370                 375                 380

Tyr Ala Trp Asp Asp Gly Tyr Gln Ile Val Tyr Lys Leu Glu Met Arg
385                 390                 395                 400

Lys Lys Glu Glu Glu Val
                405

<210>  39
<211>  1925
<212>  DNA
<213>  human

<220>
<221>  CDS
<222>  (127)..(1278)
<223>

<400>  39
agtggagtgg gacaggtata taaaggaagt acagggcctg gggaagaggc cctgtctagg      60

tagctggcac caggagccgt gggcaaggga agaggccaca ccctgccctg ctctgctgca     120

gccaga atg ggt gtg aag gcg tct caa aca ggc ttt gtg gtc ctg gtg        168
       Met Gly Val Lys Ala Ser Gln Thr Gly Phe Val Val Leu Val
       1               5                   10

ctg ctc cag tgc tgc tct gca tac aaa ctg gtc tgc tac tac acc agc       216
Leu Leu Gln Cys Cys Ser Ala Tyr Lys Leu Val Cys Tyr Tyr Thr Ser
15                  20                  25                  30

tgg tcc cag tac cgg gaa ggc gat ggg agc tgc ttc cca gat gcc ctt       264
Trp Ser Gln Tyr Arg Glu Gly Asp Gly Ser Cys Phe Pro Asp Ala Leu
                35                  40                  45

gac cgc ttc ctc tgt acc cac atc atc tac agc ttt gcc aat ata agc       312
Asp Arg Phe Leu Cys Thr His Ile Ile Tyr Ser Phe Ala Asn Ile Ser
            50                  55                  60

aac gat cac atc gac acc tgg gag tgg aat gat gtg acg ctc tac ggc       360
Asn Asp His Ile Asp Thr Trp Glu Trp Asn Asp Val Thr Leu Tyr Gly
        65                  70                  75

atg ctc aac aca ctc aag aac agg aac ccc aac ctg aag act ctc ttg 408
Met Leu Asn Thr Leu Lys Asn Arg Asn Pro Asn Leu Lys Thr Leu Leu
    80                  85                  90

tct gtc gga gga tgg aac ttt ggg tct caa aga ttt tcc aag ata gcc 456
Ser Val Gly Gly Trp Asn Phe Gly Ser Gln Arg Phe Ser Lys Ile Ala
95                  100                 105                 110

tcc aac acc cag agt cgc cgg act ttc atc aag tca gta ccg cca ttc 504
Ser Asn Thr Gln Ser Arg Arg Thr Phe Ile Lys Ser Val Pro Pro Phe
                115                 120                 125

ctg cgc acc cat ggc ttt gat ggg ctg gac ctt gcc tgg ctc tac cct 552
Leu Arg Thr His Gly Phe Asp Gly Leu Asp Leu Ala Trp Leu Tyr Pro
            130                 135                 140

gga cgg aga gac aaa cag cat ttt acc acc cta atc aag gaa atg aag 600
Gly Arg Arg Asp Lys Gln His Phe Thr Thr Leu Ile Lys Glu Met Lys
        145                 150                 155

gcc gaa ttt ata aag gaa gcc cag cca ggg aaa aag cag ctc ctg ctc 648
Ala Glu Phe Ile Lys Glu Ala Gln Pro Gly Lys Lys Gln Leu Leu Leu
    160                 165                 170

agc gca gca ctg tct gcg ggg aag gtc acc att gac agc agc tat gac 696
Ser Ala Ala Leu Ser Ala Gly Lys Val Thr Ile Asp Ser Ser Tyr Asp
175                 180                 185                 190

att gcc aag ata tcc caa cac ctg gat ttc att agc atc atg acc tac 744
Ile Ala Lys Ile Ser Gln His Leu Asp Phe Ile Ser Ile Met Thr Tyr
                195                 200                 205

gat ttt cat gga gcc tgg cgt ggg acc aca ggc cat cac agt ccc ctg 792
Asp Phe His Gly Ala Trp Arg Gly Thr Thr Gly His His Ser Pro Leu
            210                 215                 220

ttc cga ggt cag gag gat gca agt cct gac aga ttc agc aac act gac 840
Phe Arg Gly Gln Glu Asp Ala Ser Pro Asp Arg Phe Ser Asn Thr Asp
        225                 230                 235

tat gct gtg ggg tac atg ttg agg ctg ggg gct cct gcc agt aag ctg 888
Tyr Ala Val Gly Tyr Met Leu Arg Leu Gly Ala Pro Ala Ser Lys Leu
    240                 245                 250

gtg atg ggc atc ccc acc ttc ggg agg agc ttc act ctg gct tct tct 936

Val Met Gly Ile Pro Thr Phe Gly Arg Ser Phe Thr Leu Ala Ser Ser
255                 260                 265                 270

gag act ggt gtt gga gcc cca atc tca gga ccg gga att cca ggc cgg      984
Glu Thr Gly Val Gly Ala Pro Ile Ser Gly Pro Gly Ile Pro Gly Arg
                275                 280                 285

ttc acc aag gag gca ggg acc ctt gcc tac tat gag atc tgt gac ttc      1032
Phe Thr Lys Glu Ala Gly Thr Leu Ala Tyr Tyr Glu Ile Cys Asp Phe
            290                 295                 300

ctc cgc gga gcc aca gtc cat aga acc ctc ggc cag cag gtc ccc tat      1080
Leu Arg Gly Ala Thr Val His Arg Thr Leu Gly Gln Gln Val Pro Tyr
        305                 310                 315

gcc acc aag ggc aac cag tgg gta gga tac gac gac cag gaa agc gtc      1128
Ala Thr Lys Gly Asn Gln Trp Val Gly Tyr Asp Asp Gln Glu Ser Val
    320                 325                 330

aaa agc aag gtg cag tac ctg aag gat agg cag ctg gca ggc gcc atg      1176
Lys Ser Lys Val Gln Tyr Leu Lys Asp Arg Gln Leu Ala Gly Ala Met
335                 340                 345                 350

gta tgg gcc ctg gac ctg gat gac ttc cag ggc tcc ttc tgc ggc cag      1224
Val Trp Ala Leu Asp Leu Asp Asp Phe Gln Gly Ser Phe Cys Gly Gln
                355                 360                 365

gat ctg cgc ttc cct ctc acc aat gcc atc aag gat gca ctc gct gca      1272
Asp Leu Arg Phe Pro Leu Thr Asn Ala Ile Lys Asp Ala Leu Ala Ala
            370                 375                 380

acg tag ccctctgttc tgcacacagc acgggggcca aggatgcccc gtccccctct      1328
Thr

ggctccagct ggccgggagc ctgatcacct gccctgctga gtcccaggct gagcctcagt      1388

ctccctccct tggggcctat gcagaggtcc acaacacaca gatttgagct cagccctggt      1448

gggcagagag gtagggatgg ggctgtgggg atagtgaggc atcgcaatgt aagactcggg      1508

attagtacac acttgttgat gattaatgga aatgtttaca gatccccaag cctggcaagg      1568

gaatttcttc aactccctgc cccctagccc tccttatcaa aggacaccat tttggcaagc      1628

tctatcacca aggagccaaa catcctacaa gacacagtga ccatactaat tataccccct    1688

gcaaagccag cttgaaacct tcacttagga acgtaatcgt gtcccctatc ctacttcccc    1748

ttcctaattc cacagctgct caataaagta caagagttta acagtgtgtt ggcgctttgc    1808

tttggtctat ctttgagcgc ccactagacc cactggactc acctccccca tctcttctgg    1868

gttccttcct ctgagccttg ggacccctga gcttgcagag atgaaggccg ccatgtt    1925

<210>  40
<211>  383
<212>  PRT
<213>  human

<400>  40

Met Gly Val Lys Ala Ser Gln Thr Gly Phe Val Val Leu Val Leu Leu
1               5                   10                  15

Gln Cys Cys Ser Ala Tyr Lys Leu Val Cys Tyr Tyr Thr Ser Trp Ser
            20                  25                  30

Gln Tyr Arg Glu Gly Asp Gly Ser Cys Phe Pro Asp Ala Leu Asp Arg
        35                  40                  45

Phe Leu Cys Thr His Ile Ile Tyr Ser Phe Ala Asn Ile Ser Asn Asp
    50                  55                  60

His Ile Asp Thr Trp Glu Trp Asn Asp Val Thr Leu Tyr Gly Met Leu
65                  70                  75                  80

Asn Thr Leu Lys Asn Arg Asn Pro Asn Leu Lys Thr Leu Leu Ser Val
                85                  90                  95

Gly Gly Trp Asn Phe Gly Ser Gln Arg Phe Ser Lys Ile Ala Ser Asn
            100                 105                 110

Thr Gln Ser Arg Arg Thr Phe Ile Lys Ser Val Pro Pro Phe Leu Arg
        115                 120                 125

Thr His Gly Phe Asp Gly Leu Asp Leu Ala Trp Leu Tyr Pro Gly Arg
    130                 135                 140

Arg Asp Lys Gln His Phe Thr Thr Leu Ile Lys Glu Met Lys Ala Glu
145                 150                 155                 160

Phe Ile Lys Glu Ala Gln Pro Gly Lys Lys Gln Leu Leu Leu Ser Ala
                165                 170                 175

Ala Leu Ser Ala Gly Lys Val Thr Ile Asp Ser Ser Tyr Asp Ile Ala
            180                 185                 190

Lys Ile Ser Gln His Leu Asp Phe Ile Ser Ile Met Thr Tyr Asp Phe
        195                 200                 205

His Gly Ala Trp Arg Gly Thr Thr Gly His His Ser Pro Leu Phe Arg
    210                 215                 220

Gly Gln Glu Asp Ala Ser Pro Asp Arg Phe Ser Asn Thr Asp Tyr Ala
225                 230                 235                 240

Val Gly Tyr Met Leu Arg Leu Gly Ala Pro Ala Ser Lys Leu Val Met
                245                 250                 255

Gly Ile Pro Thr Phe Gly Arg Ser Phe Thr Leu Ala Ser Ser Glu Thr
            260                 265                 270

Gly Val Gly Ala Pro Ile Ser Gly Pro Gly Ile Pro Gly Arg Phe Thr
        275                 280                 285

Lys Glu Ala Gly Thr Leu Ala Tyr Tyr Glu Ile Cys Asp Phe Leu Arg
    290                 295                 300

Gly Ala Thr Val His Arg Thr Leu Gly Gln Gln Val Pro Tyr Ala Thr
305                 310                 315                 320

Lys Gly Asn Gln Trp Val Gly Tyr Asp Asp Gln Glu Ser Val Lys Ser
                325                 330                 335

Lys Val Gln Tyr Leu Lys Asp Arg Gln Leu Ala Gly Ala Met Val Trp
            340                 345                 350

Ala Leu Asp Leu Asp Asp Phe Gln Gly Ser Phe Cys Gly Gln Asp Leu
        355                 360                 365

Arg Phe Pro Leu Thr Asn Ala Ile Lys Asp Ala Leu Ala Ala Thr
    370                 375                 380

<210>  41
<211>  2105
<212>  DNA
<213>  human

<220>
<221>  CDS
<222>  (235)..(1752)
<223>

<400>  41
gtcccagctc gggagcacat cagaggctta gaggcgagtg ggaagggact cagacagtgc      60

aggacgagaa acgcccgcgg caccaaagcc cctcagagcg tcgcccccgc ctctagttct     120

agaaagtcag tttcccggca ctggcacccc ggaacctcag gggctgccga gctggggggg      180

cgctcaagct gcgaggatcc gggctgcccg cgagacgagg agcgggcgcc cagg atg      237
                                                            Met
                                                            1

ggg tgc atg aag tcc aag ttc ctc cag gtc gga ggc aat aca ttc tca 285
Gly Cys Met Lys Ser Lys Phe Leu Gln Val Gly Gly Asn Thr Phe Ser
            5                   10                  15

aaa act gaa acc agc gcc agc cca cac tgt cct gtg tac gtg ccg gat 333
Lys Thr Glu Thr Ser Ala Ser Pro His Cys Pro Val Tyr Val Pro Asp
        20                  25                  30

ccc aca tcc acc atc aag ccg ggg cct aat agc cac aac agc aac aca 381
Pro Thr Ser Thr Ile Lys Pro Gly Pro Asn Ser His Asn Ser Asn Thr
    35                  40                  45

cca gga atc agg gag gca ggc tct gag gac atc atc gtg gtt gcc ctg 429
Pro Gly Ile Arg Glu Ala Gly Ser Glu Asp Ile Ile Val Val Ala Leu
50                  55                  60                  65

tat gat tac gag gcc att cac cac gaa gac ctc agc ttc cag aag ggg 477
Tyr Asp Tyr Glu Ala Ile His His Glu Asp Leu Ser Phe Gln Lys Gly
                70                  75                  80

gac cag atg gtg gtc cta gag gaa tcc ggg gag tgg tgg aag gct cga 525
Asp Gln Met Val Val Leu Glu Glu Ser Gly Glu Trp Trp Lys Ala Arg
            85                  90                  95

tcc ctg gcc acc cgg aag gag ggc tac atc cca agc aac tat gtc gcc 573
Ser Leu Ala Thr Arg Lys Glu Gly Tyr Ile Pro Ser Asn Tyr Val Ala
        100                 105                 110

cgc gtt gac tct ctg gag aca gag gag tgg ttt ttc aag ggc atc agc 621
Arg Val Asp Ser Leu Glu Thr Glu Glu Trp Phe Phe Lys Gly Ile Ser
    115                 120                 125

cgg aag gac gca gag cgc caa ctg ctg gct ccc ggc aac atg ctg ggc 669
Arg Lys Asp Ala Glu Arg Gln Leu Leu Ala Pro Gly Asn Met Leu Gly
130                 135                 140                 145

tcc ttc atg atc cgg gat agc gag acc act aaa gga agc tac tct ttg 717
Ser Phe Met Ile Arg Asp Ser Glu Thr Thr Lys Gly Ser Tyr Ser Leu
                150                 155                 160

tcc gtg cga gac tac gac cct cgg cag gga gat acc gtg aaa cat tac 765
Ser Val Arg Asp Tyr Asp Pro Arg Gln Gly Asp Thr Val Lys His Tyr
            165                 170                 175

aag atc cgg acc ctg gac aac ggg ggc ttc tac ata tcc ccc cga agc 813

Lys Ile Arg Thr Leu Asp Asn Gly Gly Phe Tyr Ile Ser Pro Arg Ser
        180                 185                 190

acc ttc agc act ctg cag gag ctg gtg gac cac tac aag aag ggg aac     861
Thr Phe Ser Thr Leu Gln Glu Leu Val Asp His Tyr Lys Lys Gly Asn
    195                 200                 205

gac ggg ctc tgc cag aaa ctg tcg gtg ccc tgc atg tct tcc aag ccc     909
Asp Gly Leu Cys Gln Lys Leu Ser Val Pro Cys Met Ser Ser Lys Pro
210                 215                 220                 225

cag aag cct tgg gag aaa gat gcc tgg gag atc cct cgg gaa tcc ctc     957
Gln Lys Pro Trp Glu Lys Asp Ala Trp Glu Ile Pro Arg Glu Ser Leu
                230                 235                 240

aag ctg gag aag aaa ctt gga gct ggg cag ttt ggg gaa gtc tgg atg     1005
Lys Leu Glu Lys Lys Leu Gly Ala Gly Gln Phe Gly Glu Val Trp Met
            245                 250                 255

gcc acc tac aac aag cac acc aag gtg gca gtg aag acg atg aag cca     1053
Ala Thr Tyr Asn Lys His Thr Lys Val Ala Val Lys Thr Met Lys Pro
        260                 265                 270

ggg agc atg tcg gtg gag gcc ttc ctg gca gag gcc aac gtg atg aaa     1101
Gly Ser Met Ser Val Glu Ala Phe Leu Ala Glu Ala Asn Val Met Lys
    275                 280                 285

act ctg cag cat gac aag ctg gtc aaa ctt cat gcg gtg gtc acc aag     1149
Thr Leu Gln His Asp Lys Leu Val Lys Leu His Ala Val Val Thr Lys
290                 295                 300                 305

gag ccc atc tac atc atc acg gag ttc atg gcc aaa gga agc ttg ctg     1197
Glu Pro Ile Tyr Ile Ile Thr Glu Phe Met Ala Lys Gly Ser Leu Leu
                310                 315                 320

gac ttt ctg aaa agt gat gag ggc agc aag cag cca ttg cca aaa ctc     1245
Asp Phe Leu Lys Ser Asp Glu Gly Ser Lys Gln Pro Leu Pro Lys Leu
            325                 330                 335

att gac ttc tca gcc cag att gca gaa ggc atg gcc ttc atc gag cag     1293
Ile Asp Phe Ser Ala Gln Ile Ala Glu Gly Met Ala Phe Ile Glu Gln
        340                 345                 350

agg aac tac atc cac cga gac ctc cga gct gcc aac atc ttg gtc tct     1341
Arg Asn Tyr Ile His Arg Asp Leu Arg Ala Ala Asn Ile Leu Val Ser

355                 360                 365

gca tcc ctg gtg tgt aag att gct gac ttt ggc ctg gcc cgg gtc att      1389
Ala Ser Leu Val Cys Lys Ile Ala Asp Phe Gly Leu Ala Arg Val Ile
370                 375                 380                 385

gag gac aac gag tac acg gct cgg gaa ggg gcc aag ttc ccc atc aag      1437
Glu Asp Asn Glu Tyr Thr Ala Arg Glu Gly Ala Lys Phe Pro Ile Lys
                390                 395                 400

tgg aca gct cct gaa gcc atc aac ttt ggc tcc ttc acc atc aag tca      1485
Trp Thr Ala Pro Glu Ala Ile Asn Phe Gly Ser Phe Thr Ile Lys Ser
            405                 410                 415

gac gtc tgg tcc ttt ggt atc ctg ctg atg gag atc gtc acc tac ggc      1533
Asp Val Trp Ser Phe Gly Ile Leu Leu Met Glu Ile Val Thr Tyr Gly
        420                 425                 430

cgg atc cct tac cca ggg atg tca aac cct gaa gtg atc cga gct ctg      1581
Arg Ile Pro Tyr Pro Gly Met Ser Asn Pro Glu Val Ile Arg Ala Leu
    435                 440                 445

gag cgt gga tac cgg atg cct cgc cca gag aac tgc cca gag gag ctc      1629
Glu Arg Gly Tyr Arg Met Pro Arg Pro Glu Asn Cys Pro Glu Glu Leu
450                 455                 460                 465

tac aac atc atg atg cgc tgc tgg aaa aac cgt ccg gag gag cgg ccg      1677
Tyr Asn Ile Met Met Arg Cys Trp Lys Asn Arg Pro Glu Glu Arg Pro
                470                 475                 480

acc ttc gaa tac atc cag agt gtg ctg gat gac ttc tac acg gcc aca      1725
Thr Phe Glu Tyr Ile Gln Ser Val Leu Asp Asp Phe Tyr Thr Ala Thr
            485                 490                 495

gag agc cag tac caa cag cag cca tga tagggaggac cagggcaggg            1772
Glu Ser Gln Tyr Gln Gln Gln Pro
        500                 505

ccagggggtg cccaggtggt ggctgcaagg tggctccagc accatccgcc agggcccaca    1832

cccccttcct actcccagac acccaccctc gcttcagcca cagtttcctc atctgtccag    1892

tgggtaggtt ggactggaaa atctcttttt gactcttgca atccacaatc tgacattctc    1952

aggaagcccc caagttgata tttctatttc ctggaatggt tggattttag ttacagctgt    2012

gatttggaag ggaaactttc aaaatagtga aatgaatatt taaataaaag atataaatgc    2072

caaagtcttt accaaaaaaa aaaaaaaaaa aaa                                 2105

<210>  42
<211>  505
<212>  PRT
<213>  human

<400>  42

Met Gly Cys Met Lys Ser Lys Phe Leu Gln Val Gly Gly Asn Thr Phe
1               5                   10                  15

Ser Lys Thr Glu Thr Ser Ala Ser Pro His Cys Pro Val Tyr Val Pro
            20                  25                  30

Asp Pro Thr Ser Thr Ile Lys Pro Gly Pro Asn Ser His Asn Ser Asn
        35                  40                  45

Thr Pro Gly Ile Arg Glu Ala Gly Ser Glu Asp Ile Ile Val Val Ala
    50                  55                  60

Leu Tyr Asp Tyr Glu Ala Ile His His Glu Asp Leu Ser Phe Gln Lys
65                  70                  75                  80

Gly Asp Gln Met Val Val Leu Glu Glu Ser Gly Glu Trp Trp Lys Ala
                85                  90                  95

Arg Ser Leu Ala Thr Arg Lys Glu Gly Tyr Ile Pro Ser Asn Tyr Val
            100                 105                 110

Ala Arg Val Asp Ser Leu Glu Thr Glu Glu Trp Phe Phe Lys Gly Ile
        115                 120                 125

Ser Arg Lys Asp Ala Glu Arg Gln Leu Leu Ala Pro Gly Asn Met Leu
    130                 135                 140

Gly Ser Phe Met Ile Arg Asp Ser Glu Thr Thr Lys Gly Ser Tyr Ser
145                 150                 155                 160

Leu Ser Val Arg Asp Tyr Asp Pro Arg Gln Gly Asp Thr Val Lys His
                165                 170                 175

Tyr Lys Ile Arg Thr Leu Asp Asn Gly Gly Phe Tyr Ile Ser Pro Arg
            180                 185                 190

Ser Thr Phe Ser Thr Leu Gln Glu Leu Val Asp His Tyr Lys Lys Gly
        195                 200                 205

Asn Asp Gly Leu Cys Gln Lys Leu Ser Val Pro Cys Met Ser Ser Lys
    210                 215                 220

Pro Gln Lys Pro Trp Glu Lys Asp Ala Trp Glu Ile Pro Arg Glu Ser
225                 230                 235                 240

Leu Lys Leu Glu Lys Lys Leu Gly Ala Gly Gln Phe Gly Glu Val Trp
                245                 250                 255

Met Ala Thr Tyr Asn Lys His Thr Lys Val Ala Val Lys Thr Met Lys
            260                 265                 270

Pro Gly Ser Met Ser Val Glu Ala Phe Leu Ala Glu Ala Asn Val Met
        275                 280                 285

Lys Thr Leu Gln His Asp Lys Leu Val Lys Leu His Ala Val Val Thr
    290                 295                 300

Lys Glu Pro Ile Tyr Ile Ile Thr Glu Phe Met Ala Lys Gly Ser Leu

305                 310                 315                 320

Leu Asp Phe Leu Lys Ser Asp Glu Gly Ser Lys Gln Pro Leu Pro Lys
                325                 330                 335

Leu Ile Asp Phe Ser Ala Gln Ile Ala Glu Gly Met Ala Phe Ile Glu
            340                 345                 350

Gln Arg Asn Tyr Ile His Arg Asp Leu Arg Ala Ala Asn Ile Leu Val
        355                 360                 365

Ser Ala Ser Leu Val Cys Lys Ile Ala Asp Phe Gly Leu Ala Arg Val
    370                 375                 380

Ile Glu Asp Asn Glu Tyr Thr Ala Arg Glu Gly Ala Lys Phe Pro Ile
385                 390                 395                 400

Lys Trp Thr Ala Pro Glu Ala Ile Asn Phe Gly Ser Phe Thr Ile Lys
                405                 410                 415

Ser Asp Val Trp Ser Phe Gly Ile Leu Leu Met Glu Ile Val Thr Tyr
            420                 425                 430

Gly Arg Ile Pro Tyr Pro Gly Met Ser Asn Pro Glu Val Ile Arg Ala
        435                 440                 445

Leu Glu Arg Gly Tyr Arg Met Pro Arg Pro Glu Asn Cys Pro Glu Glu
    450                 455                 460

Leu Tyr Asn Ile Met Met Arg Cys Trp Lys Asn Arg Pro Glu Glu Arg
465                 470                 475                 480

Pro Thr Phe Glu Tyr Ile Gln Ser Val Leu Asp Asp Phe Tyr Thr Ala
                485                 490                 495

Thr Glu Ser Gln Tyr Gln Gln Gln Pro
            500                 505

<210>  43
<211>  3432
<212>  DNA
<213>  human

<220>
<221>  CDS
<222>  (248)..(2572)
<223>

<400>  43
actccagcgc gcggctacct acgcttggtg cttgctttct ccagccatcg gagaccagag      60

ccgccccctc tgctcgagaa aggggctcag cggcggcgga agcggagggg gaccaccgtg     120

gagagcgcgg tcccagcccg gccactgcgg atccctgaaa ccaaaaagct cctgctgctt     180

ctgtaccccg cctgtccctc ccagctgcgc agggcccctt cgtgggatca tcagcccgaa     240

gacaggg atg gag agg cct ctg tgc tcc cac ctc tgc agc tgc ctg gct       289
        Met Glu Arg Pro Leu Cys Ser His Leu Cys Ser Cys Leu Ala
        1               5                   10

atg ctg gcc ctc ctg tcc ccc ctg agc ctg gca cag tat gac agc tgg       337
Met Leu Ala Leu Leu Ser Pro Leu Ser Leu Ala Gln Tyr Asp Ser Trp
15                  20                  25                  30

ccc cat tac ccc gag tac ttc cag caa ccg gct cct gag tat cac cag       385
Pro His Tyr Pro Glu Tyr Phe Gln Gln Pro Ala Pro Glu Tyr His Gln
                35                  40                  45

ccc cag gcc ccc gcc aac gtg gcc aag att cag ctg cgc ctg gct ggg       433
Pro Gln Ala Pro Ala Asn Val Ala Lys Ile Gln Leu Arg Leu Ala Gly
            50                  55                  60

cag aag agg aag cac agc gag ggc cgg gtg gag gtg tac tat gat ggc       481
Gln Lys Arg Lys His Ser Glu Gly Arg Val Glu Val Tyr Tyr Asp Gly
        65                  70                  75

cag tgg ggc acc gtg tgc gat gac gac ttc tcc atc cac gct gcc cac 529
Gln Trp Gly Thr Val Cys Asp Asp Asp Phe Ser Ile His Ala Ala His
    80                  85                  90

gtc gtc tgc cgg gag ctg ggc tat gtg gag gcc aag tcc tgg act gcc 577
Val Val Cys Arg Glu Leu Gly Tyr Val Glu Ala Lys Ser Trp Thr Ala
95                  100                 105                 110

agc tcc tcc tac ggc aag gga gaa ggg ccc atc tgg tta gac aat ctc 625
Ser Ser Ser Tyr Gly Lys Gly Glu Gly Pro Ile Trp Leu Asp Asn Leu
                115                 120                 125

cac tgt act ggc aac gag gcg acc ctt gca gca tgc acc tcc aat ggc 673
His Cys Thr Gly Asn Glu Ala Thr Leu Ala Ala Cys Thr Ser Asn Gly
            130                 135                 140

tgg ggc gtc act gac tgc aag cac acg gag gat gtc ggt gtg gtg tgc 721
Trp Gly Val Thr Asp Cys Lys His Thr Glu Asp Val Gly Val Val Cys
        145                 150                 155

agc gac aaa agg att cct ggg ttc aaa ttt gac aat tcg ttg atc aac 769
Ser Asp Lys Arg Ile Pro Gly Phe Lys Phe Asp Asn Ser Leu Ile Asn
    160                 165                 170

cag ata gag aac ctg aat atc cag gtg gag gac att cgg att cga gcc 817
Gln Ile Glu Asn Leu Asn Ile Gln Val Glu Asp Ile Arg Ile Arg Ala
175                 180                 185                 190

atc ctc tca acc tac cgc aag cgc acc cca gtg atg gag ggc tac gtg 865
Ile Leu Ser Thr Tyr Arg Lys Arg Thr Pro Val Met Glu Gly Tyr Val
                195                 200                 205

gag gtg aag gag ggc aag acc tgg aag cag atc tgt gac aag cac tgg 913
Glu Val Lys Glu Gly Lys Thr Trp Lys Gln Ile Cys Asp Lys His Trp
            210                 215                 220

acg gcc aag aat tcc cgc gtg gtc tgc ggc atg ttt ggc ttc cct ggg 961
Thr Ala Lys Asn Ser Arg Val Val Cys Gly Met Phe Gly Phe Pro Gly
        225                 230                 235

gag agg aca tac aat acc aaa gtg tac aaa atg ttt gcc tca cgg agg 1009
Glu Arg Thr Tyr Asn Thr Lys Val Tyr Lys Met Phe Ala Ser Arg Arg
    240                 245                 250

aag cag cgc tac tgg cca ttc tcc atg gac tgc acc ggc aca gag gcc 1057

Lys Gln Arg Tyr Trp Pro Phe Ser Met Asp Cys Thr Gly Thr Glu Ala
255                 260                 265                 270

cac atc tcc agc tgc aag ctg ggc ccc cag gtg tca ctg gac ccc atg      1105
His Ile Ser Ser Cys Lys Leu Gly Pro Gln Val Ser Leu Asp Pro Met
                275                 280                 285

aag aat gtc acc tgc gag aat ggg ctg ccg gcc gtg gtg agt tgt gtg      1153
Lys Asn Val Thr Cys Glu Asn Gly Leu Pro Ala Val Val Ser Cys Val
            290                 295                 300

cct ggg cag gtc ttc agc cct gac gga ccc tcg aga ttc cgg aaa gca      1201
Pro Gly Gln Val Phe Ser Pro Asp Gly Pro Ser Arg Phe Arg Lys Ala
        305                 310                 315

tac aag cca gag caa ccc ctg gtg cga ctg aga ggc ggt gcc tac atc      1249
Tyr Lys Pro Glu Gln Pro Leu Val Arg Leu Arg Gly Gly Ala Tyr Ile
    320                 325                 330

ggg gag ggc cgc gtg gag gtg ctc aaa aat gga gaa tgg ggg acc gtc      1297
Gly Glu Gly Arg Val Glu Val Leu Lys Asn Gly Glu Trp Gly Thr Val
335                 340                 345                 350

tgc gac gac aag tgg gac ctg gtg tcg gcc agt gtg gtc tgc aga gag      1345
Cys Asp Asp Lys Trp Asp Leu Val Ser Ala Ser Val Val Cys Arg Glu
                355                 360                 365

ctg ggc ttt ggg agt gcc aaa gag gca gtc act ggc tcc cga ctg ggg      1393
Leu Gly Phe Gly Ser Ala Lys Glu Ala Val Thr Gly Ser Arg Leu Gly
            370                 375                 380

caa ggg atc gga ccc atc cac ctc aac gag atc cag tgc aca ggc aat      1441
Gln Gly Ile Gly Pro Ile His Leu Asn Glu Ile Gln Cys Thr Gly Asn
        385                 390                 395

gag aag tcc att ata gac tgc aag ttc aat gcc gag tct cag ggc tgc      1489
Glu Lys Ser Ile Ile Asp Cys Lys Phe Asn Ala Glu Ser Gln Gly Cys
    400                 405                 410

aac cac gag gag gat gct ggt gtg aga tgc aac acc cct gcc atg ggc      1537
Asn His Glu Glu Asp Ala Gly Val Arg Cys Asn Thr Pro Ala Met Gly
415                 420                 425                 430

ttg cag aag aag ctg cgc ctg aac ggc ggc cgc aat ccc tac gag ggc      1585
Leu Gln Lys Lys Leu Arg Leu Asn Gly Gly Arg Asn Pro Tyr Glu Gly

                435                 440                 445

cga gtg gag gtg ctg gtg gag aga aac ggg tcc ctt gtg tgg ggg atg 1633
Arg Val Glu Val Leu Val Glu Arg Asn Gly Ser Leu Val Trp Gly Met
            450                 455                 460

gtg tgt ggc caa aac tgg ggc atc gtg gag gcc atg gtg gtc tgc cgc 1681
Val Cys Gly Gln Asn Trp Gly Ile Val Glu Ala Met Val Val Cys Arg
        465                 470                 475

cag ctg ggc ctg gga ttc gcc agc aac gcc ttc cag gag acc tgg tat 1729
Gln Leu Gly Leu Gly Phe Ala Ser Asn Ala Phe Gln Glu Thr Trp Tyr
    480                 485                 490

tgg cac gga gat gtc aac agc aac aaa gtg gtc atg agt gga gtg aag 1777
Trp His Gly Asp Val Asn Ser Asn Lys Val Val Met Ser Gly Val Lys
495                 500                 505                 510

tgc tcg gga acg gag ctg tcc ctg gcg cac tgc cgc cac gac ggg gag 1825
Cys Ser Gly Thr Glu Leu Ser Leu Ala His Cys Arg His Asp Gly Glu
                515                 520                 525

gac gtg gcc tgc ccc cag ggc gga gtg cag tac ggg gcc gga gtt gcc 1873
Asp Val Ala Cys Pro Gln Gly Gly Val Gln Tyr Gly Ala Gly Val Ala
            530                 535                 540

tgc tca gaa acc gcc cct gac ctg gtc ctc aat gcg gag atg gtg cag 1921
Cys Ser Glu Thr Ala Pro Asp Leu Val Leu Asn Ala Glu Met Val Gln
        545                 550                 555

cag acc acc tac ctg gag gac cgg ccc atg ttc atg ctg cag tgt gcc 1969
Gln Thr Thr Tyr Leu Glu Asp Arg Pro Met Phe Met Leu Gln Cys Ala
    560                 565                 570

atg gag gag aac tgc ctc tcg gcc tca gcc gcg cag acc gac ccc acc 2017
Met Glu Glu Asn Cys Leu Ser Ala Ser Ala Ala Gln Thr Asp Pro Thr
575                 580                 585                 590

acg ggc tac cgc cgg ctc ctg cgc ttc tcc tcc cag atc cac aac aat 2065
Thr Gly Tyr Arg Arg Leu Leu Arg Phe Ser Ser Gln Ile His Asn Asn
                595                 600                 605

ggc cag tcc gac ttc cgg ccc aag aac ggc cgc cac gcg tgg atc tgg 2113
Gly Gln Ser Asp Phe Arg Pro Lys Asn Gly Arg His Ala Trp Ile Trp
            610                 615                 620

cac gac tgt cac agg cac tac cac agc atg gag gtg ttc acc cac tat 2161
His Asp Cys His Arg His Tyr His Ser Met Glu Val Phe Thr His Tyr
        625                 630                 635

gac ctg ctg aac ctc aat ggc acc aag gtg gca gag ggc cac aag gcc 2209
Asp Leu Leu Asn Leu Asn Gly Thr Lys Val Ala Glu Gly His Lys Ala
    640                 645                 650

agc ttc tgc ttg gag gac aca gaa tgt gaa gga gac atc cag aag aat 2257
Ser Phe Cys Leu Glu Asp Thr Glu Cys Glu Gly Asp Ile Gln Lys Asn
655                 660                 665                 670

tac gag tgt gcc aac ttc ggc gat cag ggc atc acc atg ggc tgc tgg 2305
Tyr Glu Cys Ala Asn Phe Gly Asp Gln Gly Ile Thr Met Gly Cys Trp
                675                 680                 685

gac atg tac cgc cat gac atc gac tgc cag tgg gtt gac atc act gac 2353
Asp Met Tyr Arg His Asp Ile Asp Cys Gln Trp Val Asp Ile Thr Asp
            690                 695                 700

gtg ccc cct gga gac tac ctg ttc cag gtt gtt att aac ccc aac ttc 2401
Val Pro Pro Gly Asp Tyr Leu Phe Gln Val Val Ile Asn Pro Asn Phe
        705                 710                 715

gag gtt gca gaa tcc gat tac tcc aac aac atc atg aaa tgc agg agc 2449
Glu Val Ala Glu Ser Asp Tyr Ser Asn Asn Ile Met Lys Cys Arg Ser
    720                 725                 730

cgc tat gac ggc cac cgc atc tgg atg tac aac tgc cac ata ggt ggt 2497
Arg Tyr Asp Gly His Arg Ile Trp Met Tyr Asn Cys His Ile Gly Gly
735                 740                 745                 750

tcc ttc agc gaa gag acg gaa aaa aag ttt gag cac ttc agc ggg ctc 2545
Ser Phe Ser Glu Glu Thr Glu Lys Lys Phe Glu His Phe Ser Gly Leu
                755                 760                 765

tta aac aac cag ctg tcc ccg cag taa agaagcctgc gtggtcaact 2592
Leu Asn Asn Gln Leu Ser Pro Gln
            770

cctgtcttca ggccacacca catcttccat gggacttccc cccaacaact gagtctgaac 2652

gaatgccacg tgccctcacc cagcccggcc cccaccctgt ccagacccct acagctgtgt 2712

ctaagctcag gaggaaaggg accctcccat cattcatggg gggctgctac ctgacccttg 2772

gggcctgaga aggccttggg ggggtggggt ttgtccacag agctgctgga gcagcaccaa 2832

gagccagtct tgaccgggat gaggcccaca gacaggttgt catcagcttg tcccattcaa 2892

gccaccgagc tcaccacaga cacagtggag ccgcgctctt ctccagtgac acgtggacaa 2952

atgcgggctc atcagccccc ccagagaggg tcaggccgaa ccccatttct cctcctctta 3012

ggtcattttc agcaaacttg aatatctaga cctctcttcc aatgaaaccc tccagtctat 3072

tatagtcaca tagataatgg tgccacgtgt tttctgattt ggtgagctca gacttggtgc 3132

ttccctctcc acaaccccca ccccttgttt ttcaagatac tattattata ttttcacaga 3192

cttttgaagc acaaatttat tggcatttaa tattggacat ctgggccctt ggaagtacaa 3252

atctaaggaa aaaccaaccc actgtgtaag tgactcatct tcctgttgtt ccaattctgt 3312

gggtttttga ttcaacggtg ctataaccag ggtcctgggt gacagggcgc tcactgagca 3372

ccatgtgtca tcacagacac ttacacatac ttgaaacttg gaataaaaga aagatttatg 3432

<210> 44
<211> 774
<212> PRT
<213> human

<400> 44

Met Glu Arg Pro Leu Cys Ser His Leu Cys Ser Cys Leu Ala Met Leu
1               5                   10                  15

Ala Leu Leu Ser Pro Leu Ser Leu Ala Gln Tyr Asp Ser Trp Pro His
            20                  25                  30

Tyr Pro Glu Tyr Phe Gln Gln Pro Ala Pro Glu Tyr His Gln Pro Gln
        35                  40                  45

Ala Pro Ala Asn Val Ala Lys Ile Gln Leu Arg Leu Ala Gly Gln Lys

50                  55                  60

Arg Lys His Ser Glu Gly Arg Val Glu Val Tyr Tyr Asp Gly Gln Trp
65                  70                  75                  80

Gly Thr Val Cys Asp Asp Asp Phe Ser Ile His Ala Ala His Val Val
                85                  90                  95

Cys Arg Glu Leu Gly Tyr Val Glu Ala Lys Ser Trp Thr Ala Ser Ser
            100                 105                 110

Ser Tyr Gly Lys Gly Glu Gly Pro Ile Trp Leu Asp Asn Leu His Cys
        115                 120                 125

Thr Gly Asn Glu Ala Thr Leu Ala Ala Cys Thr Ser Asn Gly Trp Gly
    130                 135                 140

Val Thr Asp Cys Lys His Thr Glu Asp Val Gly Val Val Cys Ser Asp
145                 150                 155                 160

Lys Arg Ile Pro Gly Phe Lys Phe Asp Asn Ser Leu Ile Asn Gln Ile
                165                 170                 175

Glu Asn Leu Asn Ile Gln Val Glu Asp Ile Arg Ile Arg Ala Ile Leu
            180                 185                 190

Ser Thr Tyr Arg Lys Arg Thr Pro Val Met Glu Gly Tyr Val Glu Val
        195                 200                 205

Lys Glu Gly Lys Thr Trp Lys Gln Ile Cys Asp Lys His Trp Thr Ala
    210                 215                 220

Lys Asn Ser Arg Val Val Cys Gly Met Phe Gly Phe Pro Gly Glu Arg
225                 230                 235                 240

Thr Tyr Asn Thr Lys Val Tyr Lys Met Phe Ala Ser Arg Arg Lys Gln
                245                 250                 255

Arg Tyr Trp Pro Phe Ser Met Asp Cys Thr Gly Thr Glu Ala His Ile
            260                 265                 270

Ser Ser Cys Lys Leu Gly Pro Gln Val Ser Leu Asp Pro Met Lys Asn
        275                 280                 285

Val Thr Cys Glu Asn Gly Leu Pro Ala Val Val Ser Cys Val Pro Gly
    290                 295                 300

Gln Val Phe Ser Pro Asp Gly Pro Ser Arg Phe Arg Lys Ala Tyr Lys
305                 310                 315                 320

Pro Glu Gln Pro Leu Val Arg Leu Arg Gly Gly Ala Tyr Ile Gly Glu
                325                 330                 335

Gly Arg Val Glu Val Leu Lys Asn Gly Glu Trp Gly Thr Val Cys Asp
            340                 345                 350

Asp Lys Trp Asp Leu Val Ser Ala Ser Val Val Cys Arg Glu Leu Gly
        355                 360                 365

Phe Gly Ser Ala Lys Glu Ala Val Thr Gly Ser Arg Leu Gly Gln Gly
    370                 375                 380

Ile Gly Pro Ile His Leu Asn Glu Ile Gln Cys Thr Gly Asn Glu Lys
385                 390                 395                 400

Ser Ile Ile Asp Cys Lys Phe Asn Ala Glu Ser Gln Gly Cys Asn His
                405                 410                 415

Glu Glu Asp Ala Gly Val Arg Cys Asn Thr Pro Ala Met Gly Leu Gln
            420                 425                 430

Lys Lys Leu Arg Leu Asn Gly Gly Arg Asn Pro Tyr Glu Gly Arg Val
        435                 440                 445

Glu Val Leu Val Glu Arg Asn Gly Ser Leu Val Trp Gly Met Val Cys
    450                 455                 460

Gly Gln Asn Trp Gly Ile Val Glu Ala Met Val Val Cys Arg Gln Leu
465                 470                 475                 480

Gly Leu Gly Phe Ala Ser Asn Ala Phe Gln Glu Thr Trp Tyr Trp His
                485                 490                 495

Gly Asp Val Asn Ser Asn Lys Val Val Met Ser Gly Val Lys Cys Ser
            500                 505                 510

Gly Thr Glu Leu Ser Leu Ala His Cys Arg His Asp Gly Glu Asp Val
        515                 520                 525

Ala Cys Pro Gln Gly Gly Val Gln Tyr Gly Ala Gly Val Ala Cys Ser
    530                 535                 540

Glu Thr Ala Pro Asp Leu Val Leu Asn Ala Glu Met Val Gln Gln Thr
545                 550                 555                 560

Thr Tyr Leu Glu Asp Arg Pro Met Phe Met Leu Gln Cys Ala Met Glu
                565                 570                 575

Glu Asn Cys Leu Ser Ala Ser Ala Ala Gln Thr Asp Pro Thr Thr Gly
            580                 585                 590

Tyr Arg Arg Leu Leu Arg Phe Ser Ser Gln Ile His Asn Asn Gly Gln
        595                 600                 605

Ser Asp Phe Arg Pro Lys Asn Gly Arg His Ala Trp Ile Trp His Asp
    610                 615                 620

Cys His Arg His Tyr His Ser Met Glu Val Phe Thr His Tyr Asp Leu
625                 630                 635                 640

Leu Asn Leu Asn Gly Thr Lys Val Ala Glu Gly His Lys Ala Ser Phe
                645                 650                 655

Cys Leu Glu Asp Thr Glu Cys Glu Gly Asp Ile Gln Lys Asn Tyr Glu
            660                 665                 670

Cys Ala Asn Phe Gly Asp Gln Gly Ile Thr Met Gly Cys Trp Asp Met
        675                 680                 685

Tyr Arg His Asp Ile Asp Cys Gln Trp Val Asp Ile Thr Asp Val Pro
    690                 695                 700

Pro Gly Asp Tyr Leu Phe Gln Val Val Ile Asn Pro Asn Phe Glu Val
705                 710                 715                 720

Ala Glu Ser Asp Tyr Ser Asn Asn Ile Met Lys Cys Arg Ser Arg Tyr
                725                 730                 735

Asp Gly His Arg Ile Trp Met Tyr Asn Cys His Ile Gly Gly Ser Phe
            740                 745                 750

Ser Glu Glu Thr Glu Lys Lys Phe Glu His Phe Ser Gly Leu Leu Asn
        755                 760                 765

Asn Gln Leu Ser Pro Gln

    770

<210> 45
<211> 3543
<212> DNA
<213> human

<220>
<221> CDS
<222> (373)..(3003)
<223>

<400> 45
ccccgggaac aggaaccctg gtggacgggt cccagcaggc ttcttcggtg cccgagaggg 60

agcgggtgcc caagggggtg gtccctgtgg caggtcccgg ggtgggggcg cggcgctccg 120

ggaagagcct tccgcaggtc cccgccccgt cacgtgggcg ccggccccgg ccgctgcggt 180

cggtccgctg gttggtcggg cgcttggtcc ggcagttggt cggtgggcca gtggcccgtc 240

gctcgcttct gggctctcat gtttgaaggt gggagggaca cgggagcggc ccgcacacct 300

gagccgcccg gagaggagcc tcggccccgt acccagtaag aagaggagga ggccaggcag 360

gcaaaaggag tc atg gct tct gat gct agt cat gcg ctg gaa gct gcc ctg 411
Met Ala Ser Asp Ala Ser His Ala Leu Glu Ala Ala Leu
1               5                   10

gag caa atg gac ggg atc att gca ggc act aaa aca ggt gca gat ctt 459
Glu Gln Met Asp Gly Ile Ile Ala Gly Thr Lys Thr Gly Ala Asp Leu
    15                  20                  25

agt gat ggt act tgt gag cct gga ctg gct tcc ccg gcc tcc tac atg 507
Ser Asp Gly Thr Cys Glu Pro Gly Leu Ala Ser Pro Ala Ser Tyr Met
30                  35                  40                  45

aac ccc ttc ccg gtg ctc cat ctc atc gag gac ttg agg ctg gcc ttg 555
Asn Pro Phe Pro Val Leu His Leu Ile Glu Asp Leu Arg Leu Ala Leu
                50                  55                  60

gag atg ctg gag ctt cct cag gag aga gca gcc ctc ctg agc cag atc 603
Glu Met Leu Glu Leu Pro Gln Glu Arg Ala Ala Leu Leu Ser Gln Ile
            65                  70                  75

cct ggc cca aca gct gcc tac ata aag gaa tgg ttt gaa gag agc ttg 651
Pro Gly Pro Thr Ala Ala Tyr Ile Lys Glu Trp Phe Glu Glu Ser Leu
        80                  85                  90

tcc cag gta aac cac cac agt gct gct agt aat gaa acc tac cag gaa 699
Ser Gln Val Asn His His Ser Ala Ala Ser Asn Glu Thr Tyr Gln Glu
    95                  100                 105

cgc ttg gca cgt cta gaa ggg gat aag gag tcc ctc ata ttg cag gtg 747
Arg Leu Ala Arg Leu Glu Gly Asp Lys Glu Ser Leu Ile Leu Gln Val
110                 115                 120                 125

agt gtc ctc aca gac caa gta gaa gcc cag gga gaa aag att cga gac 795
Ser Val Leu Thr Asp Gln Val Glu Ala Gln Gly Glu Lys Ile Arg Asp
                130                 135                 140

ctg gaa gtg tgt ctg gaa gga cac cag gtg aaa ctc aat gct gct gaa 843
Leu Glu Val Cys Leu Glu Gly His Gln Val Lys Leu Asn Ala Ala Glu
            145                 150                 155

gag atg ctt caa cag gag ctg cta agc cgc aca tct ctt gag acc cag 891
Glu Met Leu Gln Gln Glu Leu Leu Ser Arg Thr Ser Leu Glu Thr Gln
        160                 165                 170

aag ctc gat ctg atg act gaa gtg tct gag ctg aag ctc aag ctg gtt 939
Lys Leu Asp Leu Met Thr Glu Val Ser Glu Leu Lys Leu Lys Leu Val
    175                 180                 185

ggc atg gag aag gag cag aga gag cag gag gag aag cag aga aaa gca 987
Gly Met Glu Lys Glu Gln Arg Glu Gln Glu Glu Lys Gln Arg Lys Ala
190                 195                 200                 205

gag gag tta ctg caa gag ctc agg cac ctc aaa atc aaa gtg gaa gag 1035
Glu Glu Leu Leu Gln Glu Leu Arg His Leu Lys Ile Lys Val Glu Glu
                210                 215                 220

ttg gaa aat gaa agg aat cag tat gaa tgg aag cta aag gcc act aag 1083
Leu Glu Asn Glu Arg Asn Gln Tyr Glu Trp Lys Leu Lys Ala Thr Lys
            225                 230                 235

gct gaa gtc gcc cag ctg caa gaa cag gtg gcc ctg aaa gat gca gaa 1131
Ala Glu Val Ala Gln Leu Gln Glu Gln Val Ala Leu Lys Asp Ala Glu
        240                 245                 250

att gag cgt ctg cac agc cag ctc tcc cgg aca gca gct ctc cac agt 1179
Ile Glu Arg Leu His Ser Gln Leu Ser Arg Thr Ala Ala Leu His Ser
    255                 260                 265

gag agt cac aca gag aga gac caa gaa att caa cgt ctg aaa atg ggg 1227
Glu Ser His Thr Glu Arg Asp Gln Glu Ile Gln Arg Leu Lys Met Gly
270                 275                 280                 285

atg gaa act ttg ctg ctt gcc aat gaa gat aag gac cgt cgg ata gag 1275
Met Glu Thr Leu Leu Leu Ala Asn Glu Asp Lys Asp Arg Arg Ile Glu
                290                 295                 300

gag ctt acg ggg ctg tta aac cag tac cgg aag gta aag gag att gtg 1323
Glu Leu Thr Gly Leu Leu Asn Gln Tyr Arg Lys Val Lys Glu Ile Val
            305                 310                 315

atg gtc act caa ggg cct tcg gag aga act ctc tca atc aat gaa gaa 1371
Met Val Thr Gln Gly Pro Ser Glu Arg Thr Leu Ser Ile Asn Glu Glu
        320                 325                 330

gaa ccg gag gga ggt ttc agc aag tgg aac gct aca aat aag gac cct 1419
Glu Pro Glu Gly Gly Phe Ser Lys Trp Asn Ala Thr Asn Lys Asp Pro
    335                 340                 345

gaa gaa tta ttt aaa caa gag atg cct cca aga tgt agc tct cct aca 1467
Glu Glu Leu Phe Lys Gln Glu Met Pro Pro Arg Cys Ser Ser Pro Thr
350                 355                 360                 365

gtg ggg cca cct cca ttg cca cag aaa tca ctg gaa acc agg gct cag 1515
Val Gly Pro Pro Pro Leu Pro Gln Lys Ser Leu Glu Thr Arg Ala Gln
                370                 375                 380

aaa aag ctc tct tgt agt cta gaa gac ttg aga agt gaa tct gtg gat 1563
Lys Lys Leu Ser Cys Ser Leu Glu Asp Leu Arg Ser Glu Ser Val Asp
            385                 390                 395

aag tgt atg gat ggg aac cag ccc ttc ccg gtg tta gaa ccc aag gac 1611
Lys Cys Met Asp Gly Asn Gln Pro Phe Pro Val Leu Glu Pro Lys Asp
        400                 405                 410

agc cct ttc ttg gcg gag cac aaa tat ccc act tta cct ggg aag ctt 1659
Ser Pro Phe Leu Ala Glu His Lys Tyr Pro Thr Leu Pro Gly Lys Leu
    415                 420                 425

tca gga gcc acg ccc aat gga gag gct gcc aaa tct cct ccc acc atc 1707

Ser Gly Ala Thr Pro Asn Gly Glu Ala Ala Lys Ser Pro Pro Thr Ile
430                 435                 440                 445

tgc cag cct gac gcc acg ggg agc agc ctg ctg agg ctg aga gac aca     1755
Cys Gln Pro Asp Ala Thr Gly Ser Ser Leu Leu Arg Leu Arg Asp Thr
                450                 455                 460

gaa agt ggc tgg gac gac act gct gtg gtc aat gac ctc tca tcc aca     1803
Glu Ser Gly Trp Asp Asp Thr Ala Val Val Asn Asp Leu Ser Ser Thr
            465                 470                 475

tca tcg ggc act gaa tca ggt cct cag tct cct ctg aca cca gat ggt     1851
Ser Ser Gly Thr Glu Ser Gly Pro Gln Ser Pro Leu Thr Pro Asp Gly
        480                 485                 490

aaa cgg aat ccc aaa ggc att aag aag ttc tgg gga aaa atc cga aga     1899
Lys Arg Asn Pro Lys Gly Ile Lys Lys Phe Trp Gly Lys Ile Arg Arg
    495                 500                 505

act cag tca gga aat ttc tac act gac acg ctg ggg atg gca gag ttt     1947
Thr Gln Ser Gly Asn Phe Tyr Thr Asp Thr Leu Gly Met Ala Glu Phe
510                 515                 520                 525

cga cga ggt ggg ctc cgg gca acc gca ggg cca aga ctc tct agg acc     1995
Arg Arg Gly Gly Leu Arg Ala Thr Ala Gly Pro Arg Leu Ser Arg Thr
                530                 535                 540

agg gac tcc aag gga cag aaa agt gac gcc aat gcc ccc ttt gcc cag     2043
Arg Asp Ser Lys Gly Gln Lys Ser Asp Ala Asn Ala Pro Phe Ala Gln
            545                 550                 555

tgg agc aca gag cgt gtg tgt gca tgg ctg gag gac ttt ggc ctg gct     2091
Trp Ser Thr Glu Arg Val Cys Ala Trp Leu Glu Asp Phe Gly Leu Ala
        560                 565                 570

cag tat gtg atc ttt gcc agg cag tgg gta tct tct ggc cac acc tta     2139
Gln Tyr Val Ile Phe Ala Arg Gln Trp Val Ser Ser Gly His Thr Leu
    575                 580                 585

ttg aca gcc acc cct cag gac atg gaa aag gag cta gga att aag cac     2187
Leu Thr Ala Thr Pro Gln Asp Met Glu Lys Glu Leu Gly Ile Lys His
590                 595                 600                 605

cca ctc cac agg aag aag ctt gtt tta gca gtg aaa gcc atc aac acc     2235
Pro Leu His Arg Lys Lys Leu Val Leu Ala Val Lys Ala Ile Asn Thr

                610                 615                 620

aaa cag gag gag aag tct gca ctg cta gac cac att tgg gtg aca agg 2283
Lys Gln Glu Glu Lys Ser Ala Leu Leu Asp His Ile Trp Val Thr Arg
            625                 630                 635

tgg ctt gat gat att ggc tta ccc cag tac aaa gac cag ttt cat gaa 2331
Trp Leu Asp Asp Ile Gly Leu Pro Gln Tyr Lys Asp Gln Phe His Glu
        640                 645                 650

tct aga gtt gac aga cga atg ctg caa tac cta act gtg aac gat tta 2379
Ser Arg Val Asp Arg Arg Met Leu Gln Tyr Leu Thr Val Asn Asp Leu
    655                 660                 665

ctc ttc tta aaa gtc acc agc caa cta cat cat ctc agc atc aaa tgt 2427
Leu Phe Leu Lys Val Thr Ser Gln Leu His His Leu Ser Ile Lys Cys
670                 675                 680                 685

gcc att cac gtg ctg cat gtc aac aag ttc aac ccc cac tgc ctg cac 2475
Ala Ile His Val Leu His Val Asn Lys Phe Asn Pro His Cys Leu His
                690                 695                 700

cgg cgg cca gct gat gag agt aac ctt tct cct tca gaa gtt gta cag 2523
Arg Arg Pro Ala Asp Glu Ser Asn Leu Ser Pro Ser Glu Val Val Gln
            705                 710                 715

tgg tcc aac cac agg gtg atg gag tgg tta cga tct gtg gac ctg gca 2571
Trp Ser Asn His Arg Val Met Glu Trp Leu Arg Ser Val Asp Leu Ala
        720                 725                 730

gag tat gca ccc aat ctt cga ggg agt gga gtc cat gga ggc ctc att 2619
Glu Tyr Ala Pro Asn Leu Arg Gly Ser Gly Val His Gly Gly Leu Ile
    735                 740                 745

atc ctg gag cca cgc ttc act ggg gac acc ctg gct atg ctt ctc aac 2667
Ile Leu Glu Pro Arg Phe Thr Gly Asp Thr Leu Ala Met Leu Leu Asn
750                 755                 760                 765

atc ccc cca caa aag acg ctc ctc agg cgc cac ctg acc acc aag ttc 2715
Ile Pro Pro Gln Lys Thr Leu Leu Arg Arg His Leu Thr Thr Lys Phe
                770                 775                 780

aat gcc ttg att ggt ccg gag gct gaa cag gag aag cga gag aaa atg 2763
Asn Ala Leu Ile Gly Pro Glu Ala Glu Gln Glu Lys Arg Glu Lys Met
            785                 790                 795

gcc tca cca gct tac aca cca ctg acc acc aca gcc aaa gtc cgg cca     2811
Ala Ser Pro Ala Tyr Thr Pro Leu Thr Thr Thr Ala Lys Val Arg Pro
        800                 805                 810

agg aaa cta gga ttt tca cac ttc gga aac ata aga aaa aag aag ttc     2859
Arg Lys Leu Gly Phe Ser His Phe Gly Asn Ile Arg Lys Lys Lys Phe
    815                 820                 825

gat gaa tcg acg gac tac att tgc cca atg gag ccc agt gac ggt gtc     2907
Asp Glu Ser Thr Asp Tyr Ile Cys Pro Met Glu Pro Ser Asp Gly Val
830                 835                 840                 845

agt gat agt cac agg gtc tac agt ggc tac cgg ggc ctc agc ccc ctt     2955
Ser Asp Ser His Arg Val Tyr Ser Gly Tyr Arg Gly Leu Ser Pro Leu
                850                 855                 860

gat gcc cct gaa ctg gat ggg ctg gac cag gtg gga cag att agc tga     3003
Asp Ala Pro Glu Leu Asp Gly Leu Asp Gln Val Gly Gln Ile Ser
            865                 870                 875

tgcccttgtc acctgccctc tgtgcaccct gagagctcac agtaacactg tgtgtgtcac     3063

catataactg cacctcaccc ccgcacgtgt gcatgactcg cagagaatat tccagcaatt     3123

gtgtacccct gggccagtct ctttgaaccc tgagggtggc caggatctgg agctgcatct     3183

ctaaggggcc aggctttggg gaccattgcc aaaggtggac tcaggaggaa agacacttaa     3243

agacactttt acatgtctag taattcttga tgttcatctt cagcaccagt ggaaacacat     3303

gaacttcgat gcaggtccag agaccatgga cactcccacg aggctcagct ctcaggcacc     3363

ccctacactt cagttgaggg aaaagctcaa gtgccttagg cccgtggacc acagtcttgg     3423

ctgagatcaa agggatgagc aacagggact tctgccacag tgacaatgga attgtgttgt     3483

gccttacttc agaggtggtc tcttctttct tgtaataaaa gcaatattta tgcggaaagc     3543

<210>  46
<211>  876
<212>  PRT
<213>  human

<400> 46

Met Ala Ser Asp Ala Ser His Ala Leu Glu Ala Ala Leu Glu Gln Met
1               5                   10                  15

Asp Gly Ile Ile Ala Gly Thr Lys Thr Gly Ala Asp Leu Ser Asp Gly
            20                  25                  30

Thr Cys Glu Pro Gly Leu Ala Ser Pro Ala Ser Tyr Met Asn Pro Phe
        35                  40                  45

Pro Val Leu His Leu Ile Glu Asp Leu Arg Leu Ala Leu Glu Met Leu
    50                  55                  60

Glu Leu Pro Gln Glu Arg Ala Ala Leu Leu Ser Gln Ile Pro Gly Pro
65                  70                  75                  80

Thr Ala Ala Tyr Ile Lys Glu Trp Phe Glu Glu Ser Leu Ser Gln Val
                85                  90                  95

Asn His His Ser Ala Ala Ser Asn Glu Thr Tyr Gln Glu Arg Leu Ala
            100                 105                 110

Arg Leu Glu Gly Asp Lys Glu Ser Leu Ile Leu Gln Val Ser Val Leu
        115                 120                 125

Thr Asp Gln Val Glu Ala Gln Gly Glu Lys Ile Arg Asp Leu Glu Val
    130                 135                 140

Cys Leu Glu Gly His Gln Val Lys Leu Asn Ala Ala Glu Glu Met Leu
145                 150                 155                 160

Gln Gln Glu Leu Leu Ser Arg Thr Ser Leu Glu Thr Gln Lys Leu Asp
                165                 170                 175

Leu Met Thr Glu Val Ser Glu Leu Lys Leu Lys Leu Val Gly Met Glu
            180                 185                 190

Lys Glu Gln Arg Glu Gln Glu Glu Lys Gln Arg Lys Ala Glu Glu Leu
        195                 200                 205

Leu Gln Glu Leu Arg His Leu Lys Ile Lys Val Glu Glu Leu Glu Asn
    210                 215                 220

Glu Arg Asn Gln Tyr Glu Trp Lys Leu Lys Ala Thr Lys Ala Glu Val
225                 230                 235                 240

Ala Gln Leu Gln Glu Gln Val Ala Leu Lys Asp Ala Glu Ile Glu Arg
                245                 250                 255

Leu His Ser Gln Leu Ser Arg Thr Ala Ala Leu His Ser Glu Ser His
            260                 265                 270

Thr Glu Arg Asp Gln Glu Ile Gln Arg Leu Lys Met Gly Met Glu Thr
        275                 280                 285

Leu Leu Leu Ala Asn Glu Asp Lys Asp Arg Arg Ile Glu Glu Leu Thr
    290                 295                 300

Gly Leu Leu Asn Gln Tyr Arg Lys Val Lys Glu Ile Val Met Val Thr
305                 310                 315                 320

Gln Gly Pro Ser Glu Arg Thr Leu Ser Ile Asn Glu Glu Glu Pro Glu
                325                 330                 335

Gly Gly Phe Ser Lys Trp Asn Ala Thr Asn Lys Asp Pro Glu Glu Leu
            340                 345                 350

Phe Lys Gln Glu Met Pro Pro Arg Cys Ser Ser Pro Thr Val Gly Pro
        355                 360                 365

Pro Pro Leu Pro Gln Lys Ser Leu Glu Thr Arg Ala Gln Lys Lys Leu
    370                 375                 380

Ser Cys Ser Leu Glu Asp Leu Arg Ser Glu Ser Val Asp Lys Cys Met
385                 390                 395                 400

Asp Gly Asn Gln Pro Phe Pro Val Leu Glu Pro Lys Asp Ser Pro Phe
                405                 410                 415

Leu Ala Glu His Lys Tyr Pro Thr Leu Pro Gly Lys Leu Ser Gly Ala
            420                 425                 430

Thr Pro Asn Gly Glu Ala Ala Lys Ser Pro Pro Thr Ile Cys Gln Pro
        435                 440                 445

Asp Ala Thr Gly Ser Ser Leu Leu Arg Leu Arg Asp Thr Glu Ser Gly
    450                 455                 460

Trp Asp Asp Thr Ala Val Val Asn Asp Leu Ser Ser Thr Ser Ser Gly
465                 470                 475                 480

Thr Glu Ser Gly Pro Gln Ser Pro Leu Thr Pro Asp Gly Lys Arg Asn
                485                 490                 495

Pro Lys Gly Ile Lys Lys Phe Trp Gly Lys Ile Arg Arg Thr Gln Ser
            500                 505                 510

Gly Asn Phe Tyr Thr Asp Thr Leu Gly Met Ala Glu Phe Arg Arg Gly
        515                 520                 525

Gly Leu Arg Ala Thr Ala Gly Pro Arg Leu Ser Arg Thr Arg Asp Ser

530                 535                 540

Lys Gly Gln Lys Ser Asp Ala Asn Ala Pro Phe Ala Gln Trp Ser Thr
545                 550                 555                 560

Glu Arg Val Cys Ala Trp Leu Glu Asp Phe Gly Leu Ala Gln Tyr Val
                565                 570                 575

Ile Phe Ala Arg Gln Trp Val Ser Ser Gly His Thr Leu Leu Thr Ala
            580                 585                 590

Thr Pro Gln Asp Met Glu Lys Glu Leu Gly Ile Lys His Pro Leu His
        595                 600                 605

Arg Lys Lys Leu Val Leu Ala Val Lys Ala Ile Asn Thr Lys Gln Glu
    610                 615                 620

Glu Lys Ser Ala Leu Leu Asp His Ile Trp Val Thr Arg Trp Leu Asp
625                 630                 635                 640

Asp Ile Gly Leu Pro Gln Tyr Lys Asp Gln Phe His Glu Ser Arg Val
                645                 650                 655

Asp Arg Arg Met Leu Gln Tyr Leu Thr Val Asn Asp Leu Leu Phe Leu
            660                 665                 670

Lys Val Thr Ser Gln Leu His His Leu Ser Ile Lys Cys Ala Ile His
        675                 680                 685

Val Leu His Val Asn Lys Phe Asn Pro His Cys Leu His Arg Arg Pro
    690                 695                 700

Ala Asp Glu Ser Asn Leu Ser Pro Ser Glu Val Val Gln Trp Ser Asn
705                 710                 715                 720

His Arg Val Met Glu Trp Leu Arg Ser Val Asp Leu Ala Glu Tyr Ala
                725                 730                 735

Pro Asn Leu Arg Gly Ser Gly Val His Gly Gly Leu Ile Ile Leu Glu
            740                 745                 750

Pro Arg Phe Thr Gly Asp Thr Leu Ala Met Leu Leu Asn Ile Pro Pro
        755                 760                 765

Gln Lys Thr Leu Leu Arg Arg His Leu Thr Thr Lys Phe Asn Ala Leu
    770                 775                 780

Ile Gly Pro Glu Ala Glu Gln Glu Lys Arg Glu Lys Met Ala Ser Pro
785                 790                 795                 800

Ala Tyr Thr Pro Leu Thr Thr Thr Ala Lys Val Arg Pro Arg Lys Leu
                805                 810                 815

Gly Phe Ser His Phe Gly Asn Ile Arg Lys Lys Lys Phe Asp Glu Ser
            820                 825                 830

Thr Asp Tyr Ile Cys Pro Met Glu Pro Ser Asp Gly Val Ser Asp Ser
        835                 840                 845

His Arg Val Tyr Ser Gly Tyr Arg Gly Leu Ser Pro Leu Asp Ala Pro
    850                 855                 860

Glu Leu Asp Gly Leu Asp Gln Val Gly Gln Ile Ser
865                 870                 875

<210>  47
<211>  2819
<212>  DNA

<213>  human

<220>
<221>  CDS
<222>  (77)..(1180)
<223>

<400>  47
ctgggcccag ctcccccgag aggtggtcgg atcctctggg ctgctcggtc gatgcctgtg      60

ccactgacgt ccaggc atg agg tgg ttc ctg ccc tgg acg ctg gca gca gtg      112
                  Met Arg Trp Phe Leu Pro Trp Thr Leu Ala Ala Val
                  1               5                   10

aca gca gca gcc gcc agc acc gtc ctg gcc acg gcc ctc tct cca gcc      160
Thr Ala Ala Ala Ala Ser Thr Val Leu Ala Thr Ala Leu Ser Pro Ala
        15                  20                  25

cct acg acc atg gac ttt acc cca gct cca ctg gag gac acc tcc tca      208
Pro Thr Thr Met Asp Phe Thr Pro Ala Pro Leu Glu Asp Thr Ser Ser
    30                  35                  40

cgc ccc caa ttc tgc aag tgg cca tgt gag tgc ccg cca tcc cca ccc      256
Arg Pro Gln Phe Cys Lys Trp Pro Cys Glu Cys Pro Pro Ser Pro Pro
45                  50                  55                  60

cgc tgc ccg ctg ggg gtc agc ctc atc aca gat ggc tgt gag tgc tgt      304
Arg Cys Pro Leu Gly Val Ser Leu Ile Thr Asp Gly Cys Glu Cys Cys
                65                  70                  75

aag atg tgc gct cag cag ctt ggg gac aac tgc acg gag gct gcc atc      352
Lys Met Cys Ala Gln Gln Leu Gly Asp Asn Cys Thr Glu Ala Ala Ile
            80                  85                  90

tgt gac ccc cac cgg ggc ctc tac tgt gac tac agc ggg gac cgc ccg      400
Cys Asp Pro His Arg Gly Leu Tyr Cys Asp Tyr Ser Gly Asp Arg Pro
        95                  100                 105

agg tac gca ata gga gtg tgt gca cag gtg gtc ggt gtg ggc tgc gtc      448
Arg Tyr Ala Ile Gly Val Cys Ala Gln Val Val Gly Val Gly Cys Val
    110                 115                 120

ctg gat ggg gtg cgc tac aac aac ggc cag tcc ttc cag cct aac tgc      496
Leu Asp Gly Val Arg Tyr Asn Asn Gly Gln Ser Phe Gln Pro Asn Cys
125                 130                 135                 140

aag tac aac tgc acg tgc atc gac ggc gcg gtg ggc tgc aca cca ctg 544
Lys Tyr Asn Cys Thr Cys Ile Asp Gly Ala Val Gly Cys Thr Pro Leu
                145                 150                 155

tgc ctc cga gtg cgc ccc ccg cgt ctc tgg tgc ccc cac ccg cgg cgc 592
Cys Leu Arg Val Arg Pro Pro Arg Leu Trp Cys Pro His Pro Arg Arg
            160                 165                 170

gtg agc ata cct ggc cac tgc tgt gag cag tgg gta tgt gag gac gac 640
Val Ser Ile Pro Gly His Cys Cys Glu Gln Trp Val Cys Glu Asp Asp
        175                 180                 185

gcc aag agg cca cgc aag acc gca ccc cgt gac aca gga gcc ttc gat 688
Ala Lys Arg Pro Arg Lys Thr Ala Pro Arg Asp Thr Gly Ala Phe Asp
    190                 195                 200

gct gtg ggt gag gtg gag gca tgg cac agg aac tgc ata gcc tac aca 736
Ala Val Gly Glu Val Glu Ala Trp His Arg Asn Cys Ile Ala Tyr Thr
205                 210                 215                 220

agc ccc tgg agc cct tgc tcc acc agc tgc ggc ctg ggg gtc tcc act 784
Ser Pro Trp Ser Pro Cys Ser Thr Ser Cys Gly Leu Gly Val Ser Thr
                225                 230                 235

cgg atc tcc aat gtt aac gcc cag tgc tgg cct gag caa gag agc cgc 832
Arg Ile Ser Asn Val Asn Ala Gln Cys Trp Pro Glu Gln Glu Ser Arg
            240                 245                 250

ctc tgc aac ttg cgg cca tgc gat gtg gac atc cat aca ctc att aag 880
Leu Cys Asn Leu Arg Pro Cys Asp Val Asp Ile His Thr Leu Ile Lys
        255                 260                 265

gca ggg aag aag tgt ctg gct gtg tac cag cca gag gca tcc atg aac 928
Ala Gly Lys Lys Cys Leu Ala Val Tyr Gln Pro Glu Ala Ser Met Asn
    270                 275                 280

ttc aca ctt gcg ggc tgc atc agc aca cgc tcc tat caa ccc aag tac 976
Phe Thr Leu Ala Gly Cys Ile Ser Thr Arg Ser Tyr Gln Pro Lys Tyr
285                 290                 295                 300

tgt gga gtt tgc atg gac aat agg tgc tgc atc ccc tac aag tct aag 1024
Cys Gly Val Cys Met Asp Asn Arg Cys Cys Ile Pro Tyr Lys Ser Lys
                305                 310                 315

act atc gac gtg tcc ttc cag tgt cct gat ggg ctt ggc ttc tcc cgc     1072
Thr Ile Asp Val Ser Phe Gln Cys Pro Asp Gly Leu Gly Phe Ser Arg
            320                 325                 330

cag gtc cta tgg att aat gcc tgc ttc tgt aac ctg agc tgt agg aat     1120
Gln Val Leu Trp Ile Asn Ala Cys Phe Cys Asn Leu Ser Cys Arg Asn
        335                 340                 345

ccc aat gac atc ttt gct gac ttg gaa tcc tac cct gac ttc tca gaa     1168
Pro Asn Asp Ile Phe Ala Asp Leu Glu Ser Tyr Pro Asp Phe Ser Glu
    350                 355                 360

att gcc aac tag gcaggcacaa atcttgggtc ttggggacta acccaatgcc         1220
Ile Ala Asn
365

tgtgaagcag tcagccctta tggccaataa cttttcacca atgagcctta gttaccctga   1280

tctggaccct tggcctccat ttctgtctct aaccattcaa atgacgcctg atggtgctgc   1340

tcaggcccat gctatgagtt ttctccttga tatcattcag catctactct aaagaaaaat   1400

gcctgtctct agctgttctg gactacaccc aagcctgatc cagcctttcc aagtcactag   1460

aagtcctgct ggatcttgcc taaatcccaa gaaatggaat caggtagact tttaatatca   1520

ctaatttctt ctttagatgc caaaccacaa gactctttgg gtccattcag atgaatagat   1580

ggaatttgga acaatagaat aatctattat ttggagcctg ccaagaggta ctgtaatggg   1640

taattctgac gtcagcgcac caaaactatc ctgattccaa atatgtatgc acctcaaggt   1700

catcaaacat ttgccaagtg agttgaatag ttgcttaatt ttgattttta atggaaagtt   1760

gtatccatta acctgggcat tgttgaggtt aagtttctct tcacccctac actgtgaagg   1820

gtacagatta ggtttgtccc agtcagaaat aaaatttgat aaacattcct gttgatggga   1880

aaagccccca gttaatactc cagagacagg gaaaggtcag cccgtttcag aaggaccaat   1940

tgactctcac actgaatcag ctgctgactg gcagggcttt gggcagttgg ccaggctctt   2000

ccttgaatct tctcccttgt cctgcttggg gttcatagga attggtaagg cctctggact   2060

ggcctgtctg gcccctgaga gtggtgccct ggaacactcc tctactctta cagagccttg   2120

agagacccag ctgcagacca tgccagaccc actgaaatga ccaagacagg ttcaggtagg 2180

ggtgtgggtc aaaccaagaa gtgggtgccc ttggtagcag cctggggtga cctctagagc 2240

tggaggctgt gggactccag gggcccccgt gttcaggaca catctattgc agagactcat 2300

ttcacagcct ttcgttctgc tgaccaaatg gccagttttc tggtaggaag atggaggttt 2360

accggttgtt tagaaacaga aatagactta ataaaggttt aaagctgaag aggttgaagc 2420

taaaaggaaa aggttgttgt taatgaatat caggctatta tttattgtat taggaaaata 2480

taatatttac tgttagaatt cttttattta gggccttttc tgtgccagac attgctctca 2540

gtgctttgca tgtattagct cactgaatct tcacgacaat gttgagaagt tcccattatt 2600

atttctgttc ttacaaatgt gaaacggaag ctcatagagg tgagaaaact caaccagagt 2660

cacccagttg gtgactggga aagttaggat tcagatcgaa attggactgt ctttataacc 2720

catattttcc ccctgttttt agagcttcca aatgtgtcag aataggaaaa cattgcaata 2780

aatggcttga ttttttaaaa aaaaaaaaaa aaaaaaaaa 2819

<210> 48
<211> 367
<212> PRT
<213> human

<400> 48

Met Arg Trp Phe Leu Pro Trp Thr Leu Ala Ala Val Thr Ala Ala Ala
1               5                   10                  15

Ala Ser Thr Val Leu Ala Thr Ala Leu Ser Pro Ala Pro Thr Thr Met
            20                  25                  30

Asp Phe Thr Pro Ala Pro Leu Glu Asp Thr Ser Ser Arg Pro Gln Phe
        35                  40                  45

Cys Lys Trp Pro Cys Glu Cys Pro Pro Ser Pro Pro Arg Cys Pro Leu
    50                  55                  60

Gly Val Ser Leu Ile Thr Asp Gly Cys Glu Cys Cys Lys Met Cys Ala
65                  70                  75                  80

Gln Gln Leu Gly Asp Asn Cys Thr Glu Ala Ala Ile Cys Asp Pro His
                85                  90                  95

Arg Gly Leu Tyr Cys Asp Tyr Ser Gly Asp Arg Pro Arg Tyr Ala Ile
            100                 105                 110

Gly Val Cys Ala Gln Val Val Gly Val Gly Cys Val Leu Asp Gly Val
        115                 120                 125

Arg Tyr Asn Asn Gly Gln Ser Phe Gln Pro Asn Cys Lys Tyr Asn Cys
    130                 135                 140

Thr Cys Ile Asp Gly Ala Val Gly Cys Thr Pro Leu Cys Leu Arg Val
145                 150                 155                 160

Arg Pro Pro Arg Leu Trp Cys Pro His Pro Arg Arg Val Ser Ile Pro
                165                 170                 175

Gly His Cys Cys Glu Gln Trp Val Cys Glu Asp Asp Ala Lys Arg Pro
            180                 185                 190

Arg Lys Thr Ala Pro Arg Asp Thr Gly Ala Phe Asp Ala Val Gly Glu
        195                 200                 205

Val Glu Ala Trp His Arg Asn Cys Ile Ala Tyr Thr Ser Pro Trp Ser
    210                 215                 220

Pro Cys Ser Thr Ser Cys Gly Leu Gly Val Ser Thr Arg Ile Ser Asn

225                 230                 235                 240

Val Asn Ala Gln Cys Trp Pro Glu Gln Glu Ser Arg Leu Cys Asn Leu
                245                 250                 255

Arg Pro Cys Asp Val Asp Ile His Thr Leu Ile Lys Ala Gly Lys Lys
            260                 265                 270

Cys Leu Ala Val Tyr Gln Pro Glu Ala Ser Met Asn Phe Thr Leu Ala
        275                 280                 285

Gly Cys Ile Ser Thr Arg Ser Tyr Gln Pro Lys Tyr Cys Gly Val Cys
    290                 295                 300

Met Asp Asn Arg Cys Cys Ile Pro Tyr Lys Ser Lys Thr Ile Asp Val
305                 310                 315                 320

Ser Phe Gln Cys Pro Asp Gly Leu Gly Phe Ser Arg Gln Val Leu Trp
                325                 330                 335

Ile Asn Ala Cys Phe Cys Asn Leu Ser Cys Arg Asn Pro Asn Asp Ile
            340                 345                 350

Phe Ala Asp Leu Glu Ser Tyr Pro Asp Phe Ser Glu Ile Ala Asn
        355                 360                 365

<210>  49
<211>  1768
<212>  DNA
<213>  human

<220>
<221>  CDS
<222>  (356)..(868)
<223>

<400> 49
ggcacgaggc tgctgtctgc ggaggaaact gcatcgacgg acggccgccc agctacggga 60

ggacctggag tggcactggg cgcccgacgg accatccccg ggacccgcct gcccctcggc 120

gccccgcccc gccgggccgc tccccgtcgg gttccccagc cacagcctta cctacgggct 180

cctgactccg caaggcttcc agaagatgct cgaaccaccg gccggggcct cggggcagca 240

gtgagggagg cgtccagccc cccactcagc tcttctcctc ctgtgccagg ggctccccgg 300

gggatgagca tggtggtttt ccctcggagc cccctggctc gggacgtctg agaag atg 358
Met
1

ccg gtc atg agg ctg ttc cct tgc ttc ctg cag ctc ctg gcc ggg ctg 406
Pro Val Met Arg Leu Phe Pro Cys Phe Leu Gln Leu Leu Ala Gly Leu
            5                   10                  15

gcg ctg cct gct gtg ccc ccc cag cag tgg gcc ttg tct gct ggg aac 454
Ala Leu Pro Ala Val Pro Pro Gln Gln Trp Ala Leu Ser Ala Gly Asn
        20                  25                  30

ggc tcg tca gag gtg gaa gtg gta ccc ttc cag gaa gtg tgg ggc cgc 502
Gly Ser Ser Glu Val Glu Val Val Pro Phe Gln Glu Val Trp Gly Arg
    35                  40                  45

agc tac tgc cgg gcg ctg gag agg ctg gtg gac gtc gtg tcc gag tac 550
Ser Tyr Cys Arg Ala Leu Glu Arg Leu Val Asp Val Val Ser Glu Tyr
50                  55                  60                  65

ccc agc gag gtg gag cac atg ttc agc cca tcc tgt gtc tcc ctg ctg 598
Pro Ser Glu Val Glu His Met Phe Ser Pro Ser Cys Val Ser Leu Leu
                70                  75                  80

cgc tgc acc ggc tgc tgc ggc gat gag aat ctg cac tgt gtg ccg gtg 646
Arg Cys Thr Gly Cys Cys Gly Asp Glu Asn Leu His Cys Val Pro Val
            85                  90                  95

gag acg gcc aat gtc acc atg cag ctc cta aag atc cgt tct ggg gac 694
Glu Thr Ala Asn Val Thr Met Gln Leu Leu Lys Ile Arg Ser Gly Asp
        100                 105                 110

cgg ccc tcc tac gtg gag ctg acg ttc tct cag cac gtt cgc tgc gaa 742
Arg Pro Ser Tyr Val Glu Leu Thr Phe Ser Gln His Val Arg Cys Glu

115                 120                 125

tgc cgg cct ctg cgg gag aag atg aag ccg gaa agg agg aga ccc aag      790
Cys Arg Pro Leu Arg Glu Lys Met Lys Pro Glu Arg Arg Arg Pro Lys
130                 135                 140                 145

ggc agg ggg aag agg agg aga gag aag cag aga ccc aca gac tgc cac      838
Gly Arg Gly Lys Arg Arg Arg Glu Lys Gln Arg Pro Thr Asp Cys His
                150                 155                 160

ctg tgc ggc gat gct gtt ccc cgg agg taa cccacccctt ggaggagaga      888
Leu Cys Gly Asp Ala Val Pro Arg Arg
            165                 170

gaccccgcac ccggctcgtg tatttattac cgtcacactc ttcagtgact cctgctggta      948

cctgccctct atttattagc caactgtttc cctgctgaat gcctcgctcc cttcaagacg     1008

aggggcaggg aaggacagga ccctcaggaa ttcagtgcct tcaacaacgt gagagaaaga     1068

gagaagccag ccacagaccc ctgggagctt ccgctttgaa agaagcaaga cacgtggcct     1128

cgtgaggggc aagctaggcc ccagaggccc tggaggtctc caggggcctg cagaaggaaa     1188

gaagggggcc ctgctacctg ttcttgggcc tcaggctctg cacagtcaag cagcccttgc     1248

tttcggagct cctgtccaaa agtagggatg cggatcctgc tggggccgcc acggcctggc     1308

tggtgggaag gccggcagcg ggcggagggg atccagccac ttccccctct tcttctgaag     1368

atcagaacat tcagctctgg agaacagtgg ttgcctgggg gcttttgcca ctccttgtcc     1428

cccgtgatct cccctcacac tttgccattt gcttgtactg ggacattgtt ctttccggcc     1488

aaggtgccac caccctgccc cccctaagag acacatacag agtgggcccc gggctggaga     1548

aagagctgcc tggatgagaa acagctcagc cagtggggat gaggtcacca ggggaggagc     1608

ctgtgcgtcc cagctgaagg cagtggcagg ggagcaggtt ccccaagggc cctggcaccc     1668

ccacaagctg tccctgcagg gccatctgac tgccaagcca gattctcttg aataaagtat     1728

tctagtgtgg aaaaaaaaaa aaaaaaaaaa aaaaaaaaaa                           1768

<210> 50
<211> 170
<212> PRT
<213> human

<400> 50

Met Pro Val Met Arg Leu Phe Pro Cys Phe Leu Gln Leu Leu Ala Gly
1               5                   10                  15

Leu Ala Leu Pro Ala Val Pro Pro Gln Gln Trp Ala Leu Ser Ala Gly
            20                  25                  30

Asn Gly Ser Ser Glu Val Glu Val Val Pro Phe Gln Glu Val Trp Gly
        35                  40                  45

Arg Ser Tyr Cys Arg Ala Leu Glu Arg Leu Val Asp Val Val Ser Glu
    50                  55                  60

Tyr Pro Ser Glu Val Glu His Met Phe Ser Pro Ser Cys Val Ser Leu
65                  70                  75                  80

Leu Arg Cys Thr Gly Cys Cys Gly Asp Glu Asn Leu His Cys Val Pro
                85                  90                  95

Val Glu Thr Ala Asn Val Thr Met Gln Leu Leu Lys Ile Arg Ser Gly
            100                 105                 110

Asp Arg Pro Ser Tyr Val Glu Leu Thr Phe Ser Gln His Val Arg Cys
        115                 120                 125

Glu Cys Arg Pro Leu Arg Glu Lys Met Lys Pro Glu Arg Arg Arg Pro
    130                 135                 140

Lys Gly Arg Gly Lys Arg Arg Arg Glu Lys Gln Arg Pro Thr Asp Cys
145                 150                 155                 160

His Leu Cys Gly Asp Ala Val Pro Arg Arg
                165                 170

<210>  51
<211>  3874
<212>  DNA
<213>  human

<220>
<221>  CDS
<222>  (1)..(1902)
<223>

<400>  51
atg gct cac cta aag cga cta gta aaa tta cac att aaa aga cat tac        48
Met Ala His Leu Lys Arg Leu Val Lys Leu His Ile Lys Arg His Tyr
1               5                   10                  15

cat aaa aag ttc tgg aag ctt ggt gca gta att ttt ttc ttt ata ata        96
His Lys Lys Phe Trp Lys Leu Gly Ala Val Ile Phe Phe Phe Ile Ile
            20                  25                  30

gtt ttg gtt tta atg caa aga gaa gta agt gtt caa tat tcc aaa gag       144
Val Leu Val Leu Met Gln Arg Glu Val Ser Val Gln Tyr Ser Lys Glu
        35                  40                  45

gaa tca agg atg gaa agg aac atg aaa aac aaa aac aag atg ttg gat       192
Glu Ser Arg Met Glu Arg Asn Met Lys Asn Lys Asn Lys Met Leu Asp
    50                  55                  60

tta atg cta gaa gct gta aac aat att aag gat gcc atg cca aaa atg       240
Leu Met Leu Glu Ala Val Asn Asn Ile Lys Asp Ala Met Pro Lys Met
65                  70                  75                  80

caa ata gga gca cct gtc agg caa aac att gat gct ggt gag aga cct       288
Gln Ile Gly Ala Pro Val Arg Gln Asn Ile Asp Ala Gly Glu Arg Pro
                85                  90                  95

tgt ttg caa gga tat tat aca gca gca gaa ttg aag cct gtc ctt gac       336
Cys Leu Gln Gly Tyr Tyr Thr Ala Ala Glu Leu Lys Pro Val Leu Asp
            100                 105                 110

cgt cca cct cag gat tca aat gca cct ggt gct tct ggt aaa gca ttc 384
Arg Pro Pro Gln Asp Ser Asn Ala Pro Gly Ala Ser Gly Lys Ala Phe
        115                 120                 125

aag aca acc aat tta agt gtt gaa gag caa aag gaa aag gaa cgt ggg 432
Lys Thr Thr Asn Leu Ser Val Glu Glu Gln Lys Glu Lys Glu Arg Gly
    130                 135                 140

gaa gct aaa cac tgc ttt aat gct ttc gca agt gac agg att tct ttg 480
Glu Ala Lys His Cys Phe Asn Ala Phe Ala Ser Asp Arg Ile Ser Leu
145                 150                 155                 160

cac cga gat ctt gga cca gac act cga cct cct gaa tgt att gaa caa 528
His Arg Asp Leu Gly Pro Asp Thr Arg Pro Pro Glu Cys Ile Glu Gln
                165                 170                 175

aaa ttt aag cgc tgc cct ccc ctg ccc acc acc agt gtc ata ata gtt 576
Lys Phe Lys Arg Cys Pro Pro Leu Pro Thr Thr Ser Val Ile Ile Val
            180                 185                 190

ttt cat aat gaa gcg tgg tcc acg ttg ctt aga act gtc cac agt gtg 624
Phe His Asn Glu Ala Trp Ser Thr Leu Leu Arg Thr Val His Ser Val
        195                 200                 205

ctc tat tct tca cct gca ata ctg ctg aag gaa atc att ttg gtg gat 672
Leu Tyr Ser Ser Pro Ala Ile Leu Leu Lys Glu Ile Ile Leu Val Asp
    210                 215                 220

gat gct agt gta gat gag tac tta cat gat aaa cta gat gaa tat gta 720
Asp Ala Ser Val Asp Glu Tyr Leu His Asp Lys Leu Asp Glu Tyr Val
225                 230                 235                 240

aaa caa ttt tct ata gta aaa ata gtc aga caa aga gaa aga aaa ggt 768
Lys Gln Phe Ser Ile Val Lys Ile Val Arg Gln Arg Glu Arg Lys Gly
                245                 250                 255

ctg atc act gct cgg ttg cta gga gca aca gtc gca aca gct gaa acg 816
Leu Ile Thr Ala Arg Leu Leu Gly Ala Thr Val Ala Thr Ala Glu Thr
            260                 265                 270

ctc aca ttt tta gat gct cac tgt gag tgt ttc tat ggt tgg cta gaa 864
Leu Thr Phe Leu Asp Ala His Cys Glu Cys Phe Tyr Gly Trp Leu Glu
        275                 280                 285

cct ctg ttg gcc aga ata gct gag aac tac acg gct gtc gta agt cca 912

Pro Leu Leu Ala Arg Ile Ala Glu Asn Tyr Thr Ala Val Val Ser Pro
    290                 295                 300

gat att gca tcc ata gat ctg aac acg ttt gaa ttc aac aaa cct tct 960
Asp Ile Ala Ser Ile Asp Leu Asn Thr Phe Glu Phe Asn Lys Pro Ser
305                 310                 315                 320

cct tat gga agt aac cat aac cgt gga aat ttt gac tgg agt ctt tca 1008
Pro Tyr Gly Ser Asn His Asn Arg Gly Asn Phe Asp Trp Ser Leu Ser
                325                 330                 335

ttt ggc tgg gag tcg ctt cct gat cat gag aag caa aga agg aaa gat 1056
Phe Gly Trp Glu Ser Leu Pro Asp His Glu Lys Gln Arg Arg Lys Asp
            340                 345                 350

gaa acc tac cca att aaa aca ccc act ttt gca gga gga ctt ttt tcc 1104
Glu Thr Tyr Pro Ile Lys Thr Pro Thr Phe Ala Gly Gly Leu Phe Ser
        355                 360                 365

ata tca aaa gaa tat ttt gag tat att gga agc tat gat gaa gaa atg 1152
Ile Ser Lys Glu Tyr Phe Glu Tyr Ile Gly Ser Tyr Asp Glu Glu Met
    370                 375                 380

gaa atc tgg gga ggt gaa aat ata gaa atg tct ttc aga gta tgg caa 1200
Glu Ile Trp Gly Gly Glu Asn Ile Glu Met Ser Phe Arg Val Trp Gln
385                 390                 395                 400

tgt ggt ggg cag ttg gag att atg cct tgc tct gtt gtt gga cat gtt 1248
Cys Gly Gly Gln Leu Glu Ile Met Pro Cys Ser Val Val Gly His Val
                405                 410                 415

ttt cgc agc aaa agc cct cat agc ttt cca aaa ggc act cag gtg att 1296
Phe Arg Ser Lys Ser Pro His Ser Phe Pro Lys Gly Thr Gln Val Ile
            420                 425                 430

gct aga aac caa gtt cgc ctt gca gaa gtc tgg atg gat gaa tac aag 1344
Ala Arg Asn Gln Val Arg Leu Ala Glu Val Trp Met Asp Glu Tyr Lys
        435                 440                 445

gaa ata ttt tat agg aga aat aca gat gca gca aaa att gtt aaa caa 1392
Glu Ile Phe Tyr Arg Arg Asn Thr Asp Ala Ala Lys Ile Val Lys Gln
    450                 455                 460

aaa gca ttt ggt gat ctt tca aaa aga ttt gaa ata aaa cac cgt ctt 1440
Lys Ala Phe Gly Asp Leu Ser Lys Arg Phe Glu Ile Lys His Arg Leu

465                 470                 475                 480

cgg tgt aaa aat ttt aca tgg tat ctg aac aac att tat cca gag gtg     1488
Arg Cys Lys Asn Phe Thr Trp Tyr Leu Asn Asn Ile Tyr Pro Glu Val
                485                 490                 495

tat gtg cca gac ctt aat cct gtt ata tct gga tac att aaa agc gtt     1536
Tyr Val Pro Asp Leu Asn Pro Val Ile Ser Gly Tyr Ile Lys Ser Val
            500                 505                 510

ggt cag cct cta tgt ctg gat gtt gga gaa aac aat caa gga ggc aaa     1584
Gly Gln Pro Leu Cys Leu Asp Val Gly Glu Asn Asn Gln Gly Gly Lys
        515                 520                 525

cca tta att atg tat aca tgt cat gga ctt ggg gga aac cag tac ttt     1632
Pro Leu Ile Met Tyr Thr Cys His Gly Leu Gly Gly Asn Gln Tyr Phe
    530                 535                 540

gaa tac tct gct caa cat gaa att cgg cac aac atc cag aag gaa tta     1680
Glu Tyr Ser Ala Gln His Glu Ile Arg His Asn Ile Gln Lys Glu Leu
545                 550                 555                 560

tgt ctt cat gct gct caa ggt ctc gtt cag ctg aag gca tgt acc tac     1728
Cys Leu His Ala Ala Gln Gly Leu Val Gln Leu Lys Ala Cys Thr Tyr
                565                 570                 575

aaa ggt cac aag aca gtt gtc act gga gag cag ata tgg gag atc cag     1776
Lys Gly His Lys Thr Val Val Thr Gly Glu Gln Ile Trp Glu Ile Gln
            580                 585                 590

aag gat caa ctt cta tac aat cca ttc tta aaa atg tgc ctt tca gca     1824
Lys Asp Gln Leu Leu Tyr Asn Pro Phe Leu Lys Met Cys Leu Ser Ala
        595                 600                 605

aat gga gag cat cca agt tta gtg tca tgc aac cca tca gat cca ctc     1872
Asn Gly Glu His Pro Ser Leu Val Ser Cys Asn Pro Ser Asp Pro Leu
    610                 615                 620

caa aaa tgg ata ctt agc caa aat gat taa gtgttcctta aaattaagtt     1922
Gln Lys Trp Ile Leu Ser Gln Asn Asp
625                 630

gaaaaaggaa atattctttc tcataaaact gtgactaggc atacactgta gtttttgaaa     1982

attatgcaaa agcagctaaa tgtaacttat tccaagtgca tttttcttat ttatatcttt     2042

atgtagcact actacagaaa ttctgcaagt ttctgtttca aagcacaata actagtaata 2102

ccaaagacta tttcaaaatg tccagatgta ggggaagaga tgtttacagt atgatgaaaa 2162

taattttcca agtaaagtga tgtttgtgtg ttttgtacac ttagggatat atatatatag 2222

ctacattcac acactcacaa tttaaaatat ttcccctagt tttttggggg gataggaaga 2282

aagatttgtt actgtatttt tttaactaca taaaaataga tcaataaatg tcagcattgg 2342

cctctgtgta caaaccaaga gcttttacag atccagaatt tattagttta aaatgcaggt 2402

gaactttttt ttgcgtttgg tttacttgtc tgtcaaatgt ttccttaaac atgaaactga 2462

ataaggagaa gagtattttt aacacttaaa tttcttggca aattttaaaa cattttttag 2522

tctgtaatac actccacttg aagcacttaa gtcttcctta aatgactttt cttaagtaat 2582

gatactgtgt gttttcccaa agcactttta aaaaaatttt tataaattac tatctgttga 2642

aaaggtgtcc ttttcctttc ttctagtatt ttttttctta ccaaaattca ctaatcttga 2702

atgtttgtga tattaaattt caaatgcaga atacttgact catttaaagc taaattttgt 2762

tactgattca attataattg taatggattt ttgactttgt aatggattct tttcatcaaa 2822

aagccttatt attttttatc tatgtggaaa acacaataaa aaatcctcaa cactattgta 2882

atcatttggt taagtgctta ttcctctttt gggtaaaatc tgtaattgat aataggtggg 2942

ggaaaatgaa ttttgtatgc tgaatttcta agcgcctatt gtttgtaaaa ccatcagata 3002

tttcttatgg cacaaaaaat gaggaatagc aaaattcctg tgttcaatat ttagaaaatt 3062

ttgtattaat ttctgataaa gttccttaag catctgatag aatgatgttt taaaaaaatt 3122

tgacgcttgc ttaggagatt taccactttt tttttttgtt tttcgtcatt ttatatttag 3182

atctcctgta ttcttgttcc cgaagtaaaa tacgatcggt ttcatatttt aaatctggca 3242

gagcctcagc tgtacgaaaa agagcatata ctggttattg accctatctt ctcattgttt 3302

gtttgtaagt ttgaatttgt attaaaaagc ctgcattctg agctggacat ggtggctcag 3362

cttctaatcc cagcactttg gtaggcaaag gtgggaggat catttgagct caggagttcc 3422

agaccagcct gggcaacata gcaaaatctc atctctacaa aaagtaaaaa ttaaaaaatg 3482

aaattaaaaa taaaattacc taggtgtggt ggcacgcatc tgtagttcca gctatacagg 3542

aaggtgaggc agaagcattg cttgagcttg ggagatcgag gctacagtga gctatgatta 3602

caccactgca cttcagtctg tgtgactgag caagactctt tcaaaaaaaa aaaaaagcct 3662

acattctcca gttgattatt tccaactaat gtgtattatg tgcctaattt tctatcagaa 3722

gttgtattaa gcccgttttc acactgctgt taaagacata cctgagactg ggtaatttat 3782

aaagaaaaat aggttcaatg gacccacagg tccgcgtggc tggggaagct tcacaatcat 3842

ggcggaaggt gaaagcatgt cttacgtgga ag 3874

<210> 52
<211> 633
<212> PRT
<213> human

<400> 52

Met Ala His Leu Lys Arg Leu Val Lys Leu His Ile Lys Arg His Tyr
1               5                   10                  15

His Lys Lys Phe Trp Lys Leu Gly Ala Val Ile Phe Phe Phe Ile Ile
            20                  25                  30

Val Leu Val Leu Met Gln Arg Glu Val Ser Val Gln Tyr Ser Lys Glu
        35                  40                  45

Glu Ser Arg Met Glu Arg Asn Met Lys Asn Lys Asn Lys Met Leu Asp
    50                  55                  60

Leu Met Leu Glu Ala Val Asn Asn Ile Lys Asp Ala Met Pro Lys Met
65                  70                  75                  80

Gln Ile Gly Ala Pro Val Arg Gln Asn Ile Asp Ala Gly Glu Arg Pro
                85                  90                  95

Cys Leu Gln Gly Tyr Tyr Thr Ala Ala Glu Leu Lys Pro Val Leu Asp
            100                 105                 110

Arg Pro Pro Gln Asp Ser Asn Ala Pro Gly Ala Ser Gly Lys Ala Phe
        115                 120                 125

Lys Thr Thr Asn Leu Ser Val Glu Glu Gln Lys Glu Lys Glu Arg Gly
    130                 135                 140

Glu Ala Lys His Cys Phe Asn Ala Phe Ala Ser Asp Arg Ile Ser Leu
145                 150                 155                 160

His Arg Asp Leu Gly Pro Asp Thr Arg Pro Pro Glu Cys Ile Glu Gln
                165                 170                 175

Lys Phe Lys Arg Cys Pro Pro Leu Pro Thr Thr Ser Val Ile Ile Val
            180                 185                 190

Phe His Asn Glu Ala Trp Ser Thr Leu Leu Arg Thr Val His Ser Val
        195                 200                 205

Leu Tyr Ser Ser Pro Ala Ile Leu Leu Lys Glu Ile Ile Leu Val Asp
    210                 215                 220

Asp Ala Ser Val Asp Glu Tyr Leu His Asp Lys Leu Asp Glu Tyr Val
225                 230                 235                 240

Lys Gln Phe Ser Ile Val Lys Ile Val Arg Gln Arg Glu Arg Lys Gly
                245                 250                 255

Leu Ile Thr Ala Arg Leu Leu Gly Ala Thr Val Ala Thr Ala Glu Thr
            260                 265                 270

Leu Thr Phe Leu Asp Ala His Cys Glu Cys Phe Tyr Gly Trp Leu Glu
        275                 280                 285

Pro Leu Leu Ala Arg Ile Ala Glu Asn Tyr Thr Ala Val Val Ser Pro
    290                 295                 300

Asp Ile Ala Ser Ile Asp Leu Asn Thr Phe Glu Phe Asn Lys Pro Ser
305                 310                 315                 320

Pro Tyr Gly Ser Asn His Asn Arg Gly Asn Phe Asp Trp Ser Leu Ser
                325                 330                 335

Phe Gly Trp Glu Ser Leu Pro Asp His Glu Lys Gln Arg Arg Lys Asp
            340                 345                 350

Glu Thr Tyr Pro Ile Lys Thr Pro Thr Phe Ala Gly Gly Leu Phe Ser
        355                 360                 365

Ile Ser Lys Glu Tyr Phe Glu Tyr Ile Gly Ser Tyr Asp Glu Glu Met
    370                 375                 380

Glu Ile Trp Gly Gly Glu Asn Ile Glu Met Ser Phe Arg Val Trp Gln
385                 390                 395                 400

Cys Gly Gly Gln Leu Glu Ile Met Pro Cys Ser Val Val Gly His Val
                405                 410                 415

Phe Arg Ser Lys Ser Pro His Ser Phe Pro Lys Gly Thr Gln Val Ile
            420                 425                 430

Ala Arg Asn Gln Val Arg Leu Ala Glu Val Trp Met Asp Glu Tyr Lys

435                 440                 445

Glu Ile Phe Tyr Arg Arg Asn Thr Asp Ala Ala Lys Ile Val Lys Gln
    450                 455                 460

Lys Ala Phe Gly Asp Leu Ser Lys Arg Phe Glu Ile Lys His Arg Leu
465                 470                 475                 480

Arg Cys Lys Asn Phe Thr Trp Tyr Leu Asn Asn Ile Tyr Pro Glu Val
                485                 490                 495

Tyr Val Pro Asp Leu Asn Pro Val Ile Ser Gly Tyr Ile Lys Ser Val
            500                 505                 510

Gly Gln Pro Leu Cys Leu Asp Val Gly Glu Asn Asn Gln Gly Gly Lys
        515                 520                 525

Pro Leu Ile Met Tyr Thr Cys His Gly Leu Gly Gly Asn Gln Tyr Phe
    530                 535                 540

Glu Tyr Ser Ala Gln His Glu Ile Arg His Asn Ile Gln Lys Glu Leu
545                 550                 555                 560

Cys Leu His Ala Ala Gln Gly Leu Val Gln Leu Lys Ala Cys Thr Tyr
                565                 570                 575

Lys Gly His Lys Thr Val Val Thr Gly Glu Gln Ile Trp Glu Ile Gln
            580                 585                 590

Lys Asp Gln Leu Leu Tyr Asn Pro Phe Leu Lys Met Cys Leu Ser Ala
        595                 600                 605

Asn Gly Glu His Pro Ser Leu Val Ser Cys Asn Pro Ser Asp Pro Leu
    610                 615                 620

Gln Lys Trp Ile Leu Ser Gln Asn Asp
625                 630


<210>  53
<211>  20
<212>  DNA
<213>  Artificial

<220>
<223>  synthetic oligonucleotide

<400>  53
ccgcacgaca accgcaccat                                                    20


<210>  54
<211>  20
<212>  DNA
<213>  Artificial

<220>
<223>  synthetic oligonucleotide

<400>  54
cgctccggcc cacaaatctc                                                    20


<210>  55
<211>  21
<212>  DNA
<213>  Artificial

<220>
<223>  synthetic oligonucleotide

<400>  55
ctccaatcgt ccctacagtc g                                                  21


<210>  56
<211>  19
<212>  DNA

<213>  Artificial

<220>
<223>  synthetic oligonucleotide

<400>  56
ccaagctatc acctcggcc                                                    19


<210>  57
<211>  21
<212>  DNA
<213>  Artificial

<220>
<223>  synthetic oligonucleotide

<400>  57
tgtttctgca atggtgaggt g                                                 21


<210>  58
<211>  21
<212>  DNA
<213>  Artificial

<220>
<223>  synthetic oligonucleotide

<400>  58
gcggctccaa gacttcctaa t                                                 21


<210>  59
<211>  21
<212>  DNA
<213>  Artificial

<220>
<223>  synthetic oligonucleotide

<400>  59
gctgccctaa agccaaactc t                                                 21

<210> 60
<211> 23
<212> DNA
<213> Artificial

<220>
<223> synthetic oligonucleotide

<400> 60
agaggacagg gaggatcaag ttc 23


<210> 61
<211> 20
<212> DNA
<213> Artificial

<220>
<223> synthetic oligonucleotide

<400> 61
tggcgacact taccgagctt 20


<210> 62
<211> 23
<212> DNA
<213> Artificial

<220>
<223> synthetic oligonucleotide

<400> 62
ccatgcccct tgtagtagct gta 23


<210> 63
<211> 24
<212> DNA
<213> Artificial

<220>
<223> synthetic oligonucleotide

<400> 63

cttgactgtg gttactgctg atca                                              24

<210>  64
<211>  23
<212>  DNA
<213>  Artificial

<220>
<223>  synthetic oligonucleotide

<400>  64
gtatccaccg aatgtgaaaa cgt                                               23

<210>  65
<211>  20
<212>  DNA
<213>  Artificial

<220>
<223>  synthetic oligonucleotide

<400>  65
tgtccctcgg aaaaactggt                                                   20

<210>  66
<211>  20
<212>  DNA
<213>  Artificial

<220>
<223>  synthetic oligonucleotide

<400>  66
agccaccgtt catggtctct                                                   20

<210>  67
<211>  19
<212>  DNA
<213>  Artificial

<220>

<223>  synthetic oligonucleotide

<400>  67
agaacggcac gcctacgat                                                    19


<210>  68
<211>  25
<212>  DNA
<213>  Artificial

<220>
<223>  synthetic oligonucleotide

<400>  68
aggtagcctt tgctgtactc atcat                                             25


<210>  69
<211>  20
<212>  DNA
<213>  Artificial

<220>
<223>  synthetic oligonucleotide

<400>  69
tcacctgatt cttgcgtgct                                                   20


<210>  70
<211>  28
<212>  DNA
<213>  Artificial

<220>
<223>  synthetic oligonucleotide

<400>  70
ctgtgggtta ttatcaatct tgtttctt                                          28


<210>  71
<211>  19
<212>  DNA

<213>  Artificial

<220>
<223>  synthetic oligonucleotide

<400>  71
gcatgtacaa tggctgcgc                                                    19


<210>  72
<211>  19
<212>  DNA
<213>  Artificial

<220>
<223>  synthetic oligonucleotide

<400>  72
caagagctgt aaacggcgg                                                    19


<210>  73
<211>  24
<212>  DNA
<213>  Artificial

<220>
<223>  synthetic oligonucleotide

<400>  73
gagacaccat tgagacttga ccag                                              24


<210>  74
<211>  20
<212>  DNA
<213>  Artificial

<220>
<223>  synthetic oligonucleotide

<400>  74
caccaagatg aaggttcggg                                                   20

<210>  75
<211>  21
<212>  DNA
<213>  Artificial

<220>
<223>  synthetic oligonucleotide

<400>  75
tgtgacagcc agctcgaaaa c                                                21


<210>  76
<211>  21
<212>  DNA
<213>  Artificial

<220>
<223>  synthetic oligonucleotide

<400>  76
tggagagcca agacttttcc a                                                21


<210>  77
<211>  19
<212>  DNA
<213>  Artificial

<220>
<223>  synthetic oligonucleotide

<400>  77
gcctgccatt ctcagcaaa                                                   19


<210>  78
<211>  21
<212>  DNA
<213>  Artificial

<220>
<223>  synthetic oligonucleotide

<400>  78

acactgaatc cacccaggac a                                                21

<210>  79
<211>  19
<212>  DNA
<213>  Artificial

<220>
<223>  synthetic oligonucleotide

<400>  79
caagtccttc cggcatgaa                                                   19

<210>  80
<211>  21
<212>  DNA
<213>  Artificial

<220>
<223>  synthetic oligonucleotide

<400>  80
ccaaaatcac ccttcctttg c                                                21

<210>  81
<211>  21
<212>  DNA
<213>  Artificial

<220>
<223>  synthetic oligonucleotide

<400>  81
cttgacaaca cagcagcgtc a                                                21

<210>  82
<211>  21
<212>  DNA
<213>  Artificial

<220>

<223>  synthetic oligonucleotide

<400>  82
acttgaagat gtggaggccc a                                                21


<210>  83
<211>  21
<212>  DNA
<213>  Artificial

<220>
<223>  synthetic oligonucleotide

<400>  83
tggtcaagaa agcaccaatg c                                                21


<210>  84
<211>  21
<212>  DNA
<213>  Artificial

<220>
<223>  synthetic oligonucleotide

<400>  84
ccacacacgc catctttctt c                                                21


<210>  85
<211>  21
<212>  DNA
<213>  Artificial

<220>
<223>  synthetic oligonucleotide

<400>  85
ttgtctataa cacccgccct g                                                21


<210>  86
<211>  21
<212>  DNA

<213>  Artificial

<220>
<223>  synthetic oligonucleotide

<400>  86
cggcgtggaa aataggagag t                                                21


<210>  87
<211>  21
<212>  DNA
<213>  Artificial

<220>
<223>  synthetic oligonucleotide

<400>  87
aggagaagaa gctggcagga g                                                21


<210>  88
<211>  19
<212>  DNA
<213>  Artificial

<220>
<223>  synthetic oligonucleotide

<400>  88
cttgatggcg ttggtgagc                                                   19


<210>  89
<211>  19
<212>  DNA
<213>  Artificial

<220>
<223>  synthetic oligonucleotide

<400>  89
tgggtaccgt atgcctcga                                                   19

<210> 90
<211> 19
<212> DNA
<213> Artificial

<220>
<223> synthetic oligonucleotide

<400> 90
tgtattcaaa ggtgggccg 19


<210> 91
<211> 21
<212> DNA
<213> Artificial

<220>
<223> synthetic oligonucleotide

<400> 91
tgctgggaca tgtaccgtca t 21


<210> 92
<211> 19
<212> DNA
<213> Artificial

<220>
<223> synthetic oligonucleotide

<400> 92
ccagatgcgg tagccatca 19


<210> 93
<211> 19
<212> DNA
<213> Artificial

<220>
<223> synthetic oligonucleotide

<400> 93

gccttctctg tgcacggag                                                    19

<210>  94
<211>  21
<212>  DNA
<213>  Artificial

<220>
<223>  synthetic oligonucleotide

<400>  94
gtgagaccag cccaaagaca c                                                  21

<210>  95
<211>  20
<212>  DNA
<213>  Artificial

<220>
<223>  synthetic oligonucleotide

<400>  95
atgggagttg gtagggaccg                                                    20

<210>  96
<211>  20
<212>  DNA
<213>  Artificial

<220>
<223>  synthetic oligonucleotide

<400>  96
catgggaggg tgatccactt                                                    20

<210>  97
<211>  19
<212>  DNA
<213>  Artificial

<220>

<223>  synthetic oligonucleotide

<400>  97
ggctgcattg aaggcatgt                                                    19


<210>  98
<211>  19
<212>  DNA
<213>  Artificial

<220>
<223>  synthetic oligonucleotide

<400>  98
aagggcaaac tccacaggc                                                    19


<210>  99
<211>  20
<212>  DNA
<213>  Artificial

<220>
<223>  synthetic oligonucleotide

<400>  99
ttacccggaa gcgtatgtgc                                                   20


<210>  100
<211>  21
<212>  DNA
<213>  Artificial

<220>
<223>  synthetic oligonucleotide

<400>  100
gcctccctgg ttattctcac c                                                 21


<210>  101
<211>  32
<212>  DNA

<213>  Artificial

<220>
<223>  synthetic oligonucleotide

<400>  101
gcggccgcac catgaggggc aacccactga tc                                    32


<210>  102
<211>  29
<212>  DNA
<213>  Artificial

<220>
<223>  synthetic oligonucleotide

<400>  102
gcggccgcct agaaggcact gttccatga                                        29


<210>  103
<211>  36
<212>  DNA
<213>  Artificial

<220>
<223>  synthetic oligonucleotide

<400>  103
gacgcgttgc ggccgcagca tggcggatac cggctt                                36


<210>  104
<211>  36
<212>  DNA
<213>  Artificial

<220>
<223>  synthetic oligonucleotide

<400>  104
aaaggatggc ggccgctcac tcgctgtcga atttga                                36

<210>  105
<211>  29
<212>  DNA
<213>  Artificial

<220>
<223>  synthetic oligonucleotide

<400>  105
gcggccgcag catggaccgc gcggggcgc                                          29


<210>  106
<211>  29
<212>  DNA
<213>  Artificial

<220>
<223>  synthetic oligonucleotide

<400>  106
atgcatctag acagaaggcc ttggaggag                                          29


<210>  107
<211>  32
<212>  DNA
<213>  Artificial

<220>
<223>  synthetic oligonucleotide

<400>  107
gcggccgcag gatgggatgc gtgaagtcca gg                                      32


<210>  108
<211>  44
<212>  DNA
<213>  Artificial

<220>
<223>  synthetic oligonucleotide

<400>  108

gcggccgctc aaggctgctg ctggaactgg ctctcagtgg ccgt                    44

<210>  109
<211>  32
<212>  DNA
<213>  Artificial

<220>
<223>  synthetic oligonucleotide

<400>  109
gcggccgcag aatggctcac cttaagggac ta                                  32

<210>  110
<211>  32
<212>  DNA
<213>  Artificial

<220>
<223>  synthetic oligonucleotide

<400>  110
gcggccgctt attcattttg gctaaaaatc ca                                  32

<210>  111
<211>  33
<212>  DNA
<213>  Artificial

<220>
<223>  synthetic oligonucleotide

<400>  111
cggagcatgg cggataccgg cttgcgccgc gtg                                 33

<210>  112
<211>  21
<212>  DNA
<213>  Artificial

<220>

<223>  synthetic oligonucleotide

<400>  112
gcgcaagccg gtatccgcca t                                                21


<210>  113
<211>  21
<212>  RNA
<213>  Artificial

<220>
<223>  synthetic oligonucleotide

<400>  113
auggcggaua ccggcuugcg c                                                21


<210>  114
<211>  21
<212>  RNA
<213>  Artificial

<220>
<223>  synthetic oligonucleotide

<400>  114
gcgcaagccg guauccgcca u                                                21

## Description

### Technical Field

The present invention relates to proteins having a novel function of regulating cartilage differentiation, genes encoding such proteins, and a method for isolating such genes. Further, the present invention relates to a method for screening for therapeutic agents for a bone and/or joint disease (preferably osteoarthritis) as well as therapeutic agents for such diseases.

### Background Art

Osteoarthritis (OA), which is a bone and/or joint disease, causes cartilage degeneration and spur formation in the course of aging or receipt of dynamic stress, and OA involves a secondary synovitis. Besides aging, sexuality (female), obesity, and external injuries (such as ligament or medial meniscus injuries) can be risk factors, although causal factors of OA have not been clarified. It is reported that approximately 900,000 people are newly afflicted with osteoarthritis per year in Japan (Non-Patent Document No. 1: Nankodo, Co., Ltd., Orthopedic surgery 42: 2-6, 2002). It is considered that the number of patients will grow as the aging of the population progresses in the future. For the conservative medical management for osteoarthritis, a nonsteroidal anti-inflammatory drug is generally administered orally to a patient, and a high-molecular-weight hyaluronic acid is injected intraarticularly into a patient. However, no medicine having a mechanism that clearly inhibits cartilage degeneration and stimulates cartilage regeneration has yet been developed. Since causal factors of osteoarthritis have not yet been fully elucidated, development of such medicine necessitates the identification of genes that are deeply involved in the advancement of osteoarthritis.

In the past, human disease-associated genes the expression levels of which differ between the human pathologic tissues and the human normal tissues at the site of a primary lesion were identified via subtraction, DNA microarray analysis, differential display, EST comparison, or other means for obtaining such genes. In the case of one of bone and/or joint diseases (i.e., osteoarthritis), for example, 5,000 expressed sequenced tags (ESTs) from human normal and osteoarthritic cartilage cDNA libraries were compared, and the expression frequencies thereof were compared to identify the EST, the expression levels of which are increased in osteoarthritis (Non-Patent Document No. 2: Osteoarthritis and Cartilage, 2001, 9, 641-653). Also, genes the expression levels of which vary upon PMA, IL-1β, or TNFα treatment in the synovial cells and in the chondrocytes of a patient with one of bone and/or joint diseases, i.e., chronic rheumatism, are detected (Non-Patent Document No. 3: Proc. Natl. Acad. Sci. U.S.A., 1997, 94, 2150-2155). Separately, acquisition of disease-associated genes is attempted via comparison of gene expression levels between normal tissues and pathologic tissues with the use of animal models of disease. For example, collagen-induced arthritis (CIA) models were subjected to analysis of variations in gene expression levels using a DNA microarray, and it was reported that the level of gene expression induced was two times or more in 333 among 8,734 cDNAs and that the level of gene expression inhibited was two times or more in 55 cDNAs (Non-Patent Document No. 4: Clin. Immuno., 2002, 105, 155-168). Compared with normal tissues, however, expression levels of numerous genes can vary in pathologic tissues. Accordingly, it is deemed difficult to identify genes that play a key role in the advancement of disease via simple comparison between pathologic tissues and normal tissues.

In order to more directly identify the genes that play a key role in the advancement of disease, identification of genes the expressions of which are induced by a factor that is deeply involved in disease, such as an inflammatory cytokine, is attempted by DNA microarray analysis. For example, the inflammatory cytokine interleukin-1 is allowed to act on a chondrocyte cell line, and the genes induced thereupon are then identified (Non-Patent Document No. 5: Arthritis Res., 2001, 3, 381-388). Since an inflammatory cytokine also has enormous numbers of functions, this technique by itself cannot successfully identify the genes that play a key role in inflammation or disease.

Runx2/Cbfa1 is referred to as a polyoma enhancer-binding protein (PEBP2αA), which is a transcription factor of the gene family having the runt region (Non-Patent Document No. 6: Proc. Natl. Acad. Sci. U.S.A., 1993, 90, 6859-6863). Runx2/Cbfal forms a heterodimer with a conjugated transcription factor CbfB/PEBP2β, and it was verified *in vitio* that it enhances the DNA-binding activity (Non-Patent Document No. 7: Virology, 1993, 194, 314-331; and Non-Patent Document No. 8: Mol. Cell. Biol., 1993, 13, 3324-3339). Runx2/Cbfa1 knockout mice are known to die of respiratory failure shortly after birth and to cause complete anosteoplasia. Runx2/Cbfa1 has been found to be a transcription factor that is essential for bone differentiation (Patent Document No. 1: JP Patent Publication (Unexamined) No. 10-309148 (1998); and Non-Patent Document No. 9: Cell, 1997, 89, 755-764). Further, chondrocyte maturation is inhibited in Runx2/Cbfa1 knockout mice, and expression of the type II Runx2/Cbfa1 isoform is observed in the region from prehypertrophic chondrocytes to hypertrophic chondrocytes in the murine chondrocyte cell line (ATDC5). This indicates that Runx2/Cbfa1 plays a key role in the process of cartilage differentiation and particularly in the process from proliferated cartilage to hypertrophic cartilage (Non-Patent Document No. 10: J. Biol. Chem., 2000, 275, 8695-8702).

It is demonstrated that the expression level of markers of cartilage differentiation, which are observed in the growth cartilages such as type X collagen or osteopontin, is enhanced in the cartilage of a patient of osteoarthritis (Non-Patent Document No. 11: Arthritis Rheum, 1992, 35, 806-811; and Non-Patent Document No. 12: Matrix Biology, 2000, 19, 245-255). Also, calcification is actually observed in the cartilage of a patient of osteoarthritis (Non-Patent Document No. 13: *Hone to nankotsu no baioroji* (Biology of bones and cartilages), Kanehara & Co., Ltd., 2002). A. Robin Poole describes in the column in the Arthritis & Rheumatism that cartilage differentiation advances and hypertrophic chondrocytes are observed upon cartilage destruction in osteoarthritis, and this process is similar to enchondral ossification that takes place in the growth plates (Non-Patent Document No. 14: Arthritis Rheum., 2002, 46, 2549-2552). It is implied that conversion of permanent cartilages into growth cartilages observed in such osteoarthritis is deeply involved in the advancement of osteoarthritis (Non-Patent Document No. 15: Hiroyasu Iwamoto, *Nankotsu soshiki keisei no seigyo kikou* (Regulation mechanism for cartilage tissue generation), the academic-award-winning article of the 17^{th} Annual Meeting of the Japanese Society for Bone and Mineral Research).

When Runx2/Cbfa1 was expressed specifically in cartilage with the use of a type II collagen promoter, conversion of permanent cartilages into growth cartilages observed in osteoarthritis was also observed (Non-Patent Document No. 16: J. Cell Biol., 2001, 153, 87-99). This indicates that Runx2/Cbfal plays a key role in the advancement of osteoarthritis conditions. Runx2/Cbfa1, however, affects not only cartilage differentiation but also bone differentiation to a great extent. Also, influence of Runx2/Cbfa1 on the immune system is implied. Accordingly, a medicine that targets the direct inhibition of Runx2/Cbfa1 functions may cause side effects. Thus, Runx2/Cbfa1-regulated genes are searched for, and genes having more disease-specific functions are searched for. A Runx2/Cbfa1-regulated gene, Collagenase-3 (MMP-13) (Non-Patent Document No. 17: Mol. Cell Biol., 1999, 19, 4431-4442), has been proven to play the most important role in cartilage destruction in osteoarthritis among the 3 known collagenase species (Non-Patent Document No. 18: J. Clin. Invest., 1996, 97, 761-768). Transgenic mice that exhibit cartilage-specific expression of Collagenase 3 exhibit osteoarthritis-like symptoms, and expression of type X collagen, which is a marker of hypertrophic cartilage differentiation, is also observed (Non-Patent Document No. 19: J. Clin. Invest., 2001, 107, 35-44). Such findings indicate that some of the genes regarding which expression is induced or inhibited by Runx2/Cbfal (i.e., the downstream genes of Runx2/Cbfa1) are deeply associated with osteoarthritis. However, thorough analysis of the downstream genes of Runx2/Cbfal has not been attempted in the past.

The genes that are induced to express upon the forced expression of the transcription factors of interest in the cells have been analyzed. For example, an NF-kB subunit p65, which is a transcription factor deeply associated with inflammation, is forcedly expressed, the expression is analyzed using DNA microarrays, and the genes regarding which expression is induced or inhibited are identified (Non-Patent Document No. 20: Am J Physiol Cell Physiol, 2002, 283, C58-C65). This technique is effective when identifying a gene related to a transcription factor with well-known functions. However, a host cell has endogenous NF-kB, which induces constitutive expression of the gene. Thus, it may be difficult to detect all the NF-kB-regulated genes with high sensitivity. A method that can overcome such difficulty is deemed necessary, although such method has not yet been developed.

WISP-2, which was found to be a downstream gene of Runx2/Cbfa1 with the use of the present invention, is a growth factor that belongs to the connective tissue growth factor/cysteine-rich 61/neuroblastoma overexpressed (CCN) family (Non-Patent Document No. 21: Proc. Nat1. Acad. Sci. U.S.A., 1998, 95, 14717-14722). It is known that the WISP-2 expression is enhanced by Wnt-1 and such that expression is attenuated by 2 to 30 times in human colon cancer (Non-Patent Document No. 21: Proc. Nat1. Acad. Sci. U.S.A., 1998, 95, 14717-14722). The expression thereof is increased in MCF-7 human mammary cancer cells compared with WISP-2 normal human mammary epithelial cells, and involvement thereof in proliferation of cancer cells is implied (Non-Patent Document No. 22: Biochem. Biophys. Res. Commun., 2001, 282, 421-425). Further, the expression levels thereof were found to be high in osteoblasts and in chondrocytes by *in situ* hybridization. This indicates that WISP-2 is a control factor of osteoblast functions (Non-Patent Document No. 23: J. Biol. Chem., 1999, 274, 17123-17131), although there has been no report that WISP-2 is regulated by Runx2/Cbfa1 up to the present.

Nopp140, which was also found to be a downstream gene of Runx2/Cbfa1 with the use of the present invention, is the first identified nuclear localization signal-binding protein (140 kDa) (Non-Patent Document No. 24: J. Cell Biol., 1990, 111, 2235-2245). The polypeptide of the Nopp140 gene comprises 699 amino acid residues and has the molecular weight of 130 to 140 kd (95 kd upon dephosphorylation). The Nopp140 protein is a highly phosphorylated protein, and phosphorylation is required in order to achieve functions such as regulation of transcription factor. Such protein has the GTPase/ATPase domain.

In the early days, Nopp140 was considered to function as a chaperone for transporting RNA from the nucleus (Non-Patent Document No. 25: Cell, 1992, 70, 127-138). In a later report, however, it is demonstrated that Nopp140 binds specifically to the alpha-1 acid glycoprotein/enhancer-binding protein (AGP/EBP) and functions as a transcription activator (Non-Patent Document No. 26: Mol. Cell. Biol., 1997, 17, 230-239). Functions of the Nopp140 protein include: regulation of cell cycle (Non-Patent Document No. 27: J Cell Sci., 1995, May, p. 108); regulation of cell viability (Non-Patent Document No. 28: J Cell Sci., 1995, May, 108, Pt. 5, pp. 1911-1920); luteinization and oocyte maturation (Non-Patent Document No. 29: J Mol Endocrinol., 2000, Dec, 25(3), 275-286); nucleolus formation in the nucleus (Non-Patent Document No. 30: Mol. Biol. Cell, 2000, 11, 567-577); regulation of transcription factors (Non-Patent Document No. 31: Mol. Cell. Biol., 1996, 16, pp. 4257-4263); regulation of rRNA synthesis (Non-Patent Document No. 32: Mol. Cell. Biol., 1999, 19, pp. 8536-8546); chaperon activity and the induction of α1 acute phase proteins in the acute inflammatory reaction in the liver (Non-Patent Document No. 33: Mol. Cell. Biol., 1997, 17, pp. 230-239); and the increased expression of Nopp140 at the time of neurulation of a chicken (Non-Patent Document No. 34: Development, 2002, 129, pp. 5731-5741). The expression level of the Nopp140 protein is increased in the visceral fat of an obese OLETF rat (Non-Patent Document No. 35: J. Lipid Res., 2000, 41, pp. 1615-1622). The Nopp140 gene is structurally similar to the causative gene (TCOFI) for Treacher Collins syndrome (i.e., craniofacial hypoplasia) (Non-Patent Document No. 36: Mol Biol Cell, 2000, Sep, 11 (9), pp. 3061-3071). Even though the reports as mentioned above have been made, the role of Nopp140 in cartilage differentiation has not yet been elucidated, and there is no report that Nopp140 is regulated by Runx2/Cbfa1. At present, the correlation between Nopp140 and a bone and/or joint disease is not yet known.

Tem8, which was also found to be a downstream gene of Runx2/Cbfal with the use of the present invention, encodes a polypeptide, which is a transmembrane protein comprising 562 amino acid residues, and it serves as a marker in the vascular endothelial cell that is invasive to tumors (Non-Patent Document No. 37: Cancer Res., 2001, Sep 15, 61 (18), pp. 6649-6655). The polypeptide encoded by the Tem8 gene is highly homologous to the capillary morphogenesis protein (Non-Patent Document No. 38: Proc Natl Acad Sci U.S.A., 2003, Apr 29, 100 (9), pp. 5170-5174, Epub. Apr 16, 2003).

The correlation between Tem8 and a bone and/or joint disease has not yet been elucidated. The Tem8 protein is known to act as an anthrax toxin receptor (ATR1) (Non-Patent Document No. 39: Biochem Pharmacol., 2003, Feb 1, 65 (3), 309-314), and it is also known to bind to the C5 domain of collagen αIV (Non-Patent Document No. 40: Cancer Res., 2004, Feb 1, 64 (3), pp. 817-820).

GALNT3, which was also found to be a downstream gene of Runx2/Cbfa1 with the use of the present invention, encodes a polypeptide comprising 633 amino acid residues. GALNT3 is highly homologous to other enzymes in the GalNac-glycosyltransferase family (Non-Patent Document No. 41: J Biol Chem., 1999, Sep 3, 274 (36), 25362-25370).

The correlation between GALNT3 and a bone and/or joint disease has not yet been elucidated. The GALNT3 protein is a Golgi membrane enzyme, is a GalNac-glycosyltransferase, and is often expressed in the pancreas or in the glandular system, such as in the thyroid gland. Certain types of mutated genes of GALNT3 can cause familial tumoral calcinosis (Non-Patent Document No. 42: Nature Genetics, 2004, 36, pp. 579-581). It is also known that the GALNT3 protein is expressed in gastric or pancreatic cancer cells (Non-Patent Document No. 43: Cancer Sci., 2003, Jan, 94 (1), pp. 32-36).

HCK, which was also found to be a downstream gene of Runx2/Cbfa1 with the use of the present invention, encodes a polypeptide comprising 526 amino acid residues, is hematopoietic cell kinase, and is expressed in hematocytes. The HCK protein belongs to the Src tyrosine kinase family (Non-Patent Document No. 44: Int J Biochem Cell Biol., 1995, Jun, 27 (6), pp. 551-63). HCK has the SH2/SH3 domain and is activated upon phosphorylation. It is also activated by mercuric chloride (Non-Patent Document No. 45: Eur. J. Biochem., 2000, Dec, 267 (24), pp. 7201-8).

The correlation between HCK and a bone and/or joint disease has not yet been elucidated. The HCK protein is an important signaling factor in cell proliferation or immune responses, and it is activated by signals from gp130 (a signaling component of IL-6 or LIFR) (Non-Patent Document No. 46: Mol Cell Biol., 2001, Dec, 21 (23), pp. 8068-81). The HCK protein phosphorylates Ras GAP or STAT5 and it is involved in signal transduction of an immunoglobulin receptor (Non-Patent Document No. 47: J Biol Chem., 1995, Jun 16, 270 (24), 14718-24; and Non-Patent Document No. 48: EMBO J., 2002, Nov 1, 21 (21), pp. 5766-74). The expression of the HCK protein is enhanced and/or activated by imatinib mesylate in the LAMA84 cells (Non-Patent Document No. 49: Blood, 2004, Jul 15, 104 (2), pp. 509-18, Epub Mar 23, 2004). The HCK protein allows the proliferation of myeloma cells (Non-Patent Document No. 50: Exp Hematol., 1997, Dec, 25 (13), pp. 1367-77).

### Disclosure of the Invention

It is an object of the present invention to discover genes having a novel function of regulating cartilage differentiation and to use such genes for diagnosing, regulating, and treating a bone and/or joint disease such as osteoarthritis. Such genes can be obtained by focusing on the Runx2/Cbfa1 transcription factor deeply associated with a bone and/or joint disease, i.e., osteoarthritis, and introducing Runx2/Cbfa1 into Runx2/Cbfa1-deficient mouse-derived primary chondrocytes or Runx2/Cba1- and p53-deficient mouse-derived chondrocyte cell lines to identify the genes the expressions of which are thereby induced. Further, an object of the present invention is to provide polypeptides encoded by the aforementioned genes, antibodies against such polypeptides, transgenic animals for such genes, and animal models of a bone and/or joint disease (preferably osteoarthritis). It is another object of the present invention to screen for compounds that regulate the functions or expression of the aforementioned polypeptides using the aforementioned objects. Furthermore, an object of the present invention is to provide compounds selected by screening, a means for diagnosing diseases and therapeutic agents using the same.

The first aspect of the present invention to thereby overcome such drawbacks provides a method for obtaining a disease-associated gene, wherein a disease-associated transcription factor is expressed in a cell line that is deficient in such transcription factor or in a primary cultured cell, and the gene the expression of which is thereby induced or inhibited is screened for via subtraction, DNA chip analysis, or via other means to obtain such a disease-associated gene. Preferably, Runx2/Cbfa1 is expressed in a Runxs/Cbfa1- and p53-deficient chondrocyte cell line or in a Runx2/Cbfa1-deficient mouse-derived primary chondrocyte cell line, and the gene expression of which is thereby induced is screened for via subtraction, DNA chip analysis, or via other means to obtain a disease-associated gene concerning Runx2/Cbfa1. More specifically, the present invention provides a method for searching for a disease-associated gene with a lower background level and higher detection sensitivity compared with conventional techniques by introducing a disease-associated transcription factor into a cell that is deficient in such transcription factor. For example, Runx2/Cbfa1 is expressed in a Runx2/Cbfa1-deficient chondrocyte cell line or in a Runx2/Cbfa1-deficient primary cultured cell, and the gene expression of which is thereby induced or inhibited is screened for as a gene associated with regulation of cartilage differentiation.

The second aspect of the present invention provides primary chondrocytes derived from a Runx2/Cbfal-deficient mouse or cultured chondrocytes and chondrocytes derived from a Runx2/Cbfal- and p53-deficient mouse that are employed in the first aspect of the present invention. More specifically, the method for obtaining genes using such cells according to the first aspect of the present invention does not induce constitutive gene expression by endogenous Cbfa1 because of deficiency in Cbfa1 transcription factors and can provide a system for inducing and/or inhibiting gene expression with lower background levels and higher detection sensitivity compared with conventional techniques. The Runx2/Cbfa1- and p53-deficient mouse-derived chondrocyte cell lines that can be preferably used in the present invention are deposited as the RU-1 and RU-22 cell lines at the International Patent Organism Depositary of the National Institute of Advanced Industrial Science and Technology (Tsukuba Central 6, 1-1-1 Higashi, Tsukuba, Ibaraki, 305-8566, Japan) under the Budapest Treaty as of August 5, 2003, under the accession numbers FERM BP-10137 (the RU-1 cell line) and FERM BP-10138 (the RU-22 cell line) (the original deposit).

The third aspect of the present invention provides polynucleotides of the genes the expressions of which are induced upon forced expression of Cbfa1 with the use of the cells or cell lines according to the second aspect of the present invention. The fact that the expressions of these genes are induced by Runx2/Cbfa1 is reconfirmed via real-time PCR analysis. Also, the expressions of these genes are inhibited in the Runx2/Cbfa1-deficient murine embryonic skeleton to a greater extent than in the wild-type murine embryonic skeleton. More specifically, the present invention provides polynucleotides having the nucleotide sequence shown in SEQ ID NO: 1, 3, 5, 7, 9, 11, 13, 15, 17, 19, 21, 23, or 25 and exhibiting the expression induced via Runx2/Cbfa1 expression.

Preferably, the present invention provides a transgenic mouse exhibiting cartilage-specific expression under the control of a type II collagen promoter, wherein such transgenic mouse comprises a polynucleotide of a gene encoding a protein capable of stimulating cartilage differentiation and having the nucleotide sequence shown in SEQ ID NO: 5, a polynucleotide of a gene encoding a protein capable of inhibiting cartilage differentiation and having the nucleotide sequence shown in SEQ ID NO: 3, a polynucleotide of a gene encoding a protein capable of stimulating cartilage differentiation and having the nucleotide sequence shown in SEQ ID NO: 1, a polynucleotide of a gene encoding a protein capable of inhibiting cartilage differentiation and having the nucleotide sequence shown in SEQ ID NO: 15, and a polynucleotide of a gene encoding a protein capable of inhibiting chondrogenesis and having the nucleotide sequence shown in SEQ ID NO: 25. Further, the present invention provides a polynucleotide encoding a gene having a novel nucleotide sequence shown in SEQ ID NO: 9.

The aforementioned downstream gene of Runx2/Cbfa1 is derived from a mouse. By searching the database, a human homologue thereof can be easily identified, and it can be easily deduced that such human homologue has similar effects concerning cartilage differentiation. Based on such genetic information, homologous genes can be easily obtained by hybridization. Thus, it can be easily deduced that the resulting gene has similar functions concerning cartilages. Accordingly, the present invention also provides a human-derived polynucleotide, which is homologous to the aforementioned mouse-derived polynucleotide having the nucleotide sequence shown in SEQ ID NO: 27, 29, 31, 33, 35, 37, 39, 41, 43, 45, 47, 49, or 51.

Further, the present invention provides a polynucleotide that has 65% or more homology to the polypeptide encoded by the polynucleotide having the nucleotide sequence shown in SEQ ID NO: 1, 3, 5, 7, 9, 11, 13, 15, 17, 19, 21, 23, or 25 and that encodes a protein capable of stimulating or inhibiting cartilage differentiation. Furthermore, the present invention provides a polynucleotide that is capable of hybridizing under stringent conditions to a polynucleotide having the nucleotide sequence shown in SEQ ID NO: 1, 3, 5, 7, 9, 11, 13, 15, 17, 19, 21, 23, or 25 or a complementary strand thereof and that encodes a protein capable of stimulating or inhibiting cartilage differentiation.

The present invention provides: a recombinant DNA vector comprising the aforementioned polynucleotide or a complementary strand thereof; a transformant transformed with the recombinant DNA vector; a polypeptide encoded by the aforementioned polynucleotide (i.e., a polypeptide comprising an amino acid sequence shown in SEQ ID NO: 2, 4, 6, 8, 10, 12, 14, 16, 18, 20, 22, 24, 26, 28, 30, 32, 34, 36, 38, 40, 42, 44, 46, 48, 50, or 52; a polypeptide comprising an amino acid sequence derived from the aforementioned amino acid sequence by deletion, substitution, or addition of one or several amino acid residues and capable of stimulating or inhibiting cartilage differentiation; or a polypeptide comprising an amino acid sequence having at least 65% homology to the aforementioned amino acid sequence and capable of stimulating or inhibiting cartilage differentiation); an antisense polynucleotide which regulates the expression of the gene consisting of the aforementioned polynucleotide; an RNAi molecule which regulates the expression of the gene consisting of the aforementioned polynucleotide; and an antibody against the aforementioned polypeptide.

The fourth aspect of the present invention provides a method for screening for a therapeutic agent and/or prophylactic agent for a bone and/or joint disease. The screening method according to the present invention involves the use of the aforementioned polynucleotide (i.e., the downstream gene of Runx2/Cbfal) or a protein encoded by such polynucleotide. This method can provide a compound that regulates the expression of the downstream gene of Runx2/Cbfa1 *in vitro* and *in vivo* and a compound that regulates activity of a protein encoded by the downstream gene of Runx2/Cbfa1.

The downstream gene of Runx2/Cbfa1, which had been found to inhibit cartilage differentiation by the aforementioned method, can inhibit the stimulated cartilage differentiation observed in a bone and/or joint disease (preferably osteoarthritis) with the use of a polynucleotide of the gene, a polypeptide encoded by such gene, a vector containing the polynucleotide of the gene, a compound that activates the gene expression or protein functions, or the like. Thus, therapeutic and/or prophylactic effects on a bone and/or joint disease can be expected. In contrast, the downstream gene of Runx2/Cbfal, which had been found to stimulate cartilage differentiation, can inhibit the stimulated cartilage differentiation observed in a bone and/or joint disease (preferably osteoarthritis) with the use of a compound that inhibits the protein functions, a compound that inhibits the gene expression, an antibody, an RNAi molecule, an antisense polynucleotide, or the like. Thus, therapeutic and/or prophylactic effects on a bone and/or joint disease can be expected.

The disease-associated gene that was screened for by the aforementioned method, for example, the downstream gene of Runx2/Cbfa1 that was found to inhibit or stimulate cartilage differentiation, may be analyzed with the use of the polynucleotide of the gene, the polypeptide encoded by such gene, a vector containing the polynucleotide of the gene, a compound that activates gene expression or protein function, or the like. Thus, diseases (preferably a bone and/or joint disease such as osteoarthritis) can be diagnosed.

The fifth aspect of the present invention provides a transgenic animal associated with the downstream gene of Runx2/Cbfal, i.e., a transgenic animal model of a bone and/or joint disease with an increased or decreased expression level of the downstream gene of Runx2/Cbfa1. Preferably, the present invention provides a transgenic mouse exhibiting cartilage-specific expression of the downstream gene of Runx2/Cbfa1 under the control of a type II collagen promoter. A transgenic mouse having the downstream gene of Runx2/Cbfa1 capable of stimulating cartilage differentiation may have a phenotype similar to that of osteoarthritis. This can provide a useful animal model of osteoarthritis. Such disease model can effect a method for screening for a candidate pharmaceutical compound and it also serves as a useful tool for analyzing diseases. The present invention also provides a method for producing animal models for a bone and/or joint disease (preferably osteoarthritis) by administration of, for example, a DNA vector containing the polynucleotide of the downstream gene of Runx2/Cbfa1 or a complementary strand thereof, a transformant transformed with such DNA vector, a protein itself encoded by the polynucleotide, an antisense polynucleotide, an RNAi molecule, an antibody, or a compound selected by the aforementioned screening method.

### Brief Description of the Drawings

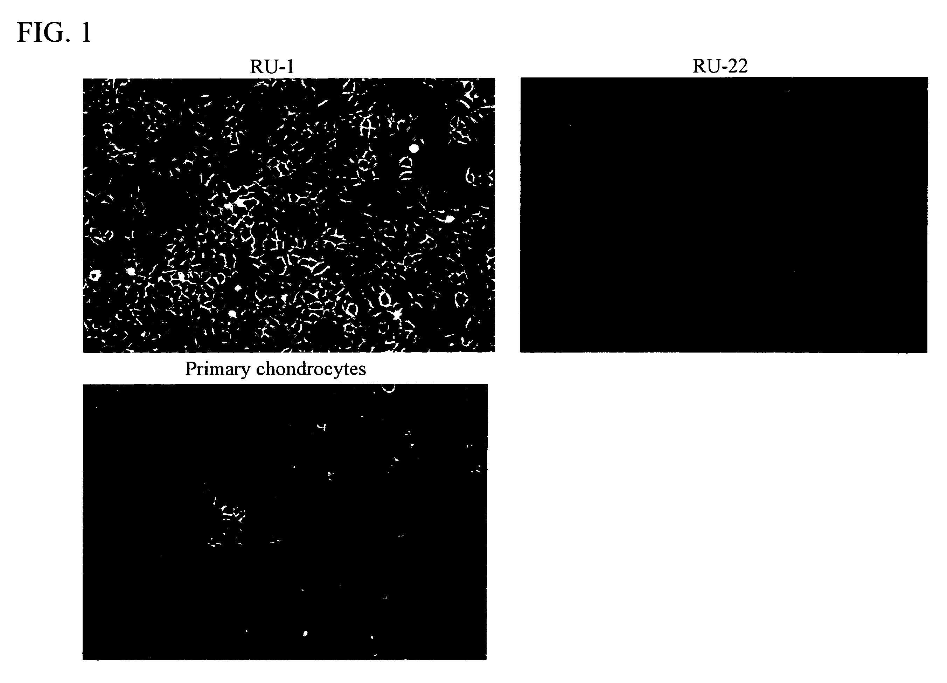

Fig. 1 is an optical phase-contrast microscope photograph (x100) showing the morphologies of the Runx2/Cbfal- and p53-deficient cell lines and the Runx2/Cbfa1-deficient mouse-derived primary cultured chondrocyte of the present invention. RU-1 and RU-22 represent Runx2/Cbfal- and p53-deficient cell lines.

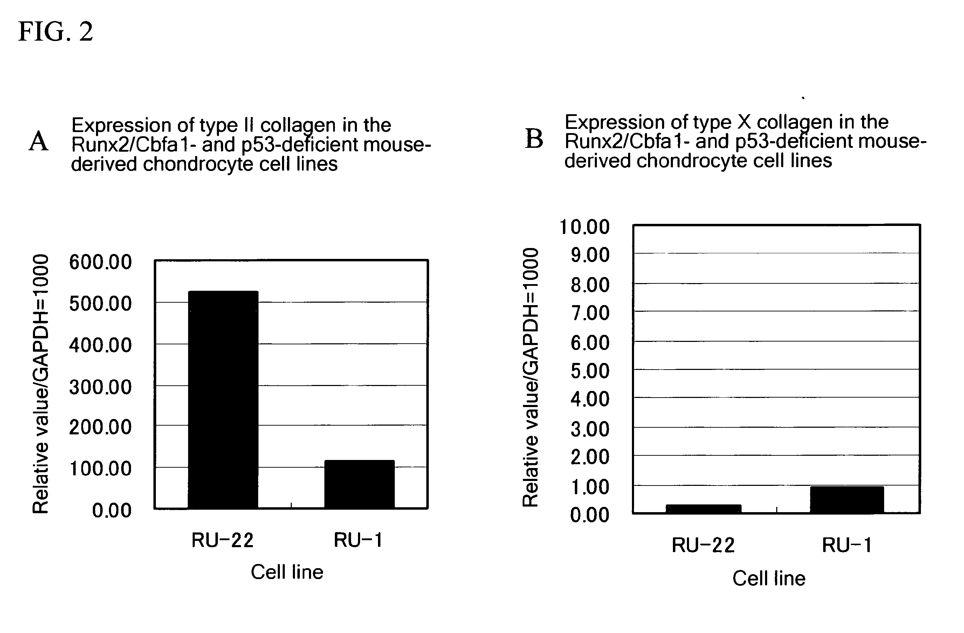

Fig. 2A and B show the results of analyzing the expression of type II collagen (A) and that of type X collagen (B) in the Runx2/Cbfa1- and p53-deficient cell lines of the present invention (RU-1 and RU-22) via real-time PCR analysis. These cell lines were cultured to be confluent, and total RNAs were extracted. Thereafter, cDNA was synthesized, and the resulting cDNA was used as a template to analyze the gene expression via real-time PCR analysis. The GAPDH expression levels in samples were simultaneously measured, and the expression levels were expressed as the relative value when the GAPDH level was determined to be 1,000.

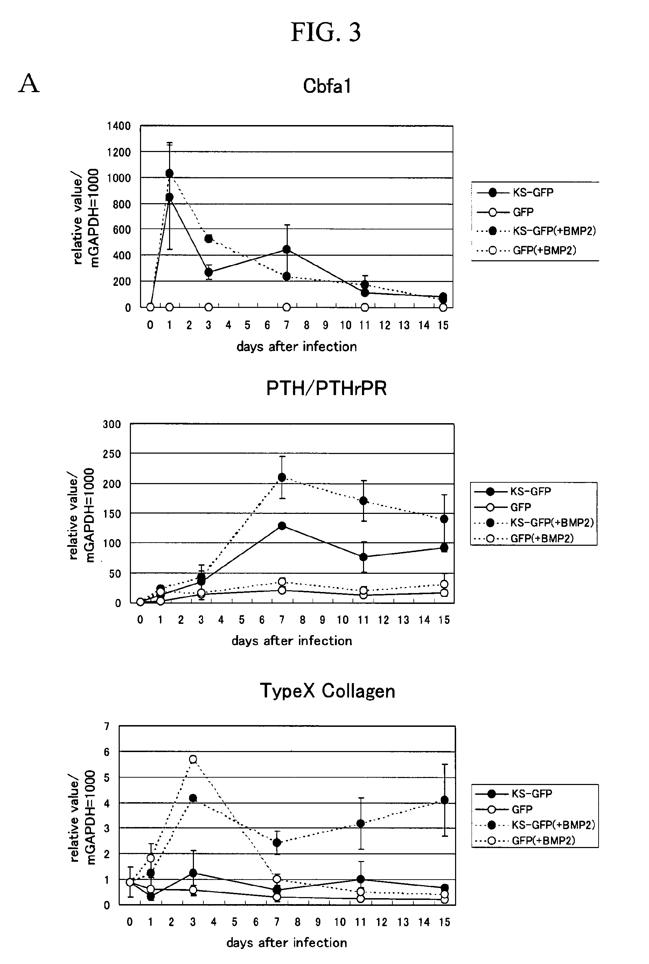

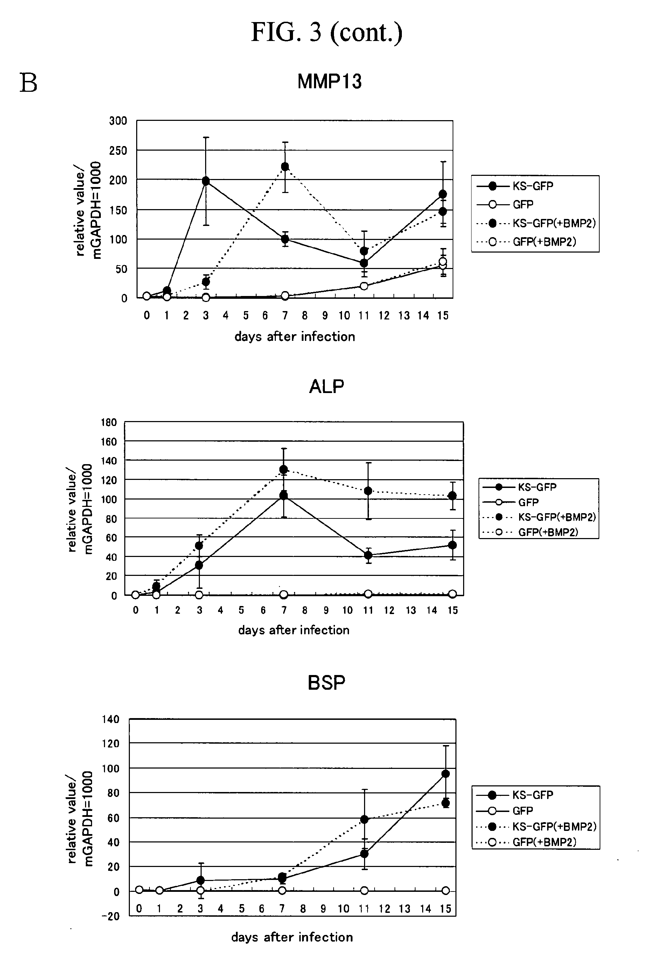

Fig. 3A and 3B show the results of analyzing the expressions of the Cbfa1 genes and of the cartilage differentiation-associated marker genes upon forced expression of Runx2/Cbfa1 in the Runx2/Cbfa1- and p53-deficient cell lines of the present invention (RU-1) with the use of an adenovirus (A: Cbfa1, PTH/PTHrPR, and type X collagen; B: MMP 13, ALP, and BSP). Changes in the gene expression levels in the presence and in the absence of BMP-2 were assayed via real-time PCR analysis over time, from 0 to 15 days after the forced expression of Runx2/Cbfa1.

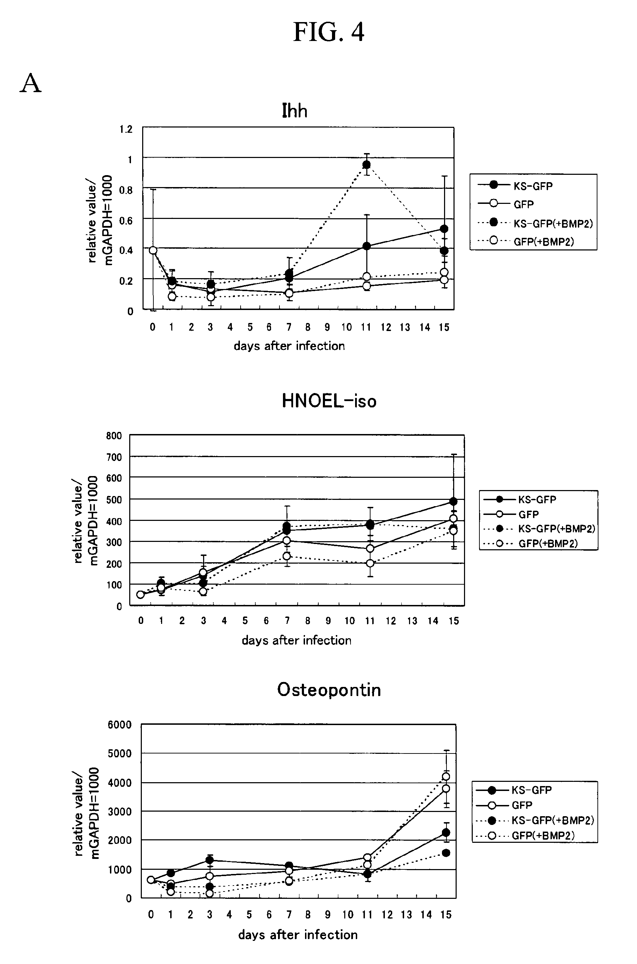

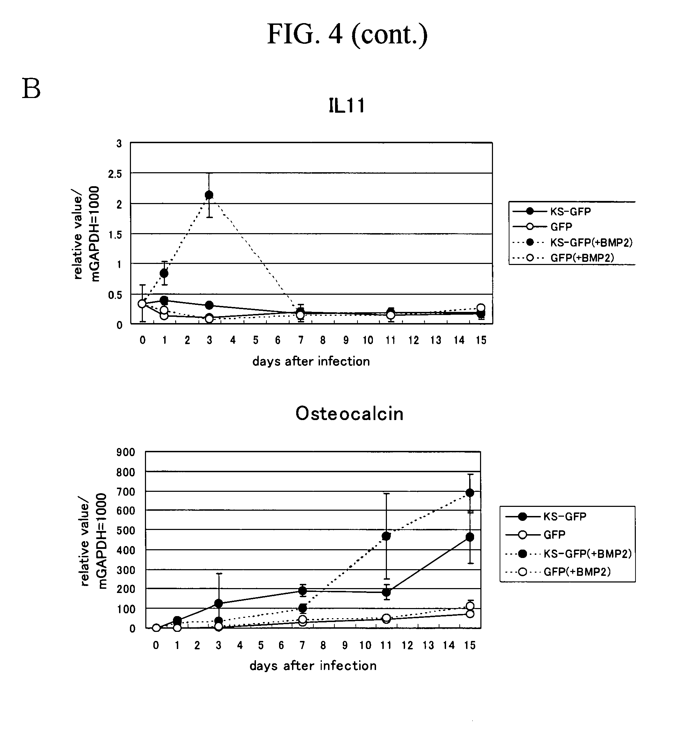

Fig. 4A and 4B show the results of analyzing the expressions of the cartilage differentiation-associated marker genes and the HNOEL-iso genes upon forced expression of Runx2/Cbfal in the Runx2/Cbfal- and p53-deficient cell lines of the present invention (RU-1) with the use of an adenovirus (A: Ihh, HNOEL-iso, and osteopontin; B: IL11 and osteocalcin). Changes in the gene expression levels in the presence and in the absence of BMP-2 were assayed via real-time PCR analysis over time, from 0 to 15 days after the forced expression of Runx2/Cbfal.

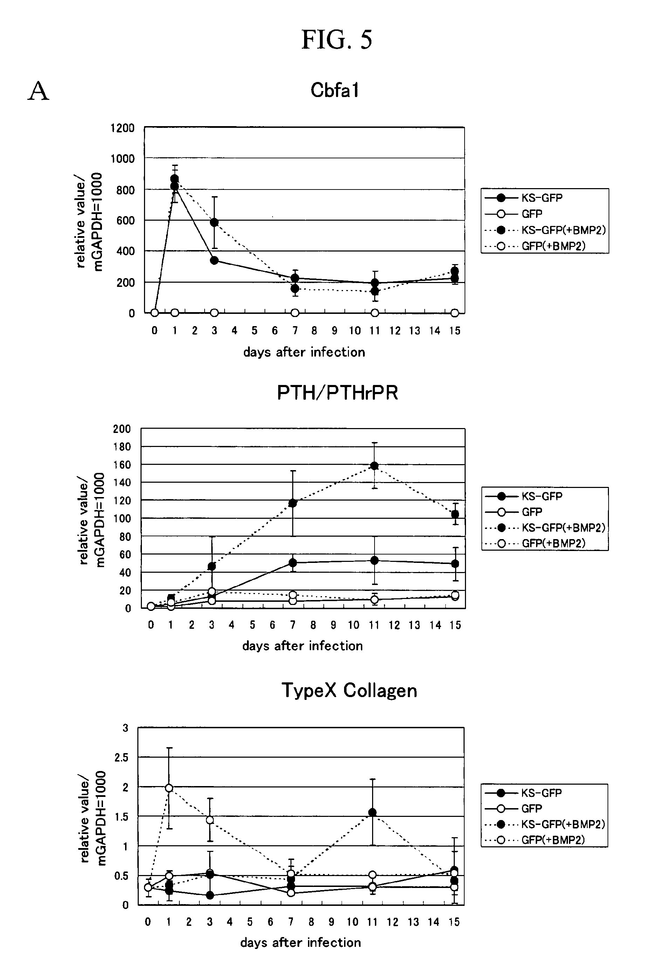

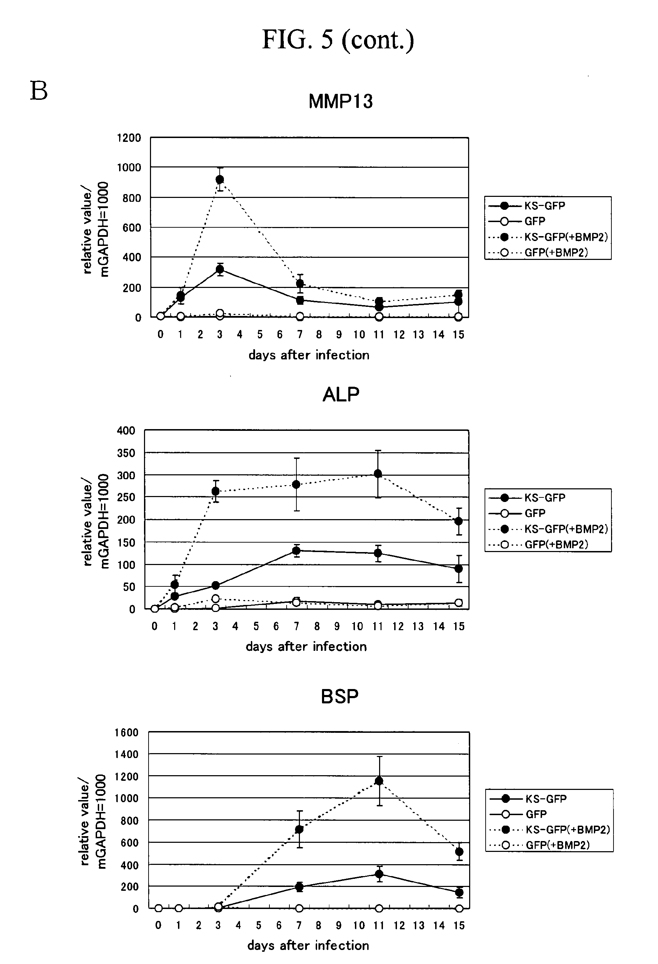

Fig. 5A and 5B show the results of analyzing the expressions of the Cbfal genes and the cartilage differentiation-associated marker genes upon forced expression of Runx2/Cbfa1 in the Runx2/Cbfa1- and p53-deficient cell lines of the present invention (RU-22) with the use of an adenovirus (A: Cbfa1, PHT/PHTrPR, and type X collagen; B: MMP13, ALP, and BSP). Changes in the gene expression levels in the presence and in the absence of BMP-2 were assayed via real-time PCR analysis over time, from 0 to 15 days after the forced expression of Runx2/Cbfa1.

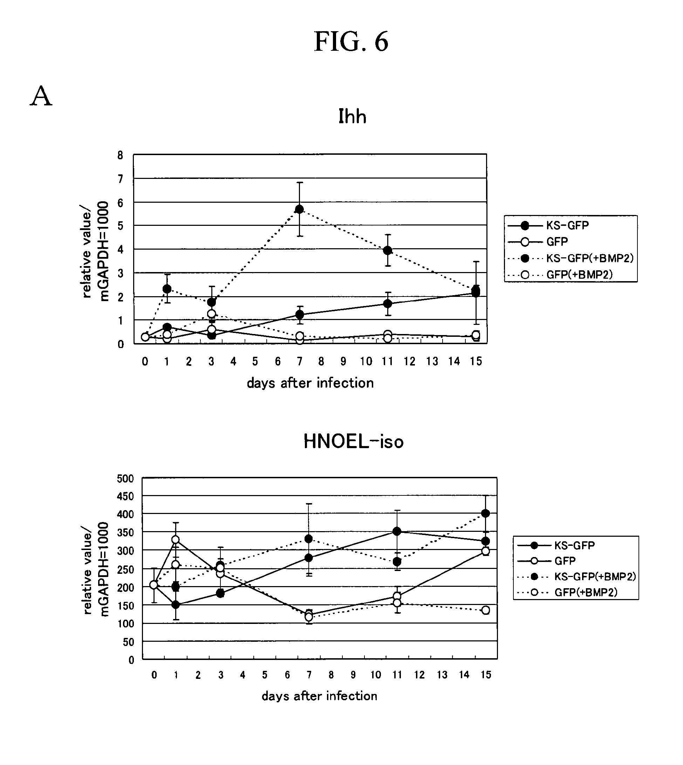

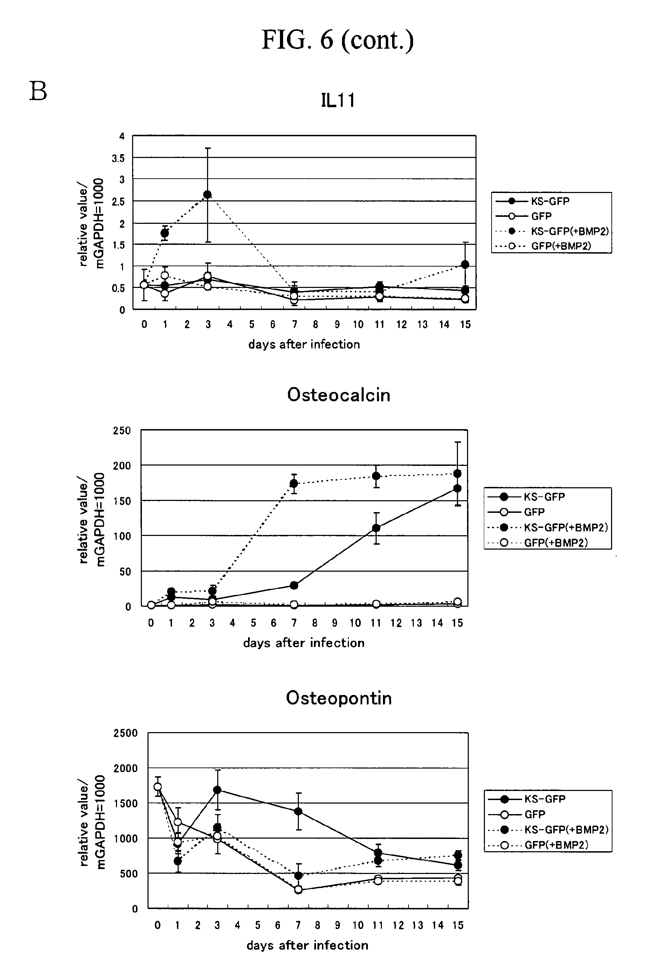

Fig. 6A and 6B show the results of analyzing the expressions of the cartilage differentiation-associated marker genes and the HNOEL-iso genes upon forced expression of Runx2/Cbfa1 in the Runx2/Cbfa1- and p53-deficient cell lines of the present invention (RU-22) with the use of an adenovirus (A: Ihh and HNOEL-iso; B: IL11, osteocalcin, and osteopontin). Changes in the gene expression levels in the presence and in the absence of BMP-2 were assayed via real-time PCR analysis over time, from 0 to 15 days after the forced expression of Runx2/Cbfal.

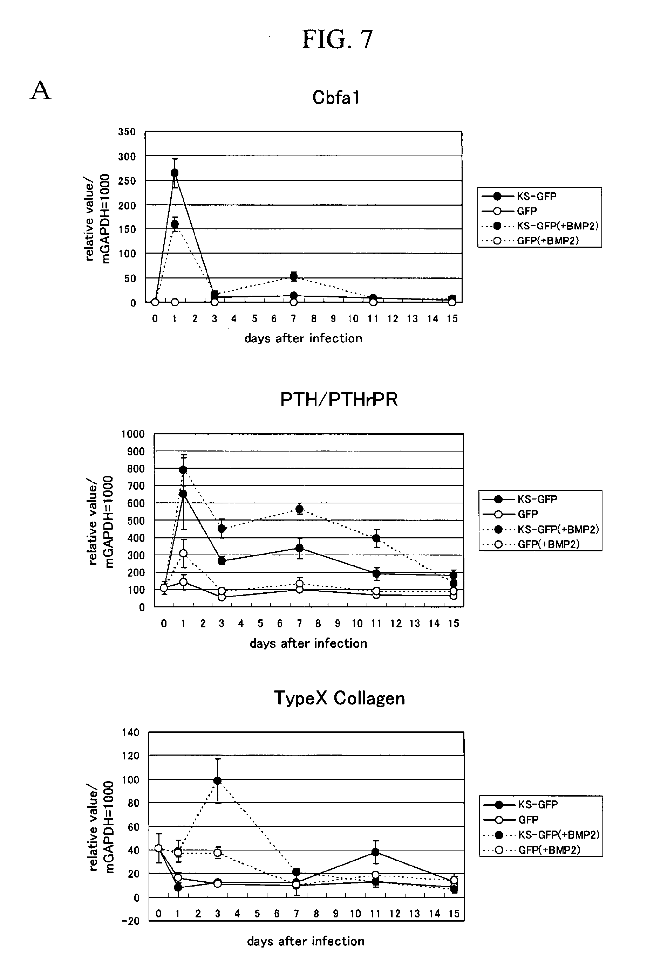

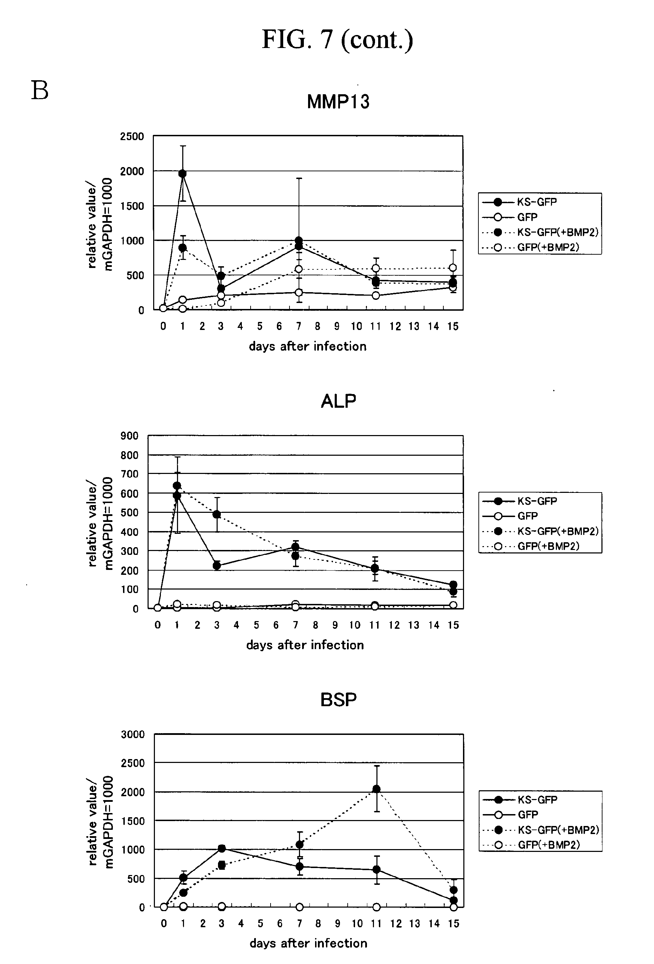

Fig. 7A and 7B show the results of analyzing the expressions of the Cbfal genes and the cartilage differentiation-associated marker genes upon forced expression of Runx2/Cbfa1 in the Runx2/Cbfa1-deficient mouse-derived primary cultured chondrocytes of the present invention with the use of an adenovirus (A: Cbfal, PHT/PHTrPR, and type X collagen; B: MMP13, ALP, and BSP). Changes in the gene expression levels in the presence and in the absence of BMP-2 were assayed via real-time PCR analysis over time, from 0 to 15 days after the forced expression of Runx2/Cbfa1.

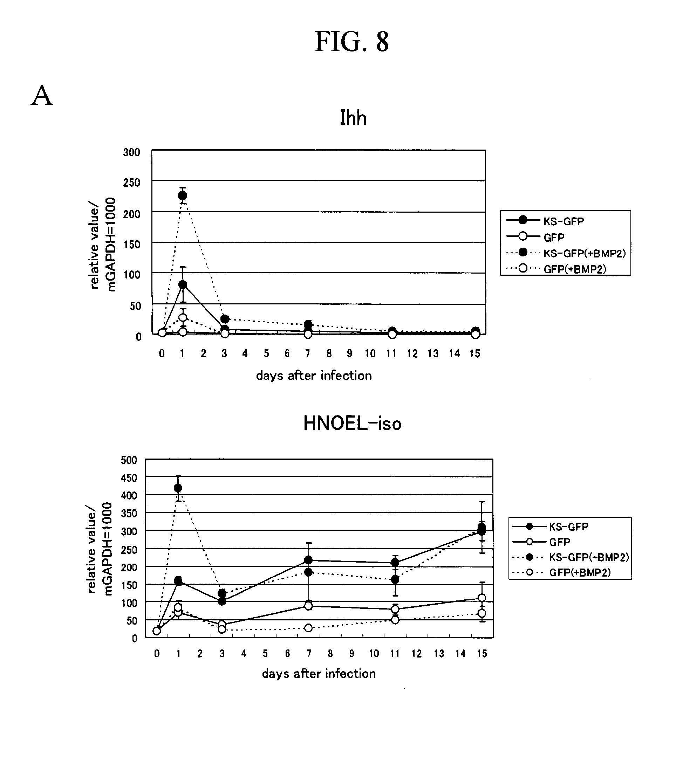

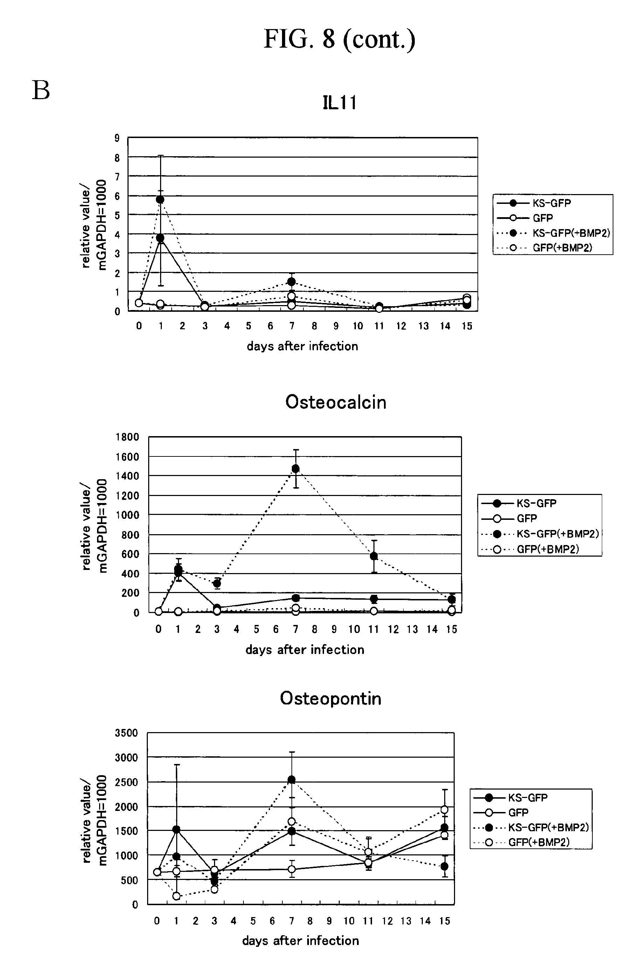

Fig. 8A and 8B show the results of analyzing the expressions of the cartilage differentiation-associated marker genes and the HNOEL-iso genes upon forced expression of Runx2/Cbfa1 in the Runx2/Cbfa1-deficient mouse-derived primary cultured chondrocytes of the present invention with the use of an adenovirus (A: Ihh and HNOEL-iso; B: IL11, osteocalcin, and osteopontin). Changes in the gene expression levels in the presence and in the absence of BMP-2 were assayed via real-time PCR analysis over time, from 0 to 15 days after the forced expression of Runx2/Cbfa1.

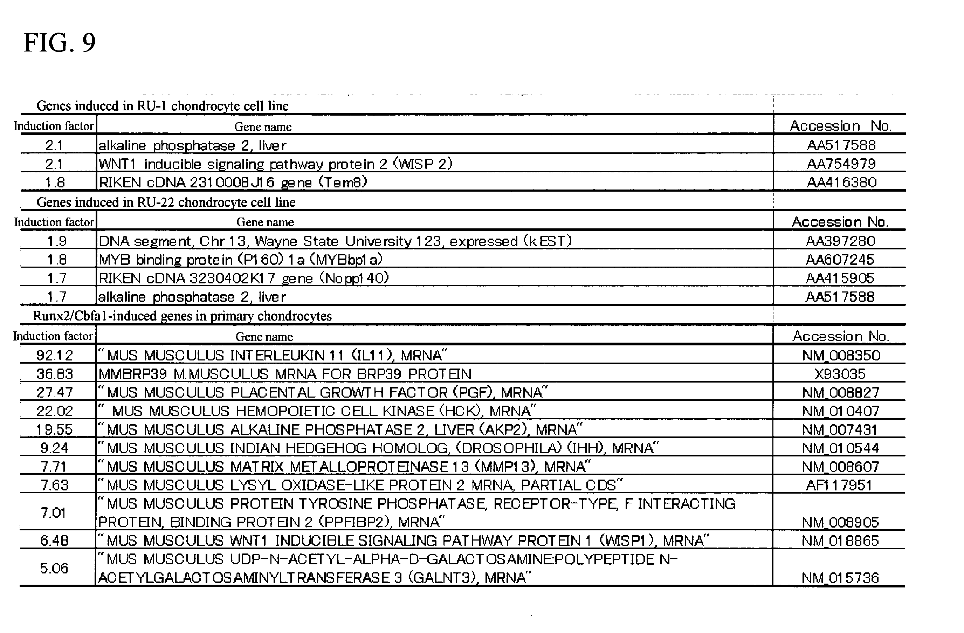

Fig. 9 shows the results of a DNA microarray analysis of the genes induced to express one day after the forced expression of Runx2/Cbfal in the Runx2/Cbfal-deficient cell lines (RU-1), the p53-deficient cell lines (RU-22), and the Runx2/Cbfal-deficient mouse-derived primary cultured cell lines, with the use of an adenovirus.

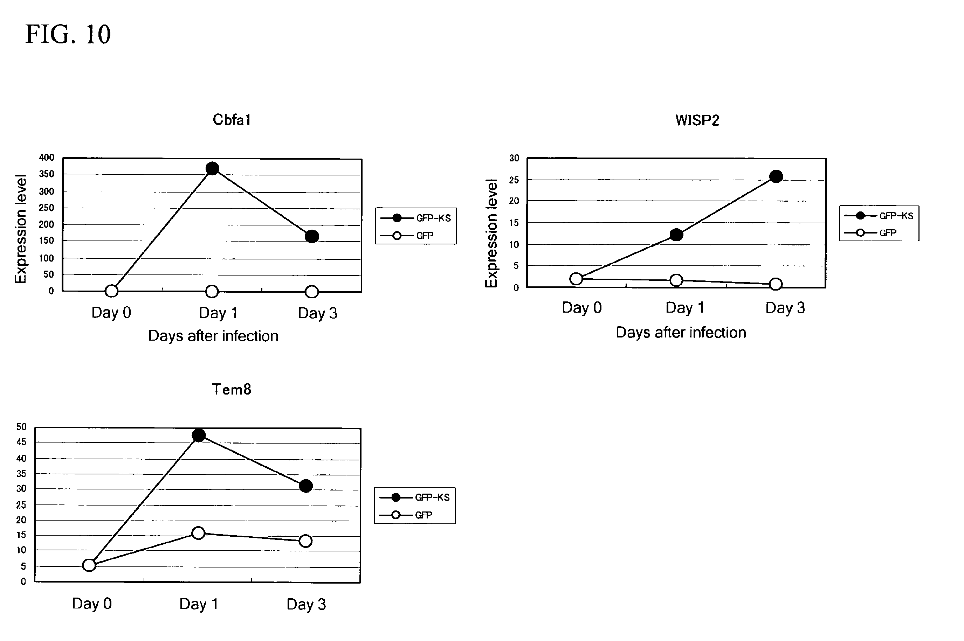

Fig. 10 shows the results of an experiment to reconfirm the Runx2/Cbfa1-induced expressions of the genes, the Runx2/Cbfa1-induced expressions of which had been found via DNA microarray analysis, via real-time PCR analysis. The Runx2/Cbfal-induced expressions of the Cbfal, Tem8, and WISP2 genes was reconfirmed with the use of the cell line (RU-1), the expression of which was found to be induced via DNA microarray analysis.

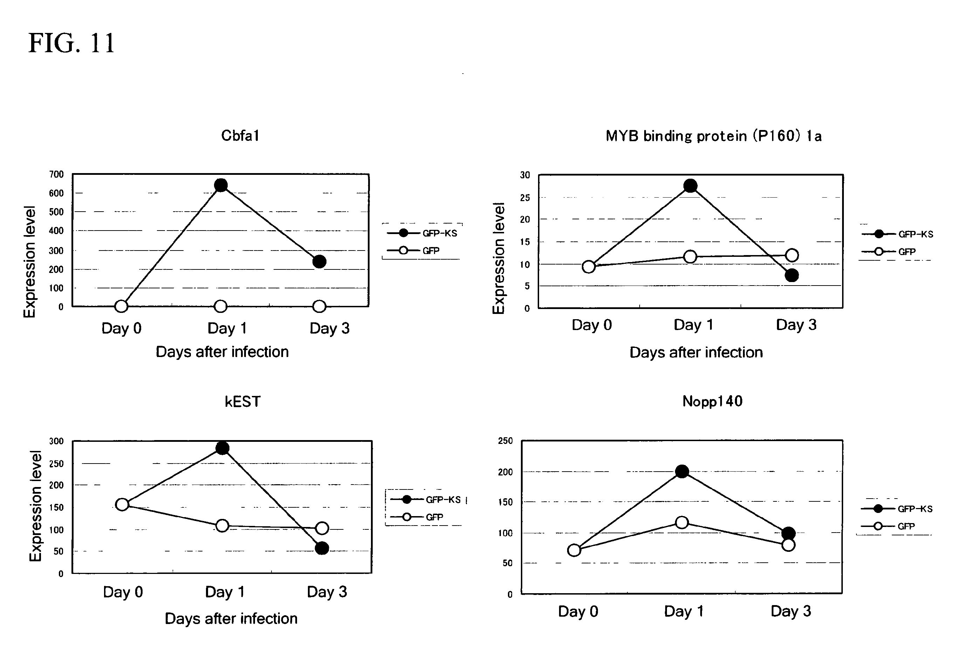

Fig. 11 shows the results of an experiment to reconfirm the Runx2/Cbfa1-induced expressions of the genes, the Runx2/Cbfa1-induced expressions of which had been found via DNA microarray analysis, via real-time PCR analysis. The Runx2/Cbfa1-induced expressions of the Cbfa1, kEST, the MYB binding protein (p160) 1a, and Nopp140 genes was reconfirmed with the use of the cell line (RU-22), the expression of which was found to be induced via DNA microarray analysis.

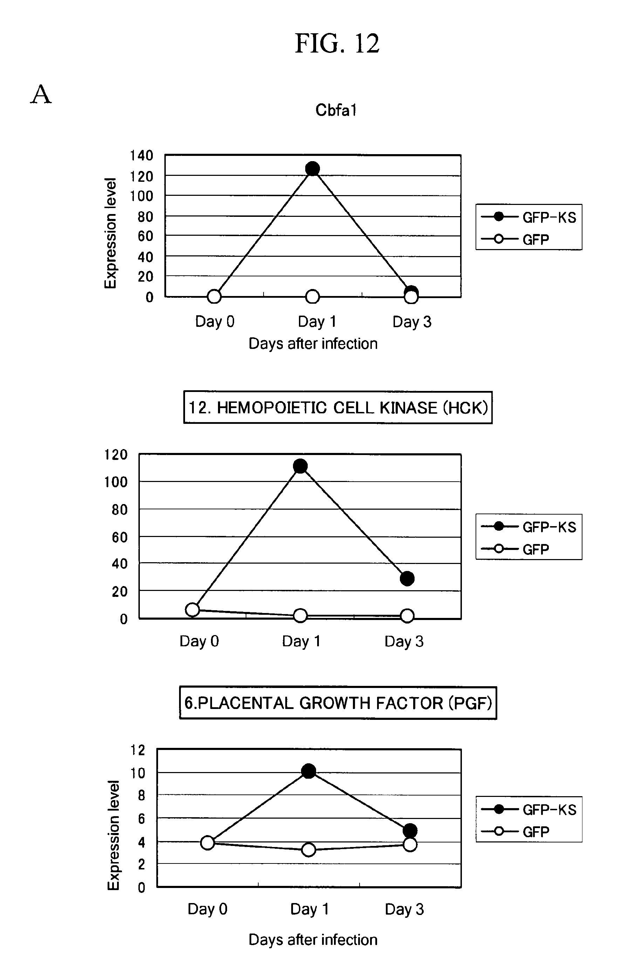

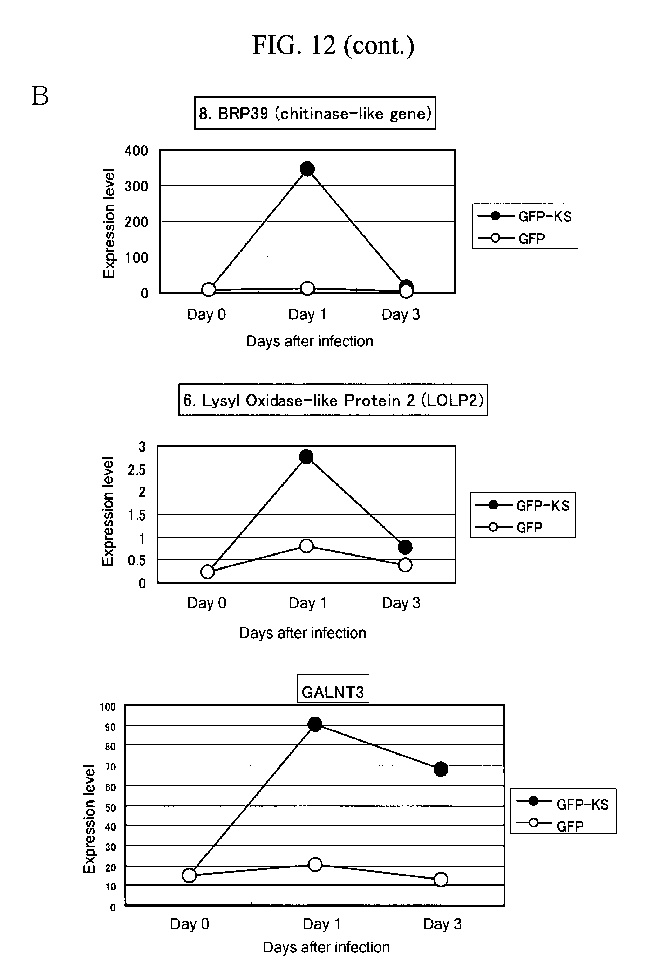

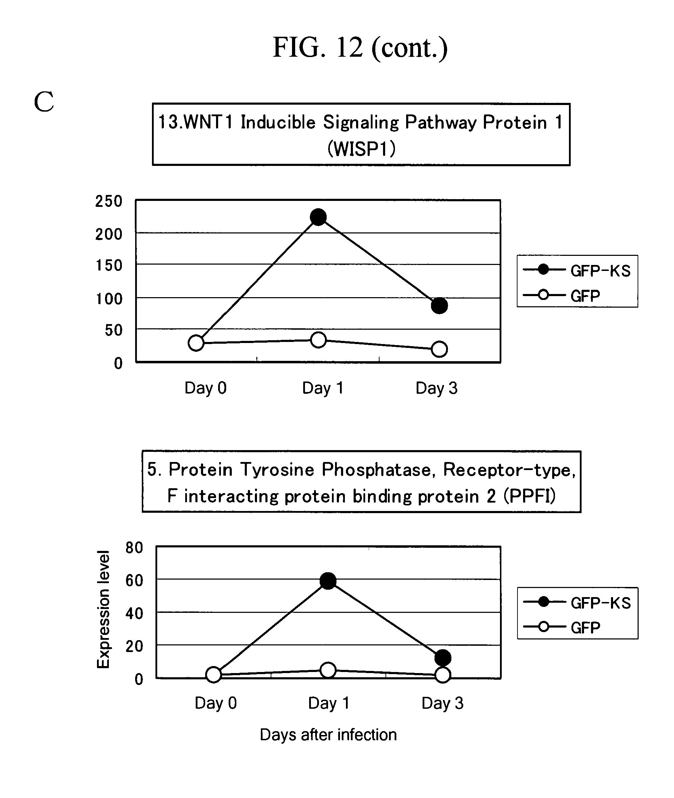

Fig. 12A to 12C show the results of an experiment to reconfirm the Runx2/Cbfa1-induced expressions of the genes, the Runx2/Cbfa1-induced expressions of which had been found via DNA microarray analysis, via real-time PCR analysis. The Runx2/Cbfa1-induced expressions of the genes were reconfirmed with the use of the primary cultured cell line, the expression of which was found to be induced via DNA microarray analysis.

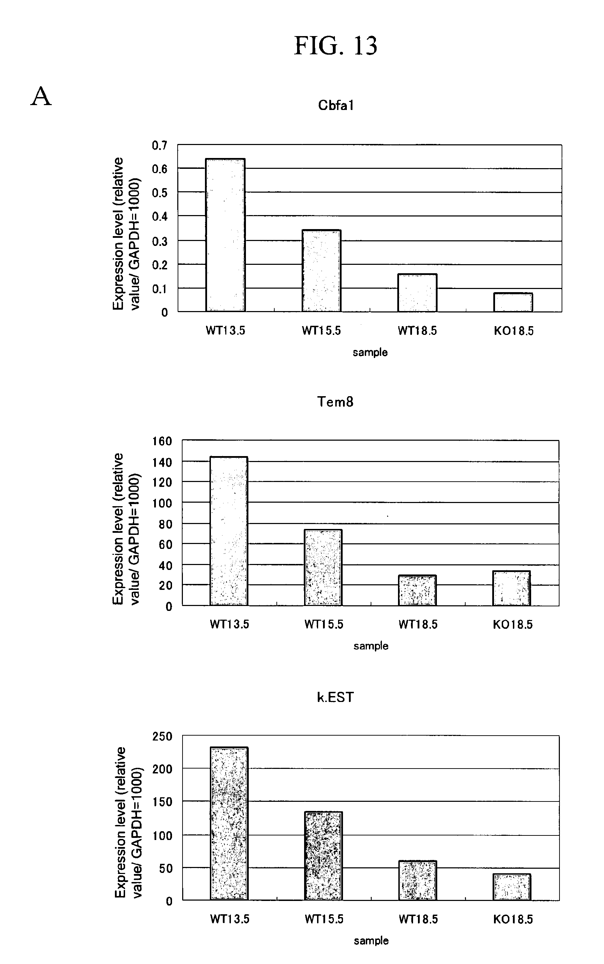

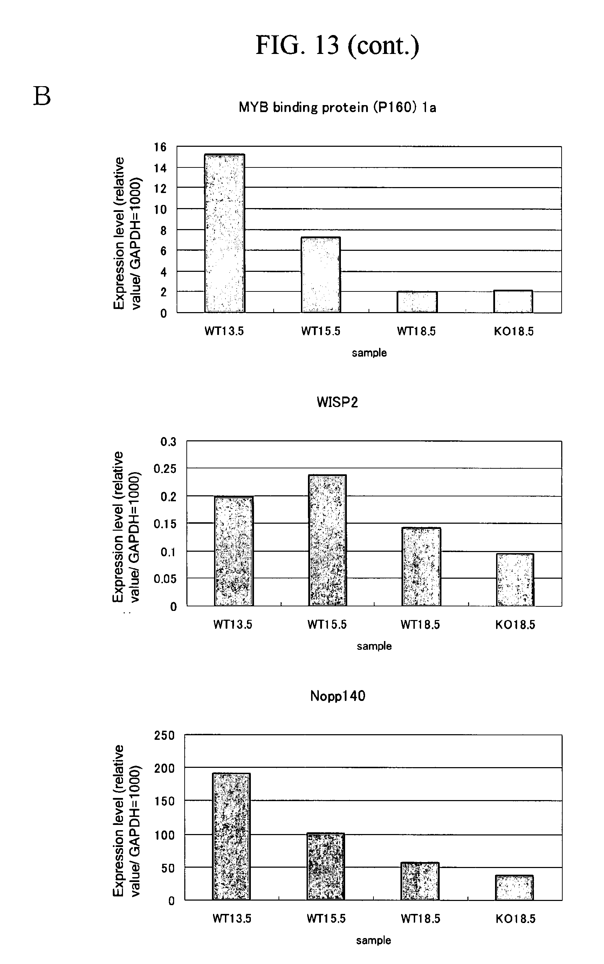

Fig. 13A and 13B show the results of analysis of the expressions of the genes, the Runx2/Cbfa1-induced expressions of which had been found via DNA microarray analysis, in an embryonic skeleton of a wild-type mouse and in that of a Runx2/Cbfa1-deficient mouse. Total RNAs were extracted from the skeleton of the wild-type mouse (WT) at days 13.5, 15.5, and 18.5 of embryonic development and from a Runx2/Cbfa1-deficient mouse (KO) at day 18.5 thereof, and cDNAs were synthesized. Thereafter, the synthesized cDNAs were used as templates to analyze the expression patterns via real-time PCR (the chondrocyte cell lines).

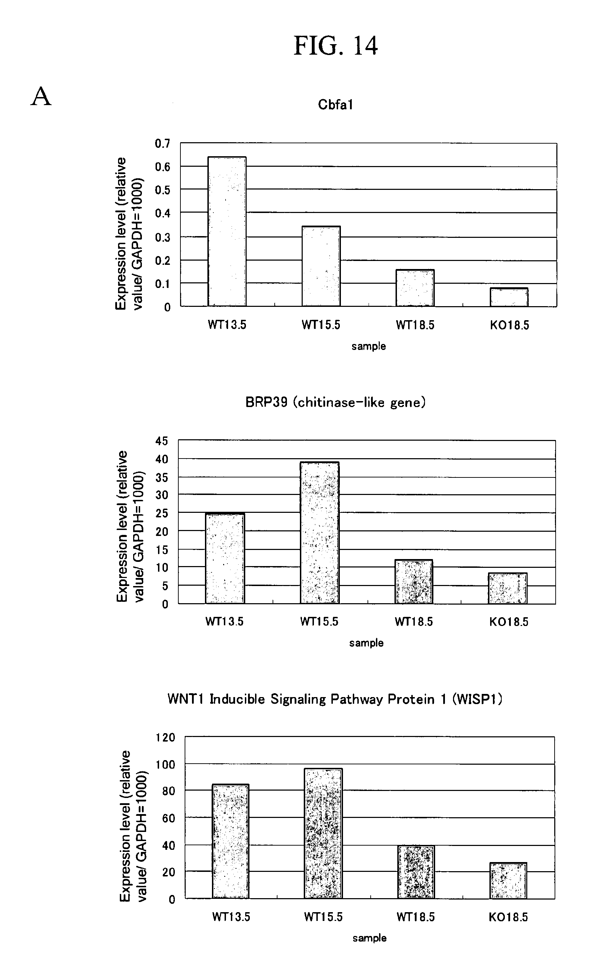

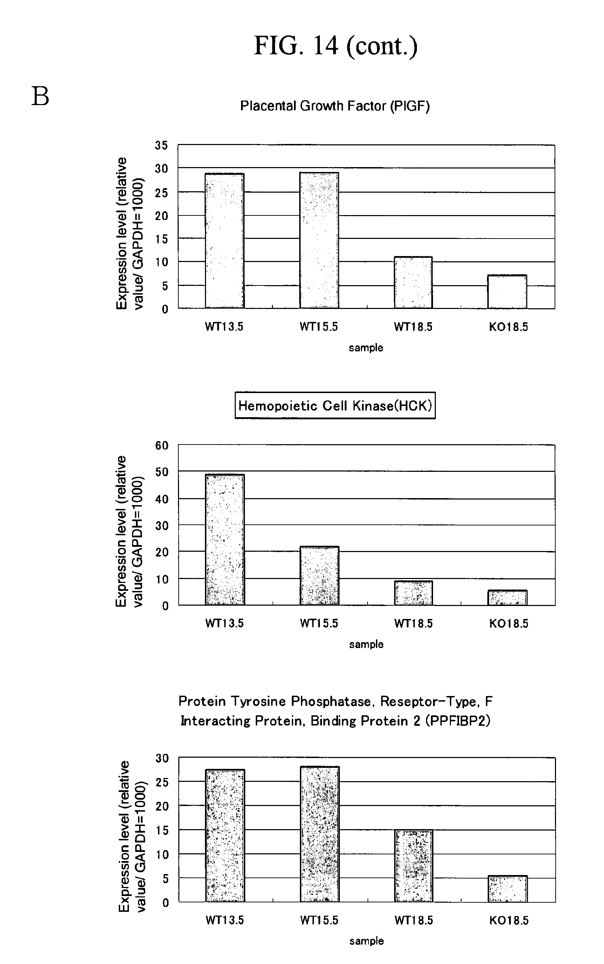

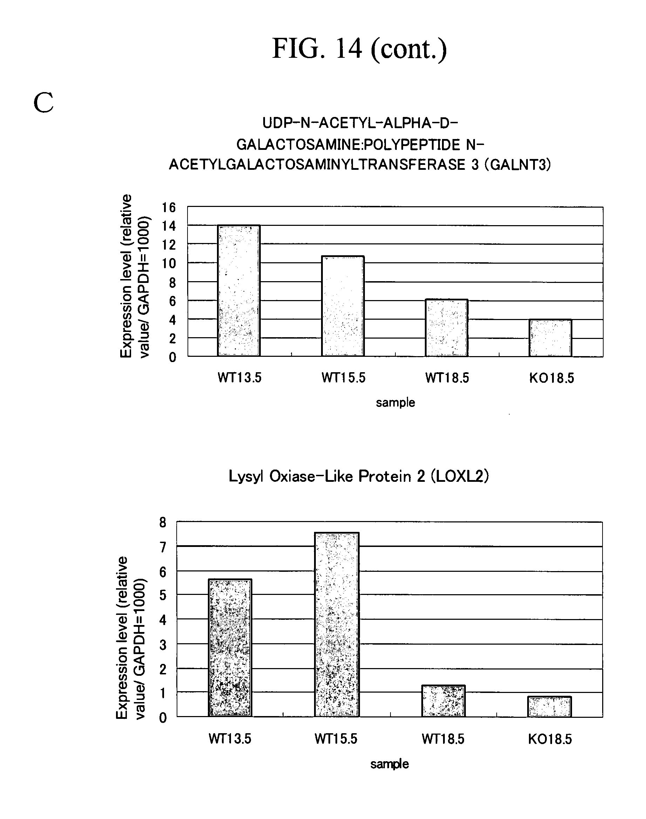

Fig. 14A to 14C show the results of expression analysis of the genes, the Runx2/Cbfa1-induced expressions of which had been found via DNA microarray analysis, in an embryonic skeleton of a wild-type mouse and in that of a Runx2/Cbfal-deficient mouse. Total RNAs were extracted from the skeleton of the wild-type mouse (WT) at days 13.5, 15.5, and 18.5 of embryonic development and from a Runx2/Cbfa1-deficient mouse (KO) at day 18.5 thereof, and cDNAs were synthesized. Thereafter, the synthesized cDNAs were used as templates to analyze the expression patterns via real-time PCR (the primary cultured chondrocytes).

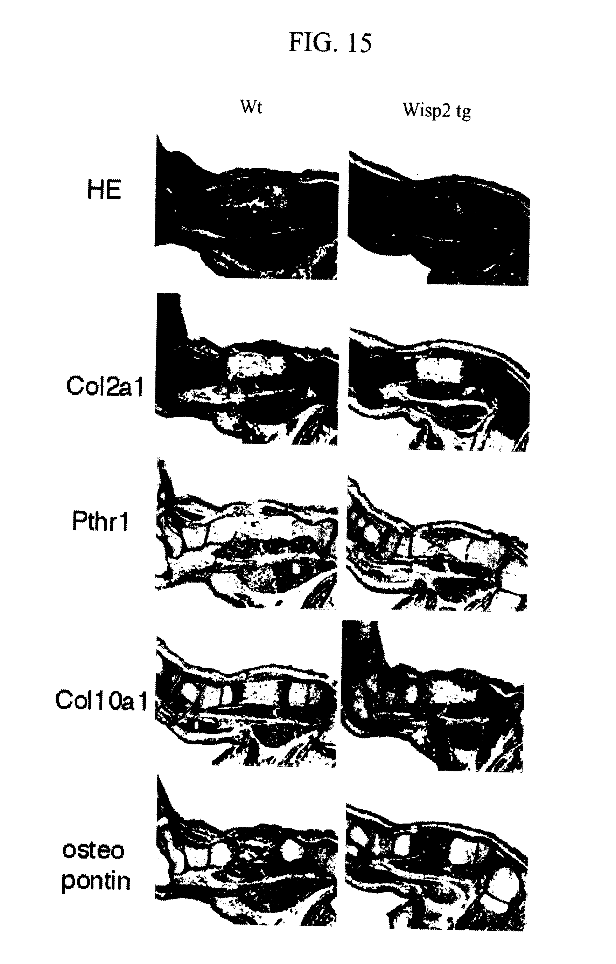

Fig. 15 shows photographs of images of HE-stained sections of the lower-body skeletons of a Wisp2 transgenic mouse (HE) and images showing the results of expression analysis via *in situ* hybridization of type II collagen (Col2al), a PTH receptor (Pthrl), type X collagen (Col10al), and osteopontin. These photographs indicate that cartilage differentiation is delayed in the Wisp2 transgenic mouse (Wisp2 tg) compared with the wild-type mouse (Wt).

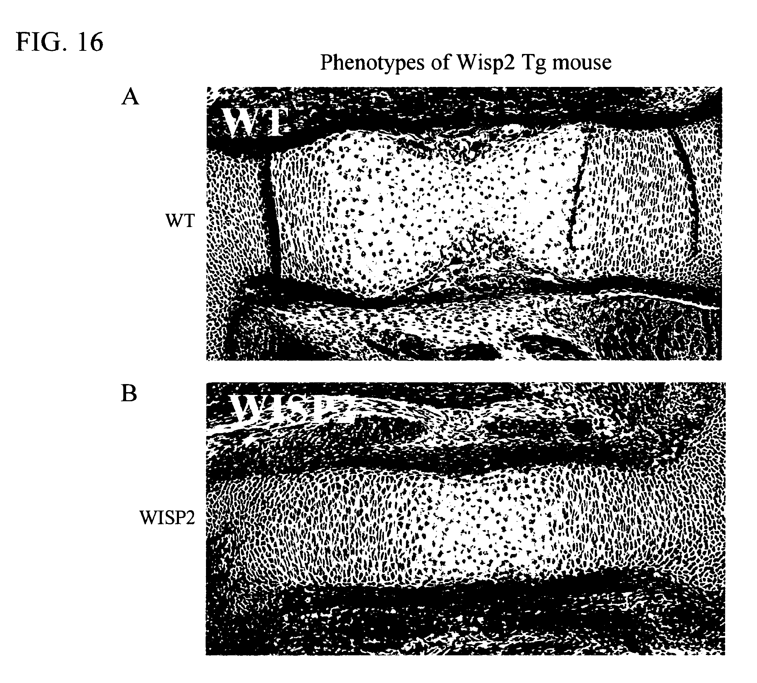

Fig. 16A and B show photographs of images of the HE-stained lower-body skeleton of a wild-type mouse (A: WT) and that of a Wisp2 transgenic mouse (B: WISP2). These photographs indicate that cartilage differentiation is delayed in the Wisp2 transgenic mouse compared with the wild-type mouse.

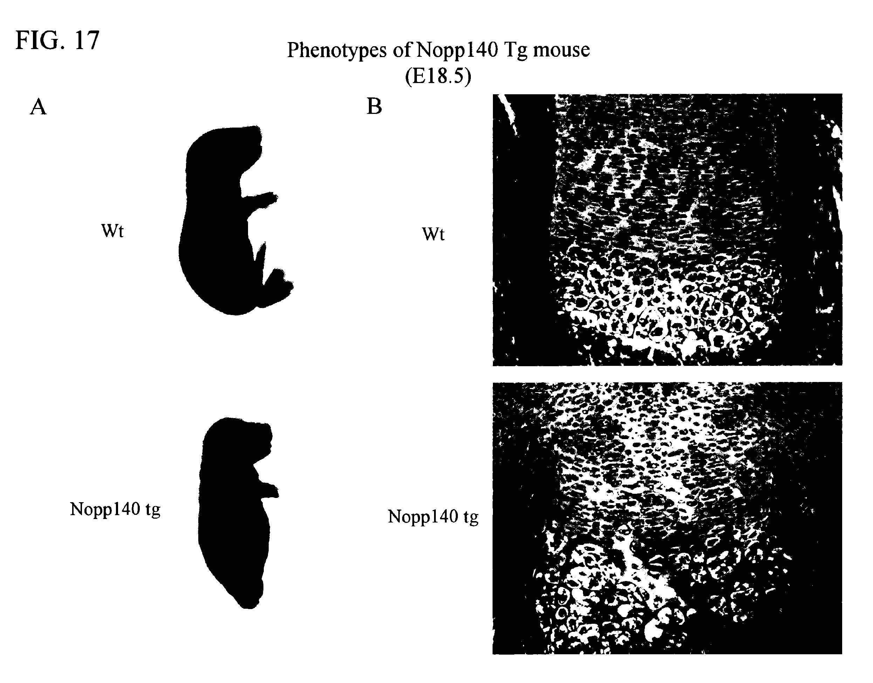

Fig. 17A and B show photographs of the appearances of the wild-type mouse (Wt) and of the Nopp 140 transgenic mouse (Nopp140 tg) (A) and photographs of images of the HE-stained tibias thereof (B). These photographs indicate that cartilage differentiation is advanced at a given stage in the Nopp 140 transgenic mouse compared with the wild-type mouse.

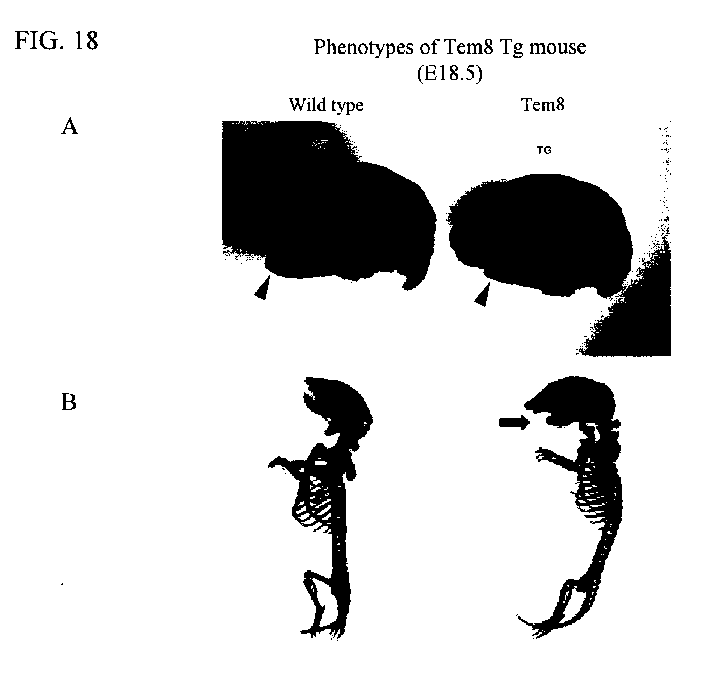

Fig. 18 shows photographs showing the head appearance of a wild-type mouse and that of the Tem8 transgenic mouse (A) and photographs of images of stained skeletons thereof (B). These photographs indicate that cartilage differentiation is advanced in the Tem8 transgenic mouse (Tem8) compared with the wild-type mouse.

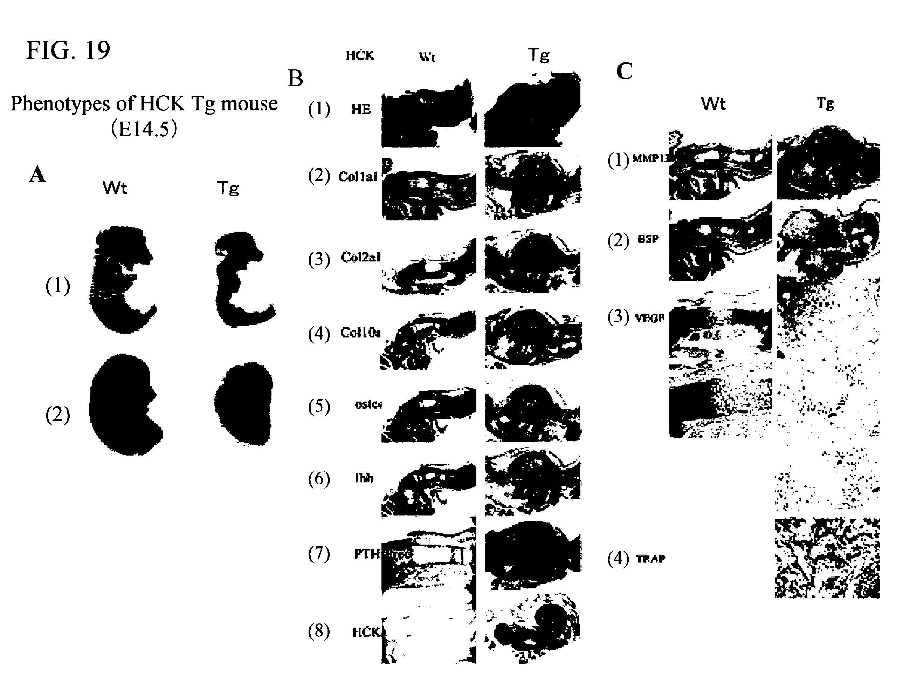

Fig. 19A shows photographs of the appearance of a wild-type mouse and that of the HCK transgenic mouse at day 14.5 of embryonic development (A-(2)) and an image of the stained skeletons thereof (A-(1)).
Fig. 19B and 19C are photographs showing images of HE staining (B-(1), HE); images showing the results of expression analysis via *in situ* hybridization for the followings: type I collagen (B-(2): Col1a1), type II collagen (B-(3): Col2a1), type X collagen (B-(4): Col10a1), osteopontin (B-(5): osteopontin), Indian hedgehog (B-(6): Ihh), a PTH receptor (B-(7): Pthr1), Hck (B-(8)), MMP13 (C-(1)), BSP (C-(2)), and VEGF (C-(3)); and images showing the results of analyzing osteoclasts by TRAP staining (C-(4)). The HCK transgenic mouse had a smaller body size and stubbier limbs, and it exhibited abnormalities in differentiation in cartilage tissues.

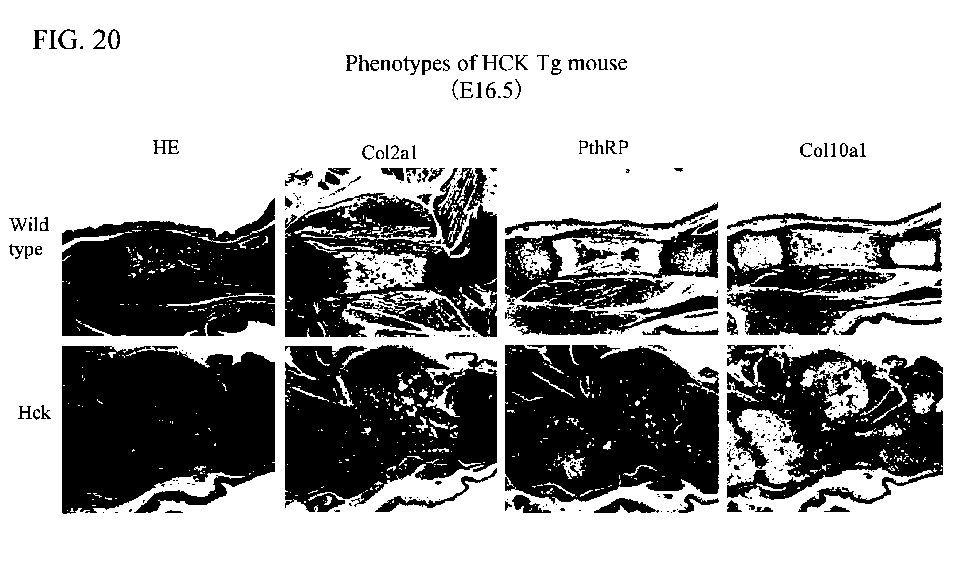

Fig. 20 shows photographs of the HE-stained HCK transgenic mouse at day 16.5 of embryonic development (HE) and the results of expression analysis by *in situ* hybridization for type II collagen (Col2a1), a PTH receptor (PthRP), and type X collagen (Col10a1). In the tibia of the HCK transgenic mouse, unorganized abnormal growth of cells inhibited the formation of the normal epiphyseal plate, and longitudinal growth was abnormal.

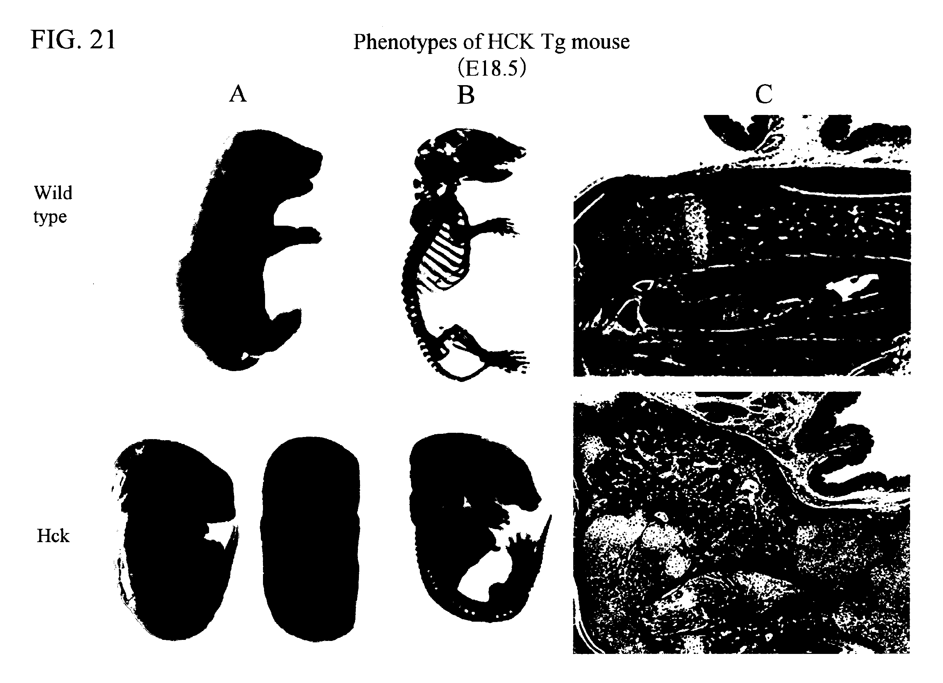

Fig. 21A to C show photographs of the appearance of the HCK transgenic mouse at day 18.5 of embryonic development (A), the images of stained skeleton thereof (B), and the results of HE staining thereof (C). Compared with a wild-type mouse, the HCK transgenic mouse had a smaller body size, a more protruding abdomen, and stubbier limbs (Fig. 21A). There was a cleavage between the nose and the upper jaw, and the process of fusion between the nose and the upper jaw was inhibited. Skeleton staining revealed that calcified tissue stained with alizarin red was decreased and extracellular matrix stained with alizarin blue was increased (Fig. 21B). The chondrocytes of the mouse were found to be immature based on the HE-stained images, and invasion and proliferation of mesenchymal cells were observed in the vicinity thereof. The enchondral bone had lost its proximal-distal direction and exhibited abnormal morphologies. The growth plates were not organized and joints had adhered to each other (Fig. 21C).

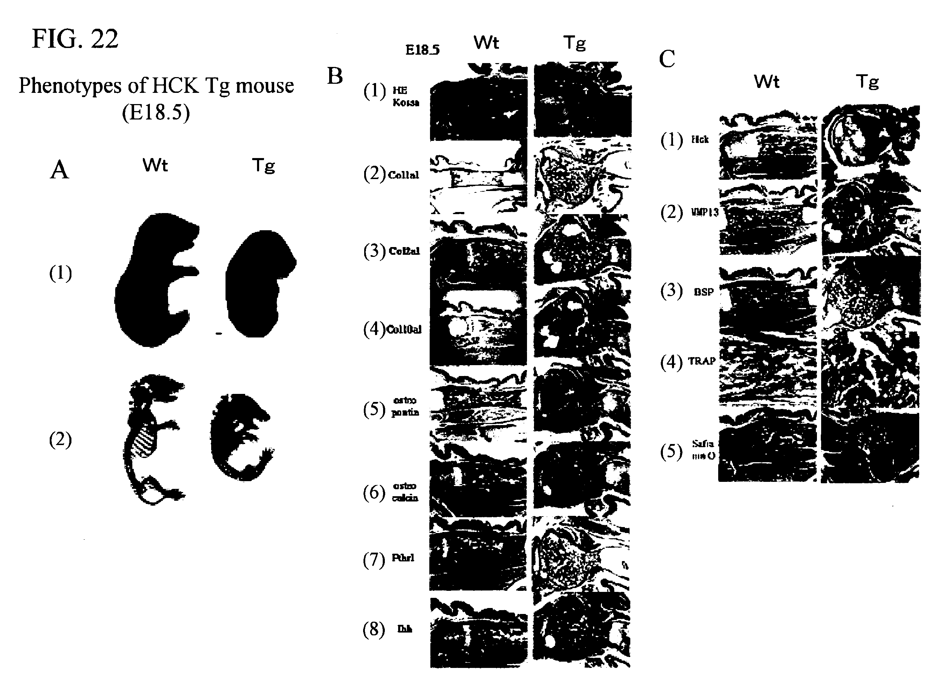

Fig. 22A is a photograph showing the appearance of the HCK transgenic mouse at day 18.5 of embryonic development (A-(1)) and an image of the stained skeleton thereof (A-(2)).
Fig. 22B and 22C are photographs of images of HE- and Kossa-staining (B-(1), HE-Kossa); images showing the results of expression analysis by *in situ* hybridization for type I collagen (B-(2): Collal), type II collagen (B-(3): Col2a1), type X collagen (B-(4): Col10a1), osteopontin (B-(5): osteopontin), osteocalcin (B-(6): osteocalcin), a PTH receptor (B-(7): Pthr1), Indian hedgehog (B-(8): Ihh), Hck (C-(1)), MMP13 (C-(2)), and BSP (C-(3)); images showing the results of analyzing osteoclasts by TRAP staining (C-(4)); and images showing the results of analyzing proteoglycan by safranin O staining (C-(5)). The number of Col2a1-expressing chondrocytes decreased, and bone formation involved mesenchymal cell invasion and proliferation without normal processes.

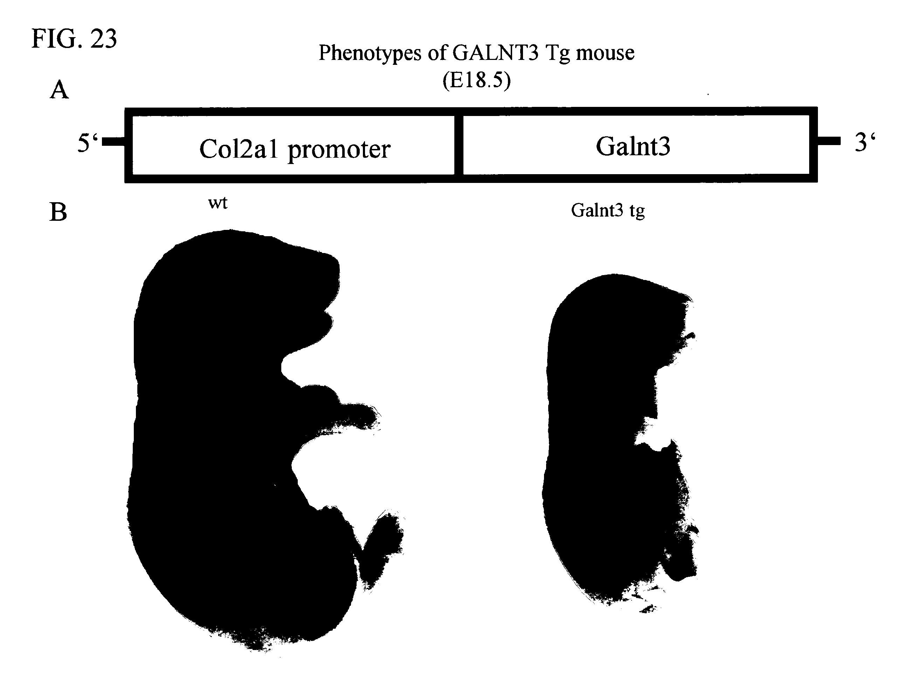

Fig. 23A and B show the structure of a gene construct for preparing the GALNT3 transgenic mouse (A) and a photograph of the appearance of the GALNT3 transgenic mouse at day 18.5 of embryonic development (B). Compared with a wild-type mouse (wt), the GALNT3 transgenic mouse (Galnt3 tg) had a smaller body size, stubbier limbs, a smaller pectoral region, and a more protruding abdomen.

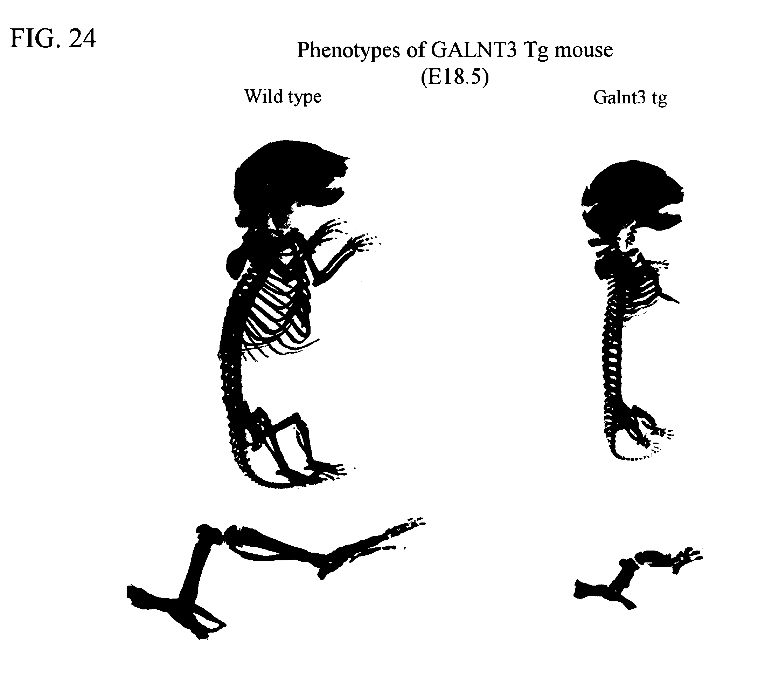

Fig. 24 is a photograph showing an image of stained skeleton of the GALNT3 transgenic mouse at day 18.5 of embryonic development. Compared with a wild-type mouse, the GALNT3 transgenic mouse (Galnt3 tg) had a smaller pectoral region and the significantly decreased bones calcified upon enchondral ossification.

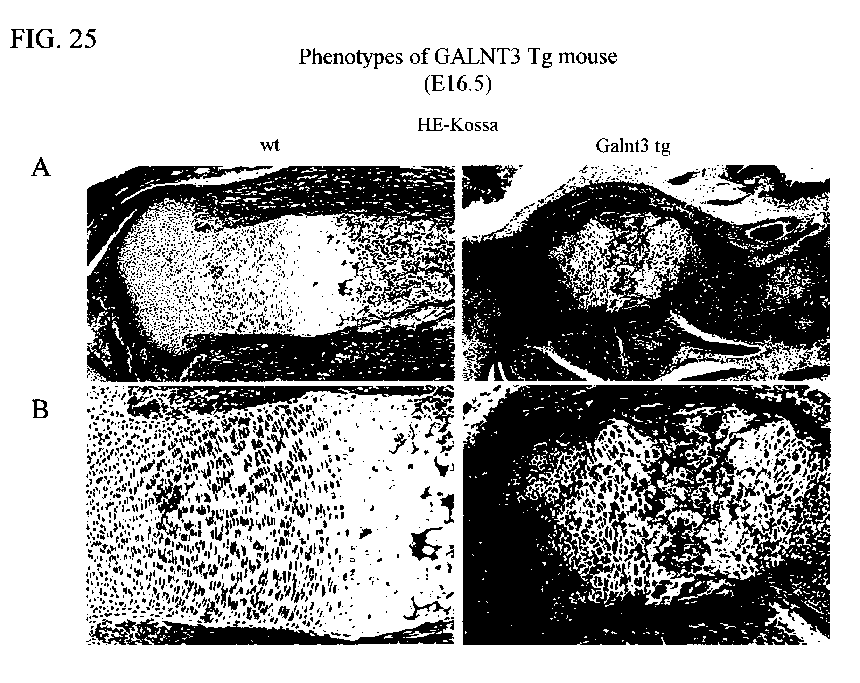

Fig. 25A and B show photographs of images of HE- and Kossa-stained tibia of the GALNT3 transgenic mouse at day 16.5 of embryonic development (B is a partial enlarged image of A). Angioinvasion was observed in a wild-type mouse (wt); however, it was not observed in the GALNT3 transgenic mouse (Galnt3 tg), and articular cavity formation was incomplete.

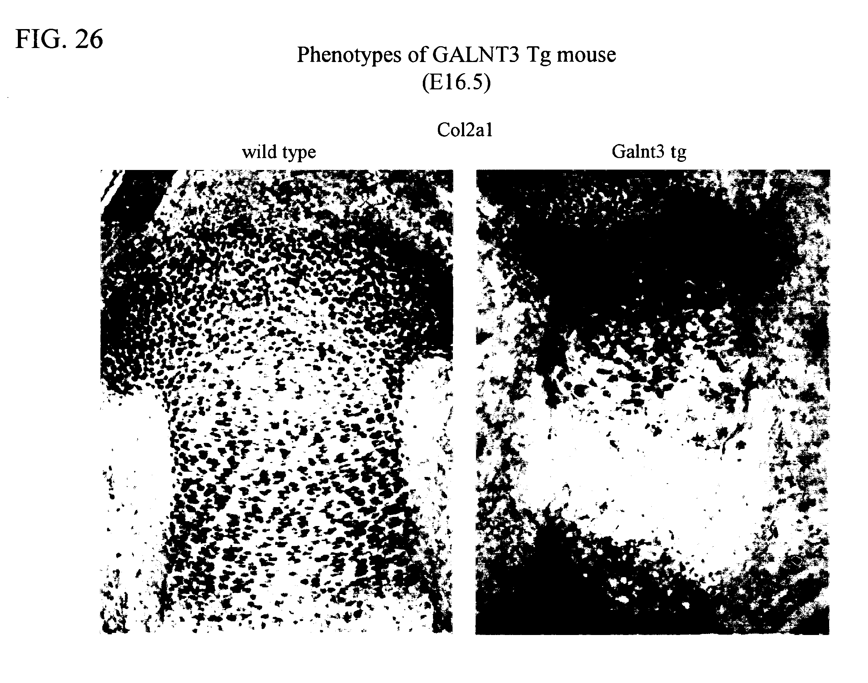

Fig. 26 is a photograph showing the results of expression analysis via *in situ* hybridization for type II collagen (Col2a1) in the tibia of the GALNT3 transgenic mouse at day 16.5 of embryonic development. Compared with a wild-type mouse, the GALNT3 transgenic mouse (Galnt3 tg) exhibited abnormal distribution of type II collagen expression.

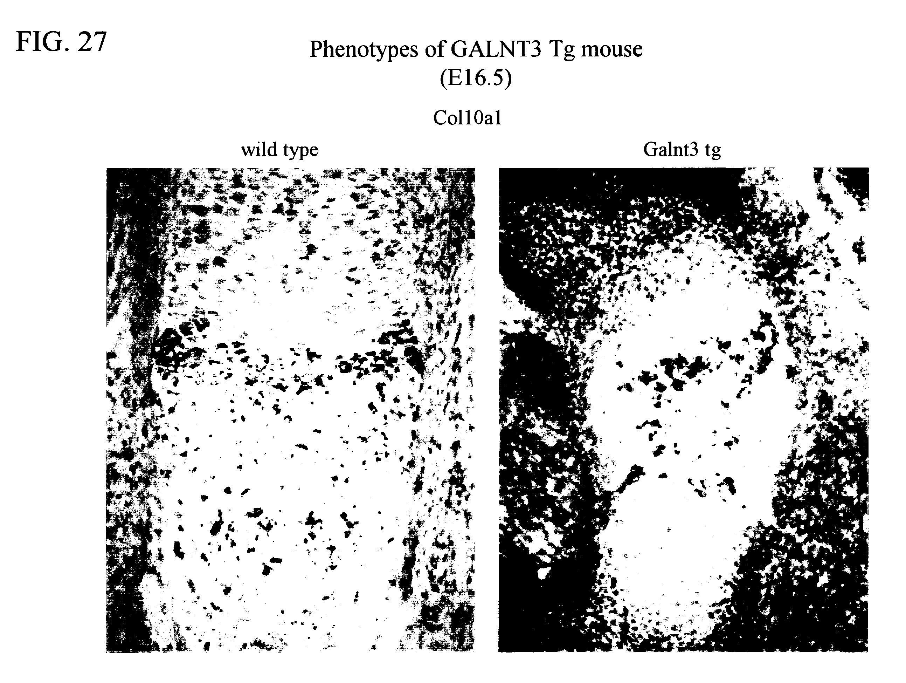

Fig. 27 is a photograph showing the results of expression analysis via *in situ* hybridization for type X collagen (Col10a1) in the tibia of the GALNT3 transgenic mouse at day 16.5 of embryonic development. Type X collagen expression was observed in hypertrophic cartilage of both the wild-type mouse and the GALNT3 transgenic mouse (Galnt3 tg).

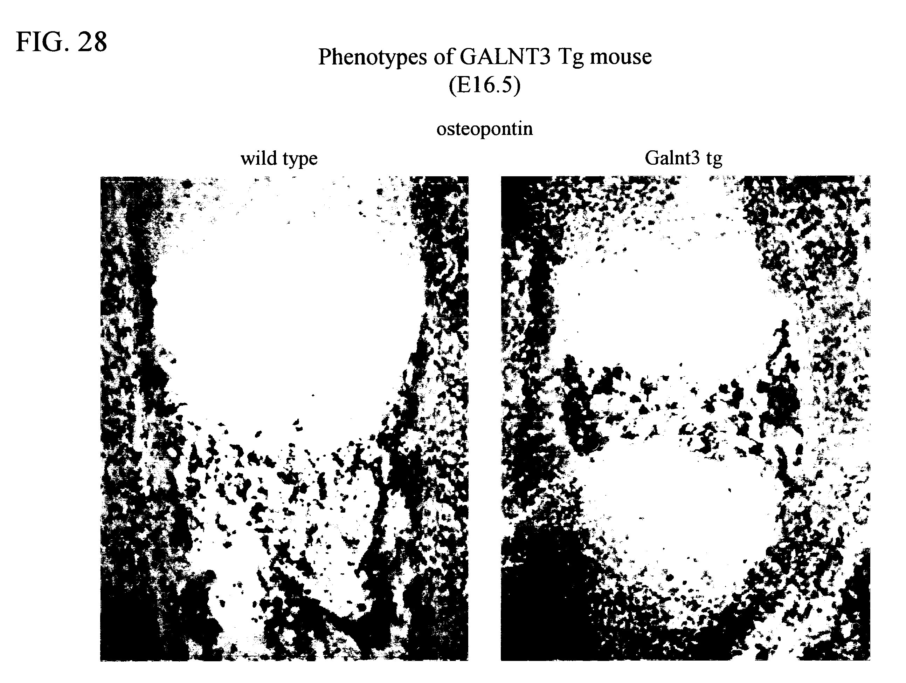

Fig. 28 is a photograph showing the results of expression analysis via *in situ* hybridization for osteopontin in the tibia of the GALNT3 transgenic mouse at day 16.5 of embryonic development. Osteopontin expression was observed in hypertrophic cartilage of both the wild-type mouse and the GALNT3 transgenic mouse (Galnt3 tg).

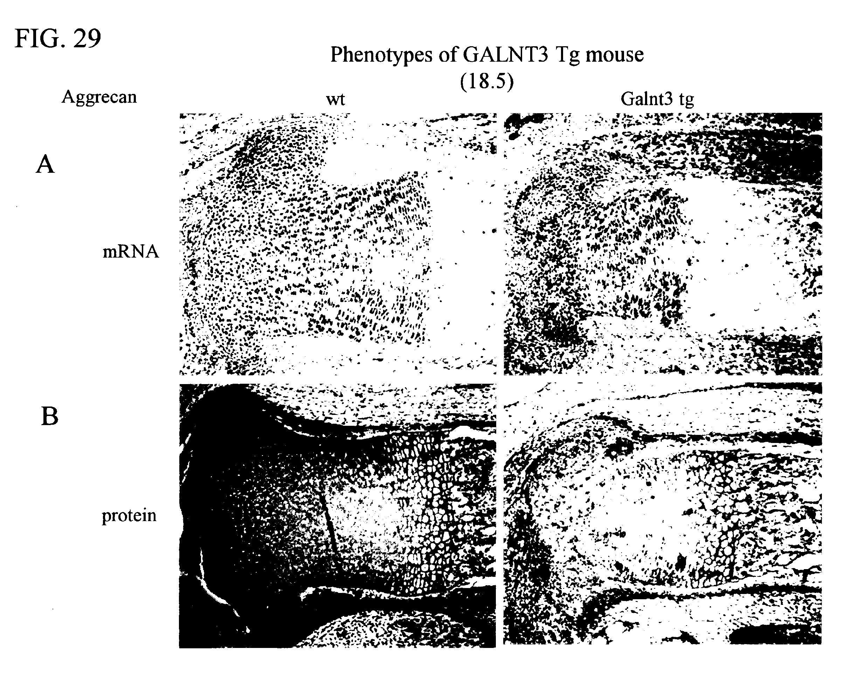

Fig. 29 shows photographs of the results of expression analysis via *in situ* hybridization (A: mRNA) and via immunostaining (B: protein) of aggrecan in the tibia of the GALNT3 transgenic mouse at day 18.5 of embryonic development. The mRNA expression of aggrecan (A) was slightly increased in the GALNT3 transgenic mouse (Galnt3 tg) compared with the wild-type mouse (wt); however, aggrecan expression was decreased at the protein level (B: protein).

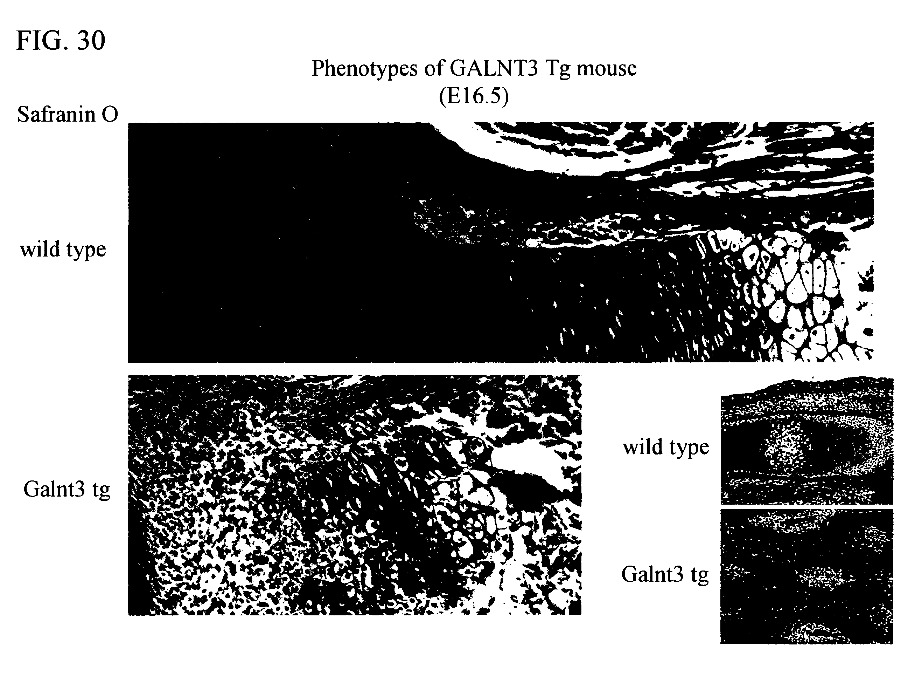

Fig. 30 is a photograph showing the results of safranin O staining of the tibia of the GALNT3 transgenic mouse at day 16.5 of embryonic development. Compared with a wild-type mouse, the staining affinity of the GALNT3 transgenic mouse (Galnt3 tg) was significantly deteriorated, which indicates significantly decreased proteoglycan content.

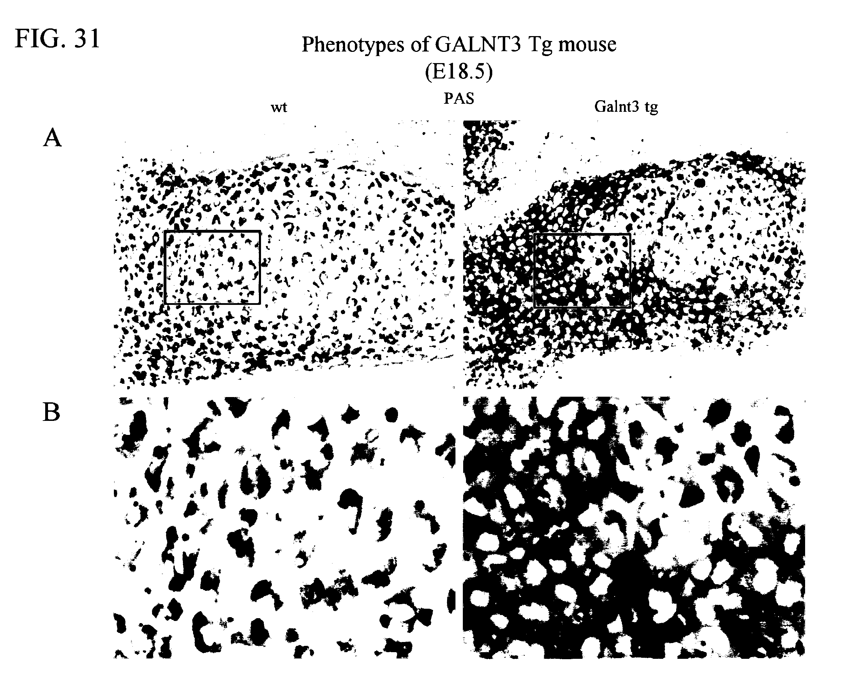

Fig. 31A and B show photographs of the results of PAS staining of the anklebone of the GALNT3 transgenic mouse at day 18.5 of embryonic development (B is a partial enlarged image of A). Compared with a wild-type mouse (wt), the staining affinity of the GALNT3 transgenic mouse (Galnt3 tg) was enhanced, which indicates an increased level of mucin-like glycoproteins.

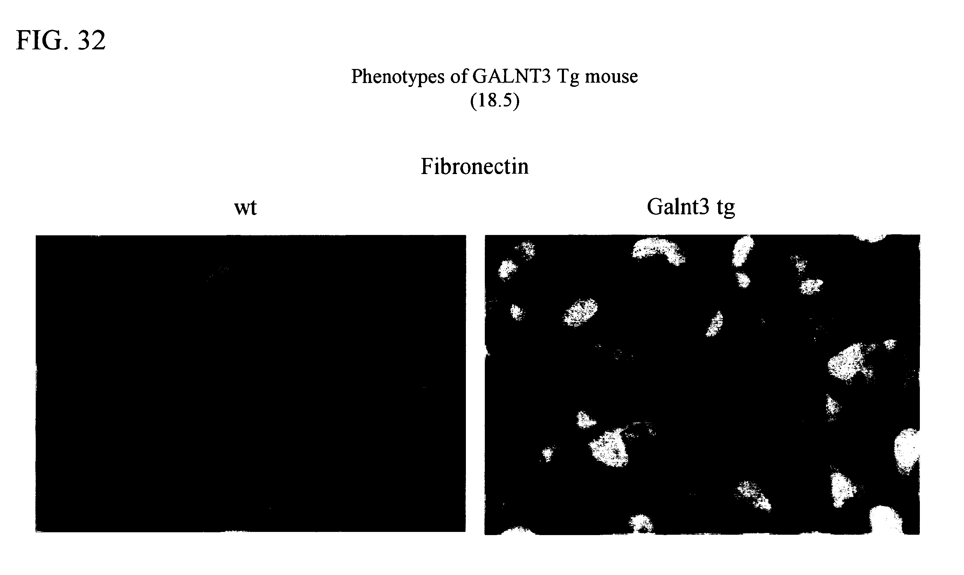

Fig. 32 is a photograph showing an image of immunostained fibronectin in the tibia of the GALNT3 transgenic mouse at day 18.5 of embryonic development. Compared with a wild-type mouse (wt), the distance between adjacent chondrocytes was small in the GALNT3 transgenic mouse (Galnt3 tg), which indicates a lowered amount of extracellular matrix.

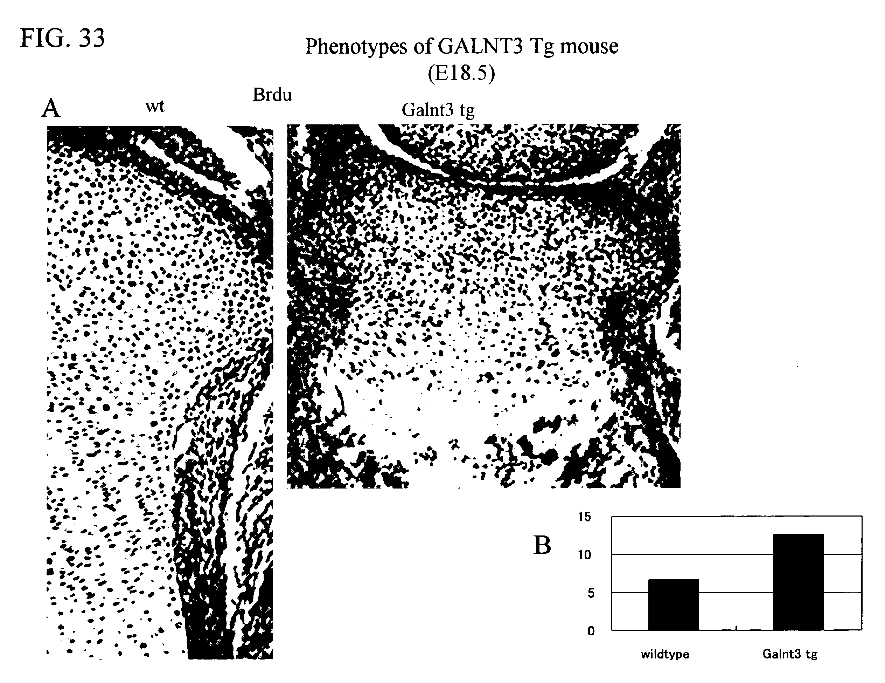

Fig. 33A is a photograph showing the results of analyzing, using Brdu labeling, the chondrocyte proliferation in the tibia of the GALNT3 transgenic mouse at day 18.5 of embryonic development. Compared with a wild-type mouse (wt), chondrocyte proliferation was enhanced in the GALNT3 transgenic mouse (Galnt3 tg).
Fig. 33B is a chart showing the number of Brdu-positive cells.

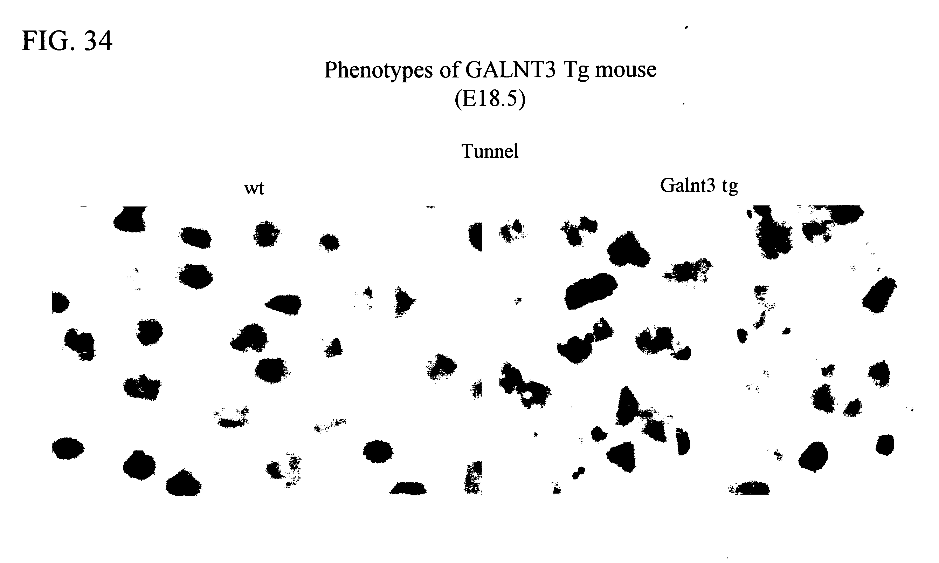

Fig. 34 is a photograph showing the results of analyzing apoptosis in the tibia of the GALNT3 transgenic mouse at day 18.5 of embryonic development by Tunnel staining. Compared with a wild-type mouse (wt), chondrocyte apoptosis was enhanced in the TALNT3 transgenic mouse (Galnt3 tg).

### Preferred Embodiments of the Invention

Hereafter, the present invention is described in detail. This patent application claims priority from Japanese Patent Application No. 2003-359172 filed on October 20, 2003, and the contents as disclosed in the description and/or drawings thereof are herein incorporated.

### [Definition of bone and/or joint disease]

In the present invention, the term "bone and/or joint disease" is a general term for systemic diseases or diseases resulting in primary lesions in joints, one of the symptoms of which is the abnormality in bones and cartilages that form skeletons. More specifically, a bone and/or joint disease can be defined by a clear correlation between bone and cartilage differentiation and lesion development. In the present invention, the bone and/or joint disease is preferably associated with cartilage differentiation. Representative examples of a bone and/or joint disease include osteoarthritis and chronic rheumatism. Also, juvenile rheumatoid arthritis, psoriatic arthritis, Reiter's syndrome, systemic lupus erythematodes (SLE), progressive systemic sclerosis, Charcot's joint (neuropathic arthropathy), CPPD crystal deposition disease, BCP crystal deposition disease, and gout may occasionally involve arthritis as a lesion. In a broad sense, the bone and joint disease includes such diseases. From the viewpoint of the correlation between bone differentiation and lesion, osteoporosis is a bone and/or joint disease in a broad sense.

Osteoarthritis is a disease characterized by degeneration and abration of joint cartilage and stiffening of and proliferative change in the subchondral bones, and secondary synovitis is also observed. Osteoarthritis is considered to be an aging-based multifactorial disease. Besides aging, sexuality (female), obesity, and external injuries (such as ligament or medial meniscus injuries) can be risk factors of osteoarthritis, although causal factors of OA have not yet been clarified. In osteoarthritic joint cartilage, the enhanced expression level of the markers for hypertrophic cartilage and calcification are observed, which are not observed in normal permanent cartilages. This implies that hypertrophied cartilage (stimulated differentiation) is associated with the osteoarthritis development. Accordingly, regulation of cartilage differentiation is considered to improve osteoarthritic symptoms.

### [Definition of the downstream gene of Runx2/Cbfa1]

In the present invention, a gene the expression of which is induced by Runx2/Cbfa1 is referred to as the downstream gene of Runx2/Cbfa1. The term "downstream gene of Runx2/Cbfa1" refers to any one of or a plurality of arbitrary genes selected from: the gene having the nucleotide sequence shown in SEQ ID NO: 1, 3, 5, 7, 9, 11, 13, 15, 17, 19, 21, 23, or 25; the human homologous gene (the human homologous polynucleotide) having the nucleotide sequence shown in SEQ ID NO: 27, 29, 31, 33, 35, 37, 39, 41, 43, 45, 47, 49, or 51; and a gene having functions equivalent to those of the gene the expression of which is induced by Runx2/Cbfal (Table 1). A specific example of the "gene having functions equivalent to those of the gene the expression of which is induced by Runx2/Cbfa1" used herein is a counterpart gene in animal species other than mice and humans. The amino acid sequences of the polypeptides encoded by such genes are shown in SEQ ID NO: 2, 4, 6, 8, 10, 12, 14, 16, 18, 20, 22, 24, 26, 28, 30, 32, 34, 36, 38, 40, 42, 44, 46, 48, 50, or 52. Since the expressions of these genes are induced by Runx2/Cbfa1, these genes may be capable of regulating cartilage differentiation, and they may be associated with diseases or pathological conditions associated with the regulation of cartilage differentiation.

**Table 1 List of the downstream genes of Runx2/Cbfa1**

| SEQ ID NO: | Mouse genes | SEQ ID NO: | Human homologue genes |
|---|---|---|---|
| 1 | Tumor endothelial marker 8 precursor (Tem8) | 27 | Homo sapiens tumor endothelial marker 8 (TEM8), transcript variant 1 |
| 3 | WNT1 inducible signaling pathway protein 2 (Wisp2) | 29 | Homo sapiens WNT1 inducible signaling pathway protein 2 (WISP2) |
| 5 | Nucleolar and coiled-body phosphoprotein 1 (Nolcl) (Nopp140) | 31 | Homo sapiens nucleolar and coiled-body phosphoprotein 1 (NOLC1) |
| 7 | MYB binding protein (P160) la (Mybbpla) | 33 | Homo sapiens MYB binding protein (P160) 1a (MYBBP1A) |
| 9 | DNA segment, Chr 13, Wayne State University 123, expressed (k. EST) | 35 | Homo sapiens hypothetical protein FLJ20303 |
| 11 | RIKEN cDNA 2810002E22 gene (HNOEL-iso homolog) | 37 | Homo sapiens HNOEL-iso protein (HNOEL-iso) |
| 13 | BRP39 | 39 | YKL40 (gp39) Homo sapiens chitinase 3-like 1 (cartilage glycoprotein-39) |
| 15 | Hemopoietic cell kinase (HCK) | 41 | Hemopoietic cell kinase (HCK) |
| 17 | Lysyl oxidase-like 2 (LOXL2) | 43 | Lysyl oxidase-like 2 (LOXL2) |
| 19 | Protein tyrosine phosphatase, receptor-type, F interacting protein, binding protein 2 (PPFIBP2) | 45 | PTPRF interacting protein (PPFIBP2) (liprin beta 2) |
| 21 | WNT1 inducible signaling pathway protein 1 (Wisp1) | 47 | WNT1 inducible signaling pathway protein 1 (WISP1) |
| 23 | Placental growth factor (PIGF) | 49 | Placental growth factor, vascular endothelial growth factor-related protein (PGF) |
| 25 | UDP-N-acetyl-alpha-D-galactosamine: polypeptide N-acetylgalactosaminyl transferase 3 (GALNT3) | 51 | UDP-N-acetyl-alpha-D-galactosamine: polypeptide N-acetylgalactosaminyltransferase 3 (GalNAc-T3) (GALNT3) |

### [Genes associated with bone and/or joint disease]

The present invention relates to a polynucleotide of a gene associated with a bone and/or joint disease. Preferably, the present invention relates to a polynucleotide of a gene the expression of which is induced by Runx2/Cbfa1 and which is capable of stimulating or inhibiting cartilage differentiation. Particularly preferably, the present invention relates to a polynucleotide of a gene associated with osteoarthritis. Specifically, such gene can be identified by introducing Runx2/Cbfa1 into Runx2/Cbfal-deficient mouse-derived primary chondrocytes or a Runx2/Cbfa1- and p53-deficient chondrocyte cell line and screening for genes the expressions of which are thereby induced or inhibited, by, for example, subtraction or DNA microarray analysis (a DNA chip).

The Runx2/Cbfa1-deficient mouse-derived primary chondrocytes and the Runx2/Cbfa1- and p53-deficient chondrocyte cell lines can be prepared in the manner as described in the section [Cells and cell lines]. A method for introducing Runx2/Cbfa1 into such cells can be carried out in accordance with a gene recombination technique known in the art (i.e., genetic transformation). An example of a more specific procedure is described in Example 2.

The polynucleotides of the genes the expressions of which are induced by Runx2/Cbfal identified above (the left column in Table 1) are derived from mice. Human homologues thereof can be easily identified by searching public databases. The nucleotide sequences of the human homologues identified by searching of public databases (e.g., the GenBank database) are shown in SEQ ID NOs: 27, 29, 31, 33, 35, 37, 39, 41, 43, 45, 47, 49, and 51 (the right column in Table 1). Human homologues can also be identified via homology search of public databases. In such a case, human homologues can be identified by selecting genes encoding proteins that are most homologous to the polypeptides encoded by the aforementioned genes, from among the genes encoding proteins having 60% or more, and preferably 65% or more, homology to such polypeptides. Homology search of proteins is known in the art, and homology can be easily determined with the use of, for example, a known protein homology search program, i.e., BLAST (Altschul S.F. et al., 1990, Basic local alignment search tool, J. Mol. Biol. 215: 403-410; and Karlin S. and Altschul S.F., 1990, Proc. Natl. Acad. Sci. U.S.A., 87: 2264-2268).

Further, the aforementioned polynucleotide encoding the gene the expression of which is induced by Runx2/Cbfa1 or a complementary strand thereof is experimentally used as a probe to obtain a polynucleotide that hybridizes under stringent conditions. Thus, a human gene that is highly homologous and functionally equivalent to a mouse-derived gene can be identified, for example. It can be readily deduced that the human homologue thus obtained retains equivalent functions concerning cartilage differentiation. In the present invention, "a polynucleotide that is capable of hybridizing under stringent conditions" can be obtained in accordance with, for example, the method described in Molecular Cloning, 2nd Ed., J. Sambrook et al., 1989. The phrase "capable of hybridizing under stringent conditions" used herein indicates that positive hybridization signals can still be observed when hybridization is carried out by heating in a solution (6x SSC, 0.5% SDS, and 50% formamide) at 42°C, followed by washing in a solution (0.1x SSC and 0.5% SDS) at 68°C, for example.

Whether or not the polynucleotide thus obtained actually encodes a protein capable of stimulating or inhibiting cartilage differentiation can be examined by, for example, the techniques described in Examples 8 and 9. For example, it can be confirmed by expressing the polynucleotide of interest and determining if cartilage differentiation can be thereby stimulated or inhibited.

Some of the polynucleotides encoding the genes the expressions of which are induced by Runx2/Cbfal, regarding which reproducibility is verified by real-time PCR analysis, and the expressions of which are inhibited in a Runx2/Cbfal-deficient murine skeleton compared to those in a wild-type murine embryonic skeleton, a novel gene having the nucleotide sequence shown in SEQ ID NO: 9 is found. Concerning this gene, the original sequence of the sequence spotted on the glass of the DNA microarray used for analysis is registered in public databases, although only a part of the sequence is available. In the present invention, the full-length sequence of the novel gene was identified via the RACE method based on the aforementioned partial sequence. Numerous genes, only the partial sequences of which are known, were spotted on the DNA microarray that was used for analysis, and the full-length sequences thereof could be determined via the RACE method. With this technique, accordingly, EST, the expression of which is induced or inhibited by Runx2/Cbfa1, can be identified, and then the full-length genes can be analyzed via the RACE method to identify the novel genes. As with the case of other downstream genes of Runx2/Cbfa1, the expressions of the genes thus identified can be regulated by Runx2/Cbfa1, they are preferably capable of regulating cartilage differentiation, and they are more preferably associated with a bone and/or joint disease such as osteoarthritis.

The polynucleotides having the nucleotide sequences shown in SEQ ID NOs: 3 and 15 are capable of inhibiting cartilage differentiation. In contrast, the polynucleotides having the nucleotide sequences shown in SEQ ID NOs: 5 and 1 are capable of stimulating cartilage differentiation. Further, the polynucleotide having the nucleotide sequence shown in SEQ ID NO: 25 is capable of inhibiting chondrogenesis.

The nucleotide sequence of the polynucleotide of the gene according to the present invention is shown in SEQ ID NO: 1, 3, 5, 7, 9, 11, 13, 15, 17, 19, 21, 23, 25, 27, 29, 31, 33, 35, 37, 39, 41, 43, 45, 47, 49, or 51. Further, the polynucleotide of the present invention may be a DNA sequence shown in SEQ ID NO: 1, 3, 5, 7, 9, 11, 13, 15, 17, 19, 21, 23, 25, 27, 29, 31, 33, 35, 37, 39, 41, 43, 45, 47, 49, or 51, or a DNA sequence derived from the nucleotide sequence registered in a public database by spontaneous or induced mutation, such as deletion, substitution, or addition, of one or several DNAs. Means for conducting deletion, substitution, addition, or insertion of DNA are known in the art. Examples thereof include a method for generating a deletion mutant with the use of exonuclease and a site-directed mutagenesis. Some downstream genes of Runx2/Cbfa1 according to the present invention may have a plurality of isoforms depending on different splicing sites. It can be readily deduced that these isoforms are functionally similar to one another. The downstream genes of Runx2/Cbfal of the present invention encompass such gene isoforms.

### [Cloning of a full-length nucleotide sequence from a partial nucleotide sequence]

Once part of the nucleotide sequence of the downstream gene of Runx2/Cbfa1 is elucidated, a person skilled in the art can define the full-length sequence of such gene based on the information concerning partial nucleotide sequence. For example, the full-length nucleotide sequence can be obtained via *in silico* cloning. Specifically, an enormous quantity of EST information accumulated in public databases is searched to inquire for the nucleotide sequence of EST (the query sequence) that constitutes part of the aforementioned gene. Based on the results of the inquiry, other piece of EST information on the sequence having the nucleotide sequence consistent with the query sequence over a given length is obtained. The other obtained piece of EST information is used as a new query sequence to repeatedly obtain other pieces of EST information. By the repeat of this procedure, a plurality of EST sets that share partial nucleotide sequences can be obtained. Such EST sets are referred to as a cluster. The EST nucleotide sequences constituting a cluster are combined and integrated into a nucleotide sequence. Thus, the nucleotide sequence of the gene of interest can be elucidated.

Further, a person skilled in the art can design primers for PCR based on the nucleotide sequence determined via *in silico* cloning. If the amplification of a gene fragment having the length as designed is confirmed by RT-PCR using such primers, this can be used to verify the actual existence of the gene consisting of the determined nucleotide sequence.

Also, the results of *in silico* cloning can be evaluated by Northern blotting. Northern blotting is carried out using the probes designed based on the determined nucleotide sequence information. If a band that is consistent with the aforementioned nucleotide sequence information is consequently detected, the existence of the gene having the determined nucleotide sequence can be verified.

In addition to *in silico* cloning, a gene of interest can be experimentally isolated. At the outset, a cDNA clone having the nucleotide sequence information registered as EST is obtained, and the full-length nucleotide sequence comprising cDNA of such clone is determined. This can consequently result in elucidation of the full-length sequence of cDNA. This procedure can at least result in the elucidation of a longer nucleotide sequence. The length of the cDNA of the clone can be experimentally confirmed in advance if the vector structure is known.

Even if the clone with the nucleotide sequence information of EST is not available, a portion of the gene with an unknown nucleotide sequence can be obtained based on the partial nucleotide sequence. Such procedure is known in the art. For example, a cDNA library may be screened using the EST probe, and this procedure can occasionally elucidate a longer nucleotide sequence. With the use of cDNA library containing a large quantity of full-length cDNAs, full-length clones can be easily isolated. For example, the cDNA library synthesized based on the principle of oligo-capping is considered to contain a large quantity of full-length cDNAs.

Also, a method for synthesizing a region of the gene containing an unknown nucleotide sequence based on the information concerning the partial nucleotide sequence is known. For example, the RACE method is a representative technique for isolating a gene containing an unknown nucleotide sequence. In the RACE method, an oligonucleotide linker is artificially ligated to the end of cDNA. The nucleotide sequence of such oligonucleotide linker is known in advance. Accordingly, primers for PCR can be designed based on a region with a known nucleotide sequence as EST and the nucleotide sequence information of the oligonucleotide linker. A region with an unknown nucleotide sequence can be specifically synthesized by PCR using the primers thus designed.

If a full-length sequence information is obtained by the RACE method, cDNA library cloning, *in silico* cloning using public databases, or other means, a person skilled in the art can prepare synthetic DNA based on the full-length sequence information and bind the synthetic DNA to obtain a polynucleotide having the full-length sequence. Via the method involving the use of synthetic DNA, mutation can be introduced into any site. Thus, a mutant comprising substitution, deletion, or addition of one or several amino acid residues can be prepared while maintaining the functions relevant to cartilage differentiation. The polynucleotide thus obtained has functions equivalent to those of a polynucleotide having the same sequence obtained via another technique, and it can be used in the same manner.

### [Gene polymorphism]

In general, the genes of higher order animals frequently involve polymorphisms. Also, a number of molecules exist, which generate isoforms consisting of alternating amino acid sequences in the process of splicing. The downstream gene of Runx2/Cbfa1 according to the present invention includes genes having activities similar to those of the downstream gene of Runx2/Cbfa1, even though such genes have different nucleotide sequences due to polymorphisms or isoforms.

### [Homologues in different species]

In the present invention, the downstream gene of Runx2/Cbfal is not limited to that of the mouse or human homologues, and it includes counterparts in other animal species. Accordingly, the downstream gene of Runx2/Cbfa1 of a species other than mice and humans refers to a homologue of the downstream gene of Runx2/Cbfa1 intrinsic to the species of interest or an extrinsic downstream gene of Runx2/Cbfa1, which has been introduced into the organism, unless otherwise specified.

In the present invention, the term "homologue of mouse downstream gene of Runx2/Cbfa1" refers to a gene derived from a species other than a mouse that can hybridize under stringent conditions using the murine gene as the probe and that has functions equivalent to those of the murine downstream gene of Runx2/Cbfal. The phrase "that hybridizes under stringent conditions" used herein indicates that positive hybridization signals can still be observed when hybridization is carried out by heating in a solution (6x SSC, 0.5% SDS, and 50% formamide) at 42°C, followed by washing in a solution (0.1x SSC and 0.5% SDS) at 68°C, for example. The temperature conditions for hybridization or washing that significantly affect stringency can be modified in accordance with the melting temperature (Tm). Tm varies depending on the proportion of constitutive nucleotides in relation to the hybridizing nucleotide pairs or the composition of the solution for hybridization (a salt concentration, a formamide concentration, a sodium dodecyl sulfate concentration, or the like). A person skilled in the art accordingly takes such conditions into consideration, he can test for the conditions that can yield equivalent stringency, or he can empirically determine such conditions. The term "gene having functions equivalent to those of the downstream gene of Runx2/Cbfa1" used herein refers to a gene encoding a protein having activity or function identical or similar to a protein the expression of which is inhibited or enhanced by the downstream gene of Runx2/Cbfa1 of a mouse or of another species, which is encoded by the downstream gene of Runx2/Cbfa1 of a mouse.

The human homologue gene of the murine downstream gene of Runx2/Cbfa1 was searched for in public databases. As a result, a human homologue exhibiting 65% or more homology in terms of the amino acid sequence of the protein was identified. When the homologues of animal species other than humans are searched for in public databases based on mouse sequences, accordingly, the searched-for gene may be a functionally similar counterpart gene, as long as it exhibits 65% or more homology at the amino acid sequence level.

### [Primer or probe]

A primer or probe can be a polynucleotide comprising the whole or a part of the nucleotide sequence of the downstream gene of Runx2/Cbfal or a polynucleotide comprising a nucleotide sequence of at least 15 nucleotides complementary to the complementary strand of the former polynucleotide. The term "complementary strand" used herein refers to one strand to the other strand of double-stranded DNA consisting of base pairs of A:T (U in the case of RNA) or G:C. The "complementary" strands are not necessarily completely complementary in a region comprising at least 15 continuous nucleotides. These strands may exhibit 70% or more, preferably 80% or more, more preferably 90% or more, and further preferably 95% or more homology to each other. Nucleotide sequence homology can be determined using an algorithm such as BLAST.

Such polynucleotide can be used as a probe for detecting the downstream gene of Runx2/Cbfa1 or as a primer for amplifying the downstream gene of Runx2/Cbfa1. A polynucleotide primer generally has a length of 15 bp to 100 bp, and preferably 15 bp to 35 bp. A polynucleotide probe has at least part of or the entire sequence of the downstream gene of Runx2/Cbfal (or a complementary strand thereof), and a DNA sequence of at least 15 bp is used. When the polynucleotide is used as a primer, the 3' region needs to be complementary, and a restriction enzyme recognition sequence, a tag, or the like can be added to the 5' end.

In the present invention, the "polynucleotide" may be DNA or RNA. Such polynucleotide may be synthetic or naturally occurring. Probe DNA used for hybridization is generally labeled. Examples of labeling methods include the following:
1) labeling by nick translation with the use of DNA polymerase I;
2) terminal labeling with the use of polynucleotide kinase;
3) fill-in labeling of the terminus with the use of the Klenow fragment (Berger SL, Kimmel AR., 1987, Guide to Molecular Cloning Techniques, Methods in Enzymology, Academic Press; Hames BD, Higgins S.J., 1985, Genes Probes: A Practical Approach, IRL Press; Sambrook J, Fritsch EF, Maniatis T., 1989, Molecular Cloning: a laboratory Manual, 2nd Ed. Cold Spring Harbor Laboratory Press);
4) labeling by transcription with the use of RNA polymerase (Melton DA, Krieg PA, Rebagkiati MR, Maniatis T, Zinn K, Green MR. Nucleic Acid Res., 1984, 12, 7035-7056); and
5) incorporation of modified nucleotides into DNA without the use of a radioisotope (Kricka L.J., 1992, Nonisotropic DNA Probing Techniques, Academic Press).

Among polynucleotides, oligonucleotides have a relatively low degree of polymerization, and polynucleotides include oligonucleotides.

### [Techniques for searching for genes]

The present invention relates to a technique for searching for a disease-associated gene by introducing a disease-associated transcription factor into a cell that is deficient in such transcription factor and then allowing the transcription factor to be expressed. Preferably, the present invention further relates to a technique for searching for a gene by introducing Runx2/Cbfa1 into a primary chondrocyte or chondrocyte cell line that is deficient in Runx2/Cbfa1 and then allowing the transcription factor to be expressed. This technique can provide a method for searching for a gene without constitutive gene expression caused by endogenous genes and with a very low background level. This may enable the detection of genes that could not be detected in the past. An example of a disease-associated transcription factor is a transcription factor associated with inflammation, such as NF-kB. Disease-associated transcription factors may be induced or inhibited under pathologic conditions or may be induced in accordance with the progress of disease.

A cell that is deficient in a transcription factor may be of an established cell line, or it can be prepared by genetic engineering based on the genetic information of the transcription factor. Alternatively, a cell that is deficient in a transcription factor can be obtained via various types of chemical treatment.

A transcription factor may be introduced into a cell by a transformation technique known in the art. The transcription factor is introduced into the cell, the expression thereof is confirmed, and the gene the expression of which is induced or inhibited is screened for via subtraction, DNA chip analysis, or via other means.

### [Cells or cell lines]

The present invention relates to the Runx2/Cbfa1-deficient mouse-derived primary cultured chondrocytes or cultured chondrocytes or Runx2/Cba1- and p53-deficient mouse-derived chondrocyte cell lines that can be employed for the aforementioned technique for searching for a gene. The Runx2/Cbfa1-deficient mouse-derived primary cultured chondrocytes are obtained by treating the skeleton of a Runx2/Cbfal-deficinet mouse at day 18.5 of embryonic development with trypsin and collagenase. The Runx2/Cbal- and p53-deficient mouse-derived chondrocyte cell lines were established by treating the skeleton of a Runx2/Cba1- and p53-deficient mouse at day 18.5 of embryonic development with trypsin and collagenase, and repeating cloning 3 or 4 times. Methods for establishing the aforementioned primary chondrocytes and chondrocyte cell lines are known, and such cells or cell lines can be obtained in accordance with other conventional techniques.

Preferable examples of Runx2/Cba1- and p53-deficient mouse-derived chondrocyte cell lines include, but are not limited to, RU-1 and RU-22. The RU-1 and RU-22 cell lines are deposited at the International Patent Organism Depositary of the National Institute of Advanced Industrial Science and Technology (Tsukuba Central 6, 1-1-1 Higashi, Tsukuba, Ibaraki, Japan) under the Budapest Treaty as of August 5, 2003 (the original deposit), under the accession numbers FERM BP-10137 (the RU-1 cell line) and FERM BP-10138 (the RU-22 cell line).

### [Polypeptide]

The polypeptide of the downstream gene of Runx2/Cbfal according to the present invention includes those represented by SEQ ID NO: 2, 4, 6, 8, 10, 12, 14, 16, 18, 20, 22, 24, 26, 28, 30, 32, 34, 36, 38, 40, 42, 44, 46, 48, 50, and 52. Polypeptide sequences can be found by searching public databases. The polypeptide sequences that are not registered with public databases can be easily deduced based on nucleotide sequences by open reading frame search. Further, a polypeptide shown in SEQ ID NO: 2, 4, 6, 8, 10, 12, 14, 16, 18, 20, 22, 24, 26, 28, 30, 32, 34, 36, 38, 40, 42, 44, 46, 48, 50, or 52 or a polypeptide registered with a public database may be employed if a new open reading frame has been discovered. Such polypeptide can be altered if functions (i.e., functions of stimulating or inhibiting cartilage differentiation) are not significantly changed. Examples of such alteration include substitution, deletion, or addition of one or several amino acid residues and modification of the amino or carboxyl group. Further, the polypeptide of the present invention also includes a polypeptide having an amino acid sequence having 65% or more homology to the amino acid sequence shown in SEQ ID NO: 2, 4, 6, 8, 10, 12, 14, 16, 18, 20, 22, 24, 26, 28, 30, 32, 34, 36, 38, 40, 42, 44, 46, 48, 50, or 52 and involving no significant changes in its functions (i.e., functions of stimulating or inhibiting cartilage differentiation).

Alternatively, a polypeptide encoded by HCK that had been demonstrated to be the downstream gene of Runx2/Cbfa1 in the present invention can be obtained as a commercially available protein (polypeptide) (e.g., Cat. No. P2908, Invitrogen). Also, the polypeptide encoded by GALNT3 that had been demonstrated to be the downstream gene of Runx2/Cbfa1 in the present invention can be obtained via purification in accordance with a conventional technique (J Biol Chem., 1997, Sep 19; 272 (38): 23503-14).

The polypeptide of the present invention itself can be used in a pharmaceutical composition for regulating its functions in organisms (for example, cartilage differentiation or osteoarthritis development). Also, the polypeptide of the present invention can be used for screening for a compound capable of regulating its functions, such as an inhibitor, antagonist, or activator, or for obtaining an antibody reacting therewith. Further, the polypeptide of the present invention can also be used as a reagent.

### [Recombinant vector]

A recombinant vector can be obtained by inserting a polynucleotide encoding the downstream gene of Runx2/Cbfa1 of the present invention into an appropriate vector DNA. Vector DNA may be obtained from nature, or a vector that partially lacks DNAs that are not required for proliferation may be employed. For example, a vector derived from ColE1 or a vector derived from a lambda phage may be used. The DNA of the present invention can be inserted into the vector DNA using a conventional technique. For example, DNA is cleaved at given sites with adequately selected restriction enzymes, the resultant is mixed with vector DNA that had been treated in the same manner, and they are then ligated using a ligase.

### [Transformant]

The novel polypeptide according to the present invention and derivatives therefrom can be obtained via gene recombination techniques using a cell-free protein expression system or conventional host cells, such as *E. coli,* yeast, *Bacillus subtilis,* insect cells, or animal cells. Transformation can be carried out in accordance with conventional techniques. For example, a plasmid, chromosome, or virus is used as a replicon to transform a host cell. From the viewpoint of gene stability, integration of genes into chromosomes is preferable. Autonomous replication involving the use of an episome is simply employed. A vector is adequately selected in accordance with a type of a host cell, and a vector is constituted by a gene sequence of interest and a sequence carrying information concerning replication and regulation of the former gene sequence. Constituents are selected depending on the type of the host cell, i.e., a procaryotic or eucaryotic cell, and a promoter, a ribosome binding site, a terminator, a signal sequence, an enhancer, and the like are combined in accordance with conventional techniques.

A transformant can be used for preparing the polypeptide of the present invention by culturing of host cells under conventional conditions optimal therefor. Culture may be conducted while determining the biological activity of a novel polypeptide to be expressed and produced as an indicator. Alternatively, subculture or batch culture may be carried out by employing the amount of transformants in a medium as an indicator.

### [Antibody]

An antibody against a protein encoded by the downstream gene of Runx2/Cbfa1 can be used for diagnosing and treating a bone and/or joint disease. For example, Western blotting, the immunoprecipitation method, or ELISA involving the use of an antibody that binds to the downstream gene of Runx2/Cbfa1 can be adopted for diagnosis. An antibody that controls activity of the protein encoded by the gene of interest can be used for treatment.

The antibody used for the aforementioned purpose can be obtained in accordance with conventional techniques. The antibody used in the present invention can be a polyclonal or monoclonal antibody (Milstein C. et al., Nature, 1983, 305, 537-540). For example, a polyclonal antibody against the downstream gene of Runx2/Cbfa1 can be obtained by collecting serum from a mammalian animal that had been sensitized using the antigen polypeptide or peptide encoded by the downstream gene. Also, an immunocyte may be collected from the mammalian animal that had been sensitized using the antigen polypeptide or peptide encoded by the downstream gene, the collected cell is fused with a myeloma cell to prepare a hybridoma, the resulting hybridoma is subjected to cloning, and an antibody is recovered from the culture product to prepare a monoclonal antibody.

Specific examples include antibodies against proteins encoded by HCK or Tem8, which had been demonstrated to be the downstream genes of Runx2/Cbfa1 in the present invention. These antibodies, such as an anti-HCK antibody (Cat. No. 610277, BD Biosciences) and an anti-Tem8 antibody (Cat. No. 200C1339, Abcam) are commercially available. Antibodies disclosed in known literature (Mol Cell Biol., Jan. 1997, 17 (1): 230-9) or antibodies prepared in the manner described in such literature can also be used as anti-Nopp140 antibodies or the like.

Proteins encoded by the downstream genes of Runx2/Cbfal may be detected using adequately labeled antibodies. Alternatively, proteins can be indirectly detected with the use of labeled substances that specifically bind to such antibodies, such as Protein A or Protein G, instead of labeling antibodies. A specific example of a detection method is ELISA.

Antigen proteins or partial peptides thereof can be obtained by, for example, inserting the downstream gene of Runx2/Cbfa1 or part thereof into an expression vector, introducing the vector into an adequate host cell to prepare a transformant, culturing the transformant to allow the expression of a recombinant protein, and purifying the expressed recombinant protein from the culture product or culture supernatant. Alternatively, an oligopeptide consisting of the amino acid sequence encoded by the aforementioned gene or a partial amino acid sequence of the amino acid sequence encoded by full-length cDNA can be chemically synthesized and used as an immunogen. Examples of animals to be immunized include mice, rats, rabbits, goats, horses, and hamsters.

### [Antisense]

If information concerning a full-length gene sequence is available, a person skilled in the art can design an antisense oligo (polynucleotide) that inhibits functions of the gene. Even if only partial gene sequence information is available, an antisense oligo can also be designed. Concerning mouse Nopp140, which had been found to play a stimulatory role in cartilage differentiation in the present invention, for example, an antisense oligo such as an antisense oligo candidate sequence 5'-GCG CAA GCC GGT ATC CGC CAT-3' (SEQ ID NO: 112) can be designed based on the sequence 5'-CGG AGC ATG GCG GAT ACC GGC TTG CGC CGC GTG-3' (SEQ ID NO: 111) located in the vicinity of the initiation codon of Nopp140. An antisense oligo undergoes various forms of modification or binding in order to avoid decomposition in the cells. A person skilled in the art can select a suitable antisense oligo form. Examples of such forms include naturally occurring (D-oligo), phosphorothioate (S-oligo), methylphosphonate (M-oligo), phosphoroamidate (A-oligo), 2'-O-methyl (D-oligo), morpholidate (Mo-oligo), and polyamide nucleic acid forms. The antisense oligo sequence has 10 to 70 nucleotides in length, and preferably 15 to 30 nucleotides in length. The antisense oligo thus prepared can, for example, inhibit the functions of Nopp140, and it can be used as a therapeutic agent for a bone and/or joint disease (preferably osteoarthritis).

### [RNAi]

RNA interference (RNAi) refers to a phenomenon of strongly inhibiting the expression of a target gene by degrading RNA of the target gene using double-stranded RNA molecules with 21 to 23 bps containing the sequence identical to the RNA of the target. Accordingly, the double-stranded RNA molecule that has the same nucleotide sequence as mRNA of the downstream gene of Runx2/Cbfa1 can be employed to inhibit the expression of the downstream gene of Runx2/Cbfa1. In order to achieve the RNAi effects, it is preferable to use double-stranded RNA molecules having nucleotide sequences comprising at least 20 continuous nucleotides. The double-strand structure may be composed of 2 different strands or the double-strand structure can be formed by one strand via a RNA stem-loop. In the present specification, the double-stranded RNA capable of such RNAi phenomenon can be employed as RNAi molecules.

In the case of mouse Nopp140, for example, 2 RNA strands; i.e., 5'-AUG GCG GAU ACC GGC UUG CGC-3' (SEQ ID NO: 113) and a complementary strand thereof 5'-GCG CAA GCC GGU AUC CGC CAU-3' (SEQ ID NO: 114), are synthesized from the sequence located in the vicinity of the initiation codon, and they are annealed to each other. Thus, double-stranded RNA molecules can be prepared, and they can be used as RNAi molecules. Provision of a 2-nucleotide overhang at each 3' end of such strands can enhance the inhibitory effects on gene expression (WO 01/75164).

Another example of such RNAi molecule that can be used is a commercially available RNAi molecule, such as an RNAi (siRNA) molecule that inhibits the expression of HCK (Cat. No. G-003100-TK-02, Dharmacon Research Inc.).

A person skilled in the art may make various forms of modification concerning the sequence used for designing RNAi molecules, and the length and the structure thereof in order to design RNAi molecules having optimal inhibitory effects on gene expression. The RNAi molecules thus obtained can be used as therapeutic agents for a bone and/or joint disease (preferably osteoarthritis) by inhibition of the expression of the downstream gene of Runx2/Cbfa1.

### [Activity assay]

According to the present invention, screening of compounds, antibodies, and the like, and diagnosis can be carried out with the use of the biological activity of a protein encoded by the downstream gene of Runx2/Cbfa1 in a biological sample as an indicator as well as the expression level of the downstream gene of Runx2/Cbfa1. For example, an increase or decrease and change in activity levels can be employed for the diagnosis of diseases associated with the Runx2/Cbfa1 gene. Also, a low-molecular-weight compound, antibody, or the like that inhibits or activates biological activity may be screened for to obtain a compound or antibody that controls activity of the protein encoded by the downstream gene of Runx2/Cbfal. The resultant can be used for treatment of a bone and/or joint disease (preferably osteoarthritis). Hereafter, general techniques for assaying activities of proteins are described.

### (1) Transcription factor or transcription regulatory factor

A transcription factor is incubated at room temperature with double-stranded oligo DNA labeled with ³²P or the like that contains the target sequence of the transcription factor to bind them together. The sample after incubation is subjected to electrophoresis on SDS-free undenatured polyacrylamide gel, and the rate of migration of the labeled oligo DNA is evaluated by employing the radioactivity of ³²P or the like as an indicator. If the transcription factor exhibits binding activity to oligo DNA, the rate of migration of the labeled oligo DNA slows down, and the oligo DNA is shifted to the higher-molecular-weight. Also, an expression vector that comprises a reporter gene such as chloramphenicol acetyltransferase (CAT) ligated to the downstream of the target sequence and an expression vector that comprises a transcription factor gene ligated to the downstream of the response gene promoter of human cytomegalovirus (CMV) can be cotransfected into cell lines such as Hela or HEK293, a homogenate solution is prepared 48 hours thereafter, and the expression level of CAT is examined. Thus, the activity of the transcription factor can also be evaluated (Zhao F. et al., J. Biol. Chem. 276, 40755-40760, 2001).

Specifically, the polypeptide encoded by Nopp140 that had been demonstrated to be the downstream gene of Runx2/Cbfa1 in the present invention is known as a transcription factor that induces the expression of the alpha-1 acid glycoprotein (AGP) gene. Accordingly, conventional techniques such as reporter gene assay that employs the AGP promoter can also be employed (Mol Cell Biol., Jan. 1997, 17 (1): 230-9; and J Biol Chem., Oct. 2002, 18, 277 (42): 39102-11).

### (2) Kinase

Kinase is added to a buffer containing the myelin basic protein as a substrate (20 mM HEPES, pH 7.5, 10 mM MgCl₂, 2 mM dithiothreitol, and 25 µM ATP), [γ-³²P]ATP is added thereto, and incubated at 37°C for 10 minutes. The reaction is terminated with the use of Laemmli buffer 10 minutes later, the reaction solution is subjected to SDS polyacrylamide gel electrophoresis, the gel is dehydrated after the electrophoresis, and the radioactivity of the phosphorylated myelin basic protein is detected using an X-ray film (Park S. Y. et al., J. Biol. Chem. 275, 19768-19777, 2000).

Specifically, the polypeptide encoded by HCK that had been demonstrated to be the downstream gene of Runx2/Cbfa1 in the present invention is known to function as kinase, and activity thereof can be assayed in accordance with conventional techniques (J. Biol. Chem., Sep. 10, 1999, Vol. 274 (37), p. 26579).

### (3) Secretory factor

The cells that are to be subjected to activity assay and are assumed to contain receptors of secretory factors are stimulated with the secretory factors, and changes in the cells resulting therefrom are assayed. Methods for assaying changes in the cells are described below.

Cells are suspended in the Hank's balanced salt solution containing the calcium-sensitive fluorescent dye fura-2 so as to stimulate the cells with secretory factors. A stimulus-induced increase in the calcium level in the cells is assayed using a fluorescence detector such as the LS50B (Perkin Elmer) (Zhou N. et al., J. Biol. Chem., 2001, 276, 42826-42833).

The cells are stimulated with secretory factors, and cell proliferation resulting therefrom is evaluated based on thymidine uptake.

Activation of a transcription factor that is considered to transmit stimuli of the secretory factor can be evaluated based on the expression of the reporter genes such as luciferases (Piek E. et al., J. Biol. Chem., 2001, 276, 19945-19953).

### (4) Receptor or membrane protein

Receptors or membrane proteins are subjected to forced expression in the cells, they are stimulated with samples that are assumed to comprise ligands (a culture solution of cells, blood serum, or the like), and changes resulting therefrom in cells are assayed in accordance with the method described in the "secretory factor" section.

Activity of a receptor can also be assayed by assaying the binding between the receptor and a substance (i.e., a ligand) that binds thereto and exerts its functions. A specific example of such receptor is a polypeptide encoded by Tem8, which had been demonstrated to be the downstream gene of Runx2/Cbfa1 in the present invention. Tem8 is also known as a receptor that binds to anthrax (J. Biol. Chem., Feb. 14, 2003, Vol. 278 (7), p. 5227), and the binding to the type VI collagen α-3 subunit is also known (Cancer Research, Feb. 1, 2004, Vol. 64 (3), p. 817). Accordingly, binding between these substances and Tem8 is assayed, and the assay result can be employed as an indicator for activity assay.

### (5) Phosphatase-regulating factor

Phosphatase that is to be regulated is subjected to activity assay, and the activity of the protein for regulating phosphatase is assayed. Phosphatase activity can be assayed in the following manner. Phosphatase is added to a buffer containing p-nitrophenyl phosphate (pNPP) as a substrate (25 mM MES, pH 5.5, 1.6 mM dithiothreitol, and 10 mM pNPP), and the resultant is incubated at 37°C for 30 minutes. Thirty minutes later, 1N NaOH is added to terminate the reaction, and the absorbance at 405 nm resulting from pNPP hydrolysis is assayed (Aoyama K. et al., J. Biol. Chem., 2001, 276, 27575-27583).

### (6) Enzymes (other than kinase or phosphatase)

A wide variety of enzymes exist in addition to the aforementioned kinases and phosphatases, and they are involved in maintenance or change in the functions or structures of organisms. Since an enzyme has specificity (i.e., substrate specificity, reaction specificity, or the like) for the substances that react therewith (e.g., a substrate, a reaction product, or the like) and the reaction mechanisms, a variety of methods for assaying activity have been formulated in accordance with enzyme types. Also, some enzymes may form enzyme groups (enzyme families) having similar structures, substrate specificity, or reaction specificity, and the members of an enzyme family occasionally share reactivity with a substrate, an inhibitor, or an activator. Thus, a novel system for evaluating enzyme activity may be developed with the use of the method for assaying activities of enzymes that belong to the same enzyme family. When the downstream gene of Runx2/Cbfa1 obtained by the method of the present invention is an enzyme, accordingly, a method for assaying its activity can be developed with the use of conventional techniques or the methods for assaying enzymes of the same enzyme family.

A specific example thereof is a case of GALNT3, which was demonstrated to be the downstream gene of Runx2/Cbfa1 in the present invention. GALNT3 (ppGaNTase-T3) is a glycosyltransferase that belongs to the ppGaNTase family, and the activity thereof can be assayed by determining the reaction that occurs when adding GaINAc to a peptide such as MUC-2 or HIV-H3 using a UDP-GaINAc substrate (J. Biol. Chem., July 19, 1996, Vol. 271 (29), p. 17006). Also, the GALNT3 protein can be purified in accordance with conventional techniques (J Biol Chem., Sep. 19, 1997, 272 (38): 23503-14). At present, 24 enzyme species of the ppGaNTase enzyme family has been reported (Glycobiology, Jan. 2003, 13 (1): 1R-16R). Since the ppGaNTase family including GALNT3 is reported to be capable of regulating enzyme activity of low-molecular-weight compounds, an activity inhibitor or activator may be obtained based on such information.

### [Diagnostic method]

In the diagnostic method of the present invention, a biological sample obtained from a subject is generally employed as a test sample. A biological sample is preferably a blood sample. Whole blood, or blood plasma or serum obtained therefrom can be used as a blood sample. In the present invention, synovial fluid, a piece of joint cartilage, synovial tissue, or the like obtained via biopsy can be used as a biological sample in addition to blood. Methods for obtaining such biological samples are known.

When the biological sample is a cell, such as a piece of joint cartilage or synovial tissue, a lysate can be prepared and used as a sample for immunological assay for the aforementioned proteins. Alternatively, mRNA may be extracted from this lysate and used as a sample for assaying mRNA for the aforementioned gene. Commercially available kits are conveniently used for extracting a lysate or mRNA of a biological sample. A liquid biological sample such as blood or synovial fluid may be diluted with a buffer or the like, if necessary, and the resultant can be used as a sample for assaying for proteins or genes.

A lysate prepared from the aforementioned biological sample can be used as a sample for immunological assay of the protein encoded by the downstream gene of Runx2/Cbfa1. mRNA extracted from such lysate can be used as a sample for assaying mRNA for the downstream gene of Runx2/Cbfa1. Commercially available kits are conveniently used for extracting a lysate or mRNA of a biological sample. When the protein encoded by the downstream gene of Runx2/Cbfa1 is secreted in the blood or synovial fluid, the amounts of target proteins in a body fluid sample such as blood or serum of the subject may be assayed to compare the expression levels of the genes encoding the same. If necessary, the aforementioned sample can be diluted with a buffer or the like and used in the method according to the present invention.

When mRNA is assayed, the measured expression level of the downstream gene of Runx2/Cbfa1 in the present invention can be corrected in accordance with conventional techniques. This correction enables the comparison of variations in gene expression levels in the cells. The measured expression levels of the downstream genes of Runx2/Cbfal in the present invention in the cells are corrected based on the measured expression levels of genes the expression levels of which do not significantly vary in cells in the biological samples (e.g., a housekeeping gene). Examples of genes the expression levels of which do not significantly vary include β-actin and GAPDH.

Further, the present invention provides a reagent (a diagnostic composition) used for the diagnostic method of the present invention. Specifically, the present invention relates to a diagnostic reagent for a bone and/or joint disease comprising a polynucleotide containing the nucleotide sequence of the downstream gene of Runx2/Cbfa1, an oligonucleotide having a nucleotide sequence complementary to the complementary strand of the polynucleotide and composed of at least 15 nucleotides, a DNA vector containing such polynucleotide, or a transformant transformed with such DNA vector. Also, the present invention relates to a diagnostic reagent for a bone and/or joint disease comprising a protein (polypeptide) encoded by the downstream gene of Runx2/Cbfa1, an antibody that recognizes a peptide containing the amino acid sequence of the protein, an antisense polynucleotide, or an RNAi molecule.

The oligonucleotide or antibody that constitutes the reagent of the present invention may be adequately labeled in accordance with an assay format. Also, the oligonucleotide or antibody that constitutes the reagent of the present invention may be immobilized on an adequate support in accordance with an assay format. The reagent of the present invention may be combined with additional elements required for detection or storage in addition to the aforementioned oligonucleotide or antibody and prepared in the form of a diagnostic kit. Additional elements (1) to (6) that can constitute the kit are listed below. These elements can be previously mixed according to need. If necessary, a preservative or antiseptic agent can be added to each element.
(1) A buffer for diluting the reagent or biological sample
(2) A positive control
(3) A negative control
(4) A substrate for assaying the labeling
(5) A reaction vessel
(6) The instructions describing the assay protocol

In the present invention, diagnosis of a bone and/or joint disease includes the following diagnosis, for example. Even if a patient exhibits symptoms suspected to indicate a bone and/or joint disease (preferably osteoarthritis) but cannot be diagnosed as having osteoarthritis by a general diagnostic procedure, by the diagnostic procedure according to the present invention, whether or not the patient is afflicted with osteoarthritis can be easily determined. More specifically, when a patient suspected of osteoarthritis exhibits an increase or decrease in the expression level of the downstream gene of Runx2/Cbfa1, the causal factor of such symptom is highly likely to be osteoarthritis.

Also, the present invention enables diagnosis whether or not the bone and/or joint disease (preferably osteoarthritis) is ameliorated. Specifically, the diagnostic method according to the present invention is useful for determining the therapeutic effects on osteoarthritis. More specifically, an increase or decrease in the expression level of the downstream gene of Runx2/Cbfa1 in a patient suspected of osteoarthritis indicates a possibility of the further progression or amelioration of osteoarthritis.

Further, the severity of osteoarthritis can be determined based on differences in the expression levels. Specifically, the degree of increase in the expression level of the downstream gene of Runx2/Cbfa1 may be correlated with the degree of severity of osteoarthritis.

### [Transgenic animal and animal model of bone and/or joint disease]

The present invention relates to an animal model of a bone and/or joint disease, and preferably an animal model of osteoarthritis, including a transgenic nonhuman animal in which the expression level of the downstream gene of Runx2/Cbfa1 or a gene functionally equivalent thereto is enhanced systemically, or preferably cartilage-specifically. According to the present invention, the downstream gene of Runx2/Cbfa1 was found to stimulate or inhibit cartilage differentiation. Accordingly, an animal in which the expression level of the downstream genes of Runx2/Cbfa1 stimulating cartilage differentiation is artificially enhanced also exhibits stimulated cartilage differentiation, which is observed in the synovial cartilage of osteoarthritis. Thus, such animal can be employed as an animal model of osteoarthritis.

The present invention relates to an animal model of a bone and/or joint disease, and preferably an animal model of osteoarthritis or chronic rheumatism, including a transgenic nonhuman animal in which the expression level of the downstream gene of Runx2/Cbfa1 or a gene functionally equivalent thereto is lowered systemically, or preferably cartilage-specifically. The animal in which the expression levels of the downstream genes of Runx2/Cbfa1 inhibiting cartilage differentiation are artificially lowered also exhibits stimulated cartilage differentiation, which is observed in the osteoarthritic joint cartilage. Thus, such animal can be employed as an animal model of osteoarthritis.

In the present invention, functionally equivalent genes refer to those encode proteins having activities equivalent to those of proteins encoded by the downstream genes of Runx2/Cbfal, which have been revealed. A representative example of the functionally equivalent gene of a test animal is a counterpart of the downstream gene of Runx2/Cbfal intrinsic to the test animal species.

Further, the present invention relates to a method for preparing an animal model of a bone and/or joint disease (preferably osteoarthritis) by the administration of the protein encoded by the downstream gene of Runx2/Cbfa1 or an antibody against the protein.

At the outset, the downstream gene of Runx2/Cbfal can induce stimulated cartilage differentiation by an increase or a decrease in its level of expression, and it can further preferably induce osteoarthritis. The correlation between the gene expression level and its influence on cartilage differentiation can be evaluated by examining whether or not the protein encoded by the gene of interest stimulates or inhibits cartilage differentiation. More specifically, an increased expression level of the downstream genes of Runx2/Cbfa1 stimulating cartilage differentiation (e.g., SEQ ID NO: 5 or 1) may result in stimulated cartilage differentiation and induction of osteoarthritis. In contrast, a decreased expression level of the downstream genes of Runx2/Cbfa1 inhibiting cartilage differentiation (e.g., SEQ ID NO: 3, 15, or 25) may result in stimulated cartilage differentiation and induction of arthritis.

In addition to the transgenic animal, the aforementioned increased gene expression level can be achieved by the administration of the protein encoded by such gene to an animal. This may induce osteoarthritis, and such animal may be used as an animal model of osteoarthritis. The protein encoded by the aforementioned gene may be a full-length protein or a protein consisting of a partial sequence containing an active site, as long as it has equivalent functions.

In addition to the transgenic animal, the aforementioned decreased gene expression level can be achieved by the administration of a substance that inhibits activity of the protein encoded by the gene of interest or a substance that decreases the expression level of the gene of interest to an animal. This may induce osteoarthritis, and such animal may be used as an animal model of osteoarthritis. Specific examples of substances that inhibit activity of the protein encoded by the gene of interest include activity inhibitors such as antibodies or compounds. Also, a decoy partial polypeptide or the like that consists of a region binding to a ligand and cannot transduce signals into the cells (i.e., an extracellular domain soluble receptor) can be used. Examples of substances decreasing the expression level of the gene include an antisense nucleic acid, a ribozyme, and an RNAi molecule. In the case of a transcription factor, a decoy nucleic acid can be designed based on a DNA sequence specific for the promoter recognized by such transcription factor. The thus designed decoy is considered to be capable of inhibiting the activation of a transcription factor, and it can be used for preparing the animal model of osteoarthritis or a pharmaceutical preparation for osteoarthritis.

The animal model of osteoarthritis according to the present invention is useful for elucidating changes in an individual suffered from osteoarthritis *in vivo.* Further elucidation of functions of the downstream gene of Runx2/Cbfa1 and evaluation of a medicine targeting the gene of interest with the use of the animal model of osteoarthritis is significant.

The animal model of osteoarthritis according to the present invention is useful for elucidation of the mechanisms of osteoarthritis and for the test of safety of the screened compound. If the animal model of osteoarthritis according to the present invention exhibits cartilage degeneration and exhibits changes in the measured value of some osteoarthritis-associated marker or cartilage differentiation marker, for example, a system for screening for a compound that can recover such change can be constructed.

In the present invention, the term "increased expression level" refers to any of the following conditions: where the downstream gene of Runx2/Cbfa1 is introduced as a extrinsic gene and forcedly expressed; where transcription of the downstream gene of Runx2/Cbfa 1 intrinsic to the test animal and translation thereof into a protein are enhanced; and where degradation of the protein as a translation product is suppressed.

In the present invention, the term "decreased expression level" refers to any of the following conditions: where transcription of the downstream gene of Runx2/Cbfa1 intrinsic to the test animal and translation thereof into a protein are blocked; or where degradation of the protein as a translation product is enhanced. The gene expression level can be determined via, for example, DNA chip analysis based on differences in signal intensity or real-time PCR analysis. Activity of the protein as a translation product can be determined in comparison with activity under normal conditions.

Representative examples of transgenic animals include: animals into which the target gene has been introduced and forcedly expressed; animals in which the downstream genes of Runx2/Cbfa1 have been knocked out; and animals in which the downstream genes of Runx2/Cbfa1 have been substituted (knocked in) with other genes. In the present invention, transgenic animals into which an antisense nucleic acid to the downstream gene of Runx2/Cbfa1, a ribozyme, DNA encoding a polynucleotide exhibiting the RNAi effects, and DNA functioning as a decoy nucleic acid have been introduced can also be used as the transgenic animals. Examples of other transgenic animals include animals prepared by introducing mutation into the coding region of the downstream gene of Runx2/Cbfa1 to enhance or inhibit its activity or to modify the amino acid sequence to make it likely or unlikely to degrade. Mutation in the amino acid sequence may include substitution, deletion, insertion, or addition. Also, the transcription regulatory region of the gene may be mutated to regulate the expression of the downstream gene of Runx2/Cbfa1 according to the present invention.

A method for obtaining a transgenic animal by targeting a given gene is a known technique. Specific examples of methods for obtaining a transgenic animal include: a method wherein mixture of a gene and an egg is treated with calcium phosphate; a method wherein a gene is directly introduced into the nucleus of the pronuclear stage egg using a micropipette under a phase contrast microscope (microinjection, U.S. Patent No. 4,873,191); and a method involving the use of an embryonic stem (ES) cell. Also, a method wherein a gene is inserted into a retrovirus vector to infect the egg, a method wherein the gene is introduced into the egg via sperm (i.e., the sperm vector method), and other methods have been developed. The sperm vector method is a technique of gene recombination wherein extrinsic genes are made to adhere to sperm or incorporated into sperm cells via electroporation or other means, and the sperms are then allowed to fertilize eggs, thereby introducing the extrinsic genes (Lavitranoet M. et al., Cell, 1989, 57, 717-723).

When a promoter the transcription of which is regulated by an adequate substance such as an agent is used as an expression vector, the expression level of the extrinsic downstream genes of Ruox2/Cbfa1 in the transgenic animal can be regulated by the administration of the aforementioned substance. An example of such promoter is, but is not limited to, a type II collagen promoter.

Transgenic animals that are used as the animal models of osteoarthritis in the present invention can be produced from all types of vertebrates except for humans. Specifically, transgenic animals are produced from vertebrates, such as mice, rats, rabbits, miniature pigs, goats, sheep, monkeys, dogs, cats, or cattle by various genes or by alteration in expression levels of various genes.

### [Method for screening for candidate compounds of therapeutic agents or prophylactic agents for a bone and/or joint disease (in vivo)]

The present invention relates to a method for screening for candidate compounds for therapeutic agents and/or prophylactic agents for a bone and/or joint disease (preferably osteoarthritis). In the present invention, the downstream gene of Runx2/Cbfa1 was found to stimulate or inhibit cartilage differentiation. Osteoarthritis involves the stimulated differentiation of permanent joint cartilage. Concerning the downstream genes of Runx2/Cbfa1 capable of inhibiting cartilage differentiation (e.g., a gene having the nucleotide sequence shown in SEQ ID NO: 5 or 9), accordingly, screening for a compound that lowers the expression level of such gene enables the provision of therapeutic agents and/or prophylactic agents for osteoarthritis. Concerning the downstream genes of Runx2/Cbfa1 capable of inhibiting cartilage differentiation (e.g., a gene having the nucleotide sequence shown in SEQ ID NO: 3, 15, or 25), accordingly, screening for a compound that enhances the expression level of such gene enables the provision of therapeutic agents and/or prophylactic agents for osteoarthritis.

In the present invention, a compound that enhances or lowers the gene expression level refers to a compound that enhances or inhibits gene transcription, translation, or expression of protein activity. A compound that lowers the gene expression level in the present invention is a compound having functions that inhibit any of such processes.

The method for screening for candidate compounds for therapeutic agents and/or prophylactic agents for a bone and/or joint disease (preferably osteoarthritis) according to the present invention may be carried out *in vivo* or *in vitro.* Such screening can be carried out in accordance with, for example, the following procedure:
(1) a step of administrating the candidate compound to the test animal;
(2) a step of assaying the expression level of the downstream gene of Runx2/Cbfa1 in the biological sample obtained from the test animal; and
(3) a step of selecting a compound that lowers the expression level of the downstream genes of Runx2/Cbfa1 in case that the downstream genes stimulate cartilage differentiation or a compound that enhances the expression level of the downstream gene of Runx2/Cbfa1 in case that the downstream genes inhibit cartilage differentiation, when compared with a control to which no candidate compound has been administered.

In the screening method according to the present invention, the downstream genes of Runx2/Cbfa1 or genes functionally equivalent thereto can be employed. In the present invention, functionally equivalent genes encode proteins having activities equivalent to those of proteins encoded by the downstream genes of Runx2/Cbfa1, which have been revealed. A representative example of such a functionally equivalent gene of a test animal is a counterpart of the downstream gene of Runx2/Cbfa1 intrinsic to the test animal species.

Test animals used in the screening method according to the present invention includes transgenic animals of a bone and/or joint disease (preferably osteoarthritis) according to the present invention, described in the section [Transgenic animal and animal model of bone and/or joint disease] and animal models of a bone and/or joint disease (preferably osteoarthritis) prepared by the administration of a polypeptide encoded by the downstream gene of Runx2/Cbfa1 or an antibody against the aforementioned polypeptide. Other conventional animal models of osteoarthritis can also be used. Examples of conventional animal models of osteoarthritis include models of spontaneous osteoarthritis (e.g., the STR/ORT mouse) and models with excised anterior cruciate ligament (e.g., mice, rats, rabbits, or dogs).

A person skilled in the art can identify the homologue in an animal species other than mice, based on the disclosure of the present invention. For example, genes (or proteins) of other species that are highly homologous to the nucleotide sequence or amino acid sequence of the human homologue can be discovered by homology search. Alternatively, a homologue in other animal species can be isolated by hybridization with the downstream gene of Runx2/Cbfal. In the screening method that involves the use of animal models to which human genes have been introduced, human genes may be occasionally detected as the downstream genes of Runx2/Cbfal, in addition to the homologues of the animal in question.

By the administration of a drug candidate compound to the test animal and the monitoring of the effects of the compound on the expression of the downstream gene of Runx2/Cbfa1 in the biological sample derived from the test animal, the effects of the drug candidate compound on the expression level of the downstream gene of Runx2/Cbfa1 can be evaluated. Change in the expression level of the downstream gene of Runx2/Cbfa1 in the biological sample obtained from the test animal can be monitored in the same manner as that of the diagnostic method according to the present invention. Based on the evaluation results, a drug candidate compound that lowers the expression level of the downstream genes of Runx2/Cbfa1 which stimulate cartilage differentiation or a drug candidate compound that enhances the expression level of the downstream gene which inhibit cartilage differentiation may be selected to screen for the drug candidate compound.

Through such screening procedure, drugs that are involved in the expression of the downstream gene of Runx2/Cbfa1 in diverse ways can be selected. Specifically, a drug candidate compound having functions as described below can be discovered. Concerning the downstream genes of Runx2/Cbfal which stimulate cartilage differentiation:
(1) inhibition of the signaling pathway that induces the expression of the downstream genes of Runx2/Cbfa1;
(2) inhibition of transcription activity of the downstream genes of Runx2/Cbfa1 ; and
(3) inhibition of stabilization or acceleration of degradation of the transcription product of the downstream genes of Runx2/Cbfa1.
Concerning the downstream genes of Runx2/Cbfa1 which inhibit cartilage differentiation:
(1) activation of the signaling pathway that induces the expression of the downstream genes of Runx2/Cbfal;
(2) acceleration of transcription activity of the downstream genes of Runx2/Cbfa1; and
(3) stabilization or inhibition of degradation of the transcription product of the downstream genes of Runx2/Cbfa1.

### [Method for screening for candidate compounds for therapeutic agents and/or prophylactic agents for a bone and/or joint disease (in vitro)]

In *in vitro* screening, for example, the candidate compound is brought into contact with a cell that expresses the downstream gene of Runx2/Cbfa1, a compound that lowers the expression level of the downstream genes of Runx2/Cbfa1 in the case that the downstream genes stimulate cartilage differentiation or a compound that enhances the expression level of the downstream gene in case that the downstream genes inhibit cartilage differentiation are selected. Such screening can be carried out in accordance with, for example, the following steps:
(1) a step of bringing the candidate compound into contact with the cell that expresses the downstream gene of Runx2/Cbfa1;
(2) a step of assaying the expression level of the downstream gene of Runx2/Cbfa1; and
(3) a step of selecting a compound that lowers the expression level of the downstream genes of Runx2/Cbfal in the case that the downstream genes stimulate cartilage differentiation or a compound that enhances the expression level of the downstream gene of Runx2/Cbfa1 in the case that the downstream genes inhibit cartilage differentiation, when compared with a control, which has not been brought into contact with candidate compound.

In the present invention, the cell that expresses the downstream gene of Runx2/Cbfa1 can be obtained by inserting the downstream gene of Runx2/Cbfa1 into an adequate expression vector and introducing the vector into an adequate host cell. Any vectors and host cells may be used as long as the downstream gene of Runx2/Cbfa1 can be expressed in the host cells. Examples of host cells that can be used in a host-vector system include *E. coli,* yeast, insect cells, and animal cells. Suitable vectors that can be used in each host cell can be adequately selected.

A vector can be introduced into a host cell via biological, physical, chemical, or other means. Examples of biological means include a method that involves the use of a virus vector, a method that involves the use of a specific receptor, the cell fusion method (hemagglutinating virus of Japan (HVJ)), a method using the polyethylene glycol (PEG), electrical cell fusion, and micronuclear fusion (chromosome transfer). Examples of physical means include microinjection, electroporation, and a method involving the use of a gene particle gun. Examples of chemical means include calcium phosphate precipitation, the liposome method, the DEAE dextran method, the protoplast method, erythrocyte ghost-mediated fusion, erythrocyte membrane ghost-mediated fusion, and microencapsulation.

In the screening method according to the present invention, a cell of the murine chondrocyte cell line, ATDC5, or the like can be used for a cell that expresses the downstream gene of Runx2/Cbfa1. The Cbfal-/- and p53-/- mouse-derived chondrocyte cell lines that were established in the present invention can be used for host cells that express the downstream gene of Runx2/Cbfa1. Also, primary cultured chondrocytes derived from rats, rabbits, chickens, or mice can be used. The primary cultured chondrocytes can be obtained from joint cartilage and epiphyseal plates in accordance with conventional techniques.

According to the screening method of the present invention, the expression levels of the downstream gene of Runx2/Cbfa1 can be compared by detecting the mRNA levels of the proteins encoded by the genes as well as the expression levels of such proteins. In order to compare the expression levels based on the mRNA levels, the process of preparing mRNA samples mentioned above is carried out instead of the process of preparing protein samples. mRNA or proteins can be detected in accordance with conventional techniques as described above.

Further, a transcription regulatory region of the downstream gene of Runx2/Cbfa1 according to the present invention is obtained based on the disclosure of the present invention, and a reporter-assay system can be constructed. The term "reporter-assay system" refers to an assay system wherein the expression level of the reporter genes located downstream of the transcription regulatory region is employed as an indicator to screen for the transcription regulatory factor that functions in the transcription regulatory region.

Specifically, the present invention relates to a method for screening for a therapeutic agent and/or prophylactic agent for a bone and/or joint disease (preferably osteoarthritis) comprising the following steps (1) to (3), wherein the agent is targeted to the downstream gene of Runx2/Cbfa1 or a gene functionally equivalent thereto:
(1) a step of bringing a cell into contact with a candidate compound, wherein a vector containing the transcription regulatory region of the downstream gene of Runx2/Cbfal and a reporter gene expressed under the control of the transcription regulatory region has been introduced into the cell;
(2) a step of assaying activity of the reporter gene; and
(3) a step of selecting a compound that lowers the expression level of the reporter gene concerning the downstream genes of Runx2/Cbfa1 which stimulate cartilage differentiation or a compound that enhances the expression level of the reporter gene concerning the downstream genes of Runx2/Cbfa1 which inhibit cartilage differentiation when compared with a control, which has not been brought into contact with the candidate compound.

Examples of transcription regulatory regions include a promoter region, an enhancer region, and a CAAT box and a TATA box generally observed in promoter regions.

Examples of reporter genes that can be used include chloramphenicol acetyltransferase (CAT) genes, luciferase genes, and growth hormone genes.

Alternatively, the transcription regulatory regions of the downstream genes of Runx2/Cbfa1 according to the present invention can be obtained in the following manner. Specifically, human genomic DNA libraries such as a BAC library and a YAC library are first screened by the method involving PCR or hybridization based on the nucleotide sequence of cDNA disclosed in the present invention to obtain genomic DNA clones containing the sequences of the cDNA. Based on the obtained genomic DNA sequences, the transcription regulatory region of the cDNA disclosed in the present invention is deduced to obtain the transcription regulatory region. The obtained transcription regulatory region is cloned so as to be located upstream of the reporter gene to form a reporter construct. The resulting reporter construct is introduced into the cultured cell line to prepare a transformant for screening. The resulting transformant is brought into contact with the candidate compound, the transformant is compared with the control that has not been brought into contact with the candidate compound, and a compound that lowers the expression level of the reporter gene concerning the downstream genes of Runx2/Cbfa1 which stimulate cartilage differentiation or a compound that enhances the expression level of the reporter gene concerning the downstream genes which inhibit cartilage differentiation are selected. Thus, the screening method according to the present invention can be carried out.

The *in vitro* screening method of the present invention can be carried out via screening based on the activity of the downstream gene of Runx2/Cbfa1. Specifically, the present invention relates to a method for screening for a therapeutic agent and/or prophylactic agent for a bone and/or joint disease (preferably osteoarthritis) comprising the following steps, wherein the agent is targeted to the downstream gene of Runx2/Cbfa1 or a gene functionally equivalent thereto:
(1) a step of bringing a protein encoded by the downstream gene of Runx2/Cbfa 1 into contact with a candidate compound;
(2) a step of assaying activity of the protein; and
(3) a step of selecting a compound that lowers the aforementioned activity concerning the downstream genes of Runx2/Cbfa1 which stimulate cartilage differentiation or a compound that enhances the aforementioned activity concerning the downstream genes of Runx2/Cbfa1 which inhibit cartilage differentiation when compared with a control, which has not been brought into contact with the candidate compound.

A compound having the activity of inhibiting the activity of the downstream genes of Runx2/Cbfa1 which stimulate cartilage differentiation can be screened for by employing the activity of the protein encoded by the downstream gene of Runx2/cbfa1 in the present invention as an indicator. The thus obtained compound inhibits functions of such genes. As a result, stimulated cartilage differentiation observed in osteoarthritis can be inhibited to regulate the osteoarthritic symptoms. Activity of the protein encoded by the downstream gene of Runx2/Cbfa1 can be assayed in accordance with the aforementioned common techniques. A person skilled in the art can optimize the assay method by modifying the composition of the reagent, the composition of the buffer, the substrate, and the like.

A compound having activity of enhancing the activity of the downstream genes of Runx2/Cbfa1 which inhibit cartilage differentiation can be screened for. The thus obtained compound enhances functions of such genes. As a result, stimulated cartilage differentiation observed in osteoarthritis can be inhibited to regulate osteoarthritic symptoms.

Examples of candidate substances used for screening include compound samples synthesized via conventional chemical techniques, such as a steroid derivative, compound samples synthesized via combinatorial chemistry, mixtures comprising a plurality of compounds such as animal or plant tissue extracts or cultured microorganisms, and samples purified therefrom.

Polynucleotides, antibodies, cell lines, or animal models required for various screening procedures according to the present invention can be combined in advance to provide a kit. Such kit can be provided with a substrate compound used for label detection, a medium or container for cell culture, a positive or negative reference sample, and instructions of the use of the kit.

The compound selected by the screening method according to the present invention is useful as a therapeutic agent and/or prophylactic agent for a bone and/or joint disease (preferably osteoarthritis). Also, the antisense nucleic acid, a ribozyme, or the RNAi effects that can suppress the expression of the downstream genes of Runx2/Cbfa1 which stimulate cartilage differentiation may be useful for the therapeutic agent and/or prophylactic agent for osteoarthritis.

Further, antibodies that recognize peptides containing the amino acid sequences of proteins encoded by the downstream genes of Runx2/Cbfa1 which stimulate cartilage differentiation are useful for the therapeutic agents and/or prophylactic agents for osteoarthritis. Furthermore, proteins encoded by the downstream gene of Runx2/Cbfa1 which inhibit cartilage differentiation are also useful for the therapeutic agents and/or prophylactic agents for osteoarthritis.

### [Pharmaceutical preparations]

The therapeutic agent and/or prophylactic agent for a bone and/or joint disease. (preferably osteoarthritis) according to the present invention comprises, as an active ingredient, the compound selected by the screening method. Such agent can be manufactured by mixture with a physiologically acceptable carrier, excipient, diluent, or the like. The therapeutic agent and/or prophylactic agent for osteoarthritis according to the present invention can be administered orally or parenterally to a patient aimed at ameliorating osteoarthritic symptoms.

Oral preparations can be the forms of, for example, granules, powders, tablets, capsules, solutions, emulsions, or suspensions. Parenteral injection may be, for example, subcutaneous, intramuscular, intra-articular cavity, or intraperitoneal injection.

When the active ingredient of the therapeutic agent and/or prophylactic agent to be administered is a protein, the gene encoding the same can be introduced into the organism by a technique of gene therapy to attain the therapeutic and/or prophylactic effects. It is a known technique to treat and/or prevent a disease by introducing the gene encoding the protein that yields the therapeutic and/or prophylactic effects into the organism and to allow the expression thereof.

Alternatively, an antisense nucleic acid, a ribozyme, or a polynucleotide that suppresses the expression of the aforementioned gene by the RNAi effects can be incorporated into a site downstream of an adequate promoter sequence, and the resultant can be administered as an expression vector for the antisense RNA, the ribozyme, or RNA that yields the RNAi effects. When such expression vector is introduced into the joint cartilage or synovial cells of the patient with osteoarthritis, the antisense nucleic acid to such gene, the ribozyme, or the polynucleotide that suppresses the expression of the aforementioned gene by the RNAi effects is expressed. This results in a lowered expression level of the gene, which in turn yields the therapeutic and/or prophylactic effects against osteoarthritis.

The antisense RNA refers to RNA having a nucleotide sequence complementary to the sense sequence of the gene. Suppression of the gene expression by antisense RNA necessitates the use of RNA having a nucleotide sequence consisting of 15 or more continuous nucleotides in general, such as 20 or more or 30 or more continuous nucleotides. For example, the antisense nucleic acid that can hybridize to a region containing an initiation codon is assumed to significantly suppress the expression of the gene of interest.

The ribozyme is RNA that has a catalytic action of cleaving RNA in a nucleotide-sequence-specific manner. The hammerhead or hairpin ribozyme is known. Such ribozyme is composed of a nucleotide sequence complementary to the region to be cleaved and a nucleotide sequence that maintains the structure necessary for exerting the catalytic activity. A nucleotide sequence complementary to the region to be cleaved can be an arbitrary sequence. By employing a nucleotide sequence complementary to the target gene in such region, accordingly, a ribozyme for regulating the expression of the downstream gene of Runx2/Cbfa1 can be designed.

The term "RNA interference (RNAi) effect" refers to the phenomenon whereby a double-stranded RNA molecule comprising a nucleotide sequence identical to that of mRNA forcedly suppresses the expression of such mRNA. Accordingly, the double-stranded RNAi molecule having a nucleotide sequence identical to that of mRNA of the downstream gene of Runx2/Cbfa1 can be used for suppressing the expression of the downstream gene of Runx2/Cbfa1. In order to attain the RNAi effects, it is preferable to use a double-stranded RNAi molecule comprising a nucleotide sequence consisting of at least 20 continuous nucleotides. The double strands may be composed of 2 different strands or the double-strand can be formed by one strand via a RNA stem-loop.

The complementary or identical nucleotide sequences among the antisense nucleic acid, the ribozyme, and the polynucleotide to realize the RNAi effects (i.e., the RNAi molecule) are not limited to completely complementary or identical nucleotide sequences. Such effects of suppressing the RNA expression are maintained if successfully high complementarity or identity is maintained. When a nucleotide sequence has identity of generally 70% or more, preferably 80% or more, more preferably 90% or more, and further preferably 95% or more (e.g., 98% or more identity) with reference to a given nucleotide sequence or a nucleotide sequence complementary thereto, their identity or complementarity can be successfully high.

The dosage varies depending on, for example, the age, sex, body weight, and symptoms of the patient, the therapeutic and/or prophylactic effects, the route of administration, the treatment period, or the type of active ingredient contained in the pharmaceutical composition. In general, dosage can be 0.1 mg to 500 mg, and preferably 0.5 mg to 20 mg, per adult. Since the dosage varies depending on various types of conditions, a dosage smaller than the lower limit of the aforementioned range may be occasionally sufficient, and a dosage exceeding the upper limit of the aforementioned range may be occasionally necessary.

### Examples

The present invention is hereafter described in detail with reference to examples, although the technical scope of the present invention is not limited thereto.

In the following examples, each procedure was carried out in accordance with the method described in the Molecular Cloning (Sambrook J, Fritsch EF and Maniatis T, Cold Spring Harbor Laboratory Press, 1989), unless otherwise specified. When commercialized reagents or kits were employed, they were used in accordance with the instructions thereof.

### [Example 1] Isolation of primary chondrocyte from Runx2/Cbfa1-deficient mouse fetus and establishment of chondrocyte cell lines from Runx2/Cbfa1- and p53-deficient murine embryonic skeleton

### (1) Sampling of primary chondrocyte derived from Runx2/Cbfa1-/- mouse

The Runx2/Cbfa1 knockout mice prepared by Komori et al. were used (Cell, 1997, 89, 755-764, JP Patent Publication (Unexamined) No. 10-309148 (1998)). The skeletons were collected from the Runx2/Cbfa1-homodeficient mice at day 18.5 of embryonic development, and the skeletons were treated with a solution containing 0.1% EDTA and 0.1% Trypsin (pH 7.4) at 37°C for 60 minutes. Thereafter, the skeletons were treated with 1.5 mg/ml collagenase and alpha modified-minimum essential medium (αMEM) for 3.5 hours to obtain a cell suspension. The cell suspension was cultured on a collagen-coated dish containing Dulbecco's Modified Eagle's Medium/Ham's F12 (1: 1) hybrid medium (Gibco BRL) containing 10% fetal bovine serum for 2 or 3 days for cell proliferation, and the resultant was used in the subsequent experiment. The morphology of the primary chondrocyte is shown in Fig. 1 (bottom row).

### (2) Generation of Runx2/Cbfa1- and p53-deficient mouse

In order to establish chondrocyte cell lines that maintain the traits of undifferentiated chondrocytes, proliferative capacity, and viability, mice that were deficient in both the Runx2/Cbfal and p53 genes were generated to obtain chondrocytes. The p53-deficient mice generated by Gond Y. et al. were used (Biochem. Biophy. Res. Commun., 1994, 202, 830-837). Since the Runx2/Cbfal-homodeficient mice (Runx2/Cbfa1-/-) die immediately after birth, they cannot be subjected to crossing. Accordingly, the Runx2/Cbfa1-heterodeficient mice (Runx2/Cbfa1+/-) were used for the generation of the Runx2/Cbfa1-deficient mice. The Runx2/Cbfal-heterodeficient mice (Runx2/Cbfa1+/-) were subjected to crossing with the p53-deficient mice (p53-/-), and part of the tails of the resulting offspring mice was sampled to determine the genomic genes. Thus, the Runx2/Cbfal-heterodeficient and p53-homodeficient mice (Runx2/Cbfa1+/- and p53-/-) were obtained. Thereafter, the Runx2/Cbfal-heterodeficient and p53-homodeficient mice (Runx2/Cbfal+/- and p53-/-) were subjected to crossing with each other, and the morphology of the resulting embryos and the genomic genes extracted from some tissues thereof were examined. Thus, Runx2/Cbfa1-homodeficient and p53-homodeficient mice (Runx2/Cbfa1-/- and p53-/-) were obtained.

### (3) Preparation of chondrocyte cell line derived from Runx2/Cbfal-/- and p53-/deficient mouse

The skeletons were sampled from the Runx2/Cbfal-homodeficient and p53-homodeficient mice at day 18.5 of embryonic development, and the sampled skeletons were treated with a solution containing 0.1% EDTA and 0.1% Trypsin (pH 7.4) at 37°C for 60 minutes. Thereafter, the skeletons were treated with 1.5 mg/ml collagenase and alpha modified-minimum essential medium (αMEM) for 3.5 hours to obtain a cell suspension. The resulting cells were cultured on a dish containing 10% fetal bovine serum and Dulbecco's Modified Eagle's Medium (DMEM) at a cell density of 50 to 200 cells per 10-cm dish and allowed to form a colony, the resulting colonies were treated with trypsin/EDTA in a stainless steel cloning ring to pick up the colonies. Further, the resulting colonies were subjected to cloning 2 to 4 times via limiting dilution again, and cells that maintain proliferative capacity and viability were selected. The selected cells were examined for the expression of type II collagen expressed specifically in chondrocytes and the expression of type X collagen, the expression level of which is low in undifferentiated chondrocytes (by the method described in (4) below), and the candidates of the cell lines that maintain the traits of undifferentiated chondrocytes were selected. The selected candidate cell lines were subjected to 5 generations of subculture and then we confirmed that they did not exhibit changes in the traits of undifferentiated chondrocytes or lowering in proliferative capacity and viability. Thus, the obtained cell lines were verified to be capable of stably maintaining the properties thereof. Finally, 2 types of Runx2/Cbfa1-/- and p53-/- mouse-derived chondrocyte cell lines, i.e., RU-1 and RU-22, were obtained through the aforementioned procedure. The morphologies of the obtained cell lines are shown in Fig. 1 (top row).

### (4) Expression analysis of type II collagen and type X collagen in RU-1 and in RU-22

In order to examine the differentiation stage and properties of the resulting cell lines, the expression patterns of type II collagen and type X collagen in RU-22 and in RU-1 were analyzed via real-time PCR. The RU-22 and RU-1 cell lines were cultured in 10% fetal bovine serum/Dulbecco's Modified Eagle's Medium (DMEM), and total RNA was purified using Isogen (Nippon Gene) when the cells became confluent. Total RNA was prepared in accordance with the method described in the instructions of Isogen. Further, single-stranded cDNA was synthesized from total RNA using a reverse transcriptase and an oligo (dT) primer. The resultant was used as a template to assay the expression level of type II collagen and type X collagen via real-time PCR analysis. Assay was conducted using the ABI PRISM 7700 (Applied Biosystems) in accordance with the instructions of the SYBR Green PCR Master Mix (Applied Biosystems). The sequences of the primers used are shown in Table 2. The Ct values obtained as a result of the assay were corrected with the expression of glyceraldehyde-3-phosphate dehydrogenase (GAPDH) and expressed as the relative value when GAPDH was determined to be 1,000 (Fig. 2).

**Table 2 List of primers used for real-time PCR analysis (1)**

| Genes | Primer sequences | | SEQ ID NO: |
|---|---|---|---|
| Runx2/Cbfa1 | Forward | CCGCACGACAACCGCACCAT | 53 |
| | Reverse | CGCTCCGGCCCACAAATCTC | 54 |
| Osteopontin (OSP) | Forward | CTCCAATCGTCCCTACAGTCG | 55 |
| | Reverse | CCAAGCTATCACCTCGGCC | 56 |
| Parathyroid hormone receptor(PTH/PTHrPR) | Forward | TGTTTCTGCAATGGTGAGGTG | 57 |
| | Reverse | GCGGCTCCAAGACTTCCTAAT | 58 |
| Osteocalcin (OC) | Forward | GCTGCCCTAAAGCCAAACTCT | 59 |
| | Reverse | AGAGGACAGGGAGGATCAAGTTC | 60 |
| Bone sialoprotein (BSP) | Forward | TGGCGACACTTACCGAGCTT | 61 |
| | Reverse | CCATGCCCCTTGTAGTAGCTGTA | 62 |
| Alkaline phosphatase (ALP) | Forward | CTTGACTGTGGTTACTGCTGATCA | 63 |
| | Reverse | GTATCCACCGAATGTGAAAACGT | 64 |
| Type II collagen | Forward | TGTCCCTCGGAAAAACTGGT | 65 |
| | Reverse | AGCCACCGTTCATGGTCTCT | 66 |
| Type X collagen | Forward | AGAACGGCACGCCTACGAT | 67 |
| | Reverse | AGGTAGCCTTTGCTGTACTCATCAT | 68 |
| Collagenase-3 (MMP-13) | Forward | TCACCTGATTCTTGCGTGCT | 69 |
| | Reverse | CTGTGGGTTATTATCAATCTTGTTTCTT | 70 |
| Interleukin-11 (IL-11) | Forward | GCATGTACAATGGCTGCGC | 71 |
| | Reverse | CAAGAGCTGTAAACGGCGG | 72 |
| Indian hedgehog (Ihh) | Forward | GAGACACCATTGAGACTTGACCAG | 73 |
| | Reverse | CACCAAGATGAAGGTTCGGG | 74 |

### (5) Results

The primary chondrocytes obtained from the Runx2/Cbfal-deficient mouse have a polygonal conformation that stores an extracellular matrix and a typical chondrocyte-like morphology (Fig. 1). The RU-1 chondrocyte cell line has morphology similar to that of the primary chondrocyte obtained from a Runx2/Cbfa1-deficient mouse. In contrast, the RU-22 chondrocyte cell line has a very flat configuration with a low extracellular matrix expression level, and it does not have chondrocyte-like morphology. The expression levels of type II collagen are high both in the RU-1 and in RU-22 chondrocyte cell lines; however, the expression level of type X collagen is very low therein (Fig. 2). Accordingly, these cell lines are considered to maintain the properties of undifferentiated chondrocytes.

### [Example 2] Construction of a system of inducing cartilage differentiation via forced expression of Runx2/Cbfa1 using an adenovirus

### (1) Construction of an adenovirus for the expression of Runx2/Cbfa1

cDNA comprising the open reading frame (ORF) of murine Runx2/Cbfal was inserted into the *Bam*HI site of pIRES2-EGFP (Biosciences Clontech), and a fragment containing Runx2/Cbfa1, the internal ribosome entry site (IRES), and the enhanced green fluorescence protein (EGFP) was inserted into the *Bam*HI*-Xba*I site of the shuttle vector pACCMV.pLpA (Proc. Natl. Acad. Sci. U.S.A., 1993, 90, 2812-2816). The constructed vector was cotransfected into the human kidney cell line 293 with the adenovirus vector pJM17 (Virology, 1988, 163, 614-617) using the Superfect transfection reagent (Qiagen). The adenovirus retaining a Runx2/Cbfa1 fragment resulting via homologous recombination was subjected to subculture 3 or 4 times with the use of the cell line 293 to multiply the viruses. A roughly purified stock solution of multiplied viruses was purified via cesium chloride density-gradient ultracentrifugation and used as a virus stock solution for infection experiment. A recombinant adenovirus only retaining IRES-EGFP was also produced in the same manner as a control virus.

### (2) Construction of a system for inducing cartilage differentiation from Runx2/Cbfal-/and p53-/- chondrocyte cell lines (RU-1 and RU-22) and Runx2/Cbfal-/- primary cultured chondrocytes

The Runx2/Cbfa1-/- and p53-/- chondrocyte cell lines (RU-1 and RU-22) were plated onto a 24-well collagen-coated dish comprising a DMEM medium containing 10% fetal bovine serum and they were cultured to be confluent. Thereafter, cells were infected with the adenovirus for the expression of Runx2/Cbfal and the control adenovirus (for the expression of EGFP only) for 15 hours, the medium was exchanged with fresh medium, and culture was continued in the presence or absence of BMP-2 until day 15 while exchanging media every 3 or 4 days. The day of infection was determined to be day 0, and sampling was carried out at days 1, 3, 7, 11, and 15 for RNA preparation.

The Runx2/Cbfa1-/- primary cultured chondrocytes were plated onto a 24-well collagen-coated dish containing Dulbecco's Modified Eagle's Medium/Ham's F12 (1:1) hybrid medium (Gibco BRL) containing 10% fetal bovine serum, and they were cultured to be confluent. Thereafter, cells were infected with the adenovirus for the expression of Runx2/Cbfa1 and the control adenovirus (for the expression of EGFP only) for 15 hours, the medium was exchanged with fresh medium, and culture was continued in the presence or absence of BMP-2 until day 15 while exchanging media every 3 or 4 days. The day of infection was determined to be day 0, and sampling was carried out at days 1, 3, 7, 11, and 15 for RNA preparation.

cDNA was prepared from the sample for RNA preparation in the same manner as in Example 1 (4), and expression patterns of Runx2/Cbfa1, collagenase-3 (MMP-13), alkaline phosphatase (ALP), bone sialoprotein (BSP), a parathyroid hormone receptor (PTH/PTHrPR), type X collagen, osteopontin (OSP), and osteocalcin (OC) were assayed via real-time PCR analysis. The sequences of the primers used are shown in Table 2.

### (3) Results and discussion

The results of real-time PCR analysis are shown in Fig. 3 to Fig. 8. The peak of Runx2/Cbfa1 expression was at day 1 in all the cell lines and primary cultured cells, and the expression level of Runx2/Cbfa1 was gradually lowered while maintaining a given level on day 3 and thereafter. The expression of a marker of a hypertrophic cartilage, i.e., type X collagen, was not induced along with the induction of Runx2/Cbfa1 expression, although the expression of an initial marker of a hypertrophic cartilage, i.e., PTH/PTHrPR, was induced in all the cell lines and primary cultured cells. This indicates that Runx2/Cbfa1 induces cartilage differentiation. The expressions of MMP-13, ALP, BSP, OSP, and OC, which are known downstream genes of Runx2/Cbfa1, were strongly induced by Runx2/Cbfa1, except for OSP. It is known that the expression of OSP is induced by serum alone, and thus, Runx2/Cbfa1-induced expression was considered to be less likely to occur in the *in vitro* culture system. Thus, this experimental system was found to be very useful for searching for the downstream gene of Runx2/Cbfa1.

cDNA was prepared from the sample for RNA preparation in the same manner as in Example 1 (4), and expression patterns of Runx2/Cbfa1, collagenase-3 (MMP-13), a parathyroid hormone receptor (PTH/PTHrPR), alkaline phosphatase (ALP), type X collagen, bone sialoprotein (BSP), Indian hedgehog (Ihh), Interleukin-11 (IL-11), HNOEL-iso, osteocalcin (OC), and osteopontin (OSP) were assayed via real-time PCR analysis. The sequences of the primers used are shown in Tables 2 and 3. The expression of Runx2/Cbfa1 was assayed in order to confirm the expressions of the genes derived from the Runx2/Cbfa1-infected viruses. MMP13, ALP, BSP, Ihh, IL-11, OC, and OSP are known downstream genes the expressions of which are induced by Runx2. If the expressions of such genes are induced, it becomes possible to verify that Runx2 derived from infecting viruses performs successfully and that cartilage differentiation is stimulated. The expression level of PTH/PTHrPR and that of type X collagen are known to increase along with cartilage differentiation. Accordingly, increased expression levels of such genes indicate that chondrocytes are differentiated. It should be noted that PTH/PTHrPR is expressed at a relatively early stage of cartilage differentiation and type X collagen is expressed at a later stage thereof. HNOEL-iso was subjected to assay as a downstream gene of Runx2, which had been newly discovered by the present inventors.

### (3) Results and discussion

The results of real-time PCR analysis are shown in Fig. 3 to Fig. 8. The results of infection with an adenovirus for the expression of Runx2/Cbfal are represented by KS-GFP (black dots), and the results of infection with the control adenovirus (for the expression of EGFP only) are represented by GFP (white dots). The presence of BMP2 is represented by "+BMP2." The peak of Runx2/Cbfa1 expression was at day 1 in all the cell lines and primary cultured cells, and the expression level of Runx2/Cbfa1 was gradually lowered while maintaining a given level on day 3 and thereafter (Figs. 3A, 5A, 7A: Cbfal). The expression of a marker of a hypertrophic cartilage, i.e., type X collagen, was not induced along with the induction of Runx2/Cbfa1 expression (Figs. 3A, 5A, 7A: type X collagen), although the expression of an initial marker of a hypertrophic cartilage, i.e., PTH/PTHrPR, was induced in all the cell lines and primary cultured cells (Figs. 3A, 5A, 7A: PTH/PTHrPR). This indicates that Runx2/Cbfa1 induces cartilage differentiation. The expressions of MMP-13, ALP, and BSP (Figs. 3A, 5A, and 7A), OSP, and OC (Figs. 4B, 6B, 8B), which are known downstream genes of Runx2/Cbfa1, were strongly induced by Runx2/Cbfa1, except for OSP. It is known that the expression of OSP is induced by serum alone, and thus, Runx2/Cbfa1-induced expression was considered to be less likely to occur in the *in vitro* culture system. Induction of such gene expression was enhanced in the presence of BMP2. A variety of growth factors including BMP2 are present in cartilage tissues in an organism, and they regulate proliferation and differentiation of chondrocytes. Runx2-induced gene expression was enhanced in the presence of BMP2. This indicates that the experimental system of the present invention involves *in vivo* cartilage differentiation. Thus, this experimental system was found to be very useful for searching for the downstream gene of Runx2/Cbfa1.

### [Example 3] Isolation of downstream gene of Runx2/Cbfa1 via subtraction

The downstream gene of Runx2/Cbfa1 was obtained via subtraction using the undifferentiated mesenchymal cell line (C3H10T1/2). At the outset, the C3H10T1/2 cell line that intensively expresses Runx2/Cbfa1 was established (C3H10T1/2-Runx2/Cbfa1). Subsequently, C3H10T1/2-Runx2/Cbfa1 and, C3H10T1/2 were used to screen for the gene that is intensively expressed in the C3H10T1/2-Runx2/Cbfa1 cell line via subtraction. Subtraction was carried out using the CLONTECH PCR-Select™ cDNA Subtraction Kit in accordance with the user's manual. As a result, the expression level of the gene shown in SEQ ID NO: 11 (Riken cDNA 2810002E22 gene (HNOEL-iso homolog)) was found to be higher in C3H10T1/2-Runx2/Cbfa1 than in C3H10T1/2.

Whether or not the HNOEL-iso homolog gene is induced by Runx2/Cbfal was analyzed via real-time PCR in RU-1, RU-22, and Runx2/Cbfal-/- mouse-derived primary cultured chondrocytes, in order to confirm that the gene is induced to be expressed by Runx2/Cbfa1. The primers used in the real-time PCR analysis are shown in Table 3. Analysis via real-time PCR was carried out in the same manner as in Example 1 (4). The results of the analysis are shown in Figs. 4A, 6A, and 8A.

As a result, it was found that the gene of interest was strongly induced to be expressed by Runx2/Cbfa1 in RU-22 chondrocytes and in primary chondrocytes (Figs. 4A, 6A, and 8A). Accordingly, the HNOEL-iso homolog gene was also found to be a downstream gene of Runx2/Cbfa1.

### [Example 4] DNA microarray analysis

### (1) DNA microarray analysis using RU-1 and RU-22 chondrocyte cell lines

The RU-1 and RU-22 cell lines were plated onto 10 wells of the 12-well collagen-coated plate, they were cultured to be confluent, and they were then infected with the adenovirus for the expression of Runx2/Cbfa1 and the control virus (for the expression of GFP only). One day after the injection, total RNA was recovered using Isogen 1 and total RNA was prepared in accordance with the instructions of the reagent. Thereafter, poly A+ RNA was prepared using the Oligotex-dT30<Super>mRNA Purification Kit (Takara) in accordance with the instructions of the kit, and the resultant was employed as a sample for DNA microarray analysis. DNA microarray analysis was carried out using LifeArray (Kurabo Industries Ltd.; the number of mouse genes: approximately 9,500).

### (2) DNA microarray analysis using primary cultured chondrocytes derived from Runx2/Cbfa1-/-

The primary cultured chondrocytes derived from Runx2/Cbfa1-/- were plated onto 10 wells of the 12-well collagen-coated plate, they were cultured to be confluent, and they were then infected with the adenovirus for the expression of Runx2/Cbfa1 and the control virus (for the expression of GFP only). One day after the injection, total RNA was recovered using Isogen 1 and total RNA was prepared in accordance with the instructions of the reagent. The resultant was employed as a sample for DNA microarray analysis. DNA microarray analysis was carried out using the CodeLink DNA microarray (Kurabo Industries Ltd.; the number of mouse genes: approximately 10,000).

### (3) Results

Fig. 9 shows some genes the expressions of which are induced along with the forced expression of Runx2/Cbfa1, which were identified via DNA microarray analysis. With the use of the LifeArray, expression of alkaline phosphatase (ALP), which is known to be regulated by Runx2/Cbfa1, was found to be induced in each cell line and in primary cultured cells. This indicates that the performance of the experimental system is successful. With the use of the CodeLink DNA microarray, the expression of alkaline phosphatase (ALP) and that of collagenase-3 (MMP-13), which are known to be induced by Runx2/Cbfa1, were found to be induced. This indicates that the performance of the experimental system is successful.

### [Example 5] Determination of cDNA sequence of kEST gene

cDNA was synthesized based on RNA obtained from the skeletal tissue of a mouse embryo at day 15 of embryonic development using the SMART™ RACE cDNA amplification kit (ClonTech) in accordance with the manufacturer's instructions. Primers were synthesized based on the partial nucleotide sequence of the mouse kEST (AA397280), and cDNA was amplified by the RACE method in accordance with the manufacturer's instructions. The resulting PCR product was electrophoresed on 1% agarose gel containing ethidium bromide, and the gel was observed under ultraviolet light to detect a DNA band. The amplified fragment was removed from the gel and purified using the QIAquick Gel Extraction Kit (Qiagen) in accordance with the manufacturer's instructions. The nucleotide sequence of the purified fragment was determined using the DNA sequencer (ABI PRISM™ 310 Genetic Analyzer, PE Applied Biosystems) and the ABI PRISM™ BigDye Terminator Cycle Sequencing Ready Reaction kit (PE Applied Biosystems) in accordance with the manufacturer's instructions.

The nucleotide sequence of the cDNA of the mouse kEST and the amino acid sequence deduced therefrom are shown in SEQ ID NO: 9.

### [Example 6] Confirmation of Runx2/Cbfa1-induced expression via real-time PCR analysis

As a result of DNA microarray analysis using the established cell lines and the primary cultured cells, the genes the expressions of which are induced by Runx2/Cbfa1 in chondrocytes were identified. Subsequently, whether or not these genes were induced to be expressed by Runx2/Cbfa1 with reproducibility was examined via real-time PCR analysis employing the samples of Example 2 (2) (days 0, 1, and 3) as the templates. Runx2/Cbfa1-induced expression was confirmed with the use of the cell lines or primary cultured cells in which such gene expression was found to be induced via DNA microarray analysis. Real-time PCR analysis was carried out in the same manner as in Example 1 (4). Primers used for such expression analysis are shown in Table 3.

**Table 3 List of primers used for real-time PCR analysis (2)**

| Murine genes | Primer sequences | | SEQ ID NO: |
|---|---|---|---|
| Tumor endothelial marker 8 precursor (Tem8) | Forward | TGTGACAGCCAGCTCGAAAAC | 75 |
| | Reverse | TGGAGAGCCAAGACTTTTCCA | 76 |
| WNT1 inducible signaling pathway protein 2 (Wisp2) | Forward | GCCTGCCATTCTCAGCAAA | 77 |
| | Reverse | ACACTGAATCCACCCAGGACA | 78 |
| Nucleolar and coiled-body phosphoprotein 1 (Nolc1) (Nopp140) | Forward | CAAGTCCTTCCGGCATGAA | 79 |
| | Reverse | CCAAAATCACCCTTCCTTTGC | 80 |
| MYB binding protein (P160) la (Mybbpla) | Forward | CTTGACAACACAGCAGCGTCA | 81 |
| | Reverse | ACTTGAAGATGTGGAGGCCCA | 82 |
| DNA segment, Chr 13, Wayne State University 123, expressed (k. EST) | Forward | TGGTCAAGAAAGCACCAATGC | 83 |
| | Reverse | CCACACACGCCATCTTTCTTC | 84 |
| RIKEN cDNA 2810002E22 gene (HNOEL-iso homolog) | Forward | TTGTCTATAACACCCGCCCTG | 85 |
| | Reverse | CGGCGTGGAAAATAGGAGAGT | 86 |
| BRP39 | Forward | AGGAGAAGAAGCTGGCAGGAG | 87 |
| | Reverse | CTTGATGGCGTTGGTGAGC | 88 |
| Hemopoietic cell kinase (HCK) | Forward | TGGGTACCGTATGCCTCGA | 89 |
| | Reverse | TGTATTCAAAGGTGGGCCG | 90 |
| Lysyl oxidase-like 2 (LOXL2) | Forward | TGCTGGGACATGTACCGTCAT | 91 |
| | Reverse | CCAGATGCGGTAGCCATCA | 92 |
| Protein tyrosine phosphatase, receptor-type. F interacting protein, binding protein 2 (PPFIBP2) | Forward | GCCTTCTCTGTGCACGGAG | 93 |
| | Reverse | GTGAGACCAGCCCAAAGACAC | 94 |
| WNT1 inducible signaling pathway protein 1 (Wisp1) | Forward | ATGGGAGTTGGTAGGGACCG | 95 |
| | Reverse | CATGGGAGGGTGATCCACTT | 96 |
| Placental growth factor (PIGF) | Forward | GGCTGCATTGAAGGCATGT | 97 |
| | Reverse | AAGGGCAAACTCCACAGGC | 98 |
| UDP-N-acetyl-alpha-D-galactosamine: polypeptide N-acetylgalactosaminyl-transferase 3 (GALNT3) | Forward | TTACCCGGAAGCGTATGTGC | 99 |

As a result, it was found that all the genes were induced to be expressed by Runx2/Cbfa1, and the results of the real-time PCR analysis were the same as those obtained via DNA microarray analysis (Figs. 10 to 12).

### [Example 7] Expression analysis of downstream gene of Runx2/Cbfa1 in embryonic skeletons of wild-type mouse and Runx2/Cbfa1-deficient mouse

The gene the expression of which was verified to be induced by Runx2/Cbfa1 via DNA microarray analysis was analyzed concerning the expression thereof in the embryonic skeleton of a wild-type mouse and of a Runx2-Cbfa1-deficient mouse. At the outset, total RNAs were prepared from the skeleton of a wild-type mouse at days 13.5, 15.5, and 18.5 of embryonic development and from that of a Runx2/Cbfa1-deficient mouse at day 18.5 of embryonic development. cDNAs were prepared therefrom. Total RNA and cDNA were prepared in the same manner as in Example 1 (4). Subsequently, 12 genes the expressions of which were found to be induced by Runx2/Cbfa1 via DNA microarray analysis were subjected to expression analysis in the embryonic skeletons via real-time PCR. Real-time PCR analysis was carried out in the same manner as in Example 1 (4).

As a result, expressions of all these 13 genes were found to be inhibited in the Runx2/Cbfal-deficient mouse at day 18.5 of embryonic development to a greater extent than in the wild-type mouse at days 13.5 and 15.5 of embryonic development (Figs. 13A and 13B and Figs. 14A, 14B, and 14C). The skeletal differentiation was significantly delayed in the Runx2/Cbfa1-deficient mouse, and the skeleton of the Runx2/Cbfa1-deficient mouse at day 18.5 of the embryonic development is equivalent to that of a wild-type mouse at days 13.5 and 15.5 of embryonic development. This indicates that the expressions of all these 13 genes were inhibited in the skeleton of the Runx2/Cbfa1 deficient mouse and that such expressions are inhibited by Runx2/Cbfa1.

### [Example 8] Generation of Wisp2 transgenic mouse using type II collagen promoter and examination of its functions in chondrocytes

A transgenic mouse, which allows cartilage-specific expression of the mouse Wisp2 gene shown in SEQ ID NO: 3 with the aid of a type II collagen promoter, was generated (a Wisp2 transgenic mouse), and functions in chondrocytes were examined.

### (1) Preparation of a construct for transgenic mouse

The niouse Wisp2 genes were amplified via PCR using the following forward primer (F) and the reverse primer (R) and using the template cDNA obtained from the skeleton of a wild-type mouse at day 13.5 of embryonic development.
<Wisp2>
F: 5' GCG GCC GCA CCA TGA GGG GCA ACC CAC TGA TC 3' (SEQ ID NO: 101)
R: 5' GCG GCC GCC TAG AAG GCA CTG TTC CAT GA 3' (SEQ ID NO: 102)

The Col2a1-based expression vector reported by Ueda et al. was employed for the transgenic mouse (J. Cell. Biol., 2001, 153, 87-99). This vector comprises a murine type II collagen promoter and an enhancer, and it has been confirmed to be expressed in a cartilage-specific manner. The obtained PCR fragment was incorporated into the *Not*I site of the Col2a1-based expression vector, and the resultant was employed as a construct for preparing a transgenic mouse.

### (2) Generation of transgenic mouse

The construct for generating a transgenic mouse was treated with *Nar*I to cleave a fragment containing a type II collagen promoter, the gene to be expressed (Wisp2), and an enhancer. The fragment was purified on agarose gel and then injected into the nucleus of the fertilized egg obtained from the F1 hybrid mouse (C57BL/6xC3H). The fertilized egg that had experienced injection was introduced into the uterus of the foster mother, the embryo was removed via Caesarean operation, the genome was extracted from the liver, and the incorporation of the fragment into the genome was confirmed via PCR. Also, total RNA was extracted from the upper-body skeleton, cDNA was synthesized, and the expression intensity was assayed via real-time PCR analysis.

### (3) Preparation, staining, and in situ hybridization of skeleton section

The lower-body skeleton was sliced, and the section was observed under an optical microscope. The lower-body skeleton of the fetus was fixed in 4% paraformaldehyde in 0.1M phosphate buffer. Thereafter, a 7-µm section was prepared and stained with hematoxylin and eosin (i.e., HE staining). Type II collagen (Col2a1), PTH receptor-1 (Pthrl), type X collagen (Col10a1), and osteopontin were subjected to *in situ* hybridization in accordance with a conventional technique using conventional probes (Dev Dyn. Apr. 1999, 214 (4), 279-90).

### (4) Results and discussion

The Wisp2 transgenic mouse was slightly smaller than the wild-type mouse. Expressions of Col2al, Pthrl, Col10al, and osteopontin at day 16.5 of embryonic development were analyzed via *in situ* hybridization, and the results thereof are shown in Fig. 15. Between the wild-type mouse (Wt) and the Wisp2 transgenic mouse (Wisp2 tg), there was no significant difference in such gene expression. Fig. 16A and 16B show images of the HE-stained skeleton sections of a wild-type mouse and of a Wisp2 transgenic mouse at day 16.5 of embryonic development. In the case of the wild-type mouse (WT; A), a blood vessel had already infiltrated the cartilage. In the case of the Wisp2 transgenic mouse (WISP2; B), a bone collar was formed. However, the hypertrophied layer was shorter compared with the wild-type, blood vessel infiltration was not observed, and cartilage differentiation and enchondral ossification were delayed. It was accordingly considered that the Wisp2 gene inhibited cartilage differentiation.

### [Example 9] Generation ofNopp140, Tem8, HCK, and GALNT3 transgenic mice using type II collagen promoter and analysis of their functions in cartilage

Transgenic mice, which allow cartilage-specific expression of the murine Nopp140 gene shown in SEQ ID NO: 5, the murine Tem8 gene shown in SEQ ID NO: 1, the HCK gene shown in SEQ ID NO: 15, and the GALNT3 gene shown in SEQ ID NO: 25 under the control of a type II collagen promoter, were prepared, and functions in chondrocytes were further examined.

### (1) Preparation of a construct for a transgenic mouse

The murine Nopp140 gene, the murine Tem8 gene, the murine HCK gene, and the murine GALNT3 gene were amplified via PCR using the following forward primers (F) and the reverse primers (R). cDNA obtained from the skeleton of a wild-type mouse at day 13.5 of embryonic development was used as a template concerning the murine Nopp140 gene and the murine Tem8 gene. cDNA obtained from the skeleton of a wild-type mouse at day 15.5 of embryonic development was used as a template concerning the murine HCK gene and the murine GALNT3 gene. A constitutive active form of HCK (499Tyr→Phe) was prepared in accordance with the previous report (J. Exp. Med., 2002, Vol. 196, No. 5, 589-604).
<Nopp 140>
F: 5'-GAC GCG TTG CGG CCG CAG CAT GGC GGA TAC CGG CTT-3' (SEQ ID NO: 103)
R: 5'-AAA GGA TGG CGG CCG CTC ACT CGC TGT CGA ATT TGA-3' (SEQ ID NO: 104)
<Tem8>
F: 5'-GCG GCC GCA GCA TGG ACC GCG CGG GGC GC-3' (SEQ ID NO: 105)
R: 5'-ATG CAT CTA GAC AGA AGG CCT TGG AGG AG-3' (SEQ ID NO: 106)
<HCK>
F: 5'-GCG GCC GCA GGA TGG GAT GCG TGA AGT CCA GG-3' (SEQ ID NO: 107)
R: 5'-GCG GCC GCT CAA GGC TGC TGC TGG AAC TGG CTC TCA GTG GCC GT -3' (SEQ ID NO: 108)
<GALNT3>
F: 5'-GCG GCC GCA GAA TGG CTC ACC TTA AGG GAC TA-3' (SEQ ID NO: 109)
R: 5'-GCG GCC GCT TAT TCA TTT TGG CTA AAA ATC CA-3' (SEQ ID NO: 110)

A vector for a transgenic mouse of each gene was prepared in the same manner as in Example 8 using the Col2a1-based expression vector described in Example 8.

### (2) Generation of a transgenic mouse

The constructs for generating transgenic mice of the Nopp140, HCK, and GALNT3 genes were treated with *Nar*I and the construct for generating transgenic mice of the Tem8 gene was treated with *Pvu*II to cleave the fragments comprising type II collagen promoters, the genes to be expressed, and the enhancers. The fragments were purified using agarose gel and then injected into the nuclei of the fertilized eggs obtained from the F1 hybrid mouse (C57BL/6×C3H). The fertilized eggs that had experienced injection were introduced into the uterus of the foster mother, the fetuses were removed via Caesarean operation, the genomes were extracted from the liver, and the incorporation of the fragments into the genomes was confirmed by PCR. Also, total RNAs were extracted from the upper-body skeletons, cDNAs were synthesized, and expression intensity was assayed via real-time PCR analysis. The skeletons of the fetuses were stained with alizarin red and with alcian blue (Cell, 1997, 89, 755-764).

### (3) Preparation of skeleton sections

The lower-body skeletons were sliced, and the sections were observed under an optical microscope. The lower-body skeletons of the fetuses were fixed in 4% paraformaldehyde in 0.1M phosphate buffer. Thereafter, 7-µm sections were prepared and stained with hematoxylin and eosin (i.e., HE staining) to analyze the skeletons (Cell, 1997, 89, 755-764). Hematoxylin-eosin and Kossa staining (HE-Kossa staining) was carried out to dye the calcified site black to analyze the skeletons and calcification (Cell, 1997, 89, 755-764). The cartilage matrix was analyzed by safranin O staining and PAS staining (Cell, 1997, 89, 755-764). Apoptosis was analyzed by Tunnel staining (Cell, 1997, 89, 755-764). Cell proliferation was analyzed by Brdu staining (Cell, 1997, 89, 755-764). *In situ* hybridization was carried out in accordance with a conventional technique using know probes (Dev Dyn. Apr. 1999, 214 (4), 279-90).

### (4) Results and discussion

Fig. 17A shows the appearance of the Nopp140 transgenic mouse at day 18.5 of embryonic development, and Fig. 17B shows images of the HE-stained sections of joints between the tibias and the thighbone. Compared with a wild-type mouse (Wt), the Nopp 140 transgenic mouse (Nopp140 tg) had significantly shorter limbs and smaller jaws (Fig. 17A, bottom row). As a result of HE staining, a disturbance was observed in a cell layer of the intrinsic proliferated chondrocytes of the Nopp140 transgenic mouse (Nopp140 tg), which suggests that differentiation was stimulated at this site (Fig. 17B, bottom row). This indicates that the Nopp140 gene may stimulate cartilage differentiation.

Fig. 18A shows the head appearance of the Tem8 transgenic mouse at day 18.5 of embryonic development, and Fig. 18B shows images of stained skeletons. Compared with a normal mouse (i.e., a wild-type mouse), the jaw of the Tem8 transgenic mouse (Tem8) was not sufficiently developed (Fig. 18A, right) and it had shorter limbs (Fig. 18B, right). By observation of HE-stained sections, calcification is observed at a site different from a site at which original bone formation takes place (i.e., allopatry). This indicates that abnormal cartilage differentiation takes place in such mouse. Compared with a wild-type mouse, tunnel-positive apoptosis took place in many more chondrocytes of the Tem8 transgenic mice. Such apoptosis-positive cells expressed osteopontin, and apoptosis involving abnormal cartilage differentiation was induced. This indicates that the Tem8 gene stimulates cartilage differentiation.

Fig. 19A-(2) shows the appearance of the HCK transgenic mouse at day 14.5 of embryonic development and Fig. 19A-(1) shows an image of the stained skeleton thereof. Fig. 19 A and B show images of HE staining (Fig. 19B-(1)), the results of expression analysis via *in situ* hybridization using the following probes: type I collagen (Col1a1: Fig. 19B-(2)), type II collagen (Col2a1: Fig. 19B-(3)), type X collagen (Col10a1: Fig. 19B-(4)), osteopontin (Fig. 19B-(5)), Indian hedgehog (Fig. 19B-(6): Ihh), a PTH receptor (Fig. 19B-(7): Pthr1), Hck (Fig. 19B-(8)), MMP13 (Fig. 19C-(1)), BSP (Fig. 19C-(2)), and VEGF (Fig. 19C-(3)), and images showing the results of analyzing osteoclasts by TRAP staining (Fig. 19C-(4)). The HCK transgenic mouse (Tg) had a smaller body size, a more protruding abdomen, and stubbier limbs compared with a wild-type mouse (Wt) (Fig. 19A-(2)). In the tibia of the HCK transgenic mouse, unorganized abnormal growth of cells inhibited the formation of the normal epiphyseal plate, and longitudinal growth was abnormal (Fig. 19A-(1)). Also, the number of chondrocytes with stimulated differentiation that express Ihh and Col10a1 was small (Fig. 19-(6) and (4)). Expression of Col2a1 and Pthr1 was observed in expanded tissue (Fig. 19-(3) and (7)). The expression level of the osteopontin that is a marker for the ultimately differentiated hypertrophic cartilage and osteoblasts was increased in cartilage tissues (Fig. 19B-(5)). Collal that is not generally expressed in cartilage was expressed in cartilage tissues (Fig. 19B-(2)). The expression level of BSP, which is usually expressed in osteoblasts, was decreased (Fig. 19C-(2)). The expression level of MMP13 and that ofVEGF, which are known to induce angioinvasion into cartilage, were increased (Fig. 19C-(1) and (3)). The TRAP-positive osteoclasts that are usually invasive to the ultimately differentiated chondrocytes were invasive to the undifferentiated chondrocytes (Fig. 19C-(4)). Brdu staining revealed that cell proliferation was enhanced in tissues.

Fig. 20 shows the results of expression analysis by HE staining (HE) of the HCK transgenic mouse at day 16.5 of embryonic development and via *in situ* hybridization of type II collagen (Col2a1), a PTH receptor (PthRP), and type X collagen (Col10a1). In the tibia of the HCK transgenic mouse, unorganized abnormal growth of cells inhibited the formation of the normal epiphyseal plate, and longitudinal growth was abnormal (Fig. 20-HE, bottom row). Expression of Col2a1 was observed in expanded tissues (Fig. 20-Cola1, bottom row). The expression level of PthRP and that of Col10a1 that are generally observed in differentiated chondrocytes was lowered (Fig. 20-PthhRP and Col10a1, bottom rows).

Fig. 21A shows the appearance of the HCK transgenic mouse at day 18.5 of embryonic development, Fig. 21B shows images of the stained skeletons thereof, and Fig. 21C shows the results of HE staining thereof. Fig. 22A-(1) shows the appearance of the HCK transgenic mouse at day 18.5 of embryonic development and Fig. 22A-(2) shows an image of the stained skeleton thereof. Fig. 22 B and C show the results of expression analysis by HE and Kossa staining (Fig. 22B-(1); HE Kossa), images showing the results of expression analysis via *in situ* hybridization of type I collagen (Fig. 22B-(2): Collal), type II collagen (Fig. 22B-(3): Col2a1), type X collagen (Fig. 22B-(4): Col10a1), osteopontin (Fig. 22B-(5): osteopontin), osteocalcin (Fig. 22B-(6): osteocalcin), a PTH receptor (Fig. 22B-(7): Pthr1), Indian hedgehog (Fig. 22B-(8): Ihh), Hck (Fig. 22C-(1)), MMP13 (Fig. 22C-(2)), and BSP (Fig. 22C-(3)), the results of analyzing osteoclasts by TRAP staining (Fig. 22C-(4)), and the results of analyzing proteoglycan by safranin O staining (Fig. 22C-(5)). Compared with a wild-type mouse (Wt), the HCK transgenic (Tg) mouse had a smaller body size, a more protruding abdomen, and stubbier limbs (Fig. 22A-(1)). There was a cleavage between the nose and the upper jaw, and the process of fusion between the nose and the upper jaw was inhibited. The chondrocranium and the nasal cartilage were covered with enchondral regions of expanded membrane bones. The directions along the proximal-distal axis were lost in enchondral bones and abnormal configurations were observed. The growth plates were not organized, and the joints were fused. Skeleton staining revealed that the amount of calcified tissues stained with alizarin red decreased and that the amount of the extracellular matrix stained with alcian blue increased (Fig. 22A-(2)). As a result of HE staining, the chondrocytes of the HCK transgenic mouse were found to be immature, and the invasion and the proliferation of mesenchymal cells were observed in the vicinity thereof (Fig. 22B-(1)). The number of Col2a1-expressing chondrocytes decreased, and the bones were formed while involving the invasion and the proliferation of mesenchymal cells without a normal procedure (Fig. 22B-(2)). The expression levels of Ihh, Pthr1, and Col10a1 were also lowered (Fig. 22B-(8), (7), and (4)). Col1a1 was expressed in bone tissues, and osteopontin was wide spread distributed (Fig. 22B-(2) and (5)). In contrast, a marker for mature osteoblast, i.e., osteocalcin, was hardly expressed, which indicates that cells constituting the bone tissue were immature (Fig. 22B-(6)). A bone collar was not organized, and continuity was lost due to the invasion of numerous blood vessels and mesenchymal cells. Such invasion of numerous blood vessels and mesenchymal cells was considered to result from an increase in TRAP-positive osteoclasts (Fig. 22C-(4)). As a result of safranin O staining, proteoglycan in cartilage tissues was assumed to be normal (Fig. 22C-(5)). Accordingly, the HCK genes were considered to enhance the proliferation of chondrocytes and to inhibit the differentiation thereof.

Fig. 23B shows the appearance of the GALNT3 transgenic mouse at day 18.5 of embryonic development. Compared with a wild-type mouse (Wt), the GALNT3 transgenic mouse (Galnt3 tg) had a smaller body size, shorter limbs, a smaller pectoral region, and a more protruding abdomen. Fig. 24 shows the result of skeleton staining. Compared with a normal (wild-type) mouse, the GALNT3 transgenic mouse (Galnt3 tg) had a smaller pectoral region and the significantly decreased bones calcified upon enchondral ossification. Fig. 25 A and B show the results of HE and Kossa staining of the tibia of the GALNT3 transgenic mouse at day 16.5 of embryonic development (Fig. 25B is an enlarged image of Fig. 25A). Angioinvasion was observed in a wild-type mouse (Wt); however, it was delayed in the GALNT3 transgenic mouse (Galnt3 tg), and articular cavity formation was incomplete (Fig. 25, right). Fig. 26 shows the results of expression analysis via *in situ* hybridization of type II collagen (Col2a1). Compared with a normal (wild-type) mouse, the GALNT3 transgenic mouse (Galnt3 tg) exhibited abnormal distribution of type II collagen expression. Fig. 27 shows the results of analysis via *in situ* hybridization of type X collagen, and Fig. 28 shows the results of analysis via *in situ* hybridization of osteopontin. Expression of type X collagen and that of osteopontin were observed in hypertrophic cartilage of both the wild-type mouse and the GALNT3 transgenic mouse (Galnt3 tg). Fig. 29A shows the results of analysis via *in situ* hybridization of mRNA expression of aggrecan (mRNA), and Fig. 29B shows an image of immunohistostaining representing expression at a protein level (protein). The mRNA expression of aggrecan was slightly increased in the GALNT3 transgenic mouse (Galnt3 tg) compared with the wild-type mouse (wt) (Fig. 29A), although aggrecan expression was decreased at the protein level (Fig. 29B). Fig. 30 shows the results of safranin O staining of the tibia at day 16.5 of embryonic development. Compared with a wild-type mouse, the staining affinity of the GALNT3 transgenic mouse (Galnt3 tg) was significantly deteriorated, which indicates that proteoglycan content was significantly decreased.

Fig. 31 A and B show the results of PAS staining of the anklebone of the GALNT3 transgenic mouse at day 18.5 of embryonic development (Fig. 31B is an enlarged image of Fig. 31A). Compared with a wild-type mouse (wt), the staining affinity of the GALNT3 transgenic mouse (Galnt3 tg) was enhanced, which indicates that the level of mucin-like glycoproteins was increased. Fig. 32 shows an image of immunostained fibronectin in the tibia of the GALNT3 transgenic mouse at day 18.5 of embryonic development. Compared with a wild-type mouse, the distance between adjacent chondrocytes was small in the GALNT3 transgenic mouse, which indicates that the amount of extracellular matrix was decreased. Fig. 33A shows the results of analyzing by Brdu labeling the chondrocyte proliferation, and Fig. 33B is a graph showing the amount of BrdU incorporated. Compared with a wild-type mouse (wt), chondrocyte proliferation was enhanced in the GALNT3 transgenic mouse (Galnt3 tg). Fig. 34 shows the results of analysis by Tunnel staining. Compared with a wild-type mouse (wt), chondrocyte apoptosis was found to be enhanced in the TALNT3 transgenic mouse (Galnt3 tg). Accordingly, the GALNT3 genes were considered to be capable of inhibiting the formation of cartilage tissues. The joint cartilage is composed of a small amount of chondrocytes, i.e., 80% of the joint cartilage is accounted for by moisture and 20% thereof is accounted for by matrix. The matrix is composed of collagen (60% of the dry weight) and proteoglycan (10% of the dry weight). Proteoglyan consists of 95% polysaccharides referred to as "glucosaminoglycan" (GAG) and 5% proteins. When proteoglycan comprises a large quantity of water, it becomes a gelatinous material. Because of the water retentivity of proteoglycan, the water content of cartilage is as high as 80%, and this can impart elasticity and stiffness to the joint cartilage. When the water retentivity is lowered due to decreased proteoglycan content, cartilage elasticity becomes deteriorated, which may lead to tissue degeneration. Accordingly, inhibition of cartilage tissue generation and decreased proteoglycan content, which were observed in the GALNT3 transgenic mouse, were considered to result from changes associated with osteoarthritis.

Thus, the Nopp140 gene and the Tem8 gene stimulate cartilage differentiation to thereby allow the osteoarthritis development. On the contrary, the HCK gene inhibits cartilage differentiation to thereby inhibit osteoarthritis. The GALNT3 gene inhibits the formation of cartilage tissues to secondarily inhibit cartilage differentiation, although such gene may be involved in the advancement of osteoarthritis.

All publications, patents, and patent applications cited herein are incorporated herein by reference in their entirety.

### Industrial Applicability

The present invention provides a method for obtaining a disease-associated gene by allowing a disease-associated transcription factor to express in a cell line that is deficient in such transcription factor or a primary cultured cell and a method for obtaining a bone and/or joint disease-associated gene, and preferably an osteoarthritis-associated gene, by allowing Runx2/Cbfal to express in the Runs2/Cbfal-deficient chondrocyte cell line or primarily chondrocytes in culture. The downstream gene of Runx2/Cbfal obtained via such method and a polypeptide encoded thereby are capable of regulating cartilage differentiation, and they can also serve as pharmaceutical preparations for a bone and/or joint disease (preferably osteoarthritis). Further, the present invention provides a method for screening for a candidate compound of a pharmaceutical preparation for a bone and/or joint disease (preferably osteoarthritis) and an animal model of a bone and/or joint disease (preferably osteoarthritis). The methods and products provided by the present invention are significantly useful in the fields of clinical and basic pharmaceutical and medical products for a bone and/or joint disease (preferably osteoarthritis).

### Sequence Listing Free Text

SEQ ID NOs: 53 to 114: synthetic oligonucleotides

## Claims

1. A method for obtaining a disease-associated gene, wherein a disease-associated transcription factor is expressed in a cell line that is deficient in said transcription factor or in a primary cultured cell, and the gene the expression of which is thereby induced or inhibited is screened.

2. A method for obtaining a Runx2/Cbfa1-related disease-associated gene, wherein Runx2/Cbfa1 is expressed in a Runxs/Cbfa1-deficient chondrocyte cell line or in a Runx2/Cbfa1-deficient primary cultured cell, and the gene the expression of which is thereby induced or inhibited is screened.

3. A method for obtaining a gene associated with regulation of cartilage differentiation, wherein Runx2/Cbfal is expressed in a Runxs/Cbfa1-deficient chondrocyte cell line or in a Runx2/Cbfa1-deficient primary cultured cell, and the gene the expression of which is thereby induced or inhibited is screened.

4. The method according to any one of claims 1 to 3, wherein said screening is carried out via subtraction or DNA chip analysis.

5. A primary chondrocyte or cultured chondrocyte derived from a Runx2/Cbfa1-deficient mouse.

6. A chondrocyte derived from a Runx2/Cbfa1- and p53-deficient mouse.

7. The chondrocyte cell line derived from the Runx2/Cbfa1- and p53-deficient mouse according to claim 6, which is the RU-1 cell line or the RU-22 cell line deposited under the accession number FERM BP-10137 or FERM BP-10138 at the International Patent Organism Depositary of the National Institute of Advanced Industrial Science and Technology.

8. A polynucleotide having the nucleotide sequence shown in SEQ ID NO: 1, 3, 5, 7, 9, 11, 13, 15, 17, 19, 21, 23, or 25, wherein the expression thereof is induced by Runx2/Cbfa1 expression.

9. A polynucleotide having the nucleotide sequence shown in SEQ ID NO: 9.

10. A polynucleotide having the nucleotide sequence shown in SEQ ID NO: 1 and encoding a protein capable of stimulating cartilage differentiation.

11. A polynucleotide having the nucleotide sequence shown in SEQ ID NO: 3 and encoding a protein capable of inhibiting cartilage differentiation.

12. A polynucleotide having the nucleotide sequence shown in SEQ ID NO: 5 and encoding a protein capable of stimulating cartilage differentiation.

13. A polynucleotide having the nucleotide sequence shown in SEQ ID NO: 15 and encoding a protein capable of inhibiting cartilage differentiation.

14. A polynucleotide having the nucleotide sequence shown in SEQ ID NO: 25 and encoding a protein capable of inhibiting chondrogenesis.

15. A human homolog polynucleotide of the polynucleotide according to claim 8, which has the nucleotide sequence shown in SEQ ID NO: 27, 29, 31, 33, 35, 37, 39, 41, 43, 45, 47, 49, or 51.

16. A polynucleotide having 65% or more homology to the polypeptide encoded by the polynucleotide according to any one of claims 8 to 15, and encoding a protein capable of stimulating or inhibiting cartilage differentiation.

17. A polynucleotide being capable of hybridizing under stringent conditions to the polynucleotide according to any one of claims 8 to 15 or a complementary strand thereof, and encoding a protein capable of stimulating or inhibiting cartilage differentiation.

18. A recombinant DNA vector comprising the polynucleotide according to any one of claims 8 to 17 or a complementary strand thereof.

19. A transformant transformed with the recombinant DNA vector according to claim 18.

20. A polypeptide comprising the amino acid sequence shown in SEQ ID NO: 2, 4, 6, 8, 10, 12, 14, 16, 18, 20, 22, 24, 26, 28, 30, 32, 34, 36, 38, 40, 42, 44, 46, 48, 50, or 52.

21. A polypeptide comprising an amino acid sequence derived from the amino acid sequence shown in SEQ ID NO: 2, 4, 6, 8, 10, 12, 14, 16, 18, 20, 22, 24, 26, 28, 30, 32, 34, 36, 38, 40, 42, 44, 46, 48, 50, or 52 by deletion, substitution, or addition of one or several amino acid residues, and capable of stimulating or inhibiting cartilage differentiation.

22. A polypeptide comprising an amino acid sequence having at least 65% homology to the amino acid sequence shown in SEQ ID NO: 2, 4, 6, 8, 10, 12, 14, 16, 18, 20, 22, 24, 26, 28, 30, 32, 34, 36, 38, 40, 42, 44, 46, 48, 50, or 52, and capable of stimulating or inhibiting cartilage differentiation.

23. An antisense polynucleotide which regulates the expression of the gene consisting of the polynucleotide according to any one of claims 8 to 17.

24. An RNAi molecule which regulates the expression of the gene consisting of the polynucleotide according to any one of claims 8 to 17.

25. An antibody against the polypeptide according to any one of claims 20 to 22.

26. A method for screening for a therapeutic agent and/or prophylactic agent for a bone and/or joint disease comprising the following steps (1) to (3):
(1) a step of bringing a candidate compound into contact with a cell that expresses the gene consisting of the polynucleotide according to any one of claims 8 to 17;
(2) a step of assaying the expression level of the gene; and
(3) a step of selecting a compound that lowers or enhances the expression level of the gene compared with a control, which has not been brought into contact with the candidate compound.

27. A method for screening for a therapeutic agent and/or prophylactic agent for a bone and/or joint disease comprising the following steps (1) to (3):
(1) a step of bringing a cell into contact with a candidate compound, wherein a vector containing the transcription regulatory region of the gene consisting of the polynucleotide according to any one of claims 8 to 17 and a reporter gene expressed under the control of the transcription regulatory region has been introduced into the cell;
(2) a step of assaying activity of the reporter gene; and
(3) a step of selecting a compound that lowers or enhances the expression level of the reporter gene compared with a control, which has not been brought into contact with the candidate compound.

28. A method for screening for a therapeutic agent and/or prophylactic agent for a bone and/or joint disease comprising the following steps (1) to (3):
(1) a step of administering a candidate compound to a test animal
(2) a step of assaying the expression level of the gene consisting of the polynucleotide according to any one of claims 8 to 17 in a biological sample obtained from the test animal; and
(3) a step of selecting a compound that lowers or enhances the expression level of the gene compared with the control to which the candidate compound has not been administered.

29. A method for screening for a therapeutic agent and/or prophylactic agent for a bone and/or joint disease comprising the following steps (1) to (3):
(1) a step of bringing a protein encoded by the gene consisting of the polynucleotide according to any one of claims 8 to 17 into contact with a candidate compound;
(2) a step of assaying activity of the protein; and
(3) a step of selecting a compound that lowers or enhances the activity of the protein compared with a control, which has not been brought into contact with the candidate compound.

30. A compound selected by the screening method according to any one of claims 26 to 29.

31. A pharmaceutical composition comprising at least one of: the polynucleotide according to any one of claims 8 to 17; the DNA vector according to claim 18; the transformant according to claim 19; the polypeptide according to any one of claims 20 to 22; the antisense polynucleotide according to claim 23; the RNAi molecule according to claim 24; the antibody according to claim 25; and the compound according to claim 30.

32. A prophylactic agent and/or therapeutic agent for a bone and/or joint disease comprising at least one of: the polynucleotide according to any one of claims 8 to 17; the DNA vector according to claim 18; the transformant according to claim 19; the polypeptide according to any one of claims 20 to 22; the antisense polynucleotide according to claim 23; the RNAi molecule according to claim 24; the antibody according to claim 25; and the compound according to claim 30.

33. The prophylactic agent and/or therapeutic agent according to claim 32, wherein the bone and/or joint disease is osteoarthritis.

34. A composition for diagnosing a disease comprising at least one of: the polynucleotide according to any one of claims 8 to 17; the DNA vector according to claim 18; the transformant according to claim 19; the polypeptide according to any one of claims 20 to 22; the antisense polynucleotide according to claim 23; the RNAi molecule according to claim 24; the antibody according to claim 25; and the compound according to claim 30.

35. A composition for diagnosing a bone and/or joint disease comprising at least one of: the polynucleotide according to any one of claims 8 to 17; the DNA vector according to claim 18; the transformant according to claim 19; the polypeptide according to any one of claims 20 to 22; the antisense polynucleotide according to claim 23; the RNAi molecule according to claim 24; the antibody according to claim 25; and the compound according to claim 30.

36. The composition according to claim 35, wherein the bone and/or joint disease is osteoarthritis.

37. A transgenic animal model of a bone and/or joint disease, in which an expression level of the gene encoded by the polynucleotide according to any one of claims 8 to 17 is enhanced or lowered.

38. A transgenic mouse model of a bone and/or joint disease, in which the gene encoded by the polynucleotide according to any one of claims 8 to 17 is expressed with the use of a type II collagen promoter.

39. A method for preparing an animal model of a bone and/or joint disease comprising administering at least one of: the DNA vector according to claim 18; the transformant according to claim 19; the polypeptide according to any one of claims 20 to 22; the antisense polynucleotide according to claim 23; the RNAi molecule according to claim 24; the antibody according to claim 25; and the compound according to claim 30.

40. The method for preparing an animal model according to claim 39, wherein the bone and/or joint disease is osteoarthritis.
